(19) Europäisches Patentamt
European Patent Office
Office européen des brevets

(11) **EP 3 301 208 B1**

(12) **EUROPEAN PATENT SPECIFICATION**

(45) Date of publication and mention
of the grant of the patent:
**04.11.2020 Bulletin 2020/45**

(51) Int Cl.:
***C40B 50/06*** *(2006.01)*   ***C40B 40/06*** *(2006.01)*
***C12P 19/34*** *(2006.01)*

(21) Application number: **17190712.4**

(22) Date of filing: **17.09.2010**

(54) **REDUCED CODON MUTAGENESIS**

REDUZIERTE CODON-MUTAGENESE

MUTAGENÈSE DE CODON RÉDUITE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **18.09.2009 US 562988**
**18.09.2009 PCT/US2009/057507**
**09.12.2009 US 283877 P**

(43) Date of publication of application:
**04.04.2018 Bulletin 2018/14**

(62) Document number(s) of the earlier application(s) in accordance with Art. 76 EPC:
**10817881.5 / 2 478 137**

(73) Proprietor: **Codexis, Inc.**
**Redwood City, CA 94063 (US)**

(72) Inventors:
• **FOX, Richard**
**Kirkwood, MO 63122 (US)**
• **GIVER, Lorraine J.**
**Redwood City, CA 94063 (US)**
• **HELD, Daniel**
**Davis, CA 95618 (US)**
• **HATTENDORF, Douglas**
**Redwood City, CA 94063 (US)**
• **CHOUDHARY, Trish**
**Redwood City, CA 94063 (US)**

(74) Representative: **Chapman, Desmond Mark**
**Carpmaels & Ransford LLP**
**One Southampton Row**
**London WC1B 5HA (GB)**

(56) References cited:
**WO-A1-2009/088933**   **US-A1- 2009 093 024**
**US-A1- 2010 093 560**

• **FOX ET AL: "Enzyme optimization: moving from blind evolution to statistical exploration of sequence-function space", TRENDS IN BIOTECHNOLOGY, ELSEVIER PUBLICATIONS, CAMBRIDGE, GB, vol. 26, no. 3, 28 January 2008 (2008-01-28), pages 132-138, XP022487056, ISSN: 0167-7799, DOI: 10.1016/J.TIBTECH.2007.12.001**
• **FOX RICHARD J ET AL: "Improving catalytic function by ProSAR-driven enzyme evolution", NATURE BIOTECHNOLOGY, NATURE PUBLISHING GROUP, NEW YORK, NY, US, vol. 25, no. 3, 1 March 2007 (2007-03-01), pages 338-344, XP002566179, ISSN: 1087-0156, DOI: 10.1038/NBT1286 [retrieved on 2007-02-18]**
• **MARCO A MENA AND PATRICK S DAUGHERTY: "Automated design of degenerate codon libraries", PROTEIN ENGINEERING, DESIGN AND SELEC, OXFORD JOURNAL, LONDON, GB, vol. 18, no. 12, 1 December 2005 (2005-12-01), pages 559-561, XP008154547, ISSN: 1741-0126, DOI: 10.1093/PROTEIN/GZI061**
• **HORTON R M ET AL: "GENE SPLICING BY OVERLAP EXTENSION TAILOR-MADE GENES USING THE POLYMERASE CHAIN REACTION", 1990, BIOTECHNIQUES, VOL. 8, NR. 5, PAGE(S) 528-530, 532, 534, XP009172492, ISSN: 0736-6205 * page 129, column 3, last paragraph - page 131, column 2, paragraph 1; figures 1,2 ***

**Description**

**FIELD OF THE INVENTION**

This invention is in the field of making mutagenic combinatorial libraries of biological molecules using optimized mutagenic codon sets, and related compositions.

**BACKGROUND OF THE INVENTION**

*In silico, in vitro* and/or *in vivo* methods for the directed evolution of biological molecules provides for the generation of active molecules with new or improved properties. In one recent example, a cytochrome P450 enzyme was evolved to have activity against substrates not normally recognized by the naturally occurring enzyme (see, e.g., Landwehr et al., 2007, Chem Biol 14(3):269-78; and Kubo et al., 2006, Chemistry 12(4):1216-20). When generating such new or improved biomolecules, a polynucleotide encoding a reference polypeptide, such as a wild type enzyme, is typically subjected to mutagenesis to produce a library of variant polynucleotides encoding polypeptide variants that display changes in amino acid sequence, relative to the wild type enzyme. Screening of the variants for a desired property, such as an improvement in enzyme activity or stability, activity against new substrates, or the like, allows for the identification of amino acid residues associated with the desired property. For a review of directed evolution and mutation approaches see, e.g.,: Fox and Huisman (2008), Trends Biotechnol. 26: 132-138; Arndt and Miller (2007) Methods in Molecular Biology, Vol. 352: Protein Engineering Protocols, Humana; Zhao (2006) Comb. Chem. High Throughput Screening 9:247- 257; Bershtein et al. (2006) Nature 444: 929-932; Brakmann and Schwienhorst (2004) Evolutionary Methods in Biotechnology: Clever Tricks for Directed Evolution, Wiley-VCH, Weinheim; and Rubin- Pitel Arnold and Georgiou (2003) Directed Enzyme Evolution: Screening and Selection Methods, 230, Humana, Totowa. For example, nucleic acid shuffling (*in vitro*, *in vivo* and/or *in silico*) has been used in a variety of ways, e.g., in combination with homology, structure or sequence based analysis and with a variety of recombination or selection protocols a variety of methods. See, e.g., WO/2000/042561 by Crameri et al. OLIGONUCLEOTIDE MEDIATED NUCLEIC ACID RECOMBINATION; WO/2000/042560 by Selifonov et al. METHODS FOR MAKING CHARACTER STRINGS, POLYNUCLEOTIDES AND POLYPEPTIDES; WO/2001/075767 by GUSTAFSSON et al. IN SILICO CROSS-OVER SITE SELECTION; and WO/2000/004190 by del Cardayre EVOLUTION OF WHOLE CELLS AND ORGANISMS BY RECURSIVE SEQUENCE RECOMBINATION.

While useful directed evolution approaches exist, many challenges remain. For example, the complete mutagenesis of a reference polypeptide, e.g., production of all possible single-amino acid sequence variants remains a challenging task, due to the large sequence space at issue for a typical polypeptide. It is difficult both to make such complete libraries, and to screen them, because of the number of library members at issue. This "numbers problem" in directed evolution has been addressed by taking account of knowledge regarding structure, sequence similarity of homologous molecules, information regarding molecule function, relatedness of amino acids, etc. This information is used to limit the number of residues that are mutated, and what amino acids the mutant residues encode. This limits the number of library members that are made and screened in a given directed evolution process. These approaches are typically reductionist in nature, seeking to limit diversity of the library both with respect to which residues are modified, and which amino acids are encoded at any given position of interest. While this simplifies library construction and screening, placing such limits on molecule diversity within the library can also reduce the chances that the library will include a molecule with a desired property.

Nevertheless, a variety of logical filters have been applied to reduce the number of mutations that are made to explore a given sequence space, e.g., by grouping amino acid residues by physico-chemical properties to create simplified amino acid sets used to reduce the overall number of mutations of a reference protein (see e.g., Li at al. (2003) "Reduction of Protein Sequence Complexity by Residue Grouping" Protein Engineering 16(5):323-330). For example, 9 or 10 amino acid "types" have been used to identify useful mutants with an efficiency argued to be similar to the use of the typical canonical 20 amino acids (e.g., Akanuma et al. (2002) "Combinatorial mutagenesis to restrict amino acid usage in an enzyme to a reduced set," PNAS 99(21):13549-13553; and Li et al. (2003, *above*). Statistical filters such as hidden Markov models (HMMs) have been used to identify and select reduced amino acid alphabets (Susko and Roger (2007) "On Reduced Amino Acid Alphabets for Phylogenetic Inference" Mol. Biol. Evol. 24(9):2139-2150) that can be used for mutagenesis. Sequence alignments of homologous enzymes have also been used to produce selected amino acid alphabets at particular sites, including the use of 2, 6, 7, and 9 amino acid alphabets (Reetz and Wu (2008) "Greatly Reduced Amino Acid Alphabets in Directed Evolution: Making the Right Choice For Saturation Mutagenesis and Homologous Amino Acid Positions" Chem. Commun. 5499-5501). "Look through mutagenesis" (LTM) and other multidimensional mutagenesis methods have also been used to simultaneously assess and optimize combinations of mutations of selected amino acids (e.g., Rajpal eta 1. (2005) "A General Method for Greatly Improving the Affinity of Antibodies Using Combinatorial Libraries." PNAS 102(24): 8466-8471). Knowledge regarding the structure of a target polypeptide can be used to integrate structure-guided design with some degree of evolutionary randomization, such as by iterative saturation mutagenesis (ISM) (see, See, e.g., Reetz et al. (2008) "Addressing the Numbers Problem in Directed Evolution" ChemBioChem 9:1797-1804 and Reetz et al. (2006) "Iterative Saturation Mutagenesis on the Basis of B Factors as a Strategy for Increasing Protein Thermostability" Angew. Chem. 118: 7907-7915).

In addition to reducing the number of amino acids used for site mutations in a library of polypeptide mutants, nucleic acid codons that are used to encode a library of polypeptides can also be selected to reduce the number of library members that code for a given polypeptide. This reduces the number of polynucleotide library members, while simultaneously increasing the frequency with which a beneficial mutation appears in the library. One conventional example of this approach is the use of "NNK" or "NNS" degenerate codon sets, where N = A, C, G, or T, K = G or T and S = C or G. Degenerate codons that encode selected amino acids at particular sites can also be automatically determined, simplifying library construction. See, e.g., Mena and Daugherty (2005) "Automated design of degenerate codon libraries" Protein Engineering, Design & Selection 18(12):559-561. NDT (D = adenine, guanine or thymine; T = thymine) and NNK degenerate codon sets have been used for site saturation mutagenesis to reduce library complexity. See, e.g., Reetz et al. (2008) "Addressing the Numbers Problem in Directed Evolution" ChemBioChem 9:1797-1804. NNK encodes all 20 amino acids (plus one stop codon), using 32 codons; NDT encodes 12 amino acids using 12 codons (no stop codons).

Adding to the numbers problem, several-fold oversampling of mutagenic libraries during screening is typically performed to ensure that an adequate representative set of library members are screened during an overall mutagenesis and screening procedure. This oversampling further exacerbates the numbers problem noted above, by increasing the total number of library members that are screened in a given directed evolution method. Estimates of the degree of oversampling that is required for screening completeness typically assume an equal representation of library members, and that a random distribution of mutants is screened with each screening cycle. For example, Poisson statistics can be used to estimate the degree of library oversampling required for essentially complete coverage of the library. Under these assumptions, approximately 3x oversampling yields approximately 95% coverage of the library, or, put another way, if approximately 3 times the number of library member types (e.g., nucleic acids or polypeptides) is randomly screened during the overall screening process, then about 95% of the total member types will be tested during the screening process. Mathematically, the number of library members (T) actually screened can be transformed into the probability that a particular sequence occurs in the library can be expressed as:

$$T = -\ln(1 - P_i)/F_i, \qquad \text{Equation 1}$$

where $P_i$ denotes the probability that a particular library member sequence occurs in the library and $F_i$ is the frequency. Upon substituting for $F_i$, the relationship reduces to:

$$T = -V\ln(1 - P_i) \qquad \text{Equation 2.}$$

where V is the number of mutants in the library. See, e.g., Reetz et al. (2008), *above*. This oversampling problem exacerbates the difficulties that are encountered in sampling mutation sequence space. Indeed, the problem of oversampling has been described as leading to a "hopeless task" for comprehensive screening of more than a few residues in a polypeptide of interest. Reetz and Wu (2008), *above*.

The art would benefit from methods and compositions for reducing library complexity, improving screening efficiency, and reducing oversampling requirements during screening. The invention provides these and other features that will be apparent upon complete review of the following.

## SUMMARY OF THE INVENTION

The invention provides a library of polynucleotides, comprising: a mixture of polynucleotide variant molecules, the variant molecules comprising at least a first degenerate codon position, wherein degenerate codons of the first position encode any encoded amino acid in a selected molar ratio, and wherein each degenerate codon position comprises NNT, VWG, TGG wherein the ratio of codons is 16(NNT):6(VWG):1(TGG), or each degenerate codon position comprises (VMA, NDT, WKG) wherein the ratio of codons in the set is 6(VMA): 12(NDT):4(WKG).

The invention provides a method of making a library of polynucleotide variants, the method comprising:

providing a reference polynucleotide molecule or reference polynucleotide sequence;

selecting at least one site in the reference polynucleotide molecule or reference polynucleotide sequence to be varied; and,

producing a set of variant polynucleotides comprising degenerate codons at the site of interest, wherein the degenerate codons at each site of interest comprise (NNT, VWG, TGG) in a molar ratio of approximately 16:6:1, or wherein the degenerate codons at each site of interest comprise (VMA, NDT, WKG) and wherein the ratio of codons in the set is 6(VMA):12(NDT):4(WKG), thereby providing the library.

The invention provides a composition comprising a set of polynucleotide variants comprising at least one degenerate codon position, wherein the degenerate codons at the position comprise (NNT, VWG, TGG) in a molar ratio of approximately 16:6:1, or wherein the degenerate codons at the position comprise (VMA, NDT, WKG) and wherein the ratio of codons in the set is 6(VMA):12(NDT):4(WKG), wherein the polynucleotide variants collectively comprise all possible variants represented by the degenerate codon position.

The invention provides a method of producing a set of variant polynucleotide molecules, the method comprising:

providing a reference polynucleotide sequence;

selecting a plurality of codon sites in the reference polynucleotide sequence to be varied;

selecting a degenerate codon set for the plurality of codon sites to be varied, the degenerate codon set comprising (NNT, VWG, TGG) in a molar ratio of approximately 16:6:1 or comprising (VMA, NDT, WKG) wherein the ratio of codons in the set is 6(VMA): 12(NDT):4(WKG);

producing the set of variant polynucleotide molecules, each variant polynucleotide comprising a member of the degenerate codon set at each of the plurality of sites of codon variation, wherein the set of variant polynucleotide molecules collectively comprises all of the members of the degenerate codon set at each of the sites of codon variation.

The invention provides a method of producing a plurality of sets of variant polynucleotide molecules, the method comprising:

providing a reference polynucleotide sequence;

selecting a plurality of codon sites in the reference polynucleotide sequence to be varied;

selecting a degenerate codon set for the plurality of codon sites to be varied, the degenerate codon set comprising (NNT, VWG, TGG) in a molar ratio of approximately 16:6:1 or comprising (VMA, NDT, WKG) wherein the ratio of codons in the set is 6(VMA): 12(NDT):4(WKG);

separately producing the plurality of sets of variant polynucleotide molecules, each variant polynucleotide comprising a member of the degenerate codon set at each of the plurality of sites of codon variation, wherein the plurality of sets of variant polynucleotide molecules collectively comprises all of the members of the degenerate codon set at each of the sites of codon variation.

The invention provides a library made by a method of the invention. Disclosed herein are methods and compositions that reduce complexity of libraries of variant biological molecules, that reduce oversampling of these libraries during screening and that improve screening efficiency. For example, several sets of minimal degenerate codon sets are described that efficiently encode all, or nearly all canonical amino acids. This reduces the number of overall nucleic acid sequence variants that are made to code for amino acid variants during mutagenesis. Correspondingly, the use of such efficient codon sets increases the frequency with which a mutant of interest appears in a library, reducing the number of screening operations performed in analyzing the library. Oversampling during screening can also be decreased, due, in part, to improved library quality, and in part to a recognition that extensive oversampling provides diminishing returns during screening (analysis models that prioritize screening approaches are also described herein). The use of a single efficient codon set across several or all of the sites of variation in a nucleic acid of interest also simplifies construction of degenerate oligonucleotides used in the synthesis of variants, and can be used to improve automation of library construction, or to simplify manual library construction. In some aspects, variant pooling strategies are also used during library construction, in which separately synthesized variants or sets of variants are pooled before screening. This reduces the number of operations that are performed during library construction and screening. Logical filtering can be applied to select codon sites for mutagenesis, and/or to select amino acid sets to be incorporated at such sites.

Accordingly, in a first instance, disclosed herein are methods of making libraries of polynucleotide variants. In the methods, a target reference polynucleotide molecule or sequence of interest is provided. A plurality of codon sites in the reference polynucleotide molecule or sequence to be varied is selected, based upon criteria from the user, such as knowledge regarding structure, activity, homology, mutation coverage, or the like. A degenerate codon set for the plurality of codon sites to be varied is selected. For example, the degenerate set can be selected based upon maximizing amino acid diversity at each site of interest, e.g., using efficient degenerate codon sets (e.g., typically comprising more than one degenerate codon) that encode most or all amino acids such as (NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK), (NAC, VMG, DKK), (DKS, NAT, VMG), (NAC, DKS, VMG), or (TDK, VDT, VVA). These example preferred codon sets include, among others, 14 instances of 3 degenerate codon sets that encode all 20 canonical amino acids using 22 codons, 4 instances of 2 degenerate codon sets that encode 19 canonical amino acids using 21 codons, and 4 instances of 2 degenerate codon sets that encode 18 amino acids using 18 codons. The tables herein additionally list a variety of degenerate codon sets encoding at least 18 amino acids using 25 or fewer codons, using 2 degenerate codons. The tables herein also list a variety of preferred codon sets encoding 20 amino acids using 25 or fewer codons with three degenerate codons, as well as 19 amino acids using 19 codon sets with three degenerate codons, and 18 amino acids using 18 codon sets with three degenerate codons. All of these codon sets are also preferred in the present disclosure. In addition, a computer program is described herein that generates all possible codon sets with a selected number of degenerate codons. These can include, e.g., all possible codon sets encoding at least 18 amino acids using no more than 25 codons with either two or three degenerate codons being used. These also represent preferred codons for use in the present disclosure.

Optionally, any of a variety of logical filters can be applied to reduce the codon set, e.g., to simplify the codon set, or to encode amino acid sets by logically grouping amino acid residues by physico-chemical properties to create simplified amino acid sets based upon user selected properties.

These methods can include separately producing a plurality of sets of variant polynucleotide molecules, each variant polynucleotide including a member of the degenerate codon set at each site of codon variation. The sets of polynucleotide variants can be pooled to produce the library.

The variant polynucleotides can include any of a variety of sites of variation, selected based upon knowledge regarding structure or function, homology (e.g., one or more feature of a sequence alignment of homologous molecules), physico-chemical properties of encoded amino acids, statistical considerations (e.g., by applying statistical or pattern recognition software, Bayes classifiers, neural networks, Monte Carlo analysis, Principal Component Analysis (PCA), Markov modeling, neural networks, HMMs, etc.), results of mutagenesis experiments (e.g., results of a mutagenesis experiment performed on the reference polynucleotide or sequence, e.g., random mutagenesis, DNA shuffling, or alanine scanning), random or semi-random selection, or any other criteria selected by the user. The number of sites selected for variation depends on the selection criteria, the screening requirements for the particular assay, or the like. 1, 2, 3, 4, 5, 6, 7, 8, 9, 10, 11, 12, 13, 14, 15, 16, 17, 18, 19, 20, 25, 50, or 100 or more sites can be selected. Typically, several such sites (or, optionally, even all such sites) use the same codon set, though it is possible in some embodiments to use different codon sets for different sites. For example, at least 3, 5, 10, 15, 25, 50 or more sites of codon variation can be varied according to the same degenerate codon set. For example, optionally, about 5%, 10%, 20%, 30%, 40%, 50%, 60%, or 70% or more of the codons of a variant polynucleotide can be varied. For example, in one class of instances each site of codon variation in the pool of polynucleotides collectively comprises fewer than 32 codons (the size of a typical NNK codon set) and encodes more than 12 different amino acids. For example, the degenerate codon set can collectively encode at least 18, 19, or 20 different amino acids, using 25, 24, 23, 22, 20, 19, 18 or fewer degenerate codons. Example degenerate codon sets include (NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK), (NAC, VMG, DKK), (DKS, NAT, VMG), (NAC, DKS, VMG), or (TDK, VDT, VVA) and those listed in the tables herein, as well as those produced according to the program provided in the examples herein.

The ratio of amino acids that are incorporated at a position of variability in a library of polypeptides variants (or encoded in a library of nucleic acid variants) can be controlled by selecting the ratio of degenerate codons incorporated in the positions of variability. Optionally, the ratio of degenerate codons can be selected to encode amino acids in a ratio as close to 1:1 as possible. For example, when the degenerate codon position is encoded by NNT, VWG, TGG and wherein the ratio of codons can be selected to be 16(NNT):6(VWG):1(TGG); in another example, where the degenerate codon position is encoded by VMA, NDT, WKG the ratio of codons in the set can be selected to be 6(VMA):12(NDT):4(WKG). Similarly, preferred ratios for certain selected codon sets can include: (12(NDT), 9(VHG)), (12(NDC), 9(VHG)), (6(VWG), 16(NNC)), (16(NNT), 6(VWG)), (6(VMA), 12(NDT)), (12(NDC), 6(VMA)), (12(NDT), 6(VMG)), (12(NDC), 6(VMG)), (16(NNT), 3(VAA)), (16(NNC), 3(VAA)), (16(NNT), 3(VAG)), (3(VAG), 16(NNC)), (6(VMA), 12(NDT), 4(WKG)), (12(NDT), 1(TGG), 9(VHG)), (16(NNT), 6(VWG), 1(TGG)), (12(NDC), 1(TGG), 9(VHG)), (12(NDC), 6(VMA), 4(WKG)), (12(NDT), 4(WKG), 6(VMG)), (12(NDC), 4(WKG), 6(VMG)), (6(VMA), 4(NAT), 12(DKK)), (6(VMA), 4(NAC), 12(DKK)), (6(VMA), 12(DKS), 4(NAT)), (6(VMA), 4(NAC), 12(DKS)), (4(NAT), 6(VMG), 12(DKK)), (4(NAC), 6(VMG), 12(DKK)), (12(DKS), 4(NAT), 6(VMG)), (4(NAC), 12(DKS), 6(VMG)), (6(TDK), 9(VDT), 9(VVA)), etc. This ensures that amino acids of interest are not underrepresented in the overall library, thereby reducing oversampling requirements. In some instances, the user can, optionally, select different amino acid coding ratios, e.g., where over-representation of a selected amino acid (or selected amino acids) is desired, e.g., taking any of the logical filters noted herein into account.

The sets of polynucleotide variants can be separately synthesized prior to pooling. For example, separate partial or full-length variants can be synthesized prior to pooling, e.g., by various degenerate oligonucleotide synthesis and polymerase or ligase mediated assembly methods. For example, sets of degenerate oligonucleotides comprising the degenerate codons can be synthesized and used as amplification primers to amplify the reference polynucleotide, e.g., in polymerase or ligase amplification reactions. In one class of preferred embodiments, the sets of polynucleotide variants are produced by performing PCR using a primer that includes a variant sequence. The primer is bound to a circular template nucleic acid during the PCR reaction. In one example, the primer can be a megaprimer comprising a polynucleotide variant of interest. In another example, the PCR reaction can be primed from two abutting primers, at least one of which comprises a variant sequence. In yet another example, the PCR reaction is primed from two overlapping primers, at least one of which comprises a variant sequence. In another example, variant segments can be made using degenerate primers (oligonucleotides comprising degenerate codons) and a template of interest (e.g., a reference polynucleotide), the segments can then be spliced to form full length genes by SOE (Splicing by Overlap Extension).

Once synthesized, nucleic acid variants can be pooled to produce a library. The variants can be cloned prior to (or after) pooling, e.g., by assembly into expression vectors that facilitate expression and screening. Thus, the library may exist as a pool of nucleic acid variants cloned into expression vectors. The methods optionally further include expressing the library members in host cells and screening the resulting expression library for one or more properties of interest. In one aspect, oversampling is limited such that fewer than 95%, e.g., fewer than about 80%, 60%, or even about 50% or fewer of the total set of variant types are screened. Thus, oversampling efforts can be reduced both by reducing over- or under-representation of library members by codon selection, and also by considering the costs of additional oversampling versus the benefits that are achieved by incremental improvements in library screening coverage achieved by any additional oversampling.

Once library members of interest are identified, they can be further manipulated according to any available method. For example, members that display one or more properties of interest can be recombined or recursively recombined to produce a secondary library of variants, which can then be screened to identify additional library members that comprise improved properties of interest. Similarly, members of interest can be analyzed, e.g., by sequencing, to determine beneficial variations. These beneficial variations can be combined to produce the secondary library of variants, e.g., by recombination, recursive recombination or simply by constructing a variant that comprises multiple beneficial variations. Secondary (or tertiary or later) libraries can be screened using increasing stringency screening conditions to identify members that comprise improved properties.

In one set of related instances, disclosed herein are similar, overlapping or additional methods of making a library of polynucleotide variants. The methods include providing a reference polynucleotide molecule or reference polynucleotide sequence; selecting at least one site in the reference polynucleotide molecule or reference polynucleotide sequence to be varied; and, producing a set of variant polynucleotides comprising degenerate codons at the site of interest, wherein the degenerate codons are selected from the group consisting of: ((NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK), (NAC, VMG, DKK), (DKS, NAT, VMG), (NAC, DKS, VMG), or (TDK, VDT, VVA), or any of the sets provided in the tables or examples herein, thereby providing the library. All of the features noted above can be applied to these methods as well.

In one additional instance, disclosed herein are additional methods of making libraries of nucleic acid variants. The method includes providing a reference polynucleotide sequence to be varied, which reference polynucleotide sequence is present as a subsequence of a circular nucleic acid template. The circular nucleic acid template is amplified in a plurality of separate polymerase reactions. Each polymerase reaction has at least one polymerase primer that includes one or more variant sequence that is partially complementary to the reference sequence. The primer also includes at least one nucleotide difference as compared to the variant sequence. Each reaction comprises at least one unique variant primer as compared to at least one other polymerase reaction. The resulting variant amplicons are pooled and transformed into a population of host cells. All of the features noted above can be applicable to these methods as well. For example, the circular nucleic acid can be an expression vector comprising a reference polynucleotide. In one set of preferred instances, each reaction includes abutting or overlapping pairs of polymerase primers, at least one of which is at least partially complementary to the reference sequence to be varied. In another related preferred instance, the method includes separately producing a set of variant nucleic acids in separate PCR reactions each using a member of a degenerate oligonucleotide set as at least one PCR primer and using the resulting variant nucleic acid amplicons as the polymerase primers (e.g., the variants are used as megaprimers for subsequent PCR), during amplification of the circular nucleic acid template. As with the other methods already described, the method can include selecting a degenerate codon set and incorporating the degenerate codon set into at least one site of variation in the polymerase primers. Features noted in the methods above can be used in combination with this set of methods.

Libraries made by any of the methods herein, including any of those methods noted above, are a feature of the disclosure. In one example, disclosed herein are a library of polynucleotides. The library can include, e.g., a mixture of polynucleotide variant molecules, that include at least a first degenerate codon position. The degenerate codons of the first position can encode any encoded amino acid, e.g., in a selected molar ratio, e.g., where the degenerate codons at each of the positions collectively comprise fewer than 32 codons and encode more than 12 different amino acids. As noted above with reference to the methods herein, the selected molar ratio can be, e.g., approximately 1:1 for each encoded amino acid, though the user can, optionally, select different ratios where over-representation of a selected amino acid (or selected amino acids) is desired (e.g., taking any of the logical filters noted above into account). As noted above, the library can include degenerate codon sets that collectively encode at least 18 different amino acids, e.g., using 25, 24, 23, 22, 21, 20, 19, 18 or fewer codons, or any of the other ratios noted above. Preferred codon sets include: (NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK), (NAC, VMG, DKK), (DKS, NAT, VMG), (NAC, DKS, VMG), or (TDK, VDT, VVA) and those sets provided in the tables and examples herein. The ratios of codons in these degenerate codon sets can include any of those noted above.

The library of polynucleotide variants can collectively include a plurality of degenerate codon positions, e.g., each comprising one of the enumerated codon sets herein. Similarly, the variant molecules can collectively include a first set of polynucleotide variants comprising at least a first degenerate codon position, and a second set of polynucleotide variants comprising at least a second degenerate codon position different from the first position. The first and the second position optionally include the same degenerate codon set. The ratios of any encoded amino acids can be the same at each position or different; in one example, the degenerate codons are present at a ratio of 1:1 in both the first and second set. The variants can collectively include a wild-type codon at each position of variation, e.g., where the degenerate codon set at the position encodes all possible amino acids, including the wild-type amino acids. Each nucleic acid variant can include a wild type or other reference amino acid or a variant amino acid at each position. Thus, one variant may have a wild-type amino acid at a first position, and a variant amino acid at a second position, while another variant may have a variant amino acid at the first position and a wild type amino acid at the second position. A third variant can include variants at both the first and second position, can include, e.g., a wild type or other reference codon at both position, and a variant at a third position. For example, the library can include (a) the first set of nucleic acid variants, which includes a wild-type or other reference codon for a plurality of members of the first set in a codon position corresponding to the second degenerate codon position in the second set; and (b) the second set of nucleic acid variants comprises a reference codon for a plurality of members of the second set in a codon position corresponding to the first degenerate codon position in the first set; or both (a) and (b). The first and second set of polynucleotide variants in this example can include members that have the same sequences (overlapping members). The variant molecules can include full-length variants, partial length variants or degenerate oligonucleotides, e.g., each member of the set including at least one degenerate codon position, e.g., where the degenerate codons of the position collectively encode amino acids in a selected molar ratio (e.g., 1:1 or another selected ratio for each encoded amino acid).

Similarly, disclosed is a composition that includes a set of polynucleotide variants as noted above. For example, the variants can include at least one degenerate codon position, e.g., with codons at the position being selected from the group consisting of: (NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK), (NAC, VMG, DKK), (DKS, NAT, VMG), (NAC, DKS, VMG), or (TDK, VDT, VVA), or any other set in the tables and examples herein. In one example, the polynucleotide variants collectively comprise all possible variants represented by the degenerate codon position, e.g., a position of variation. The variants can collectively comprise all possible variants represented by more than one degenerate codon position, e.g., at each position of variation. For example, the variants can include a plurality of degenerate codon positions, with codons at each position being selected from the group consisting of: (NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK),

(NAC, VMG, DKK), (DKS, NAT, VMG), (NAC, DKS, VMG), or (TDK, VDT, VVA), or those of the tables and example herein.

Systems and methods comprising user executable instructions relating to the other methods and compositions herein are also a feature of the disclosure. In one aspect, the disclosure includes a method (e.g., a computer assisted method) that allows a user to determine codon sets comprising degenerate codons that encode a desired set of amino acids. For example, the method can include providing a user interface that permits a user to input a desired number (and/or composition) of amino acids to be encoded, a total number of codons to encode the desired number of amino acids, and, optionally, a number of degenerate codons to be used in codon sets comprising the total number of codons. This information (amino acid number and/or composition, codon number, and, optionally, the number of degenerate codons) is input by the user through the user interface. Computer executable instructions are provided to output a list of degenerate codon sets, amino acids, and/or codons to the user (e.g., to a display or printout), to determine which codons encode the amino acids specified by the user.

For example, the number of degenerate codons can be between, e.g., 2 and 5, but is more typically between 2 and 4 and usually between 2 and 3. The number of amino acids can be between 12 and 20, and, in many preferred instances, is typically between 18 and 20 (e.g., to provide maximum diversity at each position to be varied in a variant polynucleotide or polypeptide). The number of total codons is less than 32, and is typically less than 25, and, in some preferred aspects, can be 22 or less. All ranges herein are inclusive, unless specifically indicated otherwise.

A corresponding system comprising a computer readable medium containing computer interpretable logic or instructions is also a feature of the disclosure. The system accepts a user instruction specifying a desired number of amino acids to be encoded, a total number of codons to encode the desired number of amino acids, and a number of degenerate codons to be used in the codon sets. The logic or instructions outputs a list of degenerate codons to the user. The disclosure also includes degenerate codon sets output by the systems and methods.

For example, the codons at a position can include any of those noted above, in a ratio selected to control representation of any or all encoded amino acids. For example, the ratio can be selected to provide for even representation of encoded amino acids. For example, if the degenerate codon set is (NNT, VWG, TGG) for variant positions, the encoded amino acids can be encoded in the set at a position in a molar ratio of approximately 16:6:1; similarly, where the codons are NNT, VWG, the degenerate codons are present in the set at each position of interest in a molar ratio of approximately 16:6, or where the codons at the position comprise (VMA, NDT, WKG) and the ratio of codons in the set is 6(VMA):12(NDT):4(WKG) (which is equivalent to 3(VMA):6(NDT):2(WKG)). The compositions can include synthetic oligonucleotides, nucleic acid amplicons, or the like.

The libraries and compositions herein can be associated with other components to provide a kit, e.g., to practice the methods herein. Such kits can further include packaging materials, containers that contain the libraries or compositions, software or system instructions for making variants using the codon sets herein, or the like. Systems comprising fluid handling apparatus, e.g., coupled to a computer that includes system instructions for practicing the methods or using the compositions is also a feature of the disclosure. The kits or systems can also include appropriate instructional materials, reagents or buffers useful in manipulating the compositions or libraries, cloning vectors for cloning library members, screening or assay components or reagents for screening the libraries, or the like. The methods, compositions, libraries, kits, systems and other features noted herein can be used in combination. The methods herein can, in some cases, use the compositions, libraries, kits or systems noted herein, and the methods herein include features that can be used to modify these components.

**BRIEF DESCRIPTION OF THE DRAWINGS**

Figure 1 provides a schematic overview of Automated Parallel SOEing (APS).

Figure 2 provides a process flow chart for mutagenesis by APS ("MAPS")

Figure 3 provides a schematic overview and flow chart of a megaprimer PCR cloning method.

Figure 4 provides a schematic overview of megaprimer PCR cloning.

Figure 5 provides a schematic overview of megaprimer PCR cloning applications.

Figure 6 provides a schematic overview of abutting primer Site Directed Mutagenesis.

Figure 7 provides a schematic overview of overlapping primer PCR, showing use of 5'overlapping primers to improve the megaprimer method.

Figure 8 provides an example flow chart for a combined APS procedure (CAPS), as compared to MAPS.

Figure 9 provides a schematic overview of SNOCAPS performed with a codon set of the invention.

Figure 10 provides a schematic of the plate manipulations involved in making libraries of the invention, e.g., via SNOCAPS fragment assembly.

Figures 11 provides a schematic of the plate manipulations involved in making libraries, e.g., via SNOCAPS library assembly showing resulting pooled libraries for each codon site position.

Figure 12 provides a screening resource model to optimize resource allocation during screening.

Figure 13 graphically illustrates application of a resource model.

Figure 14 provides a generalized workflow schematic.

Figure 15 provides a schematic system for determining degenerate codons.

Figure 16 provides a flow chart of an overall process for inputting information into a system that provides degenerate codon information.

## DETAILED DESCRIPTION

Disclosed herein are methods and compositions for reducing mutant library complexity, simplifying library construction, improving library screening efficiency, and reducing oversampling requirements during screening. This is achieved, e.g., by one or more of: (a) providing efficient and tunable codon sets for mutagenesis; (b) optimizing library construction through the use of highly efficient restriction site- independent cloning methods; (c) by pooling library members before transformation into host cells, reducing parallel screening operations; and (d) by optimizing screening, e.g., by consideration of resource models, to reduce oversampling requirements. These improvements to library construction and screening methodologies are optionally used in conjunction with additional strategies for library optimization, such as the use of logical filters to guide residue selection for mutagenesis.

As noted, disclosed herein are efficient codon sets for mutagenesis. Preferred codon sets include (NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK), (NAC, VMG, DKK), (DKS, NAT, VMG), (NAC, DKS, VMG) and (TDK, VDT, VVA), as well as those listed in the tables and examples herein. These example preferred codon sets encode most or all of the 20 canonical amino acids, using, e.g., 25 or fewer codons. In the context of mutagenesis, this is a significant improvement over the canonical 64 codons that nature uses to encode the 20 cannonical amino acids; the codon sets of the disclosure are also more efficient than the commonly used NNK and NNS codon sets, which code for the 20 canonical amino acids using 32 total codons. The use of a single efficient codon set across several or all of the sites of variation in a reference nucleic acid of interest also simplifies construction of degenerate oligonucleotides used in the synthesis of such variants, and can be used to improve automation of library construction, and/or to simplify library construction.

In some examples, disclosed herein are improvements to mutant library construction methods, e.g., by incorporating restriction site-independent mutagenesis methods. For example, degenerate primers constructed to comprise efficient codon sets at each site of variation are used to amplify a circular template comprising a reference polynucleotide, thereby incorporating variant residues into amplicons. These amplicons can be pooled and directly transformed into cells that comprise ligase activity, or ligation can be performed in vitro, prior to or after pooling. Three examples of this include MEGA WHOP (megaprimer PCR of whole plasmid, also denoted "megaprimer PCR"), abutting primer amplification, and overlapping primer amplification, performed, e.g., on circular templates. Further details on each of these approaches is found herein.

A variety of library pooling construction strategies are described in USSN 61/061,581 filed June 13, 2008; USSN 12/483,089 filed June 11, 2009; PCT/US2009/047046 filed June 11, 2009; USSN 12/562,988 filed September 18, 2009; and PCT/US2009/057507 filed September 18, 2009. These approaches are useful in the context of the current invention. In general, pooling of variants prior to transformation into a population of host cells reduces library complexity by eliminating any need to physically separate library members; library members can also be screened together, simplifying overall screening methods.

Screening can be simplified by reducing oversampling requirements. The present invention accomplishes this in at least two ways. First, by selecting codon sets to evenly encode (or, alternately to tunably/ selectably encode) amino acids, the problem of finding a rare variant in a library is eliminated. By way of illustration, in a canonical 64 codon amino acid set, some amino acids are encoded by more codons than are other more rarely encoded amino acids:

Inverse Codon Table

| Ala/A | GCU, GCC, GCA, GCG | Leu/L | UUA, UUG, CUU, CUC, CUA, CUG |
|---|---|---|---|
| Arg/R | CGU, CGC, CGA, CGG, AGA, AGG | Lys/K | AAA, AAG |
| Asn/N | AAU, AAC | Met/M | AUG |
| Asp/D | GAU, GAC | Phe/F | UUU, UUC |
| Cys/C | UGU, UGC | Pro/P | CCU, CCC, CCA, CCG |
| Gln/Q | CAA, CAG | Ser/S | UCU, UCC, UCA, UCG, AGU, AGC |
| Glu/E | GAA, GAG | Thr/T | ACU, ACC, ACA, ACG |
| Gly/G | GGU, GGC, GGA, GGG | Trp/W | UGG |
| His/H | CAU, CAC | Tyr/Y | UAU, UAC |
| Ile/I | AUU, AUC, AUA | Val/V | GUU, GUC, GUA, GUG |
| START | AUG | STOP | UAA, UGA, UAG |

Thus, for example, M and W are encoded by 1 codon each, while L and S are encoded by 6 codons each. If a position of interest includes all 61 possible coding codons (64 minus 3 stop codons) at equal frequency, then there is a 6/61 or 9.8% chance that the position will be L or S, but only a 1/61 or 1.6% chance that it will encode an M or W. In order to be reasonably assured that an M or W is screened, a library constructed using an "NNN" codon set (all possible codons) is typically significantly oversampled (repetitively sampled, to increase the likelihood of finding a rare variant). In contrast, in the present invention, any oversampling burden is reduced in identifying any given amino acid variant, because the variants can all be encoded with an equal (or a tunably selected) frequency. Secondly, oversampling can be reduced when the likelihood of finding an additional desirable variant is outweighed by the costs of rescreening the library, or when a resource allocation model suggests that it would be more productive to place additional resources into making and screening additional libraries, rather than in additional oversampling. In many instances, particularly where active variants are identified early in screening, it can be desirable to sample the library only 1x; in other instances the library can be partly or fully resampled, one or more times. In many instances, resampling can be performed such that there is only about a 50%-85% or lower likelihood that any given variant will be screened, e.g., considering standard Poisson probability metrics. Sufficient oversampling can often be achieved with a single (or even a partial) oversampling pass of the library. As is discussed in more detail herein, adding rounds of mutation and selection is often more productive than attempts to sample any particular library exhaustively. The disclosure includes models to assess the relative benefits of performing additional rounds of library construction and screening, as compared to resampling a given library with an additional round of screening.

DEGENERATE CODON SETS AND OLIGONUCLEOTIDES

Codon sets are selected to comprise a high level of diversity, e.g., to code for, e.g., 12 or more, 13 or more, 14 or more, 15 or more, 16 or more, 17 or more, 18 or more, 19 or more, or 20 codons/ amino acid. If non-canonical amino acids are desired at positions of interest, then the codon set can also be selected to code for any such non-canonical amino acids. For a description of genetically encoding non-canonical amino acids see, e.g., Wang and Schultz, (2005) "Expanding the Genetic Code," Angewandte Chemie Int Ed 44: 34-66; Xie and Schultz, (2005) "An Expanding Genetic Code," Methods 36: 227-238; Xie and Schultz, (2005) "Adding Amino Acids to the Genetic Repertoire," Curr Opinion in Chemical Biology 9: 548-554; and Wang, et al., (2006) "Expanding the Genetic Code," Annu Rev Biophys Biomol Struct 35: 225-249. In general, to encode a non-canonical amino acid, a degenerate codon set of the disclosure can additionally include one or more codon coding for the non-canonical amino acid (stop and four base codons are useful for this purpose).

Degenerate codon sets are preferably selected to efficiently encode the desired amino acid set at issue. Algorithms can be designed to identify efficient codon sets, or the sets can be determined by consideration of the genetic code, e.g., by considering redundancies in the inverse codon table noted above, or by considering equivalent information in a standard codon table, such as may be found in Molecular Biology of the Cell: Reference Edition (2007) Alberts et al. Garland Science, 5th edition ISBN-10: 0815341113). Optimization algorithms such as Monte Carlo or genetic algorithms can be used to sample the sequence possibilities of possible codon sets. Desirable properties of the candidate sets can be used, e.g., to guide the optimization process using a weighted fitness function. The function can include, e.g., numerical scores for each property and weighting of the scores to form linear or nonlinear combinations expressing the overall fitness of a candidate codon set. Such desirable properties include minimizing the number of degenerate codons, maximizing the number of amino acids encoded, and minimizing the redundancy of the coded amino acids. One advantage of using codon sets and corresponding degenerate oligonucleotides, as compared to specific primers for each alternate amino acid variant at a codon site is reduced oligonucleotide synthesis cost and a reduction in physical manipulations to incorporate mutagenic codons into final variant products. This is because it is less expensive to make and manipulate 2 or 3 degenerate oligonucleotides comprising the possible variants at a codon site than it is to make, e.g., 19 separate primers to encode the possible variants at the site.

Degenerate base symbols in a degenerate codon provide an IUPAC approved representation for a position within the codon that can have multiple possible alternatives. These are not to be confused with non-canonical bases such as inosine, in that each sequence that is made has one of the regular canonical bases. The following table provides standard IUPAC degenerate nucleotide nomenclature (see also Biochemical Nomenclature and Related Documents, 2nd edition, Portland Press, 1992).

IUPAC Nucleotide Nomenclature Table

| symbol | base | symbol | base |
|---|---|---|---|
| A | adenosine | M | A C (amino) |
| C | cytidine | S | G C (strong) |
| G | guanine | w | A T (weak) |
| T | thymidine | B | G T C |
| U | uridine | D | G A T |
| R | G A (purine) | H | A C T |
| Y | T C (pyrimidine) | V | G C A |
| K | G T (keto) | N | A G C T (any) |

Preferred codon sets that encode many amino acids, using a minimal number of codons include (NDT, VHG), (NDC, VHG), (VWG, NNC), (NNT, VWG), (VMA, NDT), (NDC, VMA), (NDT,VMG), (NDC, VMG), (NNT,VAA), (NNC, VAA), (NNT,VAG), (VAG, NNC), (VMA, NDT, WKG), (NDT, TGG, VHG), (NNT, VWG, TGG), (NDC, TGG, VHG), (NDC,VMA,WKG), (NDT,WKG,VMG), (NDC,WKG,VMG), (VMA, NAT, DKK), (VMA, NAC, DKK), (VMA, DKS, NAT), (VMA, NAC, DKS), (NAT, VMG, DKK), (NAC, VMG, DKK), (DKS, NAT, VMG), (NAC, DKS, VMG), and (TDK, VDT, VVA), as well as a variety of additional preferred sets described in the tables herein. The following table provides additional information for these preferred codon sets.

Table of Example Degenerate Codon Sets

| Design | Codons | Residues | Missing | |
|---|---|---|---|---|
| NNK | 32 | 20 | - | Available as an NNN alternative - reduces screening requirements while retaining all amino acids. |

(continued)

| Design | Codons | Residues | Missing | |
|---|---|---|---|---|
| (NDT, TGG, VHG) | | | | |
| (NDC, TGG, VHG) | | | | |
| (VMA, NDT, WKG) | | | | |
| (NDC, VMA, WKG) | | | | |
| (NDT, WKG, VMG) | | | | |
| (NDC, WKG, VMG) | | | | |
| (VMA, NAT, DKK) | | | | |
| (VMA, NAC, DKK) | | | | |
| (VMA, DKS, NAT) | | | | |
| (VMA, NAC, DKS) | | | | |
| (NAT, VMG, DKK) | | | | |
| (NAC, VMG, DKK) | | | | |
| (DKS,NAT, VMG) | 22 | 20 | - | Includes preferred alternatives for screening all amino acids-significantly reduces library complexity while retaining all amino acids. |
| (NDT, VHG) (NDC, VHG) | 21 | 19 | W | Only uses 2 degenerate codons, encodes 19 amino acids (omits W). |
| (VMA, NDT) (NDC, VMA) (NDT, VMG) (NDC, VMG) | 18 | 18 | M,W | Only uses 2 degenerate codons, encodes 18 amino acids (omits M,W) with 18 codons |
| (NNT, VAA) (NNC, VAA) (NNT, VAG) (VAG, NNC) | 19 | 18 | M,W | Almost as efficient as above, only uses 2 degenerate codons, encodes 18 amino acids with 19 codons. |
| (VWG, NNT, TGG) | 23 | 20 | - | Additional example alternative. Retains all amino acids, almost as efficient as 22 codon set; note: tables herein provide many similar examples. |
| (TDK, VDT, VVA) | 24 | 19 | | Includes 1 stop codon. |

The following table provides preferred degenerate codon sets to produce relatively complete sets of canonical amino acids. The codon sets each use two degenerate codons, which encode 18 or 19 amino acids, using 25 or fewer codons (e.g., 18, 19, 20, 21, 22, 23, 24 or 25 codons, as indicated.

Table: Two Degenerate Codon Solutions

| Codons | AA | Degenerate Codons | Amino Acids |
|---|---|---|---|
| 21 | 19 | NDT,VHG, | F,K,T,R,M,D,C,E,N,G,Q,S,V, Y,A,P,H,L,I, |

(continued)

| Codons | AA | Degenerate Codons | Amino Acids |
|---|---|---|---|
| 21 | 19 | NDC,VHG, | F,K,T,R,M,D,C,E,N,G,Q,S,V,Y,A,P,H,L,I, |
| 22 | 19 | VWG,NNT, | F,K,T,R,M,D,C,E,N,G,S,Q,Y,V,A,P,H,L,I, |
| 22 | 19 | VWG,NNC, | F,K,R,T,M,D,C,E,N,G,S,Q,Y,V,A,P,H,L,I, |
| 24 | 19 | DKK,VMK, | F,K,R,T,M,C,D,E,N,G,Q,S,V,A,TRP,P,L,H,I, |
| 24 | 19 | DKS,VMK, | F,K,R,T,M,C,D,E,N,G,Q,S,V,A,TRP,P,L,H,I, |
| 24 | 19 | VMM,DKK, | F,K,R,T,M,C,D,E,N,G,Q,S,V,A,TRP,P,L,H,I, |
| 24 | 19 | DKS,VMM, | F,K,R,T,M,C,D,E,N,G,Q,S,V,A,TRP,P,L,H,I, |
| 24 | 19 | VMS,DKK, | F,K,R,T,M,C,D,E,N,G,Q,S,V,A,TRP,P,L,H,I, |
| 24 | 19 | DKS,VMS, | F,K,R,T,M,C,D,E,N,G,Q,S,V,A,TRP,P,L,H,I, |
| 24 | 19 | VMW,DKK, | F,K,R,T,M,C,D,E,N,G,Q,S,V,A,TRP,P,L,H,I, |
| 24 | 19 | VMW,DKS, | F,K,R,T,M,C,D,E,N,G,Q,S,V,A,TRP,P,L,H,I, |
| 24 | 19 | NDT,VNG, | F,K,R,T,M,D,C,E,N,G,Q,S,Y,V,A,P,H,L,I, |
| 24 | 19 | NDC,VNG, | F,K,R,T,M,D,C,E,N,G,Q,S,Y,V,A,P,H,L,I, |
| 25 | 19 | NNT,VHG, | F,K,T,R,M,D,C,E,N,G,S,Q,Y,V,A,P,H,L,I, |
| 25 | 19 | NNC,VHG, | F,K,T,R,M,D,C,E,N,G,S,Q,Y,V,A,P,H,L,I, |
| 25 | 19 | NNT,VDG, | F,K,R,T,M,D,C,E,N,G,S,Q,Y,V,A,P,H,L,I, |
| 25 | 19 | NNC,VDG, | F,K,R,T,M,D,C,E,N,G,S,Q,Y,V,A,P,H,L,I, |
| 18 | 18 | VMA,NDT, | F,K,T,R,C,D,E,N,G,Q,S,Y,V,A,P,L,H,I, |
| 18 | 18 | NDC,VMA, | F,K,T,R,C,D,E,N,G,Q,S,V,Y,A,P,H,L,I, |
| 18 | 18 | NDT,VMG, | F,K,T,R,C,D,E,N,G,Q,S,Y,V,A,P,L,H,I, |
| 18 | 18 | NDC,VMG, | F,K,T,R,C,D,E,N,G,Q,S,V,Y,A,P,H,L,I, |
| 19 | 18 | NNT,VAA, | F,K,T,R,D,C,E,N,G,Q,S,Y,V,A,P,H,L,I, |
| 19 | 18 | NNC,VAA, | F,K,R,T,D,C,E,N,G,Q,S,V,Y,A,P,H,L,I, |
| 19 | 18 | NNT,VAG, | F,K,T,R,D,C,E,N,G,Q,S,Y,V,A,P,H,L,I, |
| 19 | 18 | VAG,NNC, | F,K,R,T,D,C,E,N,G,Q,S,V,Y,A,P,H,L,I, |
| 20 | 18 | NNT,MWG, | F,K,T,R,M,D,C,N,G,Q,S,V,Y,A,P,H,L,I, |
| 20 | 18 | MWG,NNC, | F,K,R,T,M,D,C,N,G,Q,S,V,Y,A,P,H,L,I, |
| 20 | 18 | NNT,RWG, | F,K,T,R,M,D,C,E,N,G,S,V,Y,A,P,H,L,I, |
| 20 | 18 | NNC,RWG, | F,K,R,T,M,D,C,E,N,G,S,V,Y,A,P,H,L,I, |
| 21 | 18 | NDT,VVA, | F,K,R,T,D,C,E,N,G,Q,S,Y,V,A,P,L,H,I, |
| 21 | 18 | NDC,VVA, | F,K,R,T,D,C,E,N,G,Q,S,V,Y,A,P,H,L,I, |
| 21 | 18 | VVG,NDT, | F,K,R,T,D,C,E,N,G,Q,S,Y,V,A,P,L,H,I, |
| 21 | 18 | NDC,VVG, | F,K,R,T,D,C,E,N,G,Q,S,V,Y,A,P,H,L,I, |
| 21 | 18 | VHA,NDT, | F,K,T,R,D,C,E,N,G,Q,S,V,Y,A,P,H,L,I, |
| 21 | 18 | NDC,VHA, | F,K,T,R,D,C,E,N,G,Q,S,V,Y,A,P,H,L,I, |
| 21 | 18 | NHT,VDG, | F,K,R,T,M,D,E,N,G,Q,S,V,Y,A,P,H,L,I, |
| 21 | 18 | NHC,VDG, | F,K,R,T,M,D,E,N,G,Q,S,V,Y,A,P,H,L,I, |
| 21 | 18 | HDT,VNG, | F,K,R,T,M,C,E,N,G,Q,S,Y,V,A,P,H,L,I, |

(continued)

| Codons | AA | Degenerate Codons | Amino Acids |
|--------|----|--------------------|-------------|
| 21 | 18 | HDC,VNG, | F,K,R,T,M,C,E,N,G,Q,S,Y,V,A,P,H,L,I, |
| 21 | 18 | DDT,VNG, | F,K,R,T,M,D,C,E,N,G,Q,S,Y,V,A,P,L,I, |
| 21 | 18 | DDC,VNG, | F,K,R,T,M,D,C,E,N,G,Q,S,Y,V,A,P,L,I, |
| 22 | 18 | NNT,MHG, | F,K,T,R,M,D,C,N,G,Q,S,V,Y,A,P,H,L,I, |
| 22 | 18 | NNC,MHG, | F,K,T,R,M,D,C,N,G,Q,S,Y,V,A,P,H,L,I, |
| 22 | 18 | NNT,MDG, | F,K,R,T,M,D,C,N,G,Q,S,V,Y,A,P,H,L,I, |
| 22 | 18 | NNC,MDG, | F,K,R,T,M,D,C,N,G,Q,S,Y,V,A,P,H,L,I, |
| 22 | 18 | NNT,RHG, | F,K,T,R,M,D,C,E,N,G,S,Y,V,A,P,H,L,I, |
| 22 | 18 | NNC,RHG, | F,K,T,R,M,D,C,E,N,G,S,Y,V,A,P,H,L,I, |
| 22 | 18 | NNT,RDG, | F,K,R,T,M,D,C,E,N,G,S,V,Y,A,P,H,L,I, |
| 22 | 18 | NNC,RDG, | F,K,R,T,M,D,C,E,N,G,S,V,Y,A,P,H,L,I, |
| 22 | 18 | NNT,VAK, | F,K,T,R,D,C,E,N,G,Q,S,V,Y,A,P,L,H,I, |
| 22 | 18 | VAK,NNC, | F,K,R,T,D,C,E,N,G,Q,S,Y,V,A,P,L,H,I, |
| 22 | 18 | VAM,NNT, | F,K,T,R,D,C,E,N,G,Q,S,V,Y,A,P,L,H,I, |
| 22 | 18 | VAM,NNC, | F,K,R,T,D,C,E,N,G,Q,S,Y,V,A,P,L,H,I, |
| 22 | 18 | NNT,VAR, | F,K,T,R,D,C,E,N,G,Q,S,Y,V,A,P,L,H,I, |
| 22 | 18 | VAR,NNC, | F,K,R,T,D,C,E,N,G,Q,S,Y,V,A,P,L,H,I, |
| 22 | 18 | NNT,V AS, | F,K,T,R,D,C,E,N,G,Q,S,V,Y,A,P,L,H,I, |
| 22 | 18 | NNC,VAS, | F,K,R,T,D,C,E,N,G,Q,S,Y,V,A,P,L,H,I, |
| 22 | 18 | NNT,VAW, | F,K,T,R,D,C,E,N,G,Q,S,V,Y,A,P,L,H,I, |
| 22 | 18 | NNC,VAW, | F,K,R,T,D,C,E,N,G,Q,S,Y,V,A,P,L,H,I, |
| 22 | 18 | VMA,NNT, | F,K,T,R,D,C,E,N,G,Q,S,V,Y,A,P,H,L,I, |
| 22 | 18 | VMA,NNC, | F,K,T,R,D,C,E,N,G,Q,S,Y,V,A,P,H,L,I, |
| 22 | 18 | NNT,VMG, | F,K,T,R,D,C,E,N,G,Q,S,V,Y,A,P,H,L,I, |
| 22 | 18 | NNC,VMG, | F,K,T,R,D,C,E,N,G,Q,S,Y,V,A,P,H,L,I, |
| 22 | 18 | NNT,VRA, | F,K,R,T,D,C,E,N,G,Q,S,Y,V,A,P,H,L,I, |
| 22 | 18 | NNC,VRA, | F,K,R,T,D,C,E,N,G,Q,S,Y,V,A,P,H,L,I, |
| 22 | 18 | NNT,VRG, | F,K,R,T,D,C,E,N,G,Q,S,Y,V,A,P,H,L,I, |
| 22 | 18 | VRG,NNC, | F,K,R,T,D,C,E,N,G,Q,S,V,A,P,H,L,I, |
| 22 | 18 | NNT,VWA, | F,K,T,R,D,C,E,N,G,S,Q,Y,V,A,P,H,L,I, |
| 22 | 18 | VWA,NNC, | F,K,R,T,D,C,E,N,G,S,Q,Y,V,A,P,H,L,I, |
| 24 | 18 | MNK,KNT, | F,K,R,T,M,D,C,N,G,S,Q,V,Y,A,P,L,H,I, |
| 24 | 18 | KNT,MNS, | F,K,T,R,M,D,C,N,G,S,Q,V,Y,A,P,L,H,I, |
| 24 | 18 | MNK,KNC, | F,K,R,T,M,D,C,N,G,S,Q,Y,V,A,P,L,H,I, |
| 24 | 18 | KNC,MNS, | F,K,T,R,M,D,C,N,G,S,Q,Y,V,A,P,L,H,I, |
| 24 | 18 | NNT,MWK, | F,K,T,R,M,D,C,N,G,S,Q,V,Y,A,P,L,H,I, |
| 24 | 18 | NNC,MWK, | F,K,R,T,M,D,C,N,G,S,Q,Y,V,A,P,L,H,I, |
| 24 | 18 | MWR,NNT, | F,K,T,R,M,D,C,N,G,Q,S,V,Y,A,P,H,L,I, |

(continued)

| Codons | AA | Degenerate Codons | Amino Acids |
|---|---|---|---|
| 24 | 18 | MWR,NNC, | F,K,R,T,M,D,C,N,G,Q,S,Y,V,A,P,H,L,I, |
| 24 | 18 | NNT,MWS, | F,K,T,R,M,D,C,N,G,S,Q,V,Y,A,P,L,H,I, |
| 24 | 18 | MWS,NNC, | F,K,R,T,M,D,C,N,G,S,Q,Y,V,A,P,L,H,I, |
| 24 | 18 | BNT,MHK, | F,K,R,T,M,D,C,N,G,Q,S,Y,V,A,P,L,H,I, |
| 24 | 18 | BNC,MHK, | F,K,R,T,M,D,C,N,G,Q,S,V,Y,A,P,L,H,I, |
| 24 | 18 | BNT,MHS, | F,K,R,T,M,D,C,N,G,Q,S,Y,V,A,P,L,H,I, |
| 24 | 18 | BNC,MHS, | F,K,R,T ,M,D,C,N,G,Q,S,V,Y,A,P,L,H,I, |
| 24 | 18 | NNT,MNG, | F,K,R,T,M,D,C,N,G,Q,S,V,Y,A,P,H,L,I, |
| 24 | 18 | NNC,MNG, | F,K,R,T ,M,D,C,N,G,Q,S,Y,V,A,P,H,L,I, |
| 24 | 18 | NNT,RWK, | F,K,T,R,M,D,C,E,N,G,S,Y,V,A,P,H,L,I, |
| 24 | 18 | RWK,NNC, | F,K,R,T,M,D,C,E,N,G,S,Y,V,A,P,H,L,I, |
| 24 | 18 | NNT,RWR, | F,K,T,R,M,D,C,E,N,G,S,Y,V,A,P,H,L,I, |
| 24 | 18 | NNC,RWR, | F,K,R,T,M,D,C,E,N,G,S,Y,V,A,P,H,L,I, |
| 24 | 18 | NNT,RWS, | F,K,T,R,M,D,C,E,N,G,S,Y,V,A,P,H,L,I, |
| 24 | 18 | NNC,RWS, | F,K,R,T,M,D,C,E,N,G,S,Y,V,A,P,H,L,I, |
| 24 | 18 | BNT,RHK, | F,K,T,R,M,C,D,E,N,G,S,V,Y,A,P,L,H,I, |
| 24 | 18 | BNC,RHK, | F,K,T,R,M,C,D,E,N,G,S,V,Y,A,P,L,H,I, |
| 24 | 18 | BNT,RHS, | F,K,T,R,M,C,D,E,N,G,S,V,Y,A,P,L,H,I, |
| 24 | 18 | BNC,RHS, | F,K,T,R,M,C,D,E,N,G,S,V,Y,A,P,H,L,I, |
| 24 | 18 | NNT,RNG, | F,K,R,T,M,D,C,E,N,G,S,Y,V,A,P,H,L,I, |
| 24 | 18 | NNC,RNG, | F,K,R,T,M,D,C,E,N,G,S,Y,V,A,P,H,L,I, |
| 24 | 18 | YNT,RNK, | F,K,T ,R,M,C,D,E,N,G,S,Y,V,A,P,L,H,I, |
| 24 | 18 | YNC,RNK, | F,K,T,R,M,C,D,E,N,G,S,Y,V,A,P,H,L,I, |
| 24 | 18 | RNS,YNT, | F,K,T,R,M,C,D,E,N,G,S,Y,V,A,P,H,L,I, |
| 24 | 18 | RNS,YNC, | F,K,T,R,M,C,D,E,N,G,S,Y,V,A,P,H,L,I, |
| 24 | 18 | BNT,VWK, | F,K,R,M,D,C,E,N,G,S,Q,V,Y,A,P,L,H,I, |
| 24 | 18 | BNT,VWS, | F,K,R,M,D,C,E,N,G,S,Q,V,Y,A,P,L,H,I, |
| 24 | 18 | BNC,VWK, | F,K,R,M,D,C,E,N,G,S,Q,Y,V,A,P,H,L,I, |
| 24 | 18 | BNC,VWS, | F,K,R,M,D,C,E,N,G,S,Q,Y,V,A,P,H,L,I, |
| 24 | 18 | VAK,DBK, | F,K,R,T,M,C,D,E,N,G,Q,S,V,A,TRP,L,H,I, |
| 24 | 18 | DBS,VAK, | F,K,R,T,M,C,D,E,N,G,Q,S,V,A,TRP,L,H,I, |
| 24 | 18 | VAM,DBK, | F,K,R,T,M,C,D,E,N,G,Q,S,V,A,TRP,L,H,I, |
| 24 | 18 | VAM,DBS, | F,K,R,T,M,C,D,E,N,G,Q,S,V,A,TRP,L,H,I, |
| 24 | 18 | DBK,VAS, | F,K,R,T,M,C,D,E,N,G,Q,S,V,A,TRP,L,H,I, |
| 24 | 18 | DBS,VAS, | F,K,R,T,M,C,D,E,N,G,Q,S,V,A,TRP,L,H,I, |
| 24 | 18 | DBK,VAW, | F,K,R,T,M,C,D,E,N,G,Q,S,V,A,TRP,L,H,I, |
| 24 | 18 | DBS,VAW, | F,K,R,T,M,C,D,E,N,G,Q,S,V,A,TRP,L,H,I, |
| 24 | 18 | NDT,VMK, | F,K,R,T,C,D,E,N,G,Q,S,Y,V,A,P,L,H,I, |

(continued)

| Codons | AA | Degenerate Codons | Amino Acids |
|--------|----|-------------------|-------------|
| 24 | 18 | NDC,VMK, | F,K,R,T,C,D,E,N,G,Q,S,V,Y,A,P,L,H,I, |
| 24 | 18 | NDT,VMM, | F,K,R,T,C,D,E,N,G,Q,S,Y,V,A,P,L,H,I, |
| 24 | 18 | NDC,VMM, | F,K,R,T,C,D,E,N,G,Q,S,V,Y,A,P,L,H,I, |
| 24 | 18 | VMR,NDT, | F,K,T,R,C,D,E,N,G,Q,S,Y,V,A,P,L,H,I, |
| 24 | 18 | NDC,VMR, | F,K,T,R,C,D,E,N,G,Q,S,V,Y,A,P,H,L,I, |
| 24 | 18 | NDT,VMS, | F,K,R,T,C,D,E,N,G,Q,S,Y,V,A,P,L,H,I, |
| 24 | 18 | NDC,VMS, | F,K,R,T,C,D,E,N,G,Q,S,V,Y,A,P,L,H,I, |
| 24 | 18 | NDT,VMW, | F,K,R,T,C,D,E,N,G,Q,S,Y,V,A,P,L,H,I, |
| 24 | 18 | NDC,VMW, | F,K,R,T,C,D,E,N,G,Q,S,V,Y,A,P,L,H,I, |
| 24 | 18 | NBT,VWK, | F,K,T,R,M,C,D,E,N,G,Q,S,V,A,P,L,H,I, |
| 24 | 18 | NBC,VWK, | F,K,R,T,M,C,D,E,N,G,Q,S,V,A,P,L,H,I, |
| 24 | 18 | NVT,VWK, | K,T,R,M,C,D,E,N,G,Q,S,V,Y,A,P,L,H,I, |
| 24 | 18 | NVC,VWK, | K,R,T,M,C,D,E,N,G,Q,S,V,Y,A,P,L,H,I, |
| 24 | 18 | VWR,NVT, | K,R,T,M,D,C,E,N,G,Q,S,V,Y,A,P,H,L,I, |
| 24 | 18 | NVC,VWR, | K,T,R,M,D,C,E,N,G,Q,S,V,Y,A,P,H,L,I, |
| 24 | 18 | VWS,NBT, | F,K,T,R,M,C,D,E,N,G,Q,S,V,A,P,L,H,I, |
| 24 | 18 | NBC,VWS, | F,K,R,T,M,C,D,E,N,G,Q,S,V,A,P,L,H,I, |
| 24 | 18 | VWS,NVT, | K,T,R,M,C,D,E,N,G,Q,S,V,Y,A,P,L,H,I, |
| 24 | 18 | NVC,VWS, | K,R,T,M,C,D,E,N,G,Q,S,V,Y,A,P,L,H,I, |
| 24 | 18 | VHK,DGK, | K,R,T,M,C,D,E,N,G,S,Q,V,A,TRP,P,L,H,I, |
| 24 | 18 | DGS,VHK, | K,R,T,M,C,D,E,N,G,S,Q,V,A,TRP,P,L,H,I, |
| 24 | 18 | DGK,VHS, | K,R,T,M,C,D,E,N,G,S,Q,V,A,TRP,P,L,H,I, |
| 24 | 18 | DGS,VHS, | K,R,T,M,C,D,E,N,G,S,Q,V,A,TRP,P,L,H,I, |
| 24 | 18 | NDT,VNA, | F,K,R,T,D,C,E,N,G,Q,S,Y,V,A,P,H,L,I, |
| 24 | 18 | NDC,VNA, | F,K,R,T,D,C,E,N,G,Q,S,Y,V,A,P,H,L,I, |
| 24 | 18 | VNG,HNT, | F,K,R,T,M,C,E,N,G,Q,S,Y,V,A,P,H,L,I, |
| 24 | 18 | VNG,HNC, | F,K,R,T,M,C,E,N,G,Q,S,Y,V,A,P,H,L,I, |
| 24 | 18 | DNT,VNG, | F,K,R,T,M,D,C,E,N,G,Q,S,Y,V,A,P,L,I, |
| 24 | 18 | DNC,VNG, | F,K,R,T,M,D,C,E,N,G,Q,S,Y,V,A,P,L,I, |
| 24 | 18 | VNG,NHT, | F,K,R,T,M,D,E,N,G,Q,S,Y,V,A,P,H,L,I, |
| 24 | 18 | VNG,NHC, | F,K,R,T,M,D,E,N,G,Q,S,Y,V,A,P,H,L,I, |
| 25 | 18 | NNT,VAB, | F,K,T,R,D,C,E,N,G,Q,S,V,Y,A,P,L,H,I, |
| 25 | 18 | NNC,VAB, | F,K,R,T,D,C,E,N,G,Q,S,V,Y,A,P,L,H,I, |
| 25 | 18 | NNT,VAV, | F,K,T,R,D,C,E,N,G,Q,S,V,Y,A,P,L,H,I, |
| 25 | 18 | NNC,VAV, | F,K,R,T,D,C,E,N,G,Q,S,V,Y,A,P,L,H,I, |
| 25 | 18 | VAH,NNT, | F,K,T,R,D,C,E,N,G,Q,S,V,Y,A,P,L,H,I, |
| 25 | 18 | VAH,NNC, | F,K,R,T,D,C,E,N,G,Q,S,V,Y,A,P,L,H,I, |
| 25 | 18 | NNT,VAD, | F,K,T,R,D,C,E,N,G,Q,S,V,Y,A,P,L,H,I, |

(continued)

| Codons | AA | Degenerate Codons | Amino Acids |
|---|---|---|---|
| 25 | 18 | NNC,V AD, | F,K,R,T,D,C,E,N,G,Q,S,Y,V,A,P,L,H,I, |
| 25 | 18 | NNT,VVA, | F,K,R,T,D,C,E,N,G,Q,S,V,Y,A,P,H,L,I, |
| 25 | 18 | NNC,VVA, | F,K,R,T,D,C,E,N,G,Q,S,Y,V,A,P,H,L,I, |
| 25 | 18 | VVG,NNT, | F,K,R,T,D,C,E,N,G,Q,S,V,Y,A,P,H,L,I, |
| 25 | 18 | VVG,NNC, | F,K,R,T,D,C,E,N,G,Q,S,Y,V,A,P,H,L,I, |
| 25 | 18 | VHA,NNT, | F,K,T,R,D,C,E,N,G,S,Q,Y,V,A,P,H,L,I, |
| 25 | 18 | VHA,NNC, | F,K,T,R,D,C,E,N,G,S,Q,Y,V,A,P,H,L,I, |
| 25 | 18 | NNT,VDA, | F,K,R,T,D,C,E,N,G,S,Q,Y,V,A,P,H,L,I, |
| 25 | 18 | NNC,VDA, | F,K,R,T,D,C,E,N,G,S,Q,Y,V,A,P,H,L,I, |

The following table provides example preferred codon solutions that use three degenerate codons. In the interest of brevity, all possible solutions that use 25 or fewer codons to encode at least, e.g., 18 amino acids are not tabulated; however, they can be determined by running the computer program described herein, set with the appropriate parameters.

Table: Example 3 Degenerate Codon Solutions

| Codons | AA | Degenerate Codons | Amino Acids |
|---|---|---|---|
| 22 | 20 | NDT,TGG,VHG, | F,K,R,T,M,D,C,E,N,G,Q,S,V,Y,A,W,P,H,L,I, |
| 22 | 20 | NDC,TGG,VHG, | F,K,R,T,M,D,C,E,N,G,Q,S,V,Y,A,W,P,H,L,I, |
| 22 | 20 | VMA,NDT,WKG, | F,K,T,R,M,D,C,E,N,G,Q,S,V,Y,A,W,P,H,L,I, |
| 22 | 20 | NDC,VMA,WKG, | F,K,T,R,M,D,C,E,N,G,Q,S,Y,V,A,W,P,H,L,I, |
| 22 | 20 | NDT,WKG,VMG, | F,K,T,R,M,D,C,E,N,G,Q,S,V,Y,A,W,P,H,L,I, |
| 22 | 20 | NDC,WKG,VMG, | F,K,T,R,M,D,C,E,N,G,Q,S,Y,V,A,W,P,H,L,I, |
| 22 | 20 | VMA,NAT,DKK, | F,K,T,R,M,D,C,E,N,G,Q,S,Y,V,A,W,P,H,L,I, |
| 22 | 20 | VMA,NAC,DKK, | F,K,T,R,M,D,C,E,N,G,Q,S,Y,V,A,W,P,H,L,I, |
| 22 | 20 | VMA,DKS,NAT, | F,K,T,R,M,D,C,E,N,G,Q,S,Y,V,A,W,P,H,L,I, |
| 22 | 20 | VMA,NAC,DKS, | F,K,T,R,M,D,C,E,N,G,Q,S,Y,V,A,W,P,H,L,I, |
| 22 | 20 | NAT,VMG,DKK, | F,K,T,R,M,D,C,E,N,G,Q,S,Y,V,A,W,P,H,L,I, |
| 22 | 20 | NAC,VMG,DKK, | F,K,T,R,M,D,C,E,N,G,Q,S,Y,V,A,W,P,H,L,I, |
| 22 | 20 | DKS,NAT,VMG, | F,K,T,R,M,D,C,E,N,G,Q,S,Y,V,A,W,P,H,L,I, |
| 22 | 20 | NAC,DKS,VMG, | F,K,T,R,M,D,C,E,N,G,Q,S,Y,V,A,W,P,H,L,I, |
| 23 | 20 | VWG,NNT,TGG, | F,K,T,R,M,D,C,E,N,G,S,Q,Y,V,A,W,P,H,L,I, |
| 23 | 20 | VWG,NNC,TGG, | F,K,R,T,M,D,C,E,N,G,S,Q,Y,V,A,W,P,H,L,I, |
| 23 | 20 | NDT,TGK,VHG, | F,K,R,T,M,D,C,E,N,G,Q,S,Y,V,A,W,P,H,L,I, |
| 23 | 20 | NDC,TGK,VHG, | F,K,R,T,M,D,C,E,N,G,Q,S,Y,V,A,W,P,H,L,I, |
| 23 | 20 | TGK,NHT,VDG, | F,K,T,R,M,D,C,E,N,G,Q,S,V,Y,A,W,P,H,L,I, |
| 23 | 20 | TGK,NHC,VDG, | F,K,T,R,M,D,C,E,N,G,Q,S,V,Y,A,W,P,H,L,I, |
| 23 | 20 | NDT,TGS,VHG, | F,K,R,T,M,D,C,E,N,G,Q,S,Y,V,A,W,P,H,L,I, |
| 23 | 20 | NDC,TGS,VHG, | F,K,R,T,M,D,C,E,N,G,Q,S,Y,V,A,W,P,H,L,I, |
| 23 | 20 | TGS,NHT,VDG, | F,K,T,R,M,D,C,E,N,G,Q,S,V,Y,A,W,P,H,L,I, |

(continued)

| Codons | AA | Degenerate Codons | Amino Acids |
|--------|-----|-------------------|-------------|
| 23 | 20 | TGS,NHC,VDG, | F,K,T,R,M,D,C,E,N,G,Q,S,V,Y,A,W,P,H,L,I, |
| 23 | 20 | NDT,TKG,VHG, | F,K,R,T,M,D,C,E,N,G,Q,S,V,Y,A,W,P,H,L,I, |
| 23 | 20 | NDC,TKG,VHG, | F,K,R,T,M,D,C,E,N,G,Q,S,V,Y,A,W,P,H,L,I, |
| 23 | 20 | NDT,VHG,TSG, | F,K,R,T,M,D,C,E,N,G,Q,S,V,Y,A,W,P,H,L,I, |
| 23 | 20 | NDC,VHG,TSG, | F,K,R,T,M,D,C,E,N,G,Q,S,V,Y,A,W,P,H,L,I, |
| 23 | 20 | TDT,NSG,VWK, | F,K,R,T,M,C,D,E,N,G,S,Q,Y,V,A,W,P,H,L,I, |
| 23 | 20 | VWS,TDT,NSG, | F,K,R,T,M,C,D,E,N,G,S,Q,Y,V,A,W,P,H,L,I, |
| 23 | 20 | TDC,NSG,VWK, | F,K,R,T,M,C,D,E,N,G,S,Q,Y,V,A,W,P,H,L,I, |
| 23 | 20 | VWS,TDC,NSG, | F,K,R,T,M,C,D,E,N,G,S,Q,Y,V,A,W,P,H,L,I, |
| 23 | 20 | NDT,KGG,VHG, | F,K,R,T,M,D,C,E,N,G,Q,S,Y,V,A,W,P,H,L,I, |
| 23 | 20 | NDC,KGG,VHG, | F,K,R,T,M,D,C,E,N,G,Q,S,Y,V,A,W,P,H,L,I, |
| 23 | 20 | NDT,VHG,YGG, | F,K,T,R,M,D,C,E,N,G,Q,S,Y,V,A,W,P,H,L,I, |
| 23 | 20 | NDC,VHG,YGG, | F,K,T,R,M,D,C,E,N,G,Q,S,Y,V,A,W,P,H,L,I, |
| 23 | 20 | NDT,VHG,WGG, | F,K,T,R,M,D,C,E,N,G,Q,S,Y,V,A,W,P,H,L,I, |
| 23 | 20 | NDC,VHG,WGG, | F,K,T,R,M,D,C,E,N,G,Q,S,Y,V,A,W,P,H,L,I, |
| 23 | 20 | NNT,WKG,VAA, | F,K,R,T,M,D,C,E,N,G,S,Q,V,Y,A,W,P,H,L,I, |
| 23 | 20 | NNC,WKG,VAA, | F,K,R,T,M,D,C,E,N,G,S,Q,Y,V,A,W,P,H,L,I, |
| 23 | 20 | NNT,V AG,WKG, | F,K,R,T,M,D,C,E,N,G,S,Q,V,Y,A,W,P,H,L,I, |
| 23 | 20 | VAG,NNC,WKG, | F,K,R,T,M,D,C,E,N,G,S,Q,Y,V,A,W,P,H,L,I, |
| 23 | 20 | VAA,NMT,DKK, | F,K,T,R,M,D,C,E,N,G,Q,S,Y,V,A,W,P,H,L,I, |
| 23 | 20 | VAA,NMC,DKK, | F,K,T,R,M,D,C,E,N,G,Q,S,Y,V,A,W,P,H,L,I, |
| 23 | 20 | DKS,VAA,NMT, | F,K,T,R,M,D,C,E,N,G,Q,S,Y,V,A,W,P,H,L,I, |
| 23 | 20 | DKS,VAA,NMC, | F,K,T,R,M,D,C,E,N,G,Q,S,Y,V,A,W,P,H,L,I, |
| 23 | 20 | VAG,NMT,DKK, | F,K,T,R,M,D,C,E,N,G,Q,S,Y,V,A,W,P,H,L,I, |
| 23 | 20 | VAG,NMC,DKK, | F,K,T,R,M,D,C,E,N,G,Q,S,Y,V,A,W,P,H,L,I, |
| 23 | 20 | VAG,DKS,NMT, | F,K,T,R,M,D,C,E,N,G,Q,S,Y,V,A,W,P,H,L,I, |
| 23 | 20 | VAG,DKS,NMC, | F,K,T,R,M,D,C,E,N,G,Q,S,Y,V,A,W,P,H,L,I, |
| 23 | 20 | VHG,DGK,NWT, | F,K,T,R,M,D,C,E,N,G,Q,S,Y,V,A,W,P,H,L,I, |
| 23 | 20 | VHG,DGK,NWC, | F,K,T,R,M,D,C,E,N,G,Q,S,Y,V,A,W,P,H,L,I, |
| 23 | 20 | DGS,VHG,NWT, | F,K,T,R,M,D,C,E,N,G,Q,S,Y,V,A,W,P,H,L,I, |
| 23 | 20 | DGS,VHG,NWC, | F,K,T,R,M,D,C,E,N,G,Q,S,Y,V,A,W,P,H,L,I, |
| 24 | 20 | VWG,NNT,TGK, | F,K,T,R,M,C,D,E,N,G,S,Q,Y,V,A,W,P,H,L,I, |
| 24 | 20 | VWG,NNC,TGK, | F,K,R,T,M,C,D,E,N,G,S,Q,Y,V,A,W,P,H,L,I, |
| 24 | 20 | VWG,NNT,TGS, | F,K,T,R,M,C,D,E,N,G,S,Q,Y,V,A,W,P,H,L,I, |
| 24 | 20 | VWG,NNC,TGS, | F,K,R,T,M,C,D,E,N,G,S,Q,Y,V,A,W,P,H,L,I, |
| 24 | 20 | TGB,NDT,VHG, | F,K,R,T,M,D,C,E,N,G,Q,S,Y,V,A,W,P,H,L,I, |
| 24 | 20 | NDC,TGB,VHG, | F,K,R,T,M,D,C,E,N,G,Q,S,Y,V,A,W,P,H,L,I, |
| 24 | 20 | TGB,NHT,VDG, | F,K,T,R,M,D,C,E,N,G,Q,S,V,Y,A,W,P,H,L,I, |

(continued)

| Codons | AA | Degenerate Codons | Amino Acids |
|--------|-----|-------------------|-------------|
| 24 | 20 | TGB,NHC,VDG, | F,K,T,R,M,D,C,E,N,G,Q,S,V,Y,A,W,P,H,L,I, |
| 24 | 20 | VWG,NNT,TKG, | F,K,T,R,M,D,C,E,N,G,S,Q,Y,V,A,W,P,H,L,I, |
| 24 | 20 | VWG,NNC,TKG, | F,K,R,T,M,D,C,E,N,G,S,Q,Y,V,A,W,P,H,L,I, |
| 24 | 20 | VWG,NNT,TSG, | F,K,T,R,M,D,C,E,N,G,Q,S,Y,V,A,W,P,H,L,I, |
| 24 | 20 | VWG,NNC,TSG, | F,K,R,T,M,D,C,E,N,G,Q,S,Y,V,A,W,P,H,L,I, |
| 24 | 20 | TSK,VNG,NWT, | F,K,T,R,M,D,C,E,N,G,S,Q,Y,V,A,W,P,H,L,I, |
| 24 | 20 | TSK,VNG,NWC, | F,K,T,R,M,D,C,E,N,G,S,Q,Y,V,A,W,P,H,L,I, |
| 24 | 20 | TSS,VNG,NWT, | F,K,T,R,M,D,C,E,N,G,S,Q,Y,V,A,W,P,H,L,I, |
| 24 | 20 | TSS,VNG,NWC, | F,K,T,R,M,D,C,E,N,G,S,Q,Y,V,A,W,P,H,L,I, |
| 24 | 20 | NDT,TBG,VHG, | F,K,R,T,M,D,C,E,N,G,Q,S,V,Y,A,W,P,H,L,I, |
| 24 | 20 | NDC,TBG,VHG, | F,K,R,T,M,D,C,E,N,G,Q,S,V,Y,A,W,P,H,L,I, |
| 24 | 20 | TNT,NSG,VWK, | F,K,R,T,M,C,D,E,N,G,S,Q,Y,V,A,W,P,H,L,I, |
| 24 | 20 | TNT,VWS,NSG, | F,K,R,T,M,C,D,E,N,G,S,Q,Y,V,A,W,P,H,L,I, |
| 24 | 20 | TNC,NSG,VWK, | F,K,R,T,M,C,D,E,N,G,S,Q,Y,V,A,W,P,H,L,I, |
| 24 | 20 | TNC,VWS,NSG, | F,K,R,T,M,C,D,E,N,G,S,Q,Y,V,A,W,P,H,L,I, |
| 24 | 20 | VWG,NNT,KGG, | F,K,T,R,M,D,C,E,N,G,S,Q,Y,V,A,W,P,H,L,I, |
| 24 | 20 | VWG,NNC,KGG, | F,K,R,T,M,D,C,E,N,G,S,Q,Y,V,A,W,P,H,L,I, |
| 24 | 20 | VWG,NNT,YGG, | F,K,R,T,M,D,C,E,N,G,S,Q,Y,V,A,W,P,H,L,I, |
| 24 | 20 | VWG,NNC,YGG, | F,K,R,T,M,D,C,E,N,G,S,Q,Y,V,A,W,P,H,L,I, |
| 24 | 20 | VWG,NNT,WGG, | F,K,R,T,M,D,C,E,N,G,S,Q,Y,V,A,W,P,H,L,I, |
| 24 | 20 | VWG,NNC,WGG, | F,K,R,T,M,D,C,E,N,G,S,Q,Y,V,A,W,P,H,L,I, |
| 24 | 20 | WGK,VNG,NWT, | F,K,R,T,M,D,C,E,N,G,S,Q,Y,V,A,W,P,H,L,I, |
| 24 | 20 | WGK,VNG,NWC, | F,K,R,T,M,D,C,E,N,G,S,Q,Y,V,A,W,P,H,L,I, |
| 24 | 20 | WGS,VNG,NWT, | F,K,R,T,M,D,C,E,N,G,S,Q,Y,V,A,W,P,H,L,I, |
| 24 | 20 | WGS,VNG,NWC, | F,K,R,T,M,D,C,E,N,G,S,Q,Y,V,A,W,P,H,L,I, |
| 24 | 20 | WKK,NAT,VNA, | F,K,R,T,M,D,C,E,N,G,S,Q,Y,V,A,W,P,H,L,I, |
| 24 | 20 | WKK,NAC,VNA, | F,K,R,T,M,D,C,E,N,G,S,Q,Y,V,A,W,P,H,L,I, |
| 24 | 20 | WKK,NAT,VNG, | F,K,R,T,M,D,C,E,N,G,S,Q,Y,V,A,W,P,H,L,I, |
| 24 | 20 | WKK,NAC,VNG, | F,K,R,T,M,D,C,E,N,G,S,Q,Y,V,A,W,P,H,L,I, |
| 24 | 20 | NAT,WKS,VNA, | F,K,R,T,M,D,C,E,N,G,S,Q,Y,V,A,W,P,H,L,I, |
| 24 | 20 | NAC,WKS,VNA, | F,K,R,T,M,D,C,E,N,G,S,Q,Y,V,A,W,P,H,L,I, |
| 24 | 20 | NAT,WKS,VNG, | F,K,R,T,M,D,C,E,N,G,S,Q,Y,V,A,W,P,H,L,I, |
| 24 | 20 | NAC,WKS,VNG, | F,K,R,T,M,D,C,E,N,G,S,Q,Y,V,A,W,P,H,L,I, |
| 24 | 20 | VMA,NDT,WBG, | F,K,T,R,M,D,C,E,N,G,Q,S,V,Y,A,W,P,H,L,I, |
| 24 | 20 | NDC,VMA,WBG, | F,K,T,R,M,D,C,E,N,G,Q,S,Y,V,A,W,P,H,L,I, |
| 24 | 20 | NDT,WBG,VMG, | F,K,T,R,M,D,C,E,N,G,Q,S,V,Y,A,W,P,H,L,I, |
| 24 | 20 | NDC,WBG,VMG, | F,K,T,R,M,D,C,E,N,G,Q,S,Y,V,A,W,P,H,L,I, |
| 24 | 20 | VAK,NBG,WDT, | F,K,R,T,M,D,C,E,N,G,Q,S,Y,V,A,W,P,L,H,I, |

(continued)

| Codons | AA | Degenerate Codons | Amino Acids |
|--------|-----|-------------------|-------------|
| 24 | 20 | VAM,NBG,WDT, | F,K,R,T,M,D,C,E,N,G,Q,S,Y,V,A,W,P,L,H,I, |
| 24 | 20 | NBG,VAS,WDT, | F,K,R,T,M,D,C,E,N,G,Q,S,Y,V,A,W,P,L,H,I, |
| 24 | 20 | NBG,WDT,VAW, | F,K,R,T,M,D,C,E,N,G,Q,S,Y,V,A,W,P,L,H,I, |
| 24 | 20 | DKG,WDT,VMK, | F,K,R,T,M,C,D,E,N,G,S,Q,V,Y,A,W,P,L,H,I, |
| 24 | 20 | DKG,VMM,WDT, | F,K,R,T,M,C,D,E,N,G,S,Q,V,Y,A,W,P,L,H,I, |
| 24 | 20 | VMS,DKG,WDT, | F,K,R,T,M,C,D,E,N,G,S,Q,V,Y,A,W,P,L,H,I, |
| 24 | 20 | VMW,DKG,WDT, | F,K,R,T,M,C,D,E,N,G,S,Q,V,Y,A,W,P,L,H,I, |
| 24 | 20 | VAK,NBG,WDC, | F,K,R,T,M,D,C,E,N,G,Q,S,Y,V,A,W,P,L,H,I, |
| 24 | 20 | VAM,NBG,WDC, | F,K,R,T,M,D,C,E,N,G,Q,S,Y,V,A,W,P,L,H,I, |
| 24 | 20 | NBG,VAS,WDC, | F,K,R,T,M,D,C,E,N,G,Q,S,Y,V,A,W,P,L,H,I, |
| 24 | 20 | NBG,WDC,VAW, | F,K,R,T,M,D,C,E,N,G,Q,S,Y,V,A,W,P,L,H,I, |
| 24 | 20 | DKG,WDC,VMK, | F,K,R,T,M,C,D,E,N,G,S,Q,V,Y,A,W,P,L,H,I, |
| 24 | 20 | DKG,WDC,VMM, | F,K,R,T,M,C,D,E,N,G,S,Q,V,Y,A,W,P,L,H,I, |
| 24 | 20 | VMS,DKG,WDC, | F,K,R,T,M,C,D,E,N,G,S,Q,V,Y,A,W,P,L,H,I, |
| 24 | 20 | VMW,DKG,WDC, | F,K,R,T,M,C,D,E,N,G,S,Q,V,Y,A,W,P,L,H,I, |
| 24 | 20 | NDT,BGG,VHG, | F,K,T,R,M,D,C,E,N,G,Q,S,Y,V,A,W,P,H,L,I, |
| 24 | 20 | NDC,BGG,VHG, | F,K,T,R,M,D,C,E,N,G,S,Q,Y,V,A,W,P,H,L,I, |
| 24 | 20 | VWG,BGK,NHT, | F,K,T,R,M,D,C,E,N,G,Q,S,V,Y,A,W,P,H,L,I, |
| 24 | 20 | VWG,BGK,NHC, | F,K,T,R,M,D,C,E,N,G,Q,S,V,Y,A,W,P,H,L,I, |
| 24 | 20 | VWG,BGS,NHT, | F,K,T,R,M,D,C,E,N,G,Q,S,V,Y,A,W,P,H,L,I, |
| 24 | 20 | VWG,BGS,NHC, | F,K,T,R,M,D,C,E,N,G,Q,S,V,Y,A,W,P,H,L,I, |
| 24 | 20 | VMA,HKG,NDT, | F,K,R,T,M,D,C,E,N,G,Q,S,V,Y,A,W,P,H,L,I, |
| 24 | 20 | NDC,VMA,HKG, | F,K,R,T,M,D,C,E,N,G,Q,S,Y,V,A,W,P,H,L,I, |
| 24 | 20 | VMA,NDT,DKG, | F,K,R,T,M,D,C,E,N,G,Q,S,Y,V,A,W,P,H,L,I, |
| 24 | 20 | NDC,VMA,DKG, | F,K,R,T,M,D,C,E,N,G,S,Q,Y,V,A,W,P,H,L,I, |
| 24 | 20 | HKG,NDT,VMG, | F,K,R,T,M,D,C,E,N,G,Q,S,Y,V,A,W,P,H,L,I, |
| 24 | 20 | NDC,HKG,VMG, | F,K,R,T,M,D,C,E,N,G,Q,S,Y,V,A,W,P,H,L,I, |
| 24 | 20 | NDT,VMG,DKG, | F,K,R,T,M,D,C,E,N,G,Q,S,Y,V,A,W,P,H,L,I, |
| 24 | 20 | NDC,VMG,DKG, | F,K,R,T,M,D,C,E,N,G,S,Q,Y,V,A,W,P,H,L,I, |
| 24 | 20 | VWG,DGK,NHT, | F,K,T,R,M,D,C,E,N,G,S,Q,V,Y,A,W,P,H,L,I, |
| 24 | 20 | VWG,DGK,NHC, | F,K,T,R,M,D,C,E,N,G,S,Q,V,Y,A,W,P,H,L,I, |
| 24 | 20 | VWG,DGS,NHT, | F,K,T,R,M,D,C,E,N,G,S,Q,V,Y,A,W,P,H,L,I, |
| 24 | 20 | VWG,DGS,NHC, | F,K,T,R,M,D,C,E,N,G,S,Q,V,Y,A,W,P,H,L,I, |
| 24 | 20 | NDT,HGG,VHG, | F,K,R,T,M,D,C,E,N,G,Q,S,Y,V,A,W,P,H,L,I, |
| 24 | 20 | NDC,HGG,VHG, | F,K,R,T,M,D,C,E,N,G,Q,S,Y,V,A,W,P,H,L,I, |
| 24 | 20 | NDT,DGG,VHG, | F,K,R,T,M,D,C,E,N,G,Q,S,Y,V,A,W,P,H,L,I, |
| 24 | 20 | NDC,DGG,VHG, | F,K,R,T,M,D,C,E,N,G,S,Q,Y,V,A,W,P,H,L,I, |
| 25 | 20 | TAT,DKK,VMK, | F,K,R,T,M,C,D,E,N,G,Q,S,Y,V,A,W,P,L,H,I, |

(continued)

| Codons | AA | Degenerate Codons | Amino Acids |
|--------|-----|-------------------|-------------|
| 25 | 20 | DKS,TAT,VMK, | F,K,R,T,M,C,D,E,N,G,Q,S,Y,V,A,W,P,L,H,I, |
| 25 | 20 | VMM,TAT,DKK, | F,K,R,T,M,C,D,E,N,G,Q,S,Y,V,A,W,P,L,H,I, |
| 25 | 20 | DKS,VMM,TAT, | F,K,R,T,M,C,D,E,N,G,Q,S,Y,V,A,W,P,L,H,I, |
| 25 | 20 | VMS,TAT,DKK, | F,K,R,T,M,C,D,E,N,G,Q,S,Y,V,A,W,P,L,H,I, |
| 25 | 20 | DKS,VMS,TAT, | F,K,R,T,M,C,D,E,N,G,Q,S,Y,V,A,W,P,L,H,I, |
| 25 | 20 | VMW,TAT,DKK, | F,K,R,T,M,C,D,E,N,G,Q,S,Y,V,A,W,P,L,H,I, |
| 25 | 20 | VMW,DKS,TAT, | F,K,R,T,M,C,D,E,N,G,Q,S,Y,V,A,W,P,L,H,I, |
| 25 | 20 | TAC,DKK,VMK, | F,K,R,T,M,C,D,E,N,G,Q,S,Y,V,A,W,P,L,H,I, |
| 25 | 20 | TAC,DKS,VMK, | F,K,R,T,M,C,D,E,N,G,Q,S,Y,V,A,W,P,L,H,I, |
| 25 | 20 | TAC,VMM,DKK, | F,K,R,T,M,C,D,E,N,G,Q,S,Y,V,A,W,P,L,H,I, |
| 25 | 20 | TAC,DKS,VMM, | F,K,R,T,M,C,D,E,N,G,Q,S,Y,V,A,W,P,L,H,I, |
| 25 | 20 | TAC,VMS,DKK, | F,K,R,T,M,C,D,E,N,G,Q,S,Y,V,A,W,P,L,H,I, |
| 25 | 20 | TAC,DKS,VMS, | F,K,R,T,M,C,D,E,N,G,Q,S,Y,V,A,W,P,L,H,I, |
| 25 | 20 | TAC,VMW,DKK, | F,K,R,T,M,C,D,E,N,G,Q,S,Y,V,A,W,P,L,H,I, |
| 25 | 20 | TAC,VMW,DKS, | F,K,R,T,M,C,D,E,N,G,Q,S,Y,V,A,W,P,L,H,I, |
| 25 | 20 | NDT,TGG,VNG, | F,K,R,T,M,D,C,E,N,G,Q,S,Y,V,A,W,P,H,L,I, |
| 25 | 20 | NDC,TGG,VNG, | F,K,R,T,M,D,C,E,N,G,S,Q,Y,V,A,W,P,H,L,I, |
| 25 | 20 | TGB,VWG,NNT, | F,K,T,R,M,C,D,E,N,G,S,Q,Y,V,A,W,P,H,L,I, |
| 25 | 20 | TGB,VWG,NNC, | F,K,R,T,M,C,D,E,N,G,S,Q,Y,V,A,W,P,H,L,I, |
| 25 | 20 | NDT,TKK,VHG, | F,K,R,T,M,D,C,E,N,G,Q,S,Y,V,A,W,P,H,L,I, |
| 25 | 20 | NDC,TKK,VHG, | F,K,R,T,M,D,C,E,N,G,Q,S,Y,V,A,W,P,H,L,I, |
| 25 | 20 | TKK,NHT,VDG, | F,K,T,R,M,D,C,E,N,G,Q,S,V,Y,A,W,P,H,L,I, |
| 25 | 20 | TKK,NHC,VDG, | F,K,T,R,M,D,C,E,N,G,Q,S,V,Y,A,W,P,H,L,I, |
| 25 | 20 | NDT,VHG,TKS, | F,K,R,T,M,D,C,E,N,G,Q,S,Y,V,A,W,P,H,L,I, |
| 25 | 20 | NDC,VHG,TKS, | F,K,R,T,M,D,C,E,N,G,Q,S,Y,V,A,W,P,H,L,I, |
| 25 | 20 | TKS,NHT,VDG, | F,K,T,R,M,D,C,E,N,G,Q,S,V,Y,A,W,P,H,L,I, |
| 25 | 20 | TKS,NHC,VDG, | F,K,T,R,M,D,C,E,N,G,Q,S,V,Y,A,W,P,H,L,I, |
| 25 | 20 | NDT,TSK,VHG, | F,K,R,T,M,D,C,E,N,G,Q,S,Y,V,A,W,P,H,L,I, |
| 25 | 20 | NDC,TSK,VHG, | F,K,R,T,M,D,C,E,N,G,Q,S,Y,V,A,W,P,H,L,I, |
| 25 | 20 | TSK,NHT,VDG, | F,K,T,R,M,D,C,E,N,G,Q,S,V,Y,A,W,P,H,L,I, |
| 25 | 20 | TSK,NHC,VDG, | F,K,T,R,M,D,C,E,N,G,Q,S,V,Y,A,W,P,H,L,I, |
| 25 | 20 | TSS,NDT,VHG, | F,K,R,T,M,D,C,E,N,G,Q,S,Y,V,A,W,P,H,L,I, |
| 25 | 20 | NDC,TSS,VHG, | F,K,R,T,M,D,C,E,N,G,Q,S,Y,V,A,W,P,H,L,I, |
| 25 | 20 | TSS,NHT,VDG, | F,K,T,R,M,D,C,E,N,G,Q,S,V,Y,A,W,P,H,L,I, |
| 25 | 20 | TSS,NHC,VDG, | F,K,T,R,M,D,C,E,N,G,Q,S,V,Y,A,W,P,H,L,I, |
| 25 | 20 | TBG,VWG,NNT, | F,K,T,R,M,D,C,E,N,G,Q,S,Y,V,A,W,P,H,L,I, |
| 25 | 20 | TBG,VWG,NNC, | F,K,R,T,M,D,C,E,N,G,Q,S,Y,V,A,W,P,H,L,I, |
| 25 | 20 | TNT,VHK,BGG, | F,K,R,T,M,C,D,E,N,G,Q,S,V,Y,A,W,P,H,L,I, |

(continued)

| Codons | AA | Degenerate Codons | Amino Acids |
|--------|-----|-------------------|-------------|
| 25 | 20 | TNT,BGG,VHS, | F,K,R,T,M,C,D,E,N,G,Q,S,V,Y,A,W,P,H,L,I, |
| 25 | 20 | TNT,VHK,DGG, | F,K,R,T,M,C,D,E,N,G,S,Q,Y,V,A,W,P,L,H,I, |
| 25 | 20 | TNT,DGG,VHS, | F,K,R,T,M,C,D,E,N,G,S,Q,Y,V,A,W,P,L,H,I, |
| 25 | 20 | VHK,TNC,BGG, | F,K,R,T,M,C,D,E,N,G,Q,S,V,Y,A,W,P,H,L,I, |
| 25 | 20 | TNC,BGG,VHS, | F,K,R,T,M,C,D,E,N,G,Q,S,V,Y,A,W,P,H,L,I, |
| 25 | 20 | TNC,VHK,DGG, | F,K,R,T,M,C,D,E,N,G,S,Q,Y,V,A,W,P,L,H,I, |
| 25 | 20 | TNC,DGG,VHS, | F,K,R,T,M,C,D,E,N,G,S,Q,Y,V,A,W,P,L,H,I, |
| 25 | 20 | NDT,VHG,KGK, | F,K,R,T,M,D,C,E,N,G,Q,S,Y,V,A,W,P,H,L,I, |
| 25 | 20 | NDC,VHG,KGK, | F,K,R,T,M,D,C,E,N,G,S,Q,Y,V,A,W,P,H,L,I, |
| 25 | 20 | KGK,NHT,VDG, | F,K,T,R,M,D,C,E,N,G,Q,S,V,Y,A,W,P,H,L,I, |
| 25 | 20 | NHC,KGK,VDG, | F,K,T,R,M,D,C,E,N,G,Q,S,V,Y,A,W,P,H,L,I, |
| 25 | 20 | NDT,KGS,VHG, | F,K,R,T,M,D,C,E,N,G,Q,S,Y,V,A,W,P,H,L,I, |
| 25 | 20 | NDC,KGS,VHG, | F,K,R,T,M,D,C,E,N,G,S,Q,Y,V,A,W,P,H,L,I, |
| 25 | 20 | KGS,NHT,VDG, | F,K,T,R,M,D,C,E,N,G,Q,S,V,Y,A,W,P,H,L,I, |
| 25 | 20 | KGS,NHC,VDG, | F,K,T,R,M,D,C,E,N,G,Q,S,V,Y,A,W,P,H,L,I, |
| 25 | 20 | NDT,VHG,KKG, | F,K,R,T,M,D,C,E,N,G,Q,S,Y,V,A,W,P,H,L,I, |
| 25 | 20 | NDC,VHG,KKG, | F,K,R,T,M,D,C,E,N,G,Q,S,Y,V,A,W,P,H,L,I, |
| 25 | 20 | NDT,KSG,VHG, | F,K,R,T,M,D,C,E,N,G,Q,S,Y,V,A,W,P,H,L,I, |
| 25 | 20 | NDC,KSG,VHG, | F,K,R,T,M,D,C,E,N,G,Q,S,Y,V,A,W,P,H,L,I, |
| 25 | 20 | NDT,VHG,YGK, | F,K,T,R,M,D,C,E,N,G,Q,S,Y,V,A,W,P,H,L,I, |
| 25 | 20 | NDC,VHG,YGK, | F,K,T,R,M,D,C,E,N,G,S,Q,Y,V,A,W,P,H,L,I, |
| 25 | 20 | YGK,NHT,VDG, | F,K,T,R,M,D,C,E,N,G,Q,S,V,Y,A,W,P,H,L,I, |
| 25 | 20 | YGK,NHC,VDG, | F,K,T,R,M,D,C,E,N,G,Q,S,V,Y,A,W,P,H,L,I, |
| 25 | 20 | NDT,VHG,YGS, | F,K,T,R,M,D,C,E,N,G,Q,S,Y,V,A,W,P,H,L,I, |
| 25 | 20 | NDC,VHG,YGS, | F,K,T,R,M,D,C,E,N,G,S,Q,Y,V,A,W,P,H,L,I, |
| 25 | 20 | NHT,YGS,VDG, | F,K,T,R,M,D,C,E,N,G,Q,S,V,Y,A,W,P,H,L,I, |
| 25 | 20 | NHC,YGS,VDG, | F,K,T,R,M,D,C,E,N,G,Q,S,V,Y,A,W,P,H,L,I, |
| 25 | 20 | NDT,YKG,VHG, | F,K,T,R,M,D,C,E,N,G,Q,S,Y,V,A,W,P,H,L,I, |
| 25 | 20 | NDC,YKG,VHG, | F,K,T,R,M,D,C,E,N,G,Q,S,Y,V,A,W,P,H,L,I, |
| 25 | 20 | NDT,VHG,YSG, | F,K,T,R,M,D,C,E,N,G,Q,S,Y,V,A,W,P,H,L,I, |
| 25 | 20 | NDC,VHG,YSG, | F,K,T,R,M,D,C,E,N,G,Q,S,Y,V,A,W,P,H,L,I, |
| 25 | 20 | NDT,WGK,VHG, | F,K,R,T,M,D,C,E,N,G,S,Q,Y,V,A,W,P,H,L,I, |
| 25 | 20 | NDC,WGK,VHG, | F,K,R,T,M,D,C,E,N,G,S,Q,Y,V,A,W,P,H,L,I, |
| 25 | 20 | WGK,NHT,VDG, | F,K,T,R,M,D,C,E,N,G,Q,S,V,Y,A,W,P,H,L,I, |
| 25 | 20 | WGK,NHC,VDG, | F,K,T,R,M,D,C,E,N,G,Q,S,V,Y,A,W,P,H,L,I, |
| 25 | 20 | WGS,NDT,VHG, | F,K,R,T,M,D,C,E,N,G,S,Q,Y,V,A,W,P,H,L,I, |
| 25 | 20 | NDC,WGS,VHG, | F,K,R,T,M,D,C,E,N,G,S,Q,Y,V,A,W,P,H,L,I, |
| 25 | 20 | WGS,NHT,VDG, | F,K,T,R,M,D,C,E,N,G,Q,S,V,Y,A,W,P,H,L,I, |

(continued)

| Codons | AA | Degenerate Codons | Amino Acids |
|---|---|---|---|
| 25 | 20 | WGS,NHC,VDG, | F,K,T,R,M,D,C,E,N,G,Q,S,V,Y,A,W,P,H,L,I, |
| 25 | 20 | WKG,DDT,VMK, | F,K,R,T,M,C,D,E,N,G,S,Q,V,Y,A,W,P,L,H,I, |
| 25 | 20 | DDC,WKG,VMK, | F,K,R,T,M,C,D,E,N,G,S,Q,Y,V,A,W,P,L,H,I, |
| 25 | 20 | WKG,DDT,VMM, | F,K,R,T,M,C,D,E,N,G,S,Q,V,Y,A,W,P,L,H,I, |
| 25 | 20 | DDC,WKG,VMM, | F,K,R,T,M,C,D,E,N,G,S,Q,Y,V,A,W,P,L,H,I, |
| 25 | 20 | WKG,VMS,DDT, | F,K,R,T,M,C,D,E,N,G,S,Q,V,Y,A,W,P,L,H,I, |
| 25 | 20 | DDC,WKG,VMS, | F,K,R,T,M,C,D,E,N,G,S,Q,Y,V,A,W,P,L,H,I, |
| 25 | 20 | VMW,WKG,DDT, | F,K,R,T,M,C,D,E,N,G,S,Q,V,Y,A,W,P,L,H,I, |
| 25 | 20 | DDC,VMW,WKG, | F,K,R,T,M,C,D,E,N,G,S,Q,Y,V,A,W,P,L,H,I, |
| 25 | 20 | NDT,WKG,VVA, | F,K,T,R,M,D,C,E,N,G,Q,S,V,Y,A,W,P,H,L,I, |
| 25 | 20 | NDC,WKG,VVA, | F,K,T,R,M,D,C,E,N,G,Q,S,Y,V,A,W,P,H,L,I, |
| 25 | 20 | VVG,NDT,WKG, | F,K,T,R,M,D,C,E,N,G,Q,S,V,Y,A,W,P,H,L,I, |
| 25 | 20 | NDC,VVG,WKG, | F,K,T,R,M,D,C,E,N,G,Q,S,Y,V,A,W,P,H,L,I, |
| 25 | 20 | VHA,NDT,WKG, | F,K,T,R,M,D,C,E,N,G,Q,S,Y,V,A,W,P,H,L,I, |
| 25 | 20 | NDC,VHA,WKG, | F,K,T,R,M,D,C,E,N,G,S,Q,Y,V,A,W,P,H,L,I, |
| 25 | 20 | NDT,WKG,VHG, | F,K,T,R,M,D,C,E,N,G,Q,S,Y,V,A,W,P,H,L,I, |
| 25 | 20 | NDC,WKG,VHG, | F,K,T,R,M,D,C,E,N,G,Q,S,Y,V,A,W,P,H,L,I, |
| 25 | 20 | WKK,VVA,NWT, | F,K,R,T,M,D,C,E,N,G,S,Q,V,Y,A,W,P,H,L,I, |
| 25 | 20 | WKK,VVA,NWC, | F,K,R,T,M,D,C,E,N,G,S,Q,Y,V,A,W,P,H,L,I, |
| 25 | 20 | VVG,WKK,NWT, | F,K,R,T,M,D,C,E,N,G,S,Q,V,Y,A,W,P,H,L,I, |
| 25 | 20 | VVG,WKK,NWC, | F,K,R,T,M,D,C,E,N,G,S,Q,Y,V,A,W,P,H,L,I, |
| 25 | 20 | VHA,WKK,NRT, | F,K,R,T,M,D,C,E,N,G,S,Q,Y,V,A,W,P,H,L,I, |
| 25 | 20 | VHA,WKK,NRC, | F,K,R,T,M,D,C,E,N,G,S,Q,Y,V,A,W,P,H,L,I, |
| 25 | 20 | WKK,VHG,NRT, | F,K,R,T,M,D,C,E,N,G,S,Q,Y,V,A,W,P,H,L,I, |
| 25 | 20 | WKK,VHG,NRC, | F,K,R,T,M,D,C,E,N,G,S,Q,Y,V,A,W,P,H,L,I, |
| 25 | 20 | WKK,VDA,NMT, | F,K,T,R,M,D,C,E,N,G,S,Q,Y,V,A,W,P,H,L,I, |
| 25 | 20 | WKK,VDA,NMC, | F,K,T,R,M,D,C,E,N,G,S,Q,Y,V,A,W,P,H,L,I, |
| 25 | 20 | WKK,NMT,VDG, | F,K,T,R,M,D,C,E,N,G,S,Q,Y,V,A,W,P,H,L,I, |
| 25 | 20 | WKK,NMC,VDG, | F,K,T,R,M,D,C,E,N,G,S,Q,Y,V,A,W,P,H,L,I, |
| 25 | 20 | VVA,WKS,NWT, | F,K,R,T,M,D,C,E,N,G,S,Q,V,Y,A,W,P,H,L,I, |
| 25 | 20 | VVA,WKS,NWC, | F,K,R,T,M,D,C,E,N,G,S,Q,Y,V,A,W,P,H,L,I, |
| 25 | 20 | VVG,WKS,NWT, | F,K,R,T,M,D,C,E,N,G,S,Q,V,Y,A,W,P,H,L,I, |
| 25 | 20 | VVG,WKS,NWC, | F,K,R,T,M,D,C,E,N,G,S,Q,Y,V,A,W,P,H,L,I, |
| 25 | 20 | VHA,WKS,NRT, | F,K,R,T,M,D,C,E,N,G,S,Q,Y,V,A,W,P,H,L,I, |
| 25 | 20 | VHA,WKS,NRC, | F,K,R,T,M,D,C,E,N,G,S,Q,Y,V,A,W,P,H,L,I, |
| 25 | 20 | WKS,VHG,NRT, | F,K,R,T,M,D,C,E,N,G,S,Q,Y,V,A,W,P,H,L,I, |
| 25 | 20 | WKS,VHG,NRC, | F,K,R,T,M,D,C,E,N,G,S,Q,Y,V,A,W,P,H,L,I, |
| 25 | 20 | VDA,WKS,NMT, | F,K,T,R,M,D,C,E,N,G,S,Q,Y,V,A,W,P,H,L,I, |

(continued)

| Codons | AA | Degenerate Codons | Amino Acids |
|---|---|---|---|
| 25 | 20 | VDA,WKS,NMC, | F,K,T,R,M,D,C,E,N,G,S,Q,Y,V,A,W,P,H,L,I, |
| 25 | 20 | WKS,NMT,VDG, | F,K,T,R,M,D,C,E,N,G,S,Q,Y,V,A,W,P,H,L,I, |
| 25 | 20 | WKS,NMC,VDG, | F,K,T,R,M,D,C,E,N,G,S,Q,Y,V,A,W,P,H,L,I, |
| 25 | 20 | WSG,NDT,VHG, | F,K,T,R,M,D,C,E,N,G,Q,S,Y,V,A,W,P,H,L,I, |
| 25 | 20 | NDC,WSG,VHG, | F,K,T,R,M,D,C,E,N,G,Q,S,Y,V,A,W,P,H,L,I, |
| 25 | 20 | NNT,WBG,VAA, | F,K,T,R,M,D,C,E,N,G,Q,S,V,Y,A,W,P,H,L,I, |
| 25 | 20 | NNC,WBG,VAA, | F,K,T,R,M,D,C,E,N,G,Q,S,Y,V,A,W,P,H,L,I, |
| 25 | 20 | NNT,VAG,WBG, | F,K,T,R,M,D,C,E,N,G,Q,S,V,Y,A,W,P,H,L,I, |
| 25 | 20 | VAG,NNC,WBG, | F,K,T,R,M,D,C,E,N,G,Q,S,Y,V,A,W,P,H,L,I, |
| 25 | 20 | VWG,NNT,BGG, | F,K,R,T,M,D,C,E,N,G,S,Q,Y,V,A,W,P,H,L,I, |
| 25 | 20 | VWG,NNC,BGG, | F,K,R,T,M,D,C,E,N,G,S,Q,Y,V,A,W,P,H,L,I, |
| 25 | 20 | HKG,NNT,VAA, | F,K,R,T,M,D,C,E,N,G,S,Q,V,Y,A,W,P,H,L,I, |
| 25 | 20 | HKG,NNC,VAA, | F,K,R,T,M,D,C,E,N,G,S,Q,Y,V,A,W,P,H,L,I, |
| 25 | 20 | NNT,VAA,DKG, | F,K,R,T,M,D,C,E,N,G,S,Q,Y,V,A,W,P,H,L,I, |
| 25 | 20 | NNC,VAA,DKG, | F,K,R,T,M,D,C,E,N,G,S,Q,Y,V,A,W,P,H,L,I, |
| 25 | 20 | HKG,NNT,VAG, | F,K,R,T,M,D,C,E,N,G,S,Q,V,Y,A,W,P,H,L,I, |
| 25 | 20 | HKG,VAG,NNC, | F,K,R,T,M,D,C,E,N,G,S,Q,Y,V,A,W,P,H,L,I, |
| 25 | 20 | NNT,VAG,DKG, | F,K,R,T,M,D,C,E,N,G,S,Q,Y,V,A,W,P,H,L,I, |
| 25 | 20 | VAG,NNC,DKG, | F,K,R,T,M,D,C,E,N,G,S,Q,Y,V,A,W,P,H,L,I, |
| 25 | 20 | VWG,NNT,HGG, | F,K,R,T,M,D,C,E,N,G,S,Q,Y,V,A,W,P,H,L,I, |
| 25 | 20 | VWG,HGG,NNC, | F,K,R,T,M,D,C,E,N,G,S,Q,Y,V,A,W,P,H,L,I, |
| 25 | 20 | VWG,NNT,DGG, | F,K,R,T,M,D,C,E,N,G,S,Q,Y,V,A,W,P,H,L,I, |
| 25 | 20 | VWG,NNC,DGG, | F,K,R,T,M,D,C,E,N,G,S,Q,Y,V,A,W,P,H,L,I, |
| 25 | 20 | VVA,NAT,DKK, | F,K,R,T,M,D,C,E,N,G,Q,S,Y,V,A,W,P,H,L,I, |
| 25 | 20 | NAC,VVA,DKK, | F,K,R,T,M,D,C,E,N,G,Q,S,Y,V,A,W,P,H,L,I, |
| 25 | 20 | DKS,VVA,NAT, | F,K,R,T,M,D,C,E,N,G,Q,S,Y,V,A,W,P,H,L,I, |
| 25 | 20 | NAC,DKS,VVA, | F,K,R,T,M,D,C,E,N,G,Q,S,Y,V,A,W,P,H,L,I, |
| 25 | 20 | VVG,NAT,DKK, | F,K,R,T,M,D,C,E,N,G,Q,S,Y,V,A,W,P,H,L,I, |
| 25 | 20 | VVG,NAC,DKK, | F,K,R,T,M,D,C,E,N,G,Q,S,Y,V,A,W,P,H,L,I, |
| 25 | 20 | VVG,DKS,NAT, | F,K,R,T,M,D,C,E,N,G,Q,S,Y,V,A,W,P,H,L,I, |
| 25 | 20 | VVG,NAC,DKS, | F,K,R,T,M,D,C,E,N,G,Q,S,Y,V,A,W,P,H,L,I, |
| 25 | 20 | VHA,NAT,DKK, | F,K,T,R,M,D,C,E,N,G,Q,S,Y,V,A,W,P,H,L,I, |
| 25 | 20 | VHA,NAC,DKK, | F,K,T,R,M,D,C,E,N,G,Q,S,Y,V,A,W,P,H,L,I, |
| 25 | 20 | VHA,DKS,NAT, | F,K,T,R,M,D,C,E,N,G,Q,S,Y,V,A,W,P,H,L,I, |
| 25 | 20 | VHA,NAC,DKS, | F,K,T,R,M,D,C,E,N,G,Q,S,Y,V,A,W,P,H,L,I, |
| 25 | 20 | NAT,VHG,DKK, | F,K,T,R,M,D,C,E,N,G,Q,S,Y,V,A,W,P,H,L,I, |
| 25 | 20 | NAC,VHG,DKK, | F,K,T,R,M,D,C,E,N,G,Q,S,Y,V,A,W,P,H,L,I, |
| 25 | 20 | DKS,NAT,VHG, | F,K,T,R,M,D,C,E,N,G,Q,S,Y,V,A,W,P,H,L,I, |

(continued)

| Codons | AA | Degenerate Codons | Amino Acids |
|--------|-----|-------------------|-------------|
| 25 | 20 | NAC,DKS,VHG, | F,K,T,R,M,D,C,E,N,G,Q,S,Y,V,A,W,P,H,L,I, |
| 25 | 20 | NDT,VHG,NGG, | F,K,R,T,M,D,C,E,N,G,Q,S,Y,V,A,W,P,H,L,I, |
| 25 | 20 | NDC,VHG,NGG, | F,K,R,T,M,D,C,E,N,G,S,Q,Y,V,A,W,P,H,L,I, |
| 25 | 20 | NGK,VHG,NWT, | F,K,T,R,M,D,C,E,N,G,Q,S,Y,V,A,W,P,H,L,I, |
| 25 | 20 | NGK,VHG,NWC, | F,K,T,R,M,D,C,E,N,G,Q,S,Y,V,A,W,P,H,L,I, |
| 25 | 20 | NGS,VHG,NWT, | F,K,T,R,M,D,C,E,N,G,Q,S,Y,V,A,W,P,H,L,I, |
| 25 | 20 | NGS,VHG,NWC, | F,K,T,R,M,D,C,E,N,G,Q,S,Y,V,A,W,P,H,L,I, |
| 19 | 19 | ATG,VMA,NDT, | F,K,T,R,M,D,C,E,N,G,Q,S,Y,V,A,P,L,H,I, |
| 19 | 19 | NDC,ATG,VMA, | F,K,T,R,M,D,C,E,N,G,Q,S,V,Y,A,P,H,L,I, |
| 19 | 19 | ATG,NDT,VMG, | F,K,T,R,M,D,C,E,N,G,Q,S,Y,V,A,P,L,H,I, |
| 19 | 19 | NDC,ATG,VMG, | F,K,T,R,M,D,C,E,N,G,Q,S,V,Y,A,P,H,L,I, |
| 19 | 19 | NDT,SMA,AHG, | F,K,T,R,M,D,C,E,N,G,Q,S,Y,V,A,P,L,H,I, |
| 19 | 19 | NDC,SMA,AHG, | F,K,T,R,M,D,C,E,N,G,Q,S,V,Y,A,P,H,L,I, |
| 19 | 19 | NDT,AHG,SMG, | F,K,T,R,M,D,C,E,N,G,Q,S,Y,V,A,P,L,H,I, |
| 19 | 19 | NDC,AHG,SMG, | F,K,T,R,M,D,C,E,N,G,Q,S,V,Y,A,P,H,L,I, |
| 19 | 19 | VMA,NDT,TGG, | F,K,T,R,D,C,E,N,G,Q,S,Y,V,A,W,P ,L,H,I, |
| 19 | 19 | NDC,VMA,TGG, | F,K,T,R,D,C,E,N,G,Q,S,V,Y,A,W,P ,H,L,I, |
| 19 | 19 | NDT,TGG,VMG, | F,K,T,R,D,C,E,N,G,Q,S,Y,V,A,W,P ,L,H,I, |
| 19 | 19 | NDC,TGG,VMG, | F,K,T,R,D,C,E,N,G,Q,S,V,Y,A,W,P ,H,L,I, |
| 19 | 19 | VHG,SRT,WDT, | F,K,T,R,M,C,D,E,N,G,S,Q,Y,V,A,P,L,H,I, |
| 19 | 19 | VHG,WDC,SRT, | F,K,T,R,M,C,D,E,N,G,S,Q,Y,V,A,P,L,H,I, |
| 19 | 19 | VHG,SRC,WDT, | F,K,T,R,M,C,D,E,N,G,S,Q,Y,V,A,P,L,H,I, |
| 19 | 19 | VHG,WDC,SRC, | F,K,T,R,M,C,D,E,N,G,S,Q,Y,V,A,P,L,H,I, |
| 19 | 19 | WTT,VHG,NRT, | F,K,R,T,M,D,C,E,N,G,Q,S,V,Y,A,P,H,L,I, |
| 19 | 19 | WTT,VHG,NRC, | F,K,R,T,M,D,C,E,N,G,Q,S,V,Y,A,P,H,L,I, |
| 19 | 19 | WTC,VHG,NRT, | F,K,R,T,M,D,C,E,N,G,Q,S,V,Y,A,P,H,L,I, |
| 19 | 19 | WTC,VHG,NRC, | F,K,R,T,M,D,C,E,N,G,Q,S,V,Y,A,P,H,L,I, |
| 19 | 19 | VMA,WKG,DDT, | F,K,T,R,M,D,C,E,N,G,Q,S,V,Y,A,W,P,L,I, |
| 19 | 19 | DDC,VMA,WKG, | F,K,T,R,M,D,C,E,N,G,Q,S,Y,V,A,W,P,L,I, |
| 19 | 19 | WKG,DDT,VMG, | F,K,T,R,M,D,C,E,N,G,Q,S,V,Y,A,W,P,L,I, |
| 19 | 19 | DDC,WKG,VMG, | F,K,T,R,M,D,C,E,N,G,Q,S,V,Y,A,W,P,L,I, |
| 20 | 19 | ATG,NNT,VAA, | F,K,T,R,M,D,C,E,N,G,Q,S,V,Y,A,P,H,L,I, |
| 20 | 19 | ATG,NNC,VAA, | F,K,R,T,M,D,C,E,N,G,Q,S,Y,V,A,P,H,L,I, |
| 20 | 19 | ATG,NNT,VAG, | F,K,T,R,M,D,C,E,N,G,Q,S,V,Y,A,P,H,L,I, |
| 20 | 19 | ATG,VAG,NNC, | F,K,R,T,M,D,C,E,N,G,Q,S,Y,V,A,P,H,L,I, |
| 20 | 19 | VMA,NDT,ATK, | F,K,T,R,M,D,C,E,N,G,Q,S,Y,V,A,P,L,H,I, |
| 20 | 19 | NDC,VMA,ATK, | F,K,T,R,M,D,C,E,N,G,Q,S,V,Y,A,P,H,L,I, |
| 20 | 19 | NDT,VMG,ATK, | F,K,T,R,M,D,C,E,N,G,Q,S,Y,V,A,P,L,H,I, |

(continued)

| Codons | AA | Degenerate Codons | Amino Acids |
|--------|-----|-------------------|-------------|
| 20 | 19 | NDC,VMG,ATK, | F,K,T,R,M,D,C,E,N,G,Q,S,V,Y,A,P,H,L,I, |
| 20 | 19 | VMA,NDT,ATR, | F,K,T,R,M,D,C,E,N,G,Q,S,Y,V,A,P,L,H,I, |
| 20 | 19 | NDC,VMA,ATR, | F,K,T,R,M,D,C,E,N,G,Q,S,V,Y,A,P,H,L,I, |
| 20 | 19 | NDT,VMG,ATR, | F,K,T,R,M,D,C,E,N,G,Q,S,Y,V,A,P,L,H,I, |
| 20 | 19 | NDC,VMG,ATR, | F,K,T,R,M,D,C,E,N,G,Q,S,V,Y,A,P,H,L,I, |
| 20 | 19 | VMA,NDT,ATS, | F,K,T,R,M,D,C,E,N,G,Q,S,Y,V,A,P,L,H,I, |
| 20 | 19 | NDC,VMA,ATS, | F,K,T,R,M,D,C,E,N,G,Q,S,V,Y,A,P,H,L,I, |
| 20 | 19 | NDT,ATS,VMG, | F,K,T,R,M,D,C,E,N,G,Q,S,Y,V,A,P,L,H,I, |
| 20 | 19 | NDC,ATS,VMG, | F,K,T,R,M,D,C,E,N,G,Q,S,V,Y,A,P,H,L,I, |
| 20 | 19 | VMA,NDT,AKG, | F,K,T,R,M,D,C,E,N,G,Q,S,Y,V,A,P,L,H,I, |
| 20 | 19 | NDC,VMA,AKG, | F,K,T,R,M,D,C,E,N,G,Q,S,V,Y,A,P,H,L,I, |
| 20 | 19 | NDT,VMG,AKG, | F,K,T,R,M,D,C,E,N,G,Q,S,Y,V,A,P,L,H,I, |
| 20 | 19 | NDC,VMG,AKG, | F,K,T,R,M,D,C,E,N,G,Q,S,V,Y,A,P,H,L,I, |
| 20 | 19 | VMA,NDT,AYG, | F,K,T,R,M,D,C,E,N,G,Q,S,Y,V,A,P,L,H,I, |
| 20 | 19 | NDC,VMA,AYG, | F,K,T,R,M,D,C,E,N,G,Q,S,V,Y,A,P,H,L,I, |
| 20 | 19 | NDT,VMG,AYG, | F,K,T,R,M,D,C,E,N,G,Q,S,Y,V,A,P,L,H,I, |
| 20 | 19 | NDC,VMG,AYG, | F,K,T,R,M,D,C,E,N,G,Q,S,V,Y,A,P,H,L,I, |
| 20 | 19 | NNT,SAA,AWG, | F,K,T,R,M,D,C,E,N,G,Q,S,V,Y,A,P,H,L,I, |
| 20 | 19 | NNC,SAA,AWG, | F,K,R,T,M,D,C,E,N,G,Q,S,Y,V,A,P,H,L,I, |
| 20 | 19 | NNT,AWG,SAG, | F,K,T,R,M,D,C,E,N,G,Q,S,V,Y,A,P,H,L,I, |
| 20 | 19 | NNC,AWG,SAG, | F,K,R,T,M,D,C,E,N,G,Q,S,Y,V,A,P,H,L,I, |
| 20 | 19 | VMA,NDT,AWG, | F,K,T,R,M,D,C,E,N,G,Q,S,Y,V,A,P,L,H,I, |
| 20 | 19 | NDC,VMA,AWG, | F,K,T,R,M,D,C,E,N,G,Q,S,V,Y,A,P,H,L,I, |
| 20 | 19 | NDT,VMG,AWG, | F,K,T,R,M,D,C,E,N,G,Q,S,Y,V,A,P,L,H,I, |
| 20 | 19 | NDC,VMG,AWG, | F,K,T,R,M,D,C,E,N,G,Q,S,V,Y,A,P,H,L,I, |
| 20 | 19 | AHK,BNT,SAA, | F,K,T,R,M,D,C,E,N,G,Q,S,Y,V,A,P,L,H,I, |
| 20 | 19 | AHK,BNC,SAA, | F,K,T,R,M,D,C,E,N,G,Q,S,V,Y,A,P,L,H,I, |
| 20 | 19 | AHK,BNT,SAG, | F,K,T,R,M,D,C,E,N,G,Q,S,Y,V,A,P,L,H,I, |
| 20 | 19 | AHK,BNC,SAG, | F,K,T,R,M,D,C,E,N,G,Q,S,V,Y,A,P,L,H,I, |
| 20 | 19 | BNT,SAA,AHS, | F,K,T,R,M,D,C,E,N,G,Q,S,Y,V,A,P,L,H,I, |
| 20 | 19 | BNC,SAA,AHS, | F,K,T,R,M,D,C,E,N,G,Q,S,V,Y,A,P,H,L,I, |
| 20 | 19 | BNT,AHS,SAG, | F,K,T,R,M,D,C,E,N,G,Q,S,Y,V,A,P,L,H,I, |
| 20 | 19 | BNC,AHS,SAG, | F,K,T,R,M,D,C,E,N,G,Q,S,V,Y,A,P,H,L,I, |
| 20 | 19 | NDT,SMA,ANG, | F,K,T,R,M,D,C,E,N,G,Q,S,Y,V,A,P,L,H,I, |
| 20 | 19 | NDC,SMA,ANG, | F,K,T,R,M,D,C,E,N,G,Q,S,V,Y,A,P,H,L,I, |
| 20 | 19 | NDT,ANG,SMG, | F,K,T,R,M,D,C,E,N,G,Q,S,Y,V,A,P,L,H,I, |
| 20 | 19 | NDC,ANG,SMG, | F,K,T,R,M,D,C,E,N,G,Q,S,V,Y,A,P,H,L,I, |
| 20 | 19 | NNT,V AA,TGG, | F,K,T,R,D,C,E,N,G,Q,S,V,Y,A,W,P ,H,L,I, |

(continued)

| Codons | AA | Degenerate Codons | Amino Acids |
|--------|----|-------------------|-------------|
| 20 | 19 | NNC,VAA,TGG, | F,K,R,T,D,C,E,N,G,Q,S,Y,V,A,W,P ,H,L,I, |
| 20 | 19 | NNT,V AG,TGG, | F,K,T,R,D,C,E,N,G,Q,S,V,Y,A,W,P ,H,L,I, |
| 20 | 19 | VAG,NNC,TGG, | F,K,R,T,D,C,E,N,G,Q,S,Y,V,A,W,P ,H,L,I, |
| 20 | 19 | TGK,RDG,NHT, | F,K,R,T,M,D,C,E,N,G,S,V,Y,A,W,P,L,H,I, |
| 20 | 19 | TGK,RDG,NHC, | F,K,R,T,M,D,C,E,N,G,S,V,Y,A,W,P,H,L,I, |
| 20 | 19 | VMA,NDT,TGK, | F,K,T,R,D,C,E,N,G,Q,S,Y,V,A,W,P,L,H,I, |
| 20 | 19 | NDC,VMA,TGK, | F,K,T,R,D,C,E,N,G,Q,S,V,Y,A,W,P,H,L,I, |
| 20 | 19 | NDT,TGK,VMG, | F,K,T,R,D,C,E,N,G,Q,S,Y,V,A,W,P,L,H,I, |
| 20 | 19 | NDC,TGK,VMG, | F,K,T,R,D,C,E,N,G,Q,S,V,Y,A,W,P,H,L,I, |
| 20 | 19 | TGK,VRA,NHT, | F,K,R,T,D,C,E,N,G,Q,S,Y,V,A,W,P ,L,H,I, |
| 20 | 19 | TGK,VRA,NHC, | F,K,R,T,D,C,E,N,G,Q,S,V,Y,A,W,P,H,L,I, |
| 20 | 19 | VRG,TGK,NHT, | F,K,R,T,D,C,E,N,G,Q,S,Y,V,A,W,P,L,H,I, |
| 20 | 19 | VRG,TGK,NHC, | F,K,R,T,D,C,E,N,G,Q,S,V,Y,A,W,P,H,L,I, |
| 20 | 19 | TGS,RDG,NHT, | F,K,R,T,M,D,C,E,N,G,S,V,Y,A,W,P,L,H,I, |
| 20 | 19 | TGS,RDG,NHC, | F,K,R,T,M,D,C,E,N,G,S,V,Y,A,W,P,H,L,I, |
| 20 | 19 | VMA,NDT,TGS, | F,K,T,R,D,C,E,N,G,Q,S,Y,V,A,W,P,L,H,I, |
| 20 | 19 | NDC,VMA,TGS, | F,K,T,R,D,C,E,N,G,Q,S,V,Y,A,W,P,H,L,I, |
| 20 | 19 | NDT,TGS,VMG, | F,K,T,R,D,C,E,N,G,Q,S,Y,V,A,W,P,L,H,I, |
| 20 | 19 | NDC,TGS,VMG, | F,K,T,R,D,C,E,N,G,Q,S,V,Y,A,W,P,H,L,I, |
| 20 | 19 | TGS,VRA,NHT, | F,K,R,T,D,C,E,N,G,Q,S,Y,V,A,W,P,L,H,I, |
| 20 | 19 | TGS,VRA,NHC, | F,K,R,T,D,C,E,N,G,Q,S,V,Y,A,W,P,H,L,I, |
| 20 | 19 | VRG,TGS,NHT, | F,K,R,T,D,C,E,N,G,Q,S,Y,V,A,W,P,L,H,I, |
| 20 | 19 | VRG,TGS,NHC, | F,K,R,T,D,C,E,N,G,Q,S,Y,V,A,W,P,H,L,I, |
| 20 | 19 | VMA,NDT,TKG, | F,K,T,R,D,C,E,N,G,Q,S,Y,V,A,W,P,H,L,I, |
| 20 | 19 | NDC,VMA,TKG, | F,K,T,R,D,C,E,N,G,Q,S,V,Y,A,W,P,H,L,I, |
| 20 | 19 | NDT,TKG,VMG, | F,K,T,R,D,C,E,N,G,Q,S,Y,V,A,W,P,H,L,I, |
| 20 | 19 | NDC,TKG,VMG, | F,K,T,R,D,C,E,N,G,Q,S,V,Y,A,W,P,H,L,I, |
| 20 | 19 | VMA,NDT,TSG, | F,K,T,R,D,C,E,N,G,Q,S,Y,V,A,W,P,L,H,I, |
| 20 | 19 | NDC,VMA,TSG, | F,K,T,R,D,C,E,N,G,Q,S,V,Y,A,W,P,H,L,I, |
| 20 | 19 | NDT,VMG,TSG, | F,K,T ,R,D,C,E,N,G,Q,S,Y,V,A,W,P,L,H,I, |
| 20 | 19 | NDC,VMG,TSG, | F,K,T,R,D,C,E,N,G,Q,S,Y,V,A,W,P,H,L,I, |
| 20 | 19 | NDT,GMA,MHG, | F,K,T,R,M,D,C,E,N,G,Q,S,Y,V,A,P,H,L,I, |
| 20 | 19 | NDC,GMA,MHG, | F,K,T,R,M,D,C,E,N,G,Q,S,V,Y,A,P,H,L,I, |
| 20 | 19 | NDT,GMG,MHG, | F,K,T,R,M,D,C,E,N,G,Q,S,V,Y,A,P,H,L,I, |
| 20 | 19 | NDC,GMG,MHG, | F,K,T,R,M,D,C,E,N,G,Q,S,V,Y,A,P,H,L,I, |
| 20 | 19 | HDT,VHG,GRT, | F,K,R,T,M,C,D,E,N,G,Q,S,Y,V,A,P,H,L,I, |
| 20 | 19 | HDC,VHG,GRT, | F,K,R,T,M,C,D,E,N,G,Q,S,Y,V,A,P,H,L,I, |
| 20 | 19 | HDT,GRC,VHG, | F,K,R,T,M,C,D,E,N,G,Q,S,Y,V,A,P,H,L,I, |

(continued)

| Codons | AA | Degenerate Codons | Amino Acids |
|---|---|---|---|
| 20 | 19 | HDC,GRC,VHG, | F,K,R,T,M,C,D,E,N,G,Q,S,Y,V,A,P,H,L,I, |
| 20 | 19 | NDT,RHG,CMA, | F,K,T,R,M,D,C,E,N,G,Q,S,V,Y,A,P,L,H,I, |
| 20 | 19 | NDC,RHG,CMA, | F,K,T,R,M,D,C,E,N,G,Q,S,V,Y,A,P,H,L,I, |
| 20 | 19 | NDT,CMG,RHG, | F,K,T,R,M,D,C,E,N,G,Q,S,V,Y,A,P,L,H,I, |
| 20 | 19 | NDC,CMG,RHG, | F,K,T,R,M,D,C,E,N,G,Q,S,V,Y,A,P,H,L,I, |
| 20 | 19 | CRT,DDT,VHG, | F,K,T,R,M,D,C,E,N,G,Q,S,Y,V,A,P,L,H,I, |
| 20 | 19 | DDC,CRT,VHG, | F,K,T,R,M,D,C,E,N,G,Q,S,Y,V,A,P,L,H,I, |
| 20 | 19 | CRC,DDT,VHG, | F,K,T,R,M,D,C,E,N,G,Q,S,Y,V,A,P,L,H,I, |
| 20 | 19 | DDC,CRC,VHG, | F,K,T,R,M,D,C,E,N,G,Q,S,Y,V,A,P,L,H,I, |
| 20 | 19 | VMA,NDT,KGG, | F,K,T,R,D,C,E,N,G,Q,S,Y,V,A,W,P,L,H,I, |
| 20 | 19 | NDC,VMA,KGG, | F,K,T,R,D,C,E,N,G,Q,S,V,Y,A,W,P,H,L,I, |
| 20 | 19 | NDT,KGG,VMG, | F,K,T,R,D,C,E,N,G,Q,S,Y,V,A,W,P,L,H,I, |
| 20 | 19 | NDC,KGG,VMG, | F,K,T,R,D,C,E,N,G,Q,S,V,Y,A,W,P,H,L,I, |
| 20 | 19 | HDT,KGG,VHG, | F,K,R,T,M,C,E,N,G,Q,S,Y,V,A,W,P,H,L,I, |
| 20 | 19 | HDC,KGG,VHG, | F,K,R,T,M,C,E,N,G,Q,S,Y,V,A,W,P,H,L,I, |
| 20 | 19 | VMA,NDT,MTG, | F,K,T,R,M,D,C,E,N,G,Q,S,Y,V,A,P,H,L,I, |
| 20 | 19 | NDC,VMA,MTG, | F,K,T,R,M,D,C,E,N,G,Q,S,V,Y,A,P,H,L,I, |
| 20 | 19 | NDT,VMG,MTG, | F,K,T,R,M,D,C,E,N,G,Q,S,V,Y,A,P,H,L,I, |
| 20 | 19 | NDC,VMG,MTG, | F,K,T,R,M,D,C,E,N,G,Q,S,V,Y,A,P,H,L,I, |
| 20 | 19 | VMA,NDT,RTG, | F,K,T,R,M,D,C,E,N,G,Q,S,V,Y,A,P,L,H,I, |
| 20 | 19 | NDC,VMA,RTG, | F,K,T,R,M,D,C,E,N,G,Q,S,V,Y,A,P,H,L,I, |
| 20 | 19 | NDT,VMG,RTG, | F,K,T,R,M,D,C,E,N,G,Q,S,V,Y,A,P,L,H,I, |
| 20 | 19 | NDC,VMG,RTG, | F,K,T,R,M,D,C,E,N,G,Q,S,V,Y,A,P,H,L,I, |
| 20 | 19 | VMA,NDT,YGG, | F,K,T,R,D,C,E,N,G,Q,S,Y,V,A,W,P,L,H,I, |
| 20 | 19 | NDC,VMA,YGG, | F,K,T,R,D,C,E,N,G,Q,S,V,Y,A,W,P,H,L,I, |
| 20 | 19 | NDT,VMG,YGG, | F,K,T,R,D,C,E,N,G,Q,S,Y,V,A,W,P,L,H,I, |
| 20 | 19 | NDC,VMG,YGG, | F,K,T,R,D,C,E,N,G,Q,S,V,Y,A,W,P ,H,L,I, |
| 20 | 19 | DDT,VHG,YGG, | F,K,T,R,M,D,C,E,N,G,Q,S,Y,V,A,W,P,L,I, |
| 20 | 19 | DDC,VHG,YGG, | F,K,T,R,M,D,C,E,N,G,Q,S,Y,V,A,W,P,L,I, |
| 20 | 19 | SAT,VNG,WDT, | F,K,R,T,M,C,D,E,N,G,S,Q,Y,V,A,P,L,H,I, |
| 20 | 19 | SAT,VNG,WDC, | F,K,R,T,M,C,D,E,N,G,S,Q,Y,V,A,P,L,H,I, |
| 20 | 19 | SAC,VNG,WDT, | F,K,R,T,M,C,D,E,N,G,S,Q,Y,V,A,P,L,H,I, |
| 20 | 19 | SAC,VNG,WDC, | F,K,R,T,M,C,D,E,N,G,S,Q,Y,V,A,P,L,H,I, |
| 20 | 19 | VWG,SVT,WNT, | F,K,R,T,M,D,C,E,N,G,Q,S,V,Y,A,P,L,H,I, |
| 20 | 19 | VWG,SVT,WNC, | F,K,R,T,M,D,C,E,N,G,Q,S,V,Y,A,P,L,H,I, |
| 20 | 19 | VWG,SVC,WNT, | F,K,R,T,M,D,C,E,N,G,Q,S,V,Y,A,P,L,H,I, |
| 20 | 19 | VWG,SVC,WNC, | F,K,R,T,M,D,C,E,N,G,Q,S,V,Y,A,P,L,H,I, |
| 20 | 19 | WTT,VWG,NVT, | F,K,R,T,M,D,C,E,N,G,Q,S,V,Y,A,P,H,L,I, |

(continued)

| Codons | AA | Degenerate Codons | Amino Acids |
|---|---|---|---|
| 20 | 19 | WTT,NVC,VWG, | F,K,T ,R,M,D,C,E,N,G,Q,S,V,Y,A,P,H,L,I, |
| 20 | 19 | VMA,NDT,WTG, | F,K,T,R,M,D,C,E,N,G,Q,S,Y,V,A,P,H,L,I, |
| 20 | 19 | NDC,VMA,WTG, | F,K,T,R,M,D,C,E,N,G,Q,S,V,Y,A,P,H,L,I, |
| 20 | 19 | NDT,VMG,WTG, | F,K,T,R,M,D,C,E,N,G,Q,S,Y,V,A,P,H,L,I, |
| 20 | 19 | NDC,VMG,WTG, | F,K,T,R,M,D,C,E,N,G,Q,S,V,Y,A,P,H,L,I, |
| 20 | 19 | WTC,VWG,NVT, | F,K,R,T ,M,D,C,E,N,G,Q,S,V,Y,A,P,H,L,I, |
| 20 | 19 | WTC,NVC,VWG, | F,K,T,R,M,D,C,E,N,G,Q,S,V,Y,A,P,H,L,I, |
| 20 | 19 | VMA,NDT,WGG, | F,K,T,R,D,C,E,N,G,Q,S,Y,V,A,W,P ,L,H,I, |
| 20 | 19 | NDC,VMA,WGG, | F,K,T,R,D,C,E,N,G,Q,S,V,Y,A,W,P ,H,L,I, |
| 20 | 19 | NDT,VMG,WGG, | F,K,T,R,D,C,E,N,G,Q,S,Y,V,A,W,P ,L,H,I, |
| 20 | 19 | NDC,VMG,WGG, | F,K,T,R,D,C,E,N,G,Q,S,V,Y,A,W,P ,H,L,I, |
| 20 | 19 | DDT,VHG,WGG, | F,K,T,R,M,D,C,E,N,G,Q,S,Y,V,A,W,P,L,I, |
| 20 | 19 | DDC,VHG,WGG, | F,K,T,R,M,D,C,E,N,G,Q,S,Y,V,A,W,P,L,I, |
| 20 | 19 | WKT,NAT,VNG, | F,K,T,R,M,D,C,E,N,G,S,Q,Y,V,A,P,H,L,I, |
| 20 | 19 | WKT,NAC,VNG, | F,K,T,R,M,D,C,E,N,G,S,Q,Y,V,A,P,H,L,I, |
| 20 | 19 | NAT,WKC,VNG, | F,K,T,R,M,D,C,E,N,G,S,Q,Y,V,A,P,H,L,I, |
| 20 | 19 | NAC,WKC,VNG, | F,K,T,R,M,D,C,E,N,G,S,Q,Y,V,A,P,H,L,I, |
| 20 | 19 | VMA,DTK,NRT, | F,K,R,T,M,D,C,E,N,G,Q,S,V,Y,A,P,H,L,I, |
| 20 | 19 | VMA,DTK,NRC, | F,K,R,T,M,D,C,E,N,G,Q,S,V,Y,A,P,H,L,I, |
| 20 | 19 | VMA,NRT,DTS, | F,K,R,T,M,D,C,E,N,G,Q,S,V,Y,A,P,H,L,I, |
| 20 | 19 | VMA,DTS,NRC, | F,K,R,T,M,D,C,E,N,G,Q,S,V,Y,A,P,H,L,I, |
| 20 | 19 | VMA,DGK,NWT, | F,K,R,T,D,C,E,N,G,S,Q,V,Y,A,W,P,L,H,I, |
| 20 | 19 | VMA,DGK,NWC, | F,K,R,T,D,C,E,N,G,S,Q,Y,V,A,W,P,H,L,I, |
| 20 | 19 | VMA,DGS,NWT, | F,K,R,T,D,C,E,N,G,S,Q,V,Y,A,W,P,L,H,I, |
| 20 | 19 | VMA,DGS,NWC, | F,K,R,T,D,C,E,N,G,S,Q,Y,V,A,W,P,H,L,I, |
| 20 | 19 | DTK,VMG,NRT, | F,K,R,T,M,D,C,E,N,G,Q,S,V,Y,A,P,H,L,I, |
| 20 | 19 | DTK,VMG,NRC, | F,K,R,T,M,D,C,E,N,G,Q,S,V,Y,A,P,H,L,I, |
| 20 | 19 | VMG,NRT,DTS, | F,K,R,T,M,D,C,E,N,G,Q,S,V,Y,A,P,H,L,I, |
| 20 | 19 | VMG,DTS,NRC, | F,K,R,T,M,D,C,E,N,G,Q,S,V,Y,A,P,H,L,I, |
| 20 | 19 | VMG,DGK,NWT, | F,K,R,T,D,C,E,N,G,S,Q,V,Y,A,W,P,L,H,I, |
| 20 | 19 | VMG,DGK,NWC, | F,K,R,T,D,C,E,N,G,S,Q,Y,V,A,W,P,H,L,I, |
| 20 | 19 | DGS,VMG,NWT, | F,K,R,T,D,C,E,N,G,S,Q,V,Y,A,W,P,L,H,I, |
| 20 | 19 | DGS,VMG,NWC, | F,K,R,T,D,C,E,N,G,S,Q,Y,V,A,W,P,H,L,I, |
| 20 | 19 | HTT,VHG,NRT, | F,K,R,T,M,D,C,E,N,G,Q,S,V,Y,A,P,H,L,I, |
| 20 | 19 | HTT,VHG,NRC, | F,K,R,T,M,D,C,E,N,G,Q,S,V,Y,A,P,H,L,I, |
| 20 | 19 | VHG,NRT,HTC, | F,K,R,T,M,D,C,E,N,G,Q,S,V,Y,A,P,H,L,I, |
| 20 | 19 | VHG,HTC,NRC, | F,K,R,T,M,D,C,E,N,G,Q,S,V,Y,A,P,H,L,I, |
| 20 | 19 | VHG,DTT,NRT, | F,K,R,T,M,D,C,E,N,G,Q,S,V,Y,A,P,H,L,I, |

(continued)

| Codons | AA | Degenerate Codons | Amino Acids |
|---|---|---|---|
| 20 | 19 | VHG,DTT,NRC, | F,K,R,T,M,D,C,E,N,G,Q,S,V,Y,A,P,H,L,I, |
| 20 | 19 | VHG,DTC,NRT, | F,K,R,T,M,D,C,E,N,G,Q,S,V,Y,A,P,H,L,I, |
| 20 | 19 | VHG,DTC,NRC, | F,K,R,T,M,D,C,E,N,G,Q,S,V,Y,A,P,H,L,I, |
| 21 | 19 | NNT,VAA,ATK, | F,K,T,R,M,D,C,E,N,G,Q,S,V,Y,A,P,H,L,I, |
| 21 | 19 | NNC,VAA,ATK, | F,K,R,T,M,D,C,E,N,G,Q,S,Y,V,A,P,H,L,I, |
| 21 | 19 | NNT,VAG,ATK, | F,K,T,R,M,D,C,E,N,G,Q,S,V,Y,A,P,H,L,I, |
| 21 | 19 | VAG,NNC,ATK, | F,K,R,T,M,D,C,E,N,G,Q,S,Y,V,A,P,H,L,I, |
| 21 | 19 | NNT,VAA,ATR, | F,K,T,R,M,D,C,E,N,G,Q,S,V,Y,A,P,H,L,I, |
| 21 | 19 | NNC,VAA,ATR, | F,K,T,R,M,D,C,E,N,G,Q,S,V,Y,A,P,H,L,I, |
| 21 | 19 | NNC,VAG,ATR, | F,K,T,R,M,D,C,E,N,G,Q,S,V,Y,A,P,H,L,I, |
| 21 | 19 | VAG,NNC,ATR, | F,K,R,T,M,D,C,E,N,G,Q,S,Y,V,A,P,H,L,I, |
| 21 | 19 | NNT,VAA,ATS, | F,K,T,R,M,D,C,E,N,G,Q,S,V,Y,A,P,H,L,I, |
| 21 | 19 | NNC,VAA,ATS, | F,K,R,T,M,D,C,E,N,G,Q,S,Y,V,A,P,H,L,I, |
| 21 | 19 | NNT,VAG,ATS, | F,K,T,R,M,D,C,E,N,G,Q,S,V,Y,A,P,H,L,I, |
| 21 | 19 | VAG,NNC,ATS, | F,K,R,T,M,D,C,E,N,G,Q,S,Y,V,A,P,H,L,I, |
| 21 | 19 | VMA,NDT,ATB, | F,K,T,R,M,D,C,E,N,G,Q,S,Y,V,A,P,L,H,I, |
| 21 | 19 | NDC,VMA,ATB, | F,K,T,R,M,D,C,E,N,G,Q,S,V,Y,A,P,H,L,I, |
| 21 | 19 | NDT,ATB,VMG, | F,K,T,R,M,D,C,E,N,G,Q,S,Y,V,A,P,L,H,I, |
| 21 | 19 | NDC,ATB,VMG, | F,K,T,R,M,D,C,E,N,G,Q,S,V,Y,A,P,H,L,I, |
| 21 | 19 | VMA,NDT,ATV, | F,K,T,R,M,D,C,E,N,G,Q,S,Y,V,A,P,L,H,I, |
| 21 | 19 | NDC,VMA,ATV, | F,K,T,R,M,D,C,E,N,G,Q,S,V,Y,A,P,H,L,I, |
| 21 | 19 | NDT,ATV,VMG, | F,K,T,R,M,D,C,E,N,G,Q,S,Y,V,A,P,L,H,I, |
| 21 | 19 | NDC,ATV,VMG, | F,K,T,R,M,D,C,E,N,G,Q,S,V,Y,A,P,H,L,I, |
| 21 | 19 | VMA,NDT,ATD, | F,K,T,R,M,D,C,E,N,G,Q,S,Y,V,A,P,L,H,I, |
| 21 | 19 | NDC,VMA,ATD, | F,K,T,R,M,D,C,E,N,G,Q,S,V,Y,A,P,H,L,I, |
| 21 | 19 | NDT,ATD,VMG, | F,K,T,R,M,D,C,E,N,G,Q,S,Y,V,A,P,L,H,I, |
| 21 | 19 | NDC,ATD,VMG, | F,K,T,R,M,D,C,E,N,G,Q,S,V,Y,A,P,H,L,I, |
| 21 | 19 | NNT,VAA,AKG, | F,K,R,T,M,D,C,E,N,G,Q,S,V,Y,A,P,H,L,I, |
| 21 | 19 | NNC,VAA,AKG, | F,K,R,T,M,D,C,E,N,G,Q,S,Y,V,A,P,H,L,I, |
| 21 | 19 | NNT,VAG,AKG, | F,K,R,T,M,D,C,E,N,G,Q,S,V,Y,A,P,H,L,I, |
| 21 | 19 | VAG,NNC,AKG, | F,K,R,T,M,D,C,E,N,G,Q,S,V,Y,A,P,H,L,I, |
| 21 | 19 | NNT,VAA,AYG, | F,K,T,R,M,D,C,E,N,G,Q,S,V,Y,A,P,H,L,I, |
| 21 | 19 | NNC,VAA,AYG, | F,K,T,R,M,D,C,E,N,G,Q,S,Y,V,A,P,H,L,I, |
| 21 | 19 | NNT,VAG,AYG, | F,K,T,R,M,D,C,E,N,G,Q,S,V,Y,A,P,H,L,I, |
| 21 | 19 | VAG,NNC,AYG, | F,K,T,R,M,D,C,E,N,G,Q,S,Y,V,A,P,H,L,I, |
| 21 | 19 | NNT,VAA,AWG, | F,K,T,R,M,D,C,E,N,G,Q,S,V,Y,A,P,H,L,I, |
| 21 | 19 | NNC,VAA,AWG, | F,K,R,T,M,D,C,E,N,G,Q,S,Y,V,A,P,H,L,I, |
| 21 | 19 | NNT,VAG,AWG, | F,K,T,R,M,D,C,E,N,G,Q,S,V,Y,A,P,H,L,I, |

(continued)

| Codons | AA | Degenerate Codons | Amino Acids |
|--------|-----|-------------------|-------------|
| 21 | 19 | VAG,NNC,AWG, | F,K,R,T,M,D,C,E,N,G,Q,S,Y,V,A,P,H,L,I, |
| 21 | 19 | VMA,NDT,ABG, | F,K,T,R,M,D,C,E,N,G,Q,S,Y,V,A,P,L,H,I, |
| 21 | 19 | NDC,VMA,ABG, | F,K,T,R,M,D,C,E,N,G,Q,S,V,Y,A,P,H,L,I, |
| 21 | 19 | NDT,ABG,VMG, | F,K,T,R,M,D,C,E,N,G,Q,S,Y,V,A,P,L,H,I, |
| 21 | 19 | NDC,ABG,VMG, | F,K,T,R,M,D,C,E,N,G,Q,S,V,Y,A,P,H,L,I, |
| 21 | 19 | VWG,BNT,AHT, | F,K,T,R,M,C,D,E,N,G,Q,S,Y,V,A,P,H,L,I, |
| 21 | 19 | VWG,BNC,AHT, | F,K,T,R,M,C,D,E,N,G,Q,S,V,Y,A,P,L,H,I, |
| 21 | 19 | NNT,AHG,SAA, | F,K,T,R,M,D,C,E,N,G,Q,S,V,Y,A,P,H,L,I, |
| 21 | 19 | NNC,AHG,SAA, | F,K,T,R,M,D,C,E,N,G,Q,S,Y,V,A,P,H,L,I, |
| 21 | 19 | NNT,AHG,SAG, | F,K,T,R,M,D,C,E,N,G,Q,S,Y,V,A,P,H,L,I, |
| 21 | 19 | NNC,AHG,SAG, | F,K,T,R,M,D,C,E,N,G,Q,S,Y,V,A,P,H,L,I, |
| 21 | 19 | NDT,AHG,SVA, | F,K,R,T,M,D,C,E,N,G,Q,S,Y,V,A,P,L,H,I, |
| 21 | 19 | NDC,AHG,SVA, | F,K,R,T,M,D,C,E,N,G,Q,S,V,Y,A,P,H,L,I, |
| 21 | 19 | SVG,NDT,AHG, | F,K,R,T,M,D,C,E,N,G,Q,S,Y,V,A,P,L,H,I, |
| 21 | 19 | NDC,SVG,AHG, | F,K,R,T,M,D,C,E,N,G,Q,S,V,Y,A,P,H,L,I, |
| 21 | 19 | SHA,NDT,AHG, | F,K,T,R,M,D,C,E,N,G,Q,S,V,Y,A,P,H,L,I, |
| 21 | 19 | NDC,SHA,AHG, | F,K,T,R,M,D,C,E,N,G,Q,S,V,Y,A,P,H,L,I, |
| 21 | 19 | NDT,AHG,SHG, | F,K,T,R,M,D,C,E,N,G,Q,S,V,Y,A,P,H,L,I, |
| 21 | 19 | NDC,AHG,SHG, | F,K,T,R,M,D,C,E,N,G,Q,S,V,Y,A,P,H,L,I, |
| 21 | 19 | VMA,NDT,AHG, | F,K,T,R,M,D,C,E,N,G,Q,S,Y,V,A,P,L,H,I, |
| 21 | 19 | NDC,VMA,AHG, | F,K,T,R,M,D,C,E,N,G,Q,S,V,Y,A,P,H,L,I, |
| 21 | 19 | NDT,AHG,VMG, | F,K,T,R,M,D,C,E,N,G,Q,S,Y,V,A,P,L,H,I, |
| 21 | 19 | NDC,AHG,VMG, | F,K,T,R,M,D,C,E,N,G,Q,S,V,Y,A,P,H,L,I, |
| 21 | 19 | VWG,BNT,AHC, | F,K,T,R,M,C,D,E,N,G,Q,S,Y,V,A,P,H,L,I, |
| 21 | 19 | VWG,BNC,AHC, | F,K,T,R,M,C,D,E,N,G,Q,S,V,Y,A,P,L,H,I, |
| 21 | 19 | AHK,BNT,VAA, | F,K,T,R,M,C,D,E,N,G,Q,S,Y,V,A,P,H,L,I, |
| 21 | 19 | AHK,BNT,VAG, | F,K,T,R,M,C,D,E,N,G,Q,S,Y,V,A,P,H,L,I, |
| 21 | 19 | AHK,BNC,VAA, | F,K,T,R,M,C,D,E,N,G,Q,S,V,Y,A,P,L,H,I, |
| 21 | 19 | AHK,VAG,BNC, | F,K,T,R,M,C,D,E,N,G,Q,S,V,Y,A,P,L,H,I, |
| 21 | 19 | BNT,VAA,AHS, | F,K,T,R,M,C,D,E,N,G,Q,S,Y,V,A,P,H,L,I, |
| 21 | 19 | BNT,VAG,AHS, | F,K,T,R,M,C,D,E,N,G,Q,S,Y,V,A,P,H,L,I, |
| 21 | 19 | BNC,VAA,AHS, | F,K,T,R,M,C,D,E,N,G,Q,S,V,Y,A,P,L,H,I, |
| 21 | 19 | VAG,BNC,AHS, | F,K,T,R,M,C,D,E,N,G,Q,S,V,Y,A,P,L,H,I, |
| 21 | 19 | BDT,VHG,ADT, | F,K,T,R,M,D,C,E,N,G,S,Q,V,Y,A,P,L,H,I, |
| 21 | 19 | BDC,VHG,ADT, | F,K,T,R,M,D,C,E,N,G,S,Q,Y,V,A,P,H,L,I, |
| 21 | 19 | NNT,SAA,ADG, | F,K,R,T,M,D,C,E,N,G,Q,S,V,Y,A,P,H,L,I, |
| 21 | 19 | NNC,SAA,ADG, | F,K,R,T,M,D,C,E,N,G,Q,S,Y,V,A,P,H,L,I, |
| 21 | 19 | NNT,ADG,SAG, | F,K,R,T,M,D,C,E,N,G,Q,S,V,Y,A,P,H,L,I, |

(continued)

| Codons | AA | Degenerate Codons | Amino Acids |
|--------|----|-------------------|-------------|
| 21 | 19 | NNC,ADG,SAG, | F,K,R,T,M,D,C,E,N,G,Q,S,Y,V,A,P,H,L,I, |
| 21 | 19 | VMA,NDT,ADG, | F,K,T,R,M,D,C,E,N,G,Q,S,Y,V,A,P,L,H,I, |
| 21 | 19 | NDC,VMA,ADG, | F,K,T,R,M,D,C,E,N,G,Q,S,V,Y,A,P,H,L,I, |
| 21 | 19 | NDT,ADG,VMG, | F,K,T,R,M,D,C,E,N,G,Q,S,Y,V,A,P,L,H,I, |
| 21 | 19 | NDC,ADG,VMG, | F,K,T,R,M,D,C,E,N,G,Q,S,V,Y,A,P,H,L,I, |
| 21 | 19 | BDT,VHG,ADC, | F,K,T,R,M,D,C,E,N,G,S,Q,V,Y,A,P,L,H,I, |
| 21 | 19 | BDC,VHG,ADC, | F,K,T,R,M,D,C,E,N,G,S,Q,Y,V,A,P,H,L,I, |
| 21 | 19 | VMA,ADK,BDT, | F,K,T,R,M,C,D,E,N,G,Q,S,Y,V,A,P,H,L,I, |
| 21 | 19 | ADK,BDT,VMG, | F,K,T,R,M,C,D,E,N,G,Q,S,Y,V,A,P,H,L,I, |
| 21 | 19 | VMA,BDC,ADK, | F,K,T,R,M,C,D,E,N,G,Q,S,V,Y,A,P,L,H,I, |
| 21 | 19 | BDC,ADK,VMG, | F,K,T,R,M,C,D,E,N,G,Q,S,V,Y,A,P,L,H,I, |
| 21 | 19 | VMA,BDT,ADS, | F,K,T,R,M,C,D,E,N,G,Q,S,Y,V,A,P,H,L,I, |
| 21 | 19 | BDT,VMG,ADS, | F,K,T,R,M,C,D,E,N,G,Q,S,Y,V,A,P,H,L,I, |
| 21 | 19 | VMA,BDC,ADS, | F,K,T,R,M,C,D,E,N,G,Q,S,V,Y,A,P,L,H,I, |
| 21 | 19 | BDC,VMG,ADS, | F,K,T,R,M,C,D,E,N,G,Q,S,V,Y,A,P,L,H,I, |
| 21 | 19 | ANK,SMA,BDT, | F,K,T,R,M,D,C,E,N,G,Q,S,V,Y,A,P,L,H,I, |
| 21 | 19 | ANK,SMA,BDC, | F,K,T,R,M,D,C,E,N,G,Q,S,Y,V,A,P,L,H,I, |
| 21 | 19 | ANK,BDT,SMG, | F,K,T,R,M,D,C,E,N,G,Q,S,V,Y,A,P,L,H,I, |
| 21 | 19 | ANK,BDC,SMG, | F,K,T,R,M,D,C,E,N,G,Q,S,Y,V,A,P,L,H,I, |
| 21 | 19 | SMA,BDT,ANS, | F,K,T,R,M,D,C,E,N,G,Q,S,V,Y,A,P,L,H,I, |
| 21 | 19 | SMA,BDC,ANS, | F,K,T,R,M,D,C,E,N,G,Q,S,Y,V,A,P,L,H,I, |
| 21 | 19 | BDT,SMG,ANS, | F,K,T,R,M,D,C,E,N,G,Q,S,V,Y,A,P,L,H,I, |
| 21 | 19 | BDC,SMG,ANS, | F,K,T,R,M,D,C,E,N,G,Q,S,V,Y,A,P,H,L,I, |
| 21 | 19 | NNT,MWG,TGG, | F,K,T,R,M,D,C,N,G,Q,S,V,Y,A,W,P,H,L,I, |
| 21 | 19 | MWG,NNC,TGG, | F,K,R,T,M,D,C,N,G,Q,S,Y,V,A,W,P,H,L,I, |
| 21 | 19 | NNT,RWG,TGG, | F,K,T,R,M,D,C,E,N,G,S,Y,V,A,W,P,H,L,I, |
| 21 | 19 | NNC,RWG,TGG, | F,K,R,T,M,D,C,E,N,G,S,Y,V,A,W,P,H,L,I, |
| 21 | 19 | NNT,TGK,VAA, | F,K,T,R,C,D,E,N,G,Q,S,V,Y,A,W,P,H,L,I, |
| 21 | 19 | NNC,TGK,VAA, | F,K,R,T,C,D,E,N,G,Q,S,Y,V,A,W,P,H,L,I, |
| 21 | 19 | NNT,VAG,TGK, | F,K,T,R,C,D,E,N,G,Q,S,V,Y,A,W,P,H,L,I, |
| 21 | 19 | VAG,NNC,TGK, | F,K,R,T,C,D,E,N,G,Q,S,Y,V,A,W,P,H,L,I, |
| 21 | 19 | NNT,TGS,VAA, | F,K,T,R,C,D,E,N,G,Q,S,V,Y,A,W,P,H,L,I, |
| 21 | 19 | NNC,TGS,VAA, | F,K,R,T,C,D,E,N,G,Q,S,Y,V,A,W,P,H,L,I, |
| 21 | 19 | NNT,VAG,TGS, | F,K,T,R,C,D,E,N,G,Q,S,V,Y,A,W,P,H,L,I, |
| 21 | 19 | VAG,NNC,TGS, | F,K,R,T,C,D,E,N,G,Q,S,Y,V,A,W,P,H,L,I, |
| 21 | 19 | TGB,RDG,NHT, | F,K,R,T,M,D,C,E,N,G,S,V,Y,A,W,P,L,H,I, |
| 21 | 19 | TGB,RDG,NHC, | F,K,R,T,M,D,C,E,N,G,S,V,Y,A,W,P,L,H,I, |
| 21 | 19 | TGB,VMA,NDT, | F,K,T,R,D,C,E,N,G,Q,S,Y,V,A,W,P,L,H,I, |

(continued)

| Codons | AA | Degenerate Codons | Amino Acids |
|---|---|---|---|
| 21 | 19 | NDC,TGB,VMA, | F,K,T,R,D,C,E,N,G,Q,S,V,Y,A,W,P,H,L,I, |
| 21 | 19 | TGB,NDT,VMG, | F,K,T,R,D,C,E,N,G,Q,S,Y,V,A,W,P,L,H,I, |
| 21 | 19 | NDC,TGB,VMG, | F,K,T,R,D,C,E,N,G,Q,S,V,Y,A,W,P,H,L,I, |
| 21 | 19 | TGB,VRA,NHT, | F,K,R,T,D,C,E,N,G,Q,S,Y,V,A,W,P,L,H,I, |
| 21 | 19 | TGB,VRA,NHC, | F,K,R,T,D,C,E,N,G,Q,S,V,Y,A,W,P,H,L,I, |
| 21 | 19 | TGB,VRG,NHT, | F,K,R,T,D,C,E,N,G,Q,S,Y,V,A,W,P,L,H,I, |
| 21 | 19 | TGB,VRG,NHC, | F,K,R,T,D,C,E,N,G,Q,S,V,Y,A,W,P,H,L,I, |
| 21 | 19 | NNT,TKG,VAA, | F,K,T,R,D,C,E,N,G,Q,S,V,Y,A,W,P,H,L,I, |
| 21 | 19 | NNC,TKG,VAA, | F,K,R,T,D,C,E,N,G,Q,S,Y,V,A,W,P,H,L,I, |
| 21 | 19 | NNT,VAG,TKG, | F,K,T,R,D,C,E,N,G,Q,S,V,Y,A,W,P,H,L,I, |
| 21 | 19 | VAG,NNC,TKG, | F,K,R,T,D,C,E,N,G,Q,S,Y,V,A,W,P,H,L,I, |
| 21 | 19 | VHA,TKK,NRT, | F,K,R,T,D,C,E,N,G,Q,S,Y,V,A,W,P,H,L,I, |
| 21 | 19 | VHA,TKK,NRC, | F,K,R,T,D,C,E,N,G,Q,S,Y,V,A,W,P,H,L,I, |
| 21 | 19 | TKK,VHG,NRT, | F,K,R,T,M,D,C,E,N,G,Q,S,Y,V,A,W,P,H,L, |
| 21 | 19 | TKK,VHG,NRC, | F,K,R,T,M,D,C,E,N,G,Q,S,Y,V,A,W,P,H,L, |
| 21 | 19 | TKK,VDA,NMT, | F,K,T,R,D,C,E,N,G,Q,S,V,Y,A,W,P,H,L,I, |
| 21 | 19 | TKK,VDA,NMC, | F,K,T,R,D,C,E,N,G,Q,S,V,Y,A,W,P,H,L,I, |
| 21 | 19 | TKK,NMT,VDG, | F,K,T,R,M,D,C,E,N,G,Q,S,V,Y,A,W,P,H,L, |
| 21 | 19 | TKK,NMC,VDG, | F,K,T,R,M,D,C,E,N,G,Q,S,V,Y,A,W,P,H,L, |
| 21 | 19 | VHA,TKS,NRT, | F,K,R,T,D,C,E,N,G,Q,S,Y,V,A,W,P,H,L,I, |
| 21 | 19 | VHA,TKS,NRC, | F,K,R,T,D,C,E,N,G,Q,S,Y,V,A,W,P,H,L,I, |
| 21 | 19 | VHG,TKS,NRT, | F,K,R,T,M,D,C,E,N,G,Q,S,Y,V,A,W,P,H,L, |
| 21 | 19 | VHG,TKS,NRC, | F,K,R,T,M,D,C,E,N,G,Q,S,Y,V,A,W,P,H,L, |
| 21 | 19 | VDA,TKS,NMT, | F,K,T,R,D,C,E,N,G,Q,S,V,Y,A,W,P,H,L,I, |
| 21 | 19 | VDA,TKS,NMC, | F,K,T,R,D,C,E,N,G,Q,S,V,Y,A,W,P,H,L,I, |
| 21 | 19 | TKS,NMT,VDG, | F,K,T,R,M,D,C,E,N,G,Q,S,V,Y,A,W,P,H,L, |
| 21 | 19 | TKS,NMC,VDG, | F,K,T,R,M,D,C,E,N,G,Q,S,V,Y,A,W,P,H,L, |
| 21 | 19 | NNT,VAA,TSG, | F,K,T,R,D,C,E,N,G,Q,S,V,Y,A,W,P,H,L,I, |
| 21 | 19 | NNC,VAA,TSG, | F,K,R,T,D,C,E,N,G,Q,S,Y,V,A,W,P,H,L,I, |
| 21 | 19 | NNT,VAG,TSG, | F,K,T,R,D,C,E,N,G,Q,S,V,Y,A,W,P,H,L,I, |
| 21 | 19 | VAG,NNC,TSG, | F,K,R,T,D,C,E,N,G,Q,S,Y,V,A,W,P,H,L,I, |
| 21 | 19 | VVA,TSK,NWT, | F,K,R,T,D,C,E,N,G,Q,S,V,Y,A,W,P,L,H,I, |
| 21 | 19 | VVA,TSK,NWC, | F,K,R,T,D,C,E,N,G,Q,S,Y,V,A,W,P,H,L,I, |
| 21 | 19 | VVG,TSK,NWT, | F,K,R,T,D,C,E,N,G,Q,S,V,Y,A,W,P,L,H,I, |
| 21 | 19 | VVG,TSK,NWC, | F,K,R,T,D,C,E,N,G,Q,S,Y,V,A,W,P,H,L,I, |
| 21 | 19 | TSS,VVA,NWT, | F,K,R,T,D,C,E,N,G,Q,S,V,Y,A,W,P,L,H,I, |
| 21 | 19 | TSS,VVA,NWC, | F,K,R,T,D,C,E,N,G,Q,S,Y,V,A,W,P,H,L,I, |
| 21 | 19 | TSS,VVG,NWT, | F,K,R,T,D,C,E,N,G,Q,S,V,Y,A,W,P,L,H,I, |

(continued)

| Codons | AA | Degenerate Codons | Amino Acids |
|--------|-----|-------------------|-------------|
| 21 | 19 | TSS,VVG,NWC, | F,K,R,T,D,C,E,N,G,Q,S,Y,V,A,W,P,H,L,I, |
| 21 | 19 | VMA,NDT,TBG, | F,K,T,R,D,C,E,N,G,Q,S,Y,V,A,W,P,H,L,I, |
| 21 | 19 | NDC,VMA,TBG, | F,K,T,R,D,C,E,N,G,Q,S,V,Y,A,W,P,H,L,I, |
| 21 | 19 | NDT,TBG,VMG, | F,K,T,R,D,C,E,N,G,Q,S,Y,V,A,W,P,H,L,I, |
| 21 | 19 | NDC,TBG,VMG, | F,K,T,R,D,C,E,N,G,Q,S,V,Y,A,W,P,H,L,I, |
| 21 | 19 | BSG,TDT,VWK, | F,K,R,M,D,C,E,N,G,S,Q,Y,V,A,W,P,L,H,I, |
| 21 | 19 | BSG,VWS,TDT, | F,K,R,M,D,C,E,N,G,S,Q,Y,V,A,W,P,L,H,I, |
| 21 | 19 | VAK,TDT,NBG, | F,K,R,T,M,D,C,E,N,G,Q,S,Y,V,A,W,P,L,H, |
| 21 | 19 | VAM,TDT,NBG, | F,K,R,T,M,D,C,E,N,G,Q,S,Y,V,A,W,P,L,H, |
| 21 | 19 | TDT,NBG,VAS, | F,K,R,T,M,D,C,E,N,G,Q,S,Y,V,A,W,P,L,H, |
| 21 | 19 | TDT,NBG,VAW, | F,K,R,T,M,D,C,E,N,G,Q,S,Y,V,A,W,P,L,H, |
| 21 | 19 | TDT,VST,VWK, | F,K,T,R,M,D,C,E,N,G,S,Q,Y,V,A,P,L,H,I, |
| 21 | 19 | VWS,TDT,VST, | F,K,T,R,M,D,C,E,N,G,S,Q,Y,V,A,P,L,H,I, |
| 21 | 19 | TDT,VSC,VWK, | F,K,R,T ,M,D,C,E,N,G,S,Q,Y,V,A,P,L,H,I, |
| 21 | 19 | VWS,TDT,VSC, | F,K,R,T ,M,D,C,E,N,G,S,Q,Y,V,A,P,L,H,I, |
| 21 | 19 | VWG,TDT,VNT, | F,K,R,T ,M,D,C,E,N,G,Q,S,V,Y,A,P,H,L,I, |
| 21 | 19 | VWG,TDT,VNC, | F,K,T ,R,M,D,C,E,N,G,Q,S,V,Y,A,P,H,L,I, |
| 21 | 19 | DSG,TDT,VWK, | F,K,R,T,M,C,D,E,N,G,S,Q,Y,V,A,W,H,L,I, |
| 21 | 19 | DSG,VWS,TDT, | F,K,R,T,M,C,D,E,N,G,S,Q,Y,V,A,W,H,L,I, |
| 21 | 19 | VDT,TDT,VHG, | F,K,R,T ,M,D,C,E,N,G,Q,S,V,Y,A,P,H,L,I, |
| 21 | 19 | VDC,TDT,VHG, | F,K,R,T ,M,D,C,E,N,G,Q,S,V,Y,A,P,H,L,I, |
| 21 | 19 | BSG,TDC,VWK, | F,K,R,M,D,C,E,N,G,S,Q,Y,V,A,W,P,L,H,I, |
| 21 | 19 | BSG,VWS,TDC, | F,K,R,M,D,C,E,N,G,S,Q,Y,V,A,W,P,L,H,I, |
| 21 | 19 | VAK,NBG,TDC, | F,K,R,T,M,D,C,E,N,G,Q,S,Y,V,A,W,P,L,H, |
| 21 | 19 | VAM,NBG,TDC, | F,K,R,T,M,D,C,E,N,G,Q,S,Y,V,A,W,P,L,H, |
| 21 | 19 | NBG,VAS,TDC, | F,K,R,T,M,D,C,E,N,G,Q,S,Y,V,A,W,P,L,H, |
| 21 | 19 | NBG,TDC,VAW, | F,K,R,T,M,D,C,E,N,G,Q,S,Y,V,A,W,P,L,H, |
| 21 | 19 | VST,TDC,VWK, | F,K,T,R,M,D,C,E,N,G,S,Q,Y,V,A,P,L,H,I, |
| 21 | 19 | VWS,VST,TDC, | F,K,T,R,M,D,C,E,N,G,S,Q,Y,V,A,P,L,H,I, |
| 21 | 19 | VSC,TDC,VWK, | F,K,R,T,M,D,C,E,N,G,S,Q,Y,V,A,P,L,H,I, |
| 21 | 19 | VWS,VSC,TDC, | F,K,R,T,M,D,C,E,N,G,S,Q,Y,V,A,P,L,H,I, |
| 21 | 19 | VWG,VNT,TDC, | F,K,R,T,M,D,C,E,N,G,Q,S,V,Y,A,P,H,L,I, |
| 21 | 19 | VWG,TDC,VNC, | F,K,T,R,M,D,C,E,N,G,Q,S,V,Y,A,P,H,L,I, |
| 21 | 19 | DSG,TDC,VWK, | F,K,R,T,M,C,D,E,N,G,S,Q,Y,V,A,W,H,L,I, |
| 21 | 19 | DSG,VWS,TDC, | F,K,R,T,M,C,D,E,N,G,S,Q,Y,V,A,W,H,L,I, |
| 21 | 19 | VDT,VHG,TDC, | F,K,R,T,M,D,C,E,N,G,Q,S,V,Y,A,P,H,L,I, |
| 21 | 19 | VDC,VHG,TDC, | F,K,R,T,M,D,C,E,N,G,Q,S,V,Y,A,P,H,L,I, |
| 21 | 19 | NNT,MWG,GAA, | F,K,T,R,M,D,C,E,N,G,Q,S,V,Y,A,P,H,L,I, |

(continued)

| Codons | AA | Degenerate Codons | Amino Acids |
|--------|----|--------------------|-------------|
| 21 | 19 | MWG,NNC,GAA, | F,K,R,T,M,D,C,E,N,G,Q,S,Y,V,A,P,H,L,I, |
| 21 | 19 | GAG,NNT,MWG, | F,K,T,R,M,D,C,E,N,G,Q,S,V,Y,A,P,H,L,I, |
| 21 | 19 | GAG,MWG,NNC, | F,K,R,T,M,D,C,E,N,G,Q,S,Y,V,A,P,H,L,I, |
| 21 | 19 | NDT,GVA,MHG, | F,K,R,T,M,D,C,E,N,G,Q,S,Y,V,A,P,H,L,I, |
| 21 | 19 | NDC,GVA,MHG, | F,K,R,T,M,D,C,E,N,G,Q,S,V,Y,A,P,H,L,I, |
| 21 | 19 | VWG,GVT,HNT, | F,K,T,R,M,C,D,E,N,G,Q,S,V,Y,A,P,H,L,I, |
| 21 | 19 | VWG,GVT,HNC, | F,K,R,T,M,C,D,E,N,G,Q,S,V,Y,A,P,H,L,I, |
| 21 | 19 | HDT,VHG,GVT, | F,K,R,T,M,C,D,E,N,G,Q,S,Y,V,A,P,H,L,I, |
| 21 | 19 | HDC,VHG,GVT, | F,K,R,T,M,C,D,E,N,G,Q,S,Y,V,A,P,H,L,I, |
| 21 | 19 | GVG,NDT,MHG, | F,K,R,T,M,D,C,E,N,G,Q,S,Y,V,A,P,H,L,I, |
| 21 | 19 | NDC,GVG,MHG, | F,K,R,T,M,D,C,E,N,G,Q,S,V,Y,A,P,H,L,I, |
| 21 | 19 | VWG,GVC,HNT, | F,K,T,R,M,C,D,E,N,G,Q,S,V,Y,A,P,H,L,I, |
| 21 | 19 | VWG,HNC,GVC, | F,K,T,R,M,C,D,E,N,G,Q,S,V,Y,A,P,H,L,I, |
| 21 | 19 | HDT,VHG,GVC, | F,K,R,T,M,C,D,E,N,G,Q,S,Y,V,A,P,H,L,I, |
| 21 | 19 | HDC,VHG,GVC, | F,K,R,T,M,C,D,E,N,G,Q,S,Y,V,A,P,H,L,I, |
| 21 | 19 | NDT,MHG,GHA, | F,K,T,R,M,D,C,E,N,G,Q,S,V,Y,A,P,H,L,I, |
| 21 | 19 | NDC,MHG,GHA, | F,K,T,R,M,D,C,E,N,G,Q,S,V,Y,A,P,H,L,I, |
| 21 | 19 | NDT,GHG,MHG, | F,K,T,R,M,D,C,E,N,G,Q,S,V,Y,A,P,H,L,I, |
| 21 | 19 | NDC,GHG,MHG, | F,K,T,R,M,D,C,E,N,G,Q,S,V,Y,A,P,H,L,I, |
| 21 | 19 | GDT,HDT,VHG, | F,K,R,T,M,C,D,E,N,G,Q,S,Y,V,A,P,H,L,I, |
| 21 | 19 | GDT,HDC,VHG, | F,K,R,T,M,C,D,E,N,G,Q,S,Y,V,A,P,H,L,I, |
| 21 | 19 | GDC,HDT,VHG, | F,K,R,T,M,C,D,E,N,G,Q,S,Y,V,A,P,H,L,I, |
| 21 | 19 | GDC,HDC,VHG, | F,K,R,T,M,C,D,E,N,G,Q,S,Y,V,A,P,H,L,I, |
| 21 | 19 | NNT,RWG,CAA, | F,K,T,R,M,D,C,E,N,G,Q,S,Y,V,A,P,H,L,I, |
| 21 | 19 | NNC,RWG,CAA, | F,K,R,T,M,D,C,E,N,G,Q,S,Y,V,A,P,H,L,I, |
| 21 | 19 | NNT,CAG,RWG, | F,K,R,T,M,D,C,E,N,G,Q,S,Y,V,A,P,H,L,I, |
| 21 | 19 | NNC,CAG,RWG, | F,K,R,T,M,D,C,E,N,G,Q,S,Y,V,A,P,H,L,I, |
| 21 | 19 | CVA,NDT,RHG, | F,K,T,R,M,D,C,E,N,G,Q,S,V,Y,A,P,L,H,I, |
| 21 | 19 | NDC,CVA,RHG, | F,K,T,R,M,D,C,E,N,G,Q,S,V,Y,A,P,H,L,I, |
| 21 | 19 | VWG,CVT,DNT, | F,K,T,R,M,D,C,E,N,G,Q,S,V,Y,A,P,L,H,I, |
| 21 | 19 | VWG,DNC,CVT, | F,K,T,R,M,D,C,E,N,G,Q,S,V,Y,A,P,L,H,I, |
| 21 | 19 | DDT,CVT,VHG, | F,K,T,R,M,D,C,E,N,G,Q,S,Y,V,A,P,L,H,I, |
| 21 | 19 | DDC,CVT,VHG, | F,K,T,R,M,D,C,E,N,G,Q,S,Y,V,A,P,L,H,I, |
| 21 | 19 | NDT,RHG,CVG, | F,K,T,R,M,D,C,E,N,G,Q,S,V,Y,A,P,L,H,I, |
| 21 | 19 | NDC,RHG,CVG, | F,K,T,R,M,D,C,E,N,G,Q,S,V,Y,A,P,H,L,I, |
| 21 | 19 | VWG,DNT,CVC, | F,K,T,R,M,D,C,E,N,G,Q,S,V,Y,A,P,L,H,I, |
| 21 | 19 | VWG,DNC,CVC, | F,K,T,R,M,D,C,E,N,G,Q,S,V,Y,A,P,L,H,I, |
| 21 | 19 | DDT,VHG,CVC, | F,K,T,R,M,D,C,E,N,G,Q,S,Y,V,A,P,L,H,I, |

(continued)

| Codons | AA | Degenerate Codons | Amino Acids |
|--------|-----|-------------------|-------------|
| 21 | 19 | DDC,VHG,CVC, | F,K,T,R,M,D,C,E,N,G,Q,S,Y,V,A,P,L,H,I, |
| 21 | 19 | NDT,CHA,RHG, | F,K,T,R,M,D,C,E,N,G,Q,S,V,Y,A,P,H,L,I, |
| 21 | 19 | NDC,CHA,RHG, | F,K,T,R,M,D,C,E,N,G,Q,S,V,Y,A,P,H,L,I, |
| 21 | 19 | NDT,CHG,RHG, | F,K,T,R,M,D,C,E,N,G,Q,S,V,Y,A,P,H,L,I, |
| 21 | 19 | NDC,CHG,RHG, | F,K,T,R,M,D,C,E,N,G,Q,S,V,Y,A,P,H,L,I, |
| 21 | 19 | DDT,VHG,CDT, | F,K,T,R,M,D,C,E,N,G,Q,S,Y,V,A,P,H,L,I, |
| 21 | 19 | DDC,VHG,CDT, | F,K,T,R,M,D,C,E,N,G,Q,S,Y,V,A,P,H,L,I, |
| 21 | 19 | DDT,VHG,CDC, | F,K,T,R,M,D,C,E,N,G,Q,S,Y,V,A,P,L,H,I, |
| 21 | 19 | DDC,VHG,CDC, | F,K,T,R,M,D,C,E,N,G,Q,S,Y,V,A,P,L,H,I, |
| 21 | 19 | NNT,VAA,KGG, | F,K,T,R,D,C,E,N,G,Q,S,V,Y,A,W,P,H,L,I, |
| 21 | 19 | NNC,VAA,KGG, | F,K,R,T,D,C,E,N,G,Q,S,Y,V,A,W,P,H,L,I, |
| 21 | 19 | NNT,VAG,KGG, | F,K,T,R,D,C,E,N,G,Q,S,V,Y,A,W,P,H,L,I, |
| 21 | 19 | VAG,NNC,KGG, | F,K,R,T,D,C,E,N,G,Q,S,Y,V,A,W,P,H,L,I, |
| 21 | 19 | MDT,VHG,KDT, | F,K,T,R,M,C,D,E,N,G,S,Q,Y,V,A,P,L,H,I, |
| 21 | 19 | MDC,VHG,KDT, | F,K,T,R,M,C,D,E,N,G,S,Q,Y,V,A,P,H,L,I, |
| 21 | 19 | MDT,VHG,KDC, | F,K,T,R,M,C,D,E,N,G,S,Q,V,Y,A,P,L,H,I, |
| 21 | 19 | MDC,VHG,KDC, | F,K,T,R,M,C,D,E,N,G,S,Q,V,Y,A,P,H,L,I, |
| 21 | 19 | NNT,VAA,MTG, | F,K,T,R,M,D,C,E,N,G,Q,S,V,Y,A,P,H,L,I, |
| 21 | 19 | NNC,VAA,MTG, | F,K,R,T,M,D,C,E,N,G,Q,S,Y,V,A,P,H,L,I, |
| 21 | 19 | NNT,VAG,MTG, | F,K,T,R,M,D,C,E,N,G,Q,S,V,Y,A,P,H,L,I, |
| 21 | 19 | VAG,NNC,MTG, | F,K,R,T,M,D,C,E,N,G,Q,S,Y,V,A,P,H,L,I, |
| 21 | 19 | NNT,VAA,RTG, | F,K,T,R,M,D,C,E,N,G,Q,S,Y,V,A,P,H,L,I, |
| 21 | 19 | NNC,VAA,RTG, | F,K,R,T,M,D,C,E,N,G,Q,S,Y,V,A,P,H,L,I, |
| 21 | 19 | NNT,VAG,RTG, | F,K,T,R,M,D,C,E,N,G,Q,S,Y,V,A,P,H,L,I, |
| 21 | 19 | VAG,NNC,RTG, | F,K,R,T,M,D,C,E,N,G,Q,S,Y,V,A,P,H,L,I, |
| 21 | 19 | VHG,YDT,RDT, | F,K,T,R,M,C,D,E,N,G,S,Q,Y,V,A,P,L,H,I, |
| 21 | 19 | YDC,VHG,RDT, | F,K,T,R,M,C,D,E,N,G,S,Q,Y,V,A,P,H,L,I, |
| 21 | 19 | VHG,YDT,RDC, | F,K,T,R,M,C,D,E,N,G,S,Q,Y,V,A,P,L,H,I, |
| 21 | 19 | YDC,VHG,RDC, | F,K,T,R,M,C,D,E,N,G,S,Q,Y,V,A,P,H,L,I, |
| 21 | 19 | NNT,VAA,YGG, | F,K,R,T,D,C,E,N,G,Q,S,V,Y,A,W,P,H,L,I, |
| 21 | 19 | NNC,VAA,YGG, | F,K,R,T,D,C,E,N,G,Q,S,Y,V,A,W,P,H,L,I, |
| 21 | 19 | NNT,VAG,YGG, | F,K,R,T,D,C,E,N,G,Q,S,V,Y,A,W,P,H,L,I, |
| 21 | 19 | VAG,NNC,YGG, | F,K,R,T,D,C,E,N,G,Q,S,Y,V,A,W,P,H,L,I, |
| 21 | 19 | WNT,SMT,VDG, | F,K,R,T,M,C,D,E,N,G,S,Q,Y,V,A,P,L,H,I, |
| 21 | 19 | WNC,SMT,VDG, | F,K,R,T,M,C,D,E,N,G,S,Q,Y,V,A,P,L,H,I, |
| 21 | 19 | SMC,WNT,VDG, | F,K,R,T,M,C,D,E,N,G,S,Q,Y,V,A,P,L,H,I, |
| 21 | 19 | SMC,WNC,VDG, | F,K,R,T,M,C,D,E,N,G,S,Q,Y,V,A,P,L,H,I, |
| 21 | 19 | VHG,WNT,SRT, | F,K,T,R,M,C,D,E,N,G,S,Q,Y,V,A,P,L,H,I, |

(continued)

| Codons | AA | Degenerate Codons | Amino Acids |
|--------|-----|-------------------|-------------|
| 21 | 19 | VHG,WNC,SRT, | F,K,T,R,M,C,D,E,N,G,S,Q,Y,V,A,P,L,H,I, |
| 21 | 19 | VHG,WNT,SRC, | F,K,T,R,M,C,D,E,N,G,S,Q,Y,V,A,P,L,H,I, |
| 21 | 19 | VHG,WNC,SRC, | F,K,T,R,M,C,D,E,N,G,S,Q,Y,V,A,P,L,H,I, |
| 21 | 19 | SVT,VHG,WDT, | F,K,T,R,M,C,D,E,N,G,S,Q,V,Y,A,P,L,H,I, |
| 21 | 19 | SVT,VHG,WDC, | F,K,T,R,M,C,D,E,N,G,S,Q,V,Y,A,P,L,H,I, |
| 21 | 19 | SVC,VHG,WDT, | F,K,T,R,M,C,D,E,N,G,S,Q,V,Y,A,P,L,H,I, |
| 21 | 19 | SVC,VHG,WDC, | F,K,T,R,M,C,D,E,N,G,S,Q,V,Y,A,P,L,H,I, |
| 21 | 19 | SDT,VHG,WDT, | F,K,T,R,M,C,D,E,N,G,S,Q,Y,V,A,P,H,L,I, |
| 21 | 19 | SDT,VHG,WDC, | F,K,T,R,M,C,D,E,N,G,S,Q,Y,V,A,P,H,L,I, |
| 21 | 19 | SDC,VHG,WDT, | F,K,T,R,M,C,D,E,N,G,S,Q,Y,V,A,P,L,H,I, |
| 21 | 19 | SDC,VHG,WDC, | F,K,T,R,M,C,D,E,N,G,S,Q,Y,V,A,P,L,H,I, |
| 21 | 19 | NNT,V AA,WTG, | F,K,T,R,M,D,C,E,N,G,Q,S,V,Y,A,P,H,L,I, |
| 21 | 19 | NNC,VAA,WTG, | F,K,R,T,M,D,C,E,N,G,Q,S,Y,V,A,P,H,L,I, |
| 21 | 19 | NNT,V AG,WTG, | F,K,T,R,M,D,C,E,N,G,Q,S,V,Y,A,P,H,L,I, |
| 21 | 19 | VAG,NNC,WTG, | F,K,R,T,M,D,C,E,N,G,Q,S,Y,V,A,P,H,L,I, |
| 21 | 19 | VHA,WTK,NRT, | F,K,R,T,M,D,C,E,N,G,Q,S,V,Y,A,P,H,L,I, |
| 21 | 19 | VHA,WTK,NRC, | F,K,R,T,M,D,C,E,N,G,Q,S,V,Y,A,P,H,L,I, |
| 21 | 19 | WTK,VHG,NRT, | F,K,R,T,M,D,C,E,N,G,Q,S,V,Y,A,P,H,L,I, |
| 21 | 19 | WTK,VHG,NRC, | F,K,R,T,M,D,C,E,N,G,Q,S,V,Y,A,P,H,L,I, |
| 21 | 19 | WTM,VHG,NRT, | F,K,R,T,M,D,C,E,N,G,Q,S,V,Y,A,P,H,L,I, |
| 21 | 19 | WTM,VHG,NRC, | F,K,R,T,M,D,C,E,N,G,Q,S,V,Y,A,P,H,L,I, |
| 21 | 19 | VHG,WTY,NRT, | F,K,R,T,M,D,C,E,N,G,Q,S,V,Y,A,P,H,L,I, |
| 21 | 19 | VHG,WTY,NRC, | F,K,R,T,M,D,C,E,N,G,Q,S,V,Y,A,P,H,L,I, |
| 21 | 19 | WTS,VHA,NRT, | F,K,R,T,M,D,C,E,N,G,Q,S,V,Y,A,P,H,L,I, |
| 21 | 19 | WTS,VHA,NRC, | F,K,R,T,M,D,C,E,N,G,Q,S,V,Y,A,P,H,L,I, |
| 21 | 19 | WTS,VHG,NRT, | F,K,R,T,M,D,C,E,N,G,Q,S,V,Y,A,P,H,L,I, |
| 21 | 19 | WTS,VHG,NRC, | F,K,R,T,M,D,C,E,N,G,Q,S,V,Y,A,P,H,L,I, |
| 21 | 19 | VHG,NRT,WTW, | F,K,R,T,M,D,C,E,N,G,Q,S,V,Y,A,P,H,L,I, |
| 21 | 19 | VHG,WTW,NRC, | F,K,R,T,M,D,C,E,N,G,Q,S,V,Y,A,P,H,L,I, |
| 21 | 19 | NNT,VAA,WGG, | F,K,R,T,D,C,E,N,G,Q,S,V,Y,A,W,P,H,L,I, |
| 21 | 19 | NNC,VAA,WGG, | F,K,R,T,D,C,E,N,G,Q,S,Y,V,A,W,P,H,L,I, |
| 21 | 19 | NNT,VAG,WGG, | F,K,R,T,D,C,E,N,G,Q,S,V,Y,A,W,P,H,L,I, |
| 21 | 19 | VAG,NNC,WGG, | F,K,R,T,D,C,E,N,G,Q,S,Y,V,A,W,P,H,L,I, |
| 21 | 19 | WGK,VVA,NWT, | F,K,T,R,D,C,E,N,G,Q,S,V,Y,A,W,P,L,H,I, |
| 21 | 19 | WGK,VVA,NWC, | F,K,T,R,D,C,E,N,G,Q,S,Y,V,A,W,P,H,L,I, |
| 21 | 19 | VVG,WGK,NWT, | F,K,T,R,D,C,E,N,G,Q,S,V,Y,A,W,P,L,H,I, |
| 21 | 19 | VVG,WGK,NWC, | F,K,T,R,D,C,E,N,G,Q,S,Y,V,A,W,P,H,L,I, |
| 21 | 19 | WGK,VHG,NWT, | F,K,R,T,M,D,C,E,N,S,Q,Y,V,A,W,P,H,L,I, |

(continued)

| Codons | AA | Degenerate Codons | Amino Acids |
|---|---|---|---|
| 21 | 19 | WGK,VHG,NWC, | F,K,R,T,M,D,C,E,N,S,Q,Y,V,A,W,P,H,L,I, |
| 21 | 19 | WGS,VVA,NWT, | F,K,T,R,D,C,E,N,G,Q,S,V,Y,A,W,P,L,H,I, |
| 21 | 19 | WGS,VVA,NWC, | F,K,T,R,D,C,E,N,G,Q,S,Y,V,A,W,P,H,L,I, |
| 21 | 19 | WGS,VVG,NWT, | F,K,T,R,D,C,E,N,G,Q,S,V,Y,A,W,P,L,H,I, |
| 21 | 19 | WGS,VVG,NWC, | F,K,T,R,D,C,E,N,G,Q,S,Y,V,A,W,P,H,L,I, |
| 21 | 19 | WGS,VHG,NWT, | F,K,R,T,M,D,C,E,N,S,Q,Y,V,A,W,P,H,L,I, |
| 21 | 19 | WGS,VHG,NWC, | F,K,R,T,M,D,C,E,N,S,Q,Y,V,A,W,P,H,L,I, |
| 21 | 19 | WKT,VHG,NRT, | F,K,R,T,M,D,C,E,N,G,S,Q,Y,V,A,P,H,L,I, |
| 21 | 19 | WKT,VHG,NRC, | F,K,R,T,M,D,C,E,N,G,S,Q,Y,V,A,P,H,L,I, |
| 21 | 19 | WKT,NMT,VDG, | F,K,T,R,M,D,C,E,N,G,Q,S,V,Y,A,P,H,L,I, |
| 21 | 19 | WKT,NMC,VDG, | F,K,T,R,M,D,C,E,N,G,Q,S,V,Y,A,P,H,L,I, |
| 21 | 19 | VHA,WKG,NRT, | K,T,R,M,D,C,E,N,G,Q,S,Y,V,A,W,P,H,L,I, |
| 21 | 19 | VHA,WKG,NRC, | K,T,R,M,D,C,E,N,G,Q,S,Y,V,A,W,P,H,L,I, |
| 21 | 19 | VHG,WKC,NRT, | F,K,R,T,M,D,C,E,N,G,S,Q,Y,V,A,P,H,L,I, |
| 21 | 19 | VHG,WKC,NRC, | F,K,R,T,M,D,C,E,N,G,S,Q,Y,V,A,P,H,L,I, |
| 21 | 19 | WKC,NMT,VDG, | F,K,T,R,M,D,C,E,N,G,Q,S,V,Y,A,P,H,L,I, |
| 21 | 19 | WKC,NMC,VDG, | F,K,T,R,M,D,C,E,N,G,Q,S,V,Y,A,P,H,L,I, |
| 21 | 19 | WKK,VVA,NAT, | F,K,R,T,M,D,C,E,N,G,S,Q,Y,A,W,P,H,L,I, |
| 21 | 19 | WKK,NAC,VVA, | F,K,R,T,M,D,C,E,N,G,S,Q,Y,A,W,P,H,L,I, |
| 21 | 19 | VVG,WKK,NAT, | F,K,R,T,M,D,C,E,N,G,S,Q,Y,A,W,P,H,L,I, |
| 21 | 19 | VVG,WKK,NAC, | F,K,R,T,M,D,C,E,N,G,S,Q,Y,A,W,P,H,L,I, |
| 21 | 19 | VHA,WKK,NAT, | F,K,R,T,M,D,C,E,N,S,Q,Y,V,A,W,P,H,L,I, |
| 21 | 19 | VHA,WKK,NAC, | F,K,R,T,M,D,C,E,N,S,Q,Y,V,A,W,P,H,L,I, |
| 21 | 19 | WKK,NAT,VHG, | F,K,R,T,M,D,C,E,N,S,Q,Y,V,A,W,P,H,L,I, |
| 21 | 19 | WKK,NAC,VHG, | F,K,R,T,M,D,C,E,N,S,Q,Y,V,A,W,P,H,L,I, |
| 21 | 19 | VVA,NAT,WKS, | F,K,R,T,M,D,C,E,N,G,S,Q,Y,A,W,P,H,L,I, |
| 21 | 19 | NAC,VVA,WKS, | F,K,R,T,M,D,C,E,N,G,S,Q,Y,A,W,P,H,L,I, |
| 21 | 19 | VVG,NAT,WKS, | F,K,R,T,M,D,C,E,N,G,S,Q,Y,A,W,P,H,L,I, |
| 21 | 19 | VVG,NAC,WKS, | F,K,R,T,M,D,C,E,N,G,S,Q,Y,A,W,P,H,L,I, |
| 21 | 19 | VHA,NAT,WKS, | F,K,R,T,M,D,C,E,N,S,Q,Y,V,A,W,P,H,L,I, |
| 21 | 19 | VHA,NAC,WKS, | F,K,R,T,M,D,C,E,N,S,Q,Y,V,A,W,P,H,L,I, |
| 21 | 19 | NAT,WKS,VHG, | F,K,R,T,M,D,C,E,N,S,Q,Y,V,A,W,P,H,L,I, |
| 21 | 19 | NAC,WKS,VHG, | F,K,R,T,M,D,C,E,N,S,Q,Y,V,A,W,P,H,L,I, |
| 21 | 19 | VHG,NRT,WYT, | F,K,R,T,M,D,C,E,N,G,Q,S,V,Y,A,P,H,L,I, |
| 21 | 19 | VHG,NRC,WYT, | F,K,R,T,M,D,C,E,N,G,Q,S,V,Y,A,P,H,L,I, |
| 21 | 19 | VHG,NRT,WYC, | F,K,R,T,M,D,C,E,N,G,Q,S,V,Y,A,P,H,L,I, |
| 21 | 19 | VHG,WYC,NRC, | F,K,R,T,M,D,C,E,N,G,Q,S,V,Y,A,P,H,L,I, |
| 21 | 19 | WWT,VHG,NRT, | F,K,R,T,M,D,C,E,N,G,S,Q,Y,V,A,P,H,L,I, |

(continued)

| Codons | AA | Degenerate Codons | Amino Acids |
|--------|----|--------------------|-------------|
| 21 | 19 | WWT,VHG,NRC, | F,K,R,T,M,D,C,E,N,G,S,Q,Y,V,A,P,H,L,I, |
| 21 | 19 | WWC,VHG,NRT, | F,K,R,T,M,D,C,E,N,G,S,Q,Y,V,A,P,H,L,I, |
| 21 | 19 | WWC,VHG,NRC, | F,K,R,T,M,D,C,E,N,G,S,Q,Y,V,A,P,H,L,I, |
| 21 | 19 | VMA,DDT,WBG, | F,K,T,R,M,D,C,E,N,G,Q,S,V,Y,A,W,P,L,I, |
| 21 | 19 | DDC,VMA,WBG, | F,K,T,R,M,D,C,E,N,G,Q,S,Y,V,A,W,P,L,I, |
| 21 | 19 | DDT,WBG,VMG, | F,K,T,R,M,D,C,E,N,G,Q,S,V,Y,A,W,P,L,I, |
| 21 | 19 | DDC,WBG,VMG, | F,K,T,R,M,D,C,E,N,G,Q,S,Y,V,A,W,P,L,I, |
| 21 | 19 | BRT,WHT,VHG, | F,K,R,T,M,D,C,E,N,G,S,Q,Y,V,A,P,L,H,I, |
| 21 | 19 | WHT,VHG,BRC, | F,K,R,T,M,D,C,E,N,G,S,Q,Y,V,A,P,L,H,I, |
| 21 | 19 | VWG,WHT,BVT, | F,K,T,R,M,C,D,E,N,G,Q,S,V,Y,A,P,L,H,I, |
| 21 | 19 | VWG,WHT,BVC, | F,K,T,R,M,C,D,E,N,G,Q,S,V,Y,A,P,L,H,I, |
| 21 | 19 | WHC,BRT,VHG, | F,K,R,T,M,D,C,E,N,G,S,Q,Y,V,A,P,L,H,I, |
| 21 | 19 | WHC,VHG,BRC, | F,K,R,T,M,D,C,E,N,G,S,Q,Y,V,A,P,L,H,I, |
| 21 | 19 | WHC,VWG,BVT, | F,K,T,R,M,C,D,E,N,G,Q,S,V,Y,A,P,L,H,I, |
| 21 | 19 | WHC,VWG,BVC, | F,K,T,R,M,C,D,E,N,G,Q,S,V,Y,A,P,L,H,I, |
| 21 | 19 | BAT,VNG,WDT, | F,K,R,T,M,D,C,E,N,G,S,Q,Y,V,A,P,L,H,I, |
| 21 | 19 | BAC,VNG,WDT, | F,K,R,T,M,D,C,E,N,G,S,Q,Y,V,A,P,L,H,I, |
| 21 | 19 | BRT,VHG,WDT, | F,K,T,R,M,D,C,E,N,G,S,Q,Y,V,A,P,L,H,I, |
| 21 | 19 | VHG,WDT,BRC, | F,K,T ,R,M,D,C,E,N,G,S,Q,Y,V,A,P,L,H,I, |
| 21 | 19 | VAT,VNG,WDT, | F,K,R,T,M,D,C,E,N,G,S,Q,Y,V,A,P,L,H,I, |
| 21 | 19 | VAC,VNG,WDT, | F,K,R,T,M,D,C,E,N,G,S,Q,Y,V,A,P,L,H,I, |
| 21 | 19 | VAK,VBG,WDT, | F,K,R,T,M,D,C,E,N,G,Q,S,Y,V,A,P,L,H,I, |
| 21 | 19 | VAK,WDT,DBG, | F,K,R,T,M,D,C,E,N,G,Q,S,Y,V,A,W,L,H,I, |
| 21 | 19 | VAM,VBG,WDT, | F,K,R,T,M,D,C,E,N,G,Q,S,Y,V,A,P,L,H,I, |
| 21 | 19 | VAM,WDT,DBG, | F,K,R,T,M,D,C,E,N,G,Q,S,Y,V,A,W,L,H,I, |
| 21 | 19 | VBG,VAS,WDT, | F,K,R,T,M,D,C,E,N,G,Q,S,Y,V,A,P,L,H,I, |
| 21 | 19 | VAS,WDT,DBG, | F,K,R,T,M,D,C,E,N,G,Q,S,Y,V,A,W,L,H,I, |
| 21 | 19 | VBG,WDT,VAW, | F,K,R,T,M,D,C,E,N,G,Q,S,Y,V,A,P,L,H,I, |
| 21 | 19 | WDT,DBG,VAW, | F,K,R,T,M,D,C,E,N,G,Q,S,Y,V,A,W,L,H,I, |
| 21 | 19 | VMT,WDT,VDG, | F,K,R,T,M,D,C,E,N,G,S,Q,Y,V,A,P,L,H,I, |
| 21 | 19 | VMC,WDT,VDG, | F,K,R,T,M,D,C,E,N,G,S,Q,Y,V,A,P,L,H,I, |
| 21 | 19 | VRT,VHG,WDT, | F,K,T,R,M,D,C,E,N,G,S,Q,Y,V,A,P,L,H,I, |
| 21 | 19 | VRC,VHG,WDT, | F,K,T,R,M,D,C,E,N,G,S,Q,Y,V,A,P,L,H,I, |
| 21 | 19 | VWG,VVT,WDT, | F,K,T,R,M,D,C,E,N,G,Q,S,V,Y,A,P,H,L,I, |
| 21 | 19 | VWG,VVC,WDT, | F,K,R,T,M,D,C,E,N,G,Q,S,V,Y,A,P,H,L,I, |
| 21 | 19 | BAT,VNG,WDC, | F,K,R,T,M,D,C,E,N,G,S,Q,Y,V,A,P,L,H,I, |
| 21 | 19 | BAC,VNG,WDC, | F,K,R,T,M,D,C,E,N,G,S,Q,Y,V,A,P,L,H,I, |
| 21 | 19 | BRT,VHG,WDC, | F,K,T,R,M,D,C,E,N,G,S,Q,Y,V,A,P,L,H,I, |

(continued)

| Codons | AA | Degenerate Codons | Amino Acids |
|---|---|---|---|
| 21 | 19 | VHG,WDC,BRC, | F,K,T,R,M,D,C,E,N,G,S,Q,Y,V,A,P,L,H,I, |
| 21 | 19 | VAT,VNG,WDC, | F,K,R,T,M,D,C,E,N,G,S,Q,Y,V,A,P,L,H,I, |
| 21 | 19 | VAC,VNG,WDC, | F,K,R,T,M,D,C,E,N,G,S,Q,Y,V,A,P,L,H,I, |
| 21 | 19 | VAK,VBG,WDC, | F,K,R,T,M,D,C,E,N,G,Q,S,Y,V,A,P,L,H,I, |
| 21 | 19 | VAK,WDC,DBG, | F,K,R,T,M,D,C,E,N,G,Q,S,Y,V,A,W,L,H,I, |
| 21 | 19 | VAM,VBG,WDC, | F,K,R,T,M,D,C,E,N,G,Q,S,Y,V,A,P,L,H,I, |
| 21 | 19 | VAM,WDC,DBG, | F,K,R,T,M,D,C,E,N,G,Q,S,Y,V,A,W,L,H,I, |
| 21 | 19 | VBG,VAS,WDC, | F,K,R,T,M,D,C,E,N,G,Q,S,Y,V,A,P,L,H,I, |
| 21 | 19 | VAS,WDC,DBG, | F,K,R,T,M,D,C,E,N,G,Q,S,Y,V,A,W,L,H,I, |
| 21 | 19 | VBG,WDC,VAW, | F,K,R,T,M,D,C,E,N,G,Q,S,Y,V,A,P,L,H,I, |
| 21 | 19 | WDC,DBG,VAW, | F,K,R,T,M,D,C,E,N,G,Q,S,Y,V,A,W,L,H,I, |
| 21 | 19 | VMT,WDC,VDG, | F,K,R,T,M,D,C,E,N,G,S,Q,Y,V,A,P,L,H,I, |
| 21 | 19 | VMC,WDC,VDG, | F,K,R,T,M,D,C,E,N,G,S,Q,Y,V,A,P,L,H,I, |
| 21 | 19 | VRT,VHG,WDC, | F,K,T,R,M,D,C,E,N,G,S,Q,Y,V,A,P,L,H,I, |
| 21 | 19 | VRC,VHG,WDC, | F,K,T,R,M,D,C,E,N,G,S,Q,Y,V,A,P,L,H,I, |
| 21 | 19 | VWG,VVT,WDC, | F,K,T,R,M,D,C,E,N,G,Q,S,V,Y,A,P,H,L,I, |
| 21 | 19 | VWG,VVC,WDC, | F,K,R,T,M,D,C,E,N,G,Q,S,V,Y,A,P,H,L,I, |
| 21 | 19 | VMA,BAT,DKK, | F,K,R,T,M,C,D,E,G,Q,S,Y,V,A,W,P,L,H,I, |
| 21 | 19 | VMA,DKS,BAT, | F,K,R,T,M,C,D,E,G,Q,S,Y,V,A,W,P,L,H,I, |
| 21 | 19 | BAT,VMG,DKK, | F,K,R,T,M,C,D,E,G,Q,S,Y,V,A,W,P,L,H,I, |
| 21 | 19 | DKS,BAT,VMG, | F,K,R,T,M,C,D,E,G,Q,S,Y,V,A,W,P,L,H,I, |
| 21 | 19 | VMA,BAC,DKK, | F,K,R,T,M,C,D,E,G,Q,S,Y,V,A,W,P,L,H,I, |
| 21 | 19 | VMA,DKS,BAC, | F,K,R,T,M,C,D,E,G,Q,S,Y,V,A,W,P,L,H,I, |
| 21 | 19 | BAC,VMG,DKK, | F,K,R,T,M,C,D,E,G,Q,S,Y,V,A,W,P,L,H,I, |
| 21 | 19 | DKS,BAC,VMG, | F,K,R,T,M,C,D,E,G,Q,S,Y,V,A,W,P,L,H,I, |
| 21 | 19 | VWG,BGT,NHT, | F,K,T,R,M,D,C,E,N,G,Q,S,V,Y,A,P,H,L,I, |
| 21 | 19 | VWG,BGT,NHC, | F,K,T,R,M,D,C,E,N,G,Q,S,V,Y,A,P,H,L,I, |
| 21 | 19 | VMA,NDT,BGG, | F,K,T,R,D,C,E,N,G,Q,S,Y,V,A,W,P,L,H,I, |
| 21 | 19 | NDC,VMA,BGG, | F,K,T,R,D,C,E,N,G,Q,S,V,Y,A,W,P,H,L,I, |
| 21 | 19 | NDT,BGG,VMG, | F,K,T,R,D,C,E,N,G,Q,S,Y,V,A,W,P,L,H,I, |
| 21 | 19 | NDC,BGG,VMG, | F,K,T,R,D,C,E,N,G,Q,S,V,Y,A,W,P,H,L,I, |
| 21 | 19 | VWG,BGG,NHT, | F,K,T,R,M,D,E,N,G,Q,S,V,Y,A,W,P,H,L,I, |
| 21 | 19 | VWG,BGG,NHC, | F,K,T,R,M,D,E,N,G,Q,S,V,Y,A,W,P,H,L,I, |
| 21 | 19 | HDT,BGG,VHG, | F,K,T,R,M,C,E,N,G,Q,S,Y,V,A,W,P,H,L,I, |
| 21 | 19 | HDC,BGG,VHG, | F,K,T,R,M,C,E,N,G,S,Q,Y,V,A,W,P,H,L,I, |
| 21 | 19 | DDT,BGG,VHG, | F,K,T,R,M,D,C,E,N,G,Q,S,Y,V,A,W,P,L,I, |
| 21 | 19 | DDC,BGG,VHG, | F,K,T,R,M,D,C,E,N,G,S,Q,Y,V,A,W,P,L,I, |
| 21 | 19 | VWG,BGC,NHT, | F,K,T,R,M,D,C,E,N,G,Q,S,V,Y,A,P,H,L,I, |

**40**

(continued)

| Codons | AA | Degenerate Codons | Amino Acids |
|--------|-----|-------------------|-------------|
| 21 | 19 | VWG,BGC,NHC, | F,K,T,R,M,D,C,E,N,G,Q,S,V,Y,A,P,H,L,I, |
| 21 | 19 | VAA,BGK,NHT, | F,K,R,T,D,C,E,N,G,Q,S,Y,V,A,W,P,L,H,I, |
| 21 | 19 | VAA,BGK,NHC, | F,K,R,T,D,C,E,N,G,Q,S,V,Y,A,W,P,H,L,I, |
| 21 | 19 | VAG,BGK,NHT, | F,K,R,T,D,C,E,N,G,Q,S,Y,V,A,W,P,L,H,I, |
| 21 | 19 | VAG,BGK,NHC, | F,K,R,T,D,C,E,N,G,Q,S,V,Y,A,W,P,H,L,I, |
| 21 | 19 | BGS,VAA,NHT, | F,K,R,T,D,C,E,N,G,Q,S,Y,V,A,W,P,L,H,I, |
| 21 | 19 | BGS,VAA,NHC, | F,K,R,T,D,C,E,N,G,Q,S,V,Y,A,W,P,L,H,I, |
| 21 | 19 | VAG,BGS,NHT, | F,K,R,T,D,C,E,N,G,Q,S,Y,V,A,W,P,L,H,I, |
| 21 | 19 | VAG,BGS,NHC, | F,K,R,T,D,C,E,N,G,Q,S,V,Y,A,W,P,L,H,I, |
| 21 | 19 | VMT,VAA,DKK, | F,K,R,T,M,C,D,E,N,G,Q,S,V,A,W,P,L,H,I, |
| 21 | 19 | DKS,VMT,VAA, | F,K,R,T,M,C,D,E,N,G,Q,S,V,A,W,P,L,H,I, |
| 21 | 19 | VMC,VAA,DKK, | F,K,R,T,M,C,D,E,N,G,Q,S,V,A,W,P,L,H,I, |
| 21 | 19 | DKS,VMC,VAA, | F,K,R,T,M,C,D,E,N,G,Q,S,V,A,W,P,L,H,I, |
| 21 | 19 | NDT,VYG,VAA, | F,K,T,R,M,D,C,E,N,G,Q,S,V,Y,A,P,H,L,I, |
| 21 | 19 | NDC,VYG,VAA, | F,K,T,R,M,D,C,E,N,G,Q,S,V,Y,A,P,H,L,I, |
| 21 | 19 | DTK,NVT,VAA, | F,K,R,T,M,D,C,E,N,G,Q,S,V,Y,A,P,H,L,I, |
| 21 | 19 | NVC,DTK,VAA, | F,K,T,R,M,D,C,E,N,G,Q,S,V,Y,A,P,H,L,I, |
| 21 | 19 | NVT,VAA,DTS, | F,K,R,T,M,D,C,E,N,G,Q,S,V,Y,A,P,H,L,I, |
| 21 | 19 | NVC,VAA,DTS, | F,K,T,R,M,D,C,E,N,G,Q,S,V,Y,A,P,H,L,I, |
| 21 | 19 | VAA,DGK,NHT, | F,K,R,T,D,C,E,N,G,S,Q,Y,V,A,W,P,L,H,I, |
| 21 | 19 | VAA,DGK,NHC, | F,K,R,T,D,C,E,N,G,S,Q,V,Y,A,W,P,L,H,I, |
| 21 | 19 | DGS,VAA,NHT, | F,K,R,T,D,C,E,N,G,S,Q,Y,V,A,W,P,L,H,I, |
| 21 | 19 | DGS,VAA,NHC, | F,K,R,T ,D,C,E,N,G,S,Q,V,Y,A,W,P,H,L,I, |
| 21 | 19 | VAA,DKG,NHT, | F,K,T,R,M,D,E,N,G,Q,S,V,Y,A,W,P,H,L,I, |
| 21 | 19 | VAA,DKG,NHC, | F,K,T,R,M,D,E,N,G,Q,S,V,Y,A,W,P,H,L,I, |
| 21 | 19 | VMA,VAT,DKK, | F,K,R,T,M,C,D,E,N,G,Q,S,V,A,W,P,L,H,I, |
| 21 | 19 | VMA,DKS,VAT, | F,K,R,T,M,C,D,E,N,G,Q,S,V,A,W,P,L,H,I, |
| 21 | 19 | VAT,VMG,DKK, | F,K,R,T,M,C,D,E,N,G,Q,S,V,A,W,P,L,H,I, |
| 21 | 19 | DKS,VAT,VMG, | F,K,R,T,M,C,D,E,N,G,Q,S,V,A,W,P,L,H,I, |
| 21 | 19 | VAG,VMT,DKK, | F,K,R,T,M,C,D,E,N,G,Q,S,V,A,W,P,L,H,I, |
| 21 | 19 | VAG,DKS,VMT, | F,K,R,T,M,C,D,E,N,G,Q,S,V,A,W,P,L,H,I, |
| 21 | 19 | VAG,VMC,DKK, | F,K,R,T,M,C,D,E,N,G,Q,S,V,A,W,P,L,H,I, |
| 21 | 19 | VAG,DKS,VMC, | F,K,R,T,M,C,D,E,N,G,Q,S,V,A,W,P,L,H,I, |
| 21 | 19 | NDT,VAG,VYG, | F,K,T,R,M,D,C,E,N,G,Q,S,V,Y,A,P,H,L,I, |
| 21 | 19 | NDC,VAG,VYG, | F,K,T,R,M,D,C,E,N,G,Q,S,V,Y,A,P,H,L,I, |
| 21 | 19 | DTK,VAG,NVT, | F,K,R,T,M,D,C,E,N,G,Q,S,V,Y,A,P,H,L,I, |
| 21 | 19 | NVC,DTK,VAG, | F,K,T,R,M,D,C,E,N,G,Q,S,V,Y,A,P,H,L,I, |
| 21 | 19 | VAG,NVT,DTS, | F,K,R,T,M,D,C,E,N,G,Q,S,V,Y,A,P,H,L,I, |

(continued)

| Codons | AA | Degenerate Codons | Amino Acids |
|---|---|---|---|
| 21 | 19 | NVC,VAG,DTS, | F,K,T,R,M,D,C,E,N,G,Q,S,V,Y,A,P,H,L,I, |
| 21 | 19 | VAG,DGK,NHT, | F,K,R,T,D,C,E,N,G,S,Q,Y,V,A,W,P,L,H,I, |
| 21 | 19 | VAG,DGK,NHC, | F,K,R,T,D,C,E,N,G,S,Q,V,Y,A,W,P,H,L,I, |
| 21 | 19 | DGS,VAG,NHT, | F,K,R,T,D,C,E,N,G,S,Q,Y,V,A,W,P,L,H,I, |
| 21 | 19 | DGS,VAG,NHC, | F,K,R,T,D,C,E,N,G,S,Q,V,Y,A,W,P,H,L,I, |
| 21 | 19 | VAG,DKG,NHT, | F,K,T,R,M,D,E,N,G,Q,S,V,Y,A,W,P,H,L,I, |
| 21 | 19 | VAG,DKG,NHC, | F,K,T,R,M,D,E,N,G,Q,S,V,Y,A,W,P,H,L,I, |
| 21 | 19 | VMA,VAC,DKK, | F,K,R,T,M,C,D,E,N,G,Q,S,V,A,W,P,L,H,I, |
| 21 | 19 | VMA,DKS,VAC, | F,K,R,T,M,C,D,E,N,G,Q,S,V,A,W,P,L,H,I, |
| 21 | 19 | VAC,VMG,DKK, | F,K,R,T,M,C,D,E,N,G,Q,S,V,A,W,P,L,H,I, |
| 21 | 19 | DKS,VAC,VMG, | F,K,R,T,M,C,D,E,N,G,Q,S,V,A,W,P,L,H,I, |
| 21 | 19 | VAK,VCA,DKK, | F,K,R,T,M,C,D,E,N,G,Q,S,V,A,W,P,L,H,I, |
| 21 | 19 | VAK,VCA,DKS, | F,K,R,T,M,C,D,E,N,G,Q,S,V,A,W,P,L,H,I, |
| 21 | 19 | VCT,VAK,DKK, | F,K,R,T,M,C,D,E,N,G,Q,S,V,A,W,P,L,H,I, |
| 21 | 19 | VCT,VAK,DKS, | F,K,R,T,M,C,D,E,N,G,Q,S,V,A,W,P,L,H,I, |
| 21 | 19 | VAK,VCG,DKK, | F,K,R,T,M,C,D,E,N,G,Q,S,V,A,W,P,L,H,I, |
| 21 | 19 | VAK,DKS,VCG, | F,K,R,T,M,C,D,E,N,G,Q,S,V,A,W,P,L,H,I, |
| 21 | 19 | VAK,VCC,DKK, | F,K,R,T,M,C,D,E,N,G,Q,S,V,A,W,P,L,H,I, |
| 21 | 19 | VAK,DKS,VCC, | F,K,R,T,M,C,D,E,N,G,Q,S,V,A,W,P,L,H,I, |
| 21 | 19 | VAM,VCA,DKK, | F,K,R,T,M,C,D,E,N,G,Q,S,V,A,W,P,L,H,I, |
| 21 | 19 | VAM,VCA,DKS, | F,K,R,T,M,C,D,E,N,G,Q,S,V,A,W,P,L,H,I, |
| 21 | 19 | VCT,VAM,DKK, | F,K,R,T,M,C,D,E,N,G,Q,S,V,A,W,P,L,H,I, |
| 21 | 19 | VCT,VAM,DKS, | F,K,R,T,M,C,D,E,N,G,Q,S,V,A,W,P,L,H,I, |
| 21 | 19 | VAM,VCG,DKK, | F,K,R,T,M,C,D,E,N,G,Q,S,V,A,W,P,L,H,I, |
| 21 | 19 | VAM,DKS,VCG, | F,K,R,T,M,C,D,E,N,G,Q,S,V,A,W,P,L,H,I, |
| 21 | 19 | VAM,VCC,DKK, | F,K,R,T,M,C,D,E,N,G,Q,S,V,A,W,P,L,H,I, |
| 21 | 19 | VAM,DKS,VCC, | F,K,R,T,M,C,D,E,N,G,Q,S,V,A,W,P,L,H,I, |
| 21 | 19 | VCA,VAS,DKK, | F,K,R,T,M,C,D,E,N,G,Q,S,V,A,W,P,L,H,I, |
| 21 | 19 | VCA,DKS,VAS, | F,K,R,T,M,C,D,E,N,G,Q,S,V,A,W,P,L,H,I, |
| 21 | 19 | VCT,V AS,DKK, | F,K,R,T,M,C,D,E,N,G,Q,S,V,A,W,P,L,H,I, |
| 21 | 19 | VCT,DKS,VAS, | F,K,R,T,M,C,D,E,N,G,Q,S,V,A,W,P,L,H,I, |
| 21 | 19 | VCG,VAS,DKK, | F,K,R,T,M,C,D,E,N,G,Q,S,V,A,W,P,L,H,I, |
| 21 | 19 | DKS,VCG,VAS, | F,K,R,T,M,C,D,E,N,G,Q,S,V,A,W,P,L,H,I, |
| 21 | 19 | VCC,VAS,DKK, | F,K,R,T,M,C,D,E,N,G,Q,S,V,A,W,P,L,H,I, |
| 21 | 19 | DKS,VCC,VAS, | F,K,R,T,M,C,D,E,N,G,Q,S,V,A,W,P,L,H,I, |
| 21 | 19 | VCA,DKK,VAW, | F,K,R,T,M,C,D,E,N,G,Q,S,V,A,W,P,L,H,I, |
| 21 | 19 | VCA,DKS,VAW, | F,K,R,T,M,C,D,E,N,G,Q,S,V,A,W,P,L,H,I, |
| 21 | 19 | VCT,DKK,VAW, | F,K,R,T,M,C,D,E,N,G,Q,S,V,A,W,P,L,H,I, |

(continued)

| Codons | AA | Degenerate Codons | Amino Acids |
|--------|----|--------------------|-------------|
| 21 | 19 | VCT,DKS,VAW, | F,K,R,T,M,C,D,E,N,G,Q,S,V,A,W,P,L,H,I, |
| 21 | 19 | VCG,DKK,VAW, | F,K,R,T,M,C,D,E,N,G,Q,S,V,A,W,P,L,H,I, |
| 21 | 19 | DKS,VCG,VAW, | F,K,R,T,M,C,D,E,N,G,Q,S,V,A,W,P,L,H,I, |
| 21 | 19 | VCC,DKK,VAW, | F,K,R,T,M,C,D,E,N,G,Q,S,V,A,W,P,L,H,I, |
| 21 | 19 | DKS,VCC,VAW, | F,K,R,T,M,C,D,E,N,G,Q,S,V,A,W,P,L,H,I, |
| 21 | 19 | VMA,NDT,VTG, | F,K,T,R,M,D,C,E,N,G,Q,S,V,Y,A,P,H,L,I, |
| 21 | 19 | NDC,VMA,VTG, | F,K,T,R,M,D,C,E,N,G,Q,S,V,Y,A,P,H,L,I, |
| 21 | 19 | NDT,VTG,VMG, | F,K,T,R,M,D,C,E,N,G,Q,S,V,Y,A,P,H,L,I, |
| 21 | 19 | NDC,VTG,VMG, | F,K,T,R,M,D,C,E,N,G,Q,S,V,Y,A,P,H,L,I, |
| 21 | 19 | NDT,VWG,VCA, | F,K,T,R,M,D,C,E,N,G,Q,S,V,Y,A,P,H,L,I, |
| 21 | 19 | NDC,VWG,VCA, | F,K,T,R,M,D,C,E,N,G,Q,S,V,Y,A,P,H,L,I, |
| 21 | 19 | VCT,NDT,VWG, | F,K,T,R,M,D,C,E,N,G,Q,S,V,Y,A,P,H,L,I, |
| 21 | 19 | NDC,VCT,VWG, | F,K,T,R,M,D,C,E,N,G,Q,S,V,Y,A,P,H,L,I, |
| 21 | 19 | NDT,VWG,VCG, | F,K,T,R,M,D,C,E,N,G,Q,S,V,Y,A,P,H,L,I, |
| 21 | 19 | NDC,VWG,VCG, | F,K,T,R,M,D,C,E,N,G,Q,S,V,Y,A,P,H,L,I, |
| 21 | 19 | NDT,VWG,VCC, | F,K,T,R,M,D,C,E,N,G,Q,S,V,Y,A,P,H,L,I, |
| 21 | 19 | NDC,VWG,VCC, | F,K,T,R,M,D,C,E,N,G,Q,S,V,Y,A,P,H,L,I, |
| 21 | 19 | HAT,VMA,DKK, | F,K,R,T,M,C,E,N,G,Q,S,Y,V,A,W,P,L,H,I, |
| 21 | 19 | HAT,VMA,DKS, | F,K,R,T,M,C,E,N,G,Q,S,Y,V,A,W,P,L,H,I, |
| 21 | 19 | HAC,VMA,DKK, | F,K,R,T,M,C,E,N,G,Q,S,Y,V,A,W,P,L,H,I, |
| 21 | 19 | HAC,VMA,DKS, | F,K,R,T,M,C,E,N,G,Q,S,Y,V,A,W,P,L,H,I, |
| 21 | 19 | VMA,HTG,NDT, | F,K,T,R,M,D,C,E,N,G,Q,S,Y,V,A,P,H,L,I, |
| 21 | 19 | NDC,VMA,HTG, | F,K,T,R,M,D,C,E,N,G,Q,S,V,Y,A,P,H,L,I, |
| 21 | 19 | VMA,NDT,HGG, | F,K,R,T,D,C,E,N,G,Q,S,Y,V,A,W,P,L,H,I, |
| 21 | 19 | NDC,VMA,HGG, | F,K,R,T,D,C,E,N,G,Q,S,V,Y,A,W,P,L,H,I, |
| 21 | 19 | VMA,HKG,DDT, | F,K,R,T,M,D,C,E,N,G,Q,S,V,Y,A,W,P,L,I, |
| 21 | 19 | DDC,VMA,HKG, | F,K,R,T,M,D,C,E,N,G,Q,S,Y,V,A,W,P,L,I, |
| 21 | 19 | VMA,HDT,DKG, | F,K,T,R,M,C,E,N,G,Q,S,V,Y,A,W,P,L,H,I, |
| 21 | 19 | VMA,HDC,DKG, | F,K,T,R,M,C,E,N,G,Q,S,V,Y,A,W,P,L,H,I, |
| 21 | 19 | VMA,DKK,DAT, | F,K,R,T,M,C,D,E,N,G,Q,S,Y,V,A,W,P,L,I, |
| 21 | 19 | VMA,DKS,DAT, | F,K,R,T,M,C,D,E,N,G,Q,S,Y,V,A,W,P,L,I, |
| 21 | 19 | VMA,DKK,DAC, | F,K,R,T,M,C,D,E,N,G,Q,S,Y,V,A,W,P,L,I, |
| 21 | 19 | VMA,DKS,DAC, | F,K,R,T,M,C,D,E,N,G,Q,S,Y,V,A,W,P,L,I, |
| 21 | 19 | VMA,NDT,DTG, | F,K,T,R,M,D,C,E,N,G,Q,S,V,Y,A,P,H,L,I, |
| 21 | 19 | NDC,VMA,DTG, | F,K,T,R,M,D,C,E,N,G,Q,S,V,Y,A,P,H,L,I, |
| 21 | 19 | VMA,NDT,DGG, | F,K,R,T,D,C,E,N,G,Q,S,Y,V,A,W,P,L,H,I, |
| 21 | 19 | NDC,VMA,DGG, | F,K,R,T,D,C,E,N,G,Q,S,V,Y,A,W,P,L,H,I, |
| 21 | 19 | VMA,DDT,DKG, | F,K,R,T,M,D,C,E,N,G,Q,S,Y,V,A,W,P,L,I, |

(continued)

| Codons | AA | Degenerate Codons | Amino Acids |
|--------|----|-------------------|-------------|
| 21 | 19 | DDC,VMA,DKG, | F,K,R,T,M,D,C,E,N,G,S,Q,Y,V,A,W,P,L,I, |
| 21 | 19 | HAT,VMG,DKK, | F,K,R,T,M,C,E,N,G,Q,S,Y,V,A,W,P,L,H,I, |
| 21 | 19 | HAT,DKS,VMG, | F,K,R,T,M,C,E,N,G,Q,S,Y,V,A,W,P,L,H,I, |
| 21 | 19 | HAC,VMG,DKK, | F,K,R,T,M,C,E,N,G,Q,S,Y,V,A,W,P,L,H,I, |
| 21 | 19 | HAC,DKS,VMG, | F,K,R,T,M,C,E,N,G,Q,S,Y,V,A,W,P,L,H,I, |
| 21 | 19 | HTG,NDT,VMG, | F,K,T,R,M,D,C,E,N,G,Q,S,Y,V,A,P,H,L,I, |
| 21 | 19 | NDC,HTG,VMG, | F,K,T,R,M,D,C,E,N,G,Q,S,V,Y,A,P,H,L,I, |
| 21 | 19 | NDT,HGG,VMG, | F,K,R,T,D,C,E,N,G,Q,S,Y,V,A,W,P,L,H,I, |
| 21 | 19 | NDC,HGG,VMG, | F,K,R,T,D,C,E,N,G,Q,S,V,Y,A,W,P,L,H,I, |
| 21 | 19 | HKG,DDT,VMG, | F,K,R,T,M,D,C,E,N,G,Q,S,V,Y,A,W,P,L,I, |
| 21 | 19 | DDC,HKG,VMG, | F,K,R,T,M,D,C,E,N,G,Q,S,Y,V,A,W,P,L,I, |
| 21 | 19 | HDT,DKG,VMG, | F,K,T,R,M,C,E,N,G,Q,S,V,Y,A,W,P,H,L,I, |
| 21 | 19 | HDC,DKG,VMG, | F,K,T,R,M,C,E,N,G,Q,S,V,Y,A,W,P,L,H,I, |
| 21 | 19 | VMG,DKK,DAT, | F,K,R,T,M,C,D,E,N,G,Q,S,Y,V,A,W,P,L,I, |
| 21 | 19 | DKS,VMG,DAT, | F,K,R,T,M,C,D,E,N,G,Q,S,Y,V,A,W,P,L,I, |
| 21 | 19 | VMG,DKK,DAC, | F,K,R,T,M,C,D,E,N,G,Q,S,Y,V,A,W,P,L,I, |
| 21 | 19 | DKS,VMG,DAC, | F,K,R,T,M,C,D,E,N,G,Q,S,Y,V,A,W,P,L,I, |
| 21 | 19 | NDT,VMG,DTG, | F,K,T,R,M,D,C,E,N,G,Q,S,V,Y,A,P,H,L,I, |
| 21 | 19 | NDC,VMG,DTG, | F,K,T,R,M,D,C,E,N,G,Q,S,V,Y,A,P,H,L,I, |
| 21 | 19 | NDT,DGG,VMG, | F,K,R,T,D,C,E,N,G,Q,S,Y,V,A,W,P,L,H,I, |
| 21 | 19 | NDC,DGG,VMG, | F,K,R,T,D,C,E,N,G,Q,S,V,Y,A,W,P,L,H,I, |
| 21 | 19 | DDT,VMG,DKG, | F,K,R,T,M,D,C,E,N,G,Q,S,Y,V,A,W,P,L,I, |
| 21 | 19 | DDC,VMG,DKG, | F,K,R,T,M,D,C,E,N,G,S,Q,Y,V,A,W,P,L,I, |
| 21 | 19 | VWG,HTT,NVT, | F,K,R,T,M,D,C,E,N,G,Q,S,V,Y,A,P,H,L,I, |
| 21 | 19 | NVC,VWG,HTT, | F,K,T,R,M,D,C,E,N,G,Q,S,V,Y,A,P,H,L,I, |
| 21 | 19 | VWG,NVT,HTC, | F,K,R,T,M,D,C,E,N,G,Q,S,V,Y,A,P,H,L,I, |
| 21 | 19 | NVC,VWG,HTC, | F,K,T,R,M,D,C,E,N,G,Q,S,V,Y,A,P,H,L,I, |
| 21 | 19 | VWG,NVT,DTT, | F,K,R,T,M,D,C,E,N,G,Q,S,V,Y,A,P,H,L,I, |
| 21 | 19 | NVC,VWG,DTT, | F,K,T,R,M,D,C,E,N,G,Q,S,V,Y,A,P,H,L,I, |
| 21 | 19 | VWG,NVT,DTC, | F,K,R,T,M,D,C,E,N,G,Q,S,V,Y,A,P,H,L,I, |
| 21 | 19 | NVC,VWG,DTC, | F,K,T,R,M,D,C,E,N,G,Q,S,V,Y,A,P,H,L,I, |
| 21 | 19 | VWG,DGG,NHT, | F,K,T,R,M,D,E,N,G,Q,S,V,Y,A,W,P,H,L,I, |
| 21 | 19 | VWG,DGG,NHC, | F,K,T,R,M,D,E,N,G,Q,S,V,Y,A,W,P,H,L,I, |
| 21 | 19 | HGG,DDT,VHG, | F,K,R,T,M,D,C,E,N,G,Q,S,Y,V,A,W,P,L,I, |
| 21 | 19 | DDC,HGG,VHG, | F,K,R,T,M,D,C,E,N,G,Q,S,Y,V,A,W,P,L,I, |
| 21 | 19 | HWT,VHG,DGK, | F,K,R,T,M,C,E,N,G,S,Q,V,Y,A,W,P,L,H,I, |
| 21 | 19 | DGS,HWT,VHG, | F,K,R,T,M,C,E,N,G,S,Q,V,Y,A,W,P,L,H,I, |
| 21 | 19 | HWC,VHG,DGK, | F,K,R,T,M,C,E,N,G,S,Q,V,Y,A,W,P,L,H,I, |

(continued)

| Codons | AA | Degenerate Codons | Amino Acids |
|---|---|---|---|
| 21 | 19 | DGS,HWC,VHG, | F,K,R,T,M,C,E,N,G,S,Q,V,Y,A,W,P,L,H,I, |
| 21 | 19 | DGG,HDT,VHG, | F,K,T,R,M,C,E,N,G,Q,S,V,Y,A,W,P,L,H,I, |
| 21 | 19 | HDC,DGG,VHG, | F,K,T,R,M,C,E,N,G,Q,S,V,Y,A,W,P,L,H,I, |
| 21 | 19 | DDT,DGG,VHG, | F,K,R,T,M,D,C,E,N,G,S,Q,Y,V,A,W,P,L,I, |
| 21 | 19 | DDC,DGG,VHG, | F,K,R,T,M,D,C,E,N,G,S,Q,Y,V,A,W,P,L,I, |
| 21 | 19 | DWT,VHG,DGK, | F,K,T,R,M,D,C,E,N,G,Q,S,Y,V,A,W,P,L,I, |
| 21 | 19 | DWC,VHG,DGK, | F,K,T,R,M,D,C,E,N,G,Q,S,Y,V,A,W,P,L,I, |
| 21 | 19 | DGS,DWT,VHG, | F,K,T,R,M,D,C,E,N,G,Q,S,Y,V,A,W,P,L,I, |
| 21 | 19 | DGS,DWC,VHG, | F,K,T,R,M,D,C,E,N,G,Q,S,Y,V,A,W,P,L,I, |
| 21 | 19 | NAT,VHG,NKT, | F,K,R,T,M,C,D,E,N,G,S,Q,V,Y,A,P,L,H,I, |
| 21 | 19 | NAT,VHG,NKC, | F,K,R,T,M,C,D,E,N,G,S,Q,V,Y,A,P,L,H,I, |
| 21 | 19 | NAC,VHG,NKT, | F,K,R,T,M,C,D,E,N,G,S,Q,V,Y,A,P,L,H,I, |
| 21 | 19 | NAC,VHG,NKC, | F,K,R,T,M,C,D,E,N,G,S,Q,V,Y,A,P,L,H,I, |
| 21 | 19 | NTT,VHG,NRT, | F,K,R,T,M,D,C,E,N,G,Q,S,V,Y,A,P,H,L,I, |
| 21 | 19 | NTT,VHG,NRC, | F,K,R,T,M,D,C,E,N,G,Q,S,V,Y,A,P,H,L,I, |
| 21 | 19 | NTC,VHG,NRT, | F,K,R,T,M,D,C,E,N,G,Q,S,V,Y,A,P,H,L,I, |
| 21 | 19 | NTC,VHG,NRC, | F,K,R,T,M,D,C,E,N,G,Q,S,V,Y,A,P,H,L,I, |
| 21 | 19 | NGT,VHG,NWT, | F,K,T,R,M,D,C,E,N,G,Q,S,Y,V,A,P,H,L,I, |
| 21 | 19 | NGT,VHG,NWC, | F,K,T,R,M,D,C,E,N,G,Q,S,Y,V,A,P,H,L,I, |
| 21 | 19 | NGC,VHG,NWT, | F,K,T,R,M,D,C,E,N,G,Q,S,Y,V,A,P,H,L,I, |
| 21 | 19 | NGC,VHG,NWC, | F,K,T,R,M,D,C,E,N,G,Q,S,Y,V,A,P,H,L,I, |
| 18 | 18 | VHG,ATA,NRT, | K,T,R,M,D,C,E,N,G,Q,S,V,Y,A,P,H,L,I, |
| 18 | 18 | VHG,ATA,NRC, | K,T,R,M,D,C,E,N,G,Q,S,V,Y,A,P,H,L,I, |
| 18 | 18 | VHG,ATT,NRT, | K,T,R,M,D,C,E,N,G,Q,S,V,Y,A,P,H,L,I, |
| 18 | 18 | VHG,ATT,NRC, | K,T,R,M,D,C,E,N,G,Q,S,V,Y,A,P,H,L,I, |
| 18 | 18 | ATG,VHA,NRT, | K,T,R,M,D,C,E,N,G,Q,S,V,Y,A,P,H,L,I, |
| 18 | 18 | ATG,VHA,NRC, | K,T,R,M,D,C,E,N,G,Q,S,V,Y,A,P,H,L,I, |
| 18 | 18 | ATC,VHG,NRT, | K,T,R,M,D,C,E,N,G,Q,S,V,Y,A,P,H,L,I, |
| 18 | 18 | ATC,VHG,NRC, | K,T,R,M,D,C,E,N,G,Q,S,V,Y,A,P,H,L,I, |
| 18 | 18 | NDT,SMA,AMA, | F,K,T,R,C,D,E,N,G,Q,S,Y,V,A,P,L,H,I, |
| 18 | 18 | NDC,SMA,AMA, | F,K,T,R,C,D,E,N,G,Q,S,Y,V,A,P,H,L,I, |
| 18 | 18 | NDT,AMA,SMG, | F,K,T,R,C,D,E,N,G,Q,S,Y,V,A,P,L,H,I, |
| 18 | 18 | NDC,AMA,SMG, | F,K,T,R,C,D,E,N,G,Q,S,Y,V,A,P,H,L,I, |
| 18 | 18 | NDT,SMA,AMG, | F,K,T,R,C,D,E,N,G,Q,S,Y,V,A,P,L,H,I, |
| 18 | 18 | NDC,SMA,AMG, | F,K,T,R,C,D,E,N,G,Q,S,Y,V,A,P,H,L,I, |
| 18 | 18 | NDT,AMG,SMG, | F,K,T,R,C,D,E,N,G,Q,S,Y,V,A,P,L,H,I, |
| 18 | 18 | NDC,AMG,SMG, | F,K,T,R,C,D,E,N,G,Q,S,Y,V,A,P,H,L,I, |
| 18 | 18 | NDT,SMA,AYG, | F,T,R,M,C,D,E,N,G,Q,S,Y,V,A,P,L,H,I, |

(continued)

| Codons | AA | Degenerate Codons | Amino Acids |
|---|---|---|---|
| 18 | 18 | NDC,SMA,AYG, | F,T,R,M,C,D,E,N,G,Q,S,V,Y,A,P,H,L,I, |
| 18 | 18 | NDT,SMG,AYG, | F,T,R,M,C,D,E,N,G,Q,S,Y,V,A,P,L,H,I, |
| 18 | 18 | NDC,SMG,AYG, | F,T,R,M,C,D,E,N,G,Q,S,V,Y,A,P,H,L,I, |
| 18 | 18 | NDT,SMA,AWG, | F,K,R,M,C,D,E,N,G,Q,S,Y,V,A,P,L,H,I, |
| 18 | 18 | NDC,SMA,AWG, | F,K,R,M,C,D,E,N,G,Q,S,V,Y,A,P,H,L,I, |
| 18 | 18 | NDT,SMG,AWG, | F,K,R,M,C,D,E,N,G,Q,S,Y,V,A,P,L,H,I, |
| 18 | 18 | NDC,SMG,AWG, | F,K,R,M,C,D,E,N,G,Q,S,V,Y,A,P,H,L,I, |
| 18 | 18 | SRA,AWG,NHT, | F,K,R,T,M,D,E,N,G,Q,S,Y,V,A,P,L,H,I, |
| 18 | 18 | SRA,NHC,AWG, | F,K,R,T,M,D,E,N,G,Q,S,V,Y,A,P,H,L,I, |
| 18 | 18 | SRG,AWG,NHT, | F,K,R,T,M,D,E,N,G,Q,S,Y,V,A,P,L,H,I, |
| 18 | 18 | SRG,NHC,AWG, | F,K,R,T,M,D,E,N,G,Q,S,V,Y,A,P,H,L,I, |
| 18 | 18 | BNT,SAA,AWK, | F,K,R,M,D,C,E,N,G,Q,S,Y,V,A,P,L,H,I, |
| 18 | 18 | BNC,SAA,AWK, | F,K,R,M,D,C,E,N,G,Q,S,Y,V,A,P,L,H,I, |
| 18 | 18 | BNT,AWK,SAG, | F,K,R,M,D,C,E,N,G,Q,S,Y,V,A,P,L,H,I, |
| 18 | 18 | BNC,AWK, SAG, | F,K,R,M,D,C,E,N,G,Q,S,V,Y,A,P,L,H,I, |
| 18 | 18 | BNT,AWS,SAA, | F,K,R,M,D,C,E,N,G,Q,S,Y,V,A,P,L,H,I, |
| 18 | 18 | AWS,BNC,SAA, | F,K,R,M,D,C,E,N,G,Q,S,V,Y,A,P,L,H,I, |
| 18 | 18 | BNT,AWS,SAG, | F,K,R,M,D,C,E,N,G,Q,S,Y,V,A,P,L,H,I, |
| 18 | 18 | AWS,BNC,SAG, | F,K,R,M,D,C,E,N,G,Q,S,V,Y,A,P,L,H,I, |
| 18 | 18 | VWG,AHT,BVT, | K,R,T,M,D,C,E,N,G,S,Q,V,Y,A,P,L,H,I, |
| 18 | 18 | VWG,AHT,BVC, | K,R,T,M,D,C,E,N,G,S,Q,V,Y,A,P,L,H,I, |
| 18 | 18 | BNT,AHT,VAA, | F,K,T,R,C,D,E,N,G,Q,S,Y,V,A,P,H,L,I, |
| 18 | 18 | BNT,VAG,AHT, | F,K,T,R,C,D,E,N,G,Q,S,Y,V,A,P,H,L,I, |
| 18 | 18 | BNC,AHT,VAA, | F,K,T,R,C,D,E,N,G,Q,S,V,Y,A,P,L,H,I, |
| 18 | 18 | VAG,BNC,AHT, | F,K,T,R,C,D,E,N,G,Q,S,V,Y,A,P,L,H,I, |
| 18 | 18 | VWG,AHC,BVT, | K,R,T,M,D,C,E,N,G,S,Q,V,Y,A,P,L,H,I, |
| 18 | 18 | VWG,AHC,BVC, | K,R,T,M,D,C,E,N,G,S,Q,V,Y,A,P,L,H,I, |
| 18 | 18 | BNT,AHC,VAA, | F,K,T,R,C,D,E,N,G,Q,S,Y,V,A,P,H,L,I, |
| 18 | 18 | BNT,VAG,AHC, | F,K,T,R,C,D,E,N,G,Q,S,Y,V,A,P,H,L,I, |
| 18 | 18 | BNC,AHC,VAA, | F,K,T,R,C,D,E,N,G,Q,S,V,Y,A,P,L,H,I, |
| 18 | 18 | VAG,BNC,AHC, | F,K,T,R,C,D,E,N,G,Q,S,V,Y,A,P,L,H,I, |
| 18 | 18 | BRT,VHG,ADT, | K,T,R,M,D,C,E,N,G,S,Q,Y,V,A,P,L,H,I, |
| 18 | 18 | VHG,ADT,BRC, | K,T,R,M,D,C,E,N,G,S,Q,Y,V,A,P,L,H,I, |
| 18 | 18 | VMA,BDT,ADT, | F,K,T,R,D,C,E,N,G,S,Q,V,Y,A,P,L,H,I, |
| 18 | 18 | BDT,VMG,ADT, | F,K,T,R,D,C,E,N,G,S,Q,V,Y,A,P,L,H,I, |
| 18 | 18 | VMA,BDC,ADT, | F,K,T,R,D,C,E,N,G,S,Q,Y,V,A,P,H,L,I, |
| 18 | 18 | BDC,VMG,ADT, | F,K,T,R,D,C,E,N,G,S,Q,Y,V,A,P,L,H,I, |
| 18 | 18 | BRT,VHG,ADC, | K,T,R,M,D,C,E,N,G,S,Q,Y,V,A,P,L,H,I, |

(continued)

| Codons | AA | Degenerate Codons | Amino Acids |
|--------|-----|-------------------|-------------|
| 18 | 18 | VHG,ADC,BRC, | K,T,R,M,D,C,E,N,G,S,Q,Y,V,A,P,L,H,I, |
| 18 | 18 | VMA,BDT,ADC, | F,K,T,R,D,C,E,N,G,S,Q,V,Y,A,P,L,H,I, |
| 18 | 18 | BDT,VMG,ADC, | F,K,T,R,D,C,E,N,G,S,Q,V,Y,A,P,L,H,I, |
| 18 | 18 | VMA,BDC,ADC, | F,K,T,R,D,C,E,N,G,S,Q,Y,V,A,P,H,L,I, |
| 18 | 18 | BDC,VMG,ADC, | F,K,T,R,D,C,E,N,G,S,Q,Y,V,A,P,H,L,I, |
| 18 | 18 | VHA,TTT,NRT, | F,K,T,R,D,C,E,N,G,Q,S,V,Y,A,P,H,L,I, |
| 18 | 18 | VHA,TTT,NRC, | F,K,T,R,D,C,E,N,G,Q,S,V,Y,A,P,H,L,I, |
| 18 | 18 | TTT,VHG,NRT, | F,K,T,R,M,D,C,E,N,G,Q,S,V,Y,A,P,H,L, |
| 18 | 18 | TTT,VHG,NRC, | F,K,T,R,M,D,C,E,N,G,Q,S,V,Y,A,P,H,L, |
| 18 | 18 | TTC,VHA,NRT, | F,K,T,R,D,C,E,N,G,Q,S,V,Y,A,P,H,L,I, |
| 18 | 18 | TTC,VHA,NRC, | F,K,T,R,D,C,E,N,G,Q,S,V,Y,A,P,H,L,I, |
| 18 | 18 | TTC,VHG,NRT, | F,K,T,R,M,D,C,E,N,G,Q,S,V,Y,A,P,H,L, |
| 18 | 18 | TTC,VHG,NRC, | F,K,T,R,M,D,C,E,N,G,Q,S,V,Y,A,P,H,L, |
| 18 | 18 | VHA,TGG,NRT, | K,T,R,D,C,E,N,G,Q,S,V,Y,A,W,P,H,L,I, |
| 18 | 18 | VHA,TGG,NRC, | K,T,R,D,C,E,N,G,Q,S,V,Y,A,W,P,H,L,I, |
| 18 | 18 | TGG,VHG,NRT, | K,T,R,M,D,C,E,N,G,Q,S,V,Y,A,W,P,H,L, |
| 18 | 18 | TGG,VHG,NRC, | K,T,R,M,D,C,E,N,G,Q,S,V,Y,A,W,P,H,L, |
| 18 | 18 | TGK,NHT,RRA, | F,K,R,T,C,D,E,N,G,S,Y,V,A,W,P,L,H,I, |
| 18 | 18 | TGK,NHC,RRA, | F,K,R,T,C,D,E,N,G,S,V,Y,A,W,P,H,L,I, |
| 18 | 18 | TGK,RRG,NHT, | F,K,R,T,C,D,E,N,G,S,Y,V,A,W,P,L,H,I, |
| 18 | 18 | TGK,RRG,NHC, | F,K,R,T,C,D,E,N,G,S,V,Y,A,W,P,H,L,I, |
| 18 | 18 | TGK,SRA,NHT, | F,R,T,C,D,E,N,G,Q,S,Y,V,A,W,P,L,H,I, |
| 18 | 18 | TGK,SRA,NHC, | F,R,T,C,D,E,N,G,Q,S,V,Y,A,W,P,H,L,I, |
| 18 | 18 | SRG,TGK,NHT, | F,R,T,C,D,E,N,G,Q,S,Y,V,A,W,P,L,H,I, |
| 18 | 18 | SRG,TGK,NHC, | F,R,T,C,D,E,N,G,Q,S,V,Y,A,W,P,H,L,I, |
| 18 | 18 | TGS,NHT,RRA, | F,K,R,T,C,D,E,N,G,S,Y,V,A,W,P,L,H,I, |
| 18 | 18 | TGS,NHC,RRA, | F,K,R,T,C,D,E,N,G,S,Y,V,A,W,P,L,H,I, |
| 18 | 18 | TGS,RRG,NHT, | F,K,R,T,C,D,E,N,G,S,Y,V,A,W,P,L,H,I, |
| 18 | 18 | TGS,RRG,NHC, | F,K,R,T,C,D,E,N,G,S,Y,V,A,W,P,L,H,I, |
| 18 | 18 | SRA,TGS,NHT, | F,R,T,C,D,E,N,G,Q,S,Y,V,A,W,P,L,H,I, |
| 18 | 18 | SRA,TGS,NHC, | F,R,T,C,D,E,N,G,Q,S,V,Y,A,W,P,H,L,I, |
| 18 | 18 | SRG,TGS,NHT, | F,R,T,C,D,E,N,G,Q,S,Y,V,A,W,P,L,H,I, |
| 18 | 18 | SRG,TGS,NHC, | F,R,T,C,D,E,N,G,Q,S,V,Y,A,W,P,H,L,I, |
| 18 | 18 | RDA,TKK,NMT, | F,K,T,R,D,C,E,N,G,S,V,Y,A,W,P,H,L,I, |
| 18 | 18 | RDA,TKK,NMC, | F,K,T,R,D,C,E,N,G,S,V,Y,A,W,P,H,L,I, |
| 18 | 18 | TKK,RDG,NMT, | F,K,T,R,M,D,C,E,N,G,S,V,Y,A,W,P,H,L, |
| 18 | 18 | TKK,RDG,NMC, | F,K,T,R,M,D,C,E,N,G,S,V,Y,A,W,P,H,L, |
| 18 | 18 | RDA,TKS,NMT, | F,K,T,R,D,C,E,N,G,S,V,Y,A,W,P,H,L,I, |

47

(continued)

| Codons | AA | Degenerate Codons | Amino Acids |
|---|---|---|---|
| 18 | 18 | RDA,TKS,NMC, | F,K,T,R,D,C,E,N,G,S,V,Y,A,W,P,H,L,I, |
| 18 | 18 | RDG,TKS,NMT, | F,K,T,R,M,D,C,E,N,G,S,V,Y,A,W,P,H,L, |
| 18 | 18 | RDG,TKS,NMC, | F,K,T,R,M,D,C,E,N,G,S,V,Y,A,W,P,H,L, |
| 18 | 18 | TDT,VNT,VAA, | F,K,R,T,C,D,E,N,G,Q,S,Y,V,A,P,L,H,I, |
| 18 | 18 | TDT,VAA,VNC, | F,K,T,R,C,D,E,N,G,Q,S,Y,V,A,P,H,L,I, |
| 18 | 18 | VAG,TDT,VNT, | F,K,R,T,C,D,E,N,G,Q,S,Y,V,A,P,L,H,I, |
| 18 | 18 | VAG,TDT,VNC, | F,K,T,R,C,D,E,N,G,Q,S,Y,V,A,P,H,L,I, |
| 18 | 18 | VAK,TDT,VBT, | F,K,T,R,D,C,E,N,G,Q,S,Y,V,A,P,L,H,I, |
| 18 | 18 | VAK,VBC,TDT, | F,K,R,T,D,C,E,N,G,Q,S,Y,V,A,P,L,H,I, |
| 18 | 18 | VAK,TDT,DBG, | F,K,R,T,M,D,C,E,N,G,Q,S,Y,V,A,W,L,H, |
| 18 | 18 | VAM,TDT,VBT, | F,K,T,R,D,C,E,N,G,Q,S,Y,V,A,P,L,H,I, |
| 18 | 18 | VAM,VBC,TDT, | F,K,R,T,D,C,E,N,G,Q,S,Y,V,A,P,L,H,I, |
| 18 | 18 | VAM,TDT,DBG, | F,K,R,T,M,D,C,E,N,G,Q,S,Y,V,A,W,L,H, |
| 18 | 18 | TDT,VBT,VAS, | F,K,T,R,D,C,E,N,G,Q,S,Y,V,A,P,L,H,I, |
| 18 | 18 | VBC,TDT,VAS, | F,K,R,T,D,C,E,N,G,Q,S,Y,V,A,P,L,H,I, |
| 18 | 18 | TDT,VAS,DBG, | F,K,R,T,M,D,C,E,N,G,Q,S,Y,V,A,W,L,H, |
| 18 | 18 | TDT,VBT,VAW, | F,K,T,R,D,C,E,N,G,Q,S,Y,V,A,P,L,H,I, |
| 18 | 18 | VBC,TDT,VAW, | F,K,R,T,D,C,E,N,G,Q,S,Y,V,A,P,L,H,I, |
| 18 | 18 | TDT,DBG,VAW, | F,K,R,T,M,D,C,E,N,G,Q,S,Y,V,A,W,L,H, |
| 18 | 18 | VMA,VDT,TDT, | F,K,T,R,C,D,E,N,G,Q,S,Y,V,A,P,L,H,I, |
| 18 | 18 | VMA,VDC,TDT, | F,K,T,R,C,D,E,N,G,Q,S,Y,V,A,P,H,L,I, |
| 18 | 18 | VDT,TDT,VMG, | F,K,T,R,C,D,E,N,G,Q,S,Y,V,A,P,L,H,I, |
| 18 | 18 | VDC,TDT,VMG, | F,K,T,R,C,D,E,N,G,Q,S,Y,V,A,P,H,L,I, |
| 18 | 18 | VHA,VRT,TDT, | F,K,T,R,D,C,E,N,G,S,Q,Y,V,A,P,L,H,I, |
| 18 | 18 | VRT,TDT,VHG, | F,K,T,R,M,D,C,E,N,G,S,Q,Y,V,A,P,L,H, |
| 18 | 18 | VHA,TDT,VRC, | F,K,T,R,D,C,E,N,G,S,Q,Y,V,A,P,L,H,I, |
| 18 | 18 | TDT,VRC,VHG, | F,K,T,R,M,D,C,E,N,G,S,Q,Y,V,A,P,L,H, |
| 18 | 18 | VVT,VWA,TDT, | F,K,R,T,D,C,E,N,G,Q,S,V,Y,A,P,H,L,I, |
| 18 | 18 | VWA,VVC,TDT, | F,K,T,R,D,C,E,N,G,Q,S,V,Y,A,P,H,L,I, |
| 18 | 18 | VWG,VVT,TDT, | F,K,R,T,M,D,C,E,N,G,Q,S,V,Y,A,P,H,L, |
| 18 | 18 | VWG,VVC,TDT, | F,K,T,R,M,D,C,E,N,G,Q,S,V,Y,A,P,H,L, |
| 18 | 18 | VNT,VAA,TDC, | F,K,R,T,C,D,E,N,G,Q,S,Y,V,A,P,L,H,I, |
| 18 | 18 | VAA,TDC,VNC, | F,K,T,R,C,D,E,N,G,Q,S,Y,V,A,P,H,L,I, |
| 18 | 18 | VAG,VNT,TDC, | F,K,R,T,C,D,E,N,G,Q,S,Y,V,A,P,L,H,I, |
| 18 | 18 | VAG,TDC,VNC, | F,K,T,R,C,D,E,N,G,Q,S,Y,V,A,P,H,L,I, |
| 18 | 18 | VAK,VBT,TDC, | F,K,T,R,D,C,E,N,G,Q,S,Y,V,A,P,L,H,I, |
| 18 | 18 | VAK,VBC,TDC, | F,K,R,T,D,C,E,N,G,Q,S,Y,V,A,P,L,H,I, |
| 18 | 18 | VAK,TDC,DBG, | F,K,R,T,M,D,C,E,N,G,Q,S,Y,V,A,W,L,H, |

(continued)

| Codons | AA | Degenerate Codons | Amino Acids |
|---|---|---|---|
| 18 | 18 | VAM,VBT,TDC, | F,K,T,R,D,C,E,N,G,Q,S,Y,V,A,P,L,H,I, |
| 18 | 18 | VAM,VBC,TDC, | F,K,R,T,D,C,E,N,G,Q,S,Y,V,A,P,L,H,I, |
| 18 | 18 | VAM,TDC,DBG, | F,K,R,T,M,D,C,E,N,G,Q,S,Y,V,A,W,L,H, |
| 18 | 18 | VBT,VAS,TDC, | F,K,T,R,D,C,E,N,G,Q,S,Y,V,A,P,L,H,I, |
| 18 | 18 | VBC,VAS,TDC, | F,K,R,T,D,C,E,N,G,Q,S,Y,V,A,P,L,H,I, |
| 18 | 18 | VAS,TDC,DBG, | F,K,R,T,M,D,C,E,N,G,Q,S,Y,V,A,W,L,H, |
| 18 | 18 | VBT,TDC,VAW, | F,K,T,R,D,C,E,N,G,Q,S,Y,V,A,P,L,H,I, |
| 18 | 18 | VBC,TDC,VAW, | F,K,R,T,D,C,E,N,G,Q,S,Y,V,A,P,L,H,I, |
| 18 | 18 | TDC,DBG,VAW, | F,K,R,T,M,D,C,E,N,G,Q,S,Y,V,A,W,L,H, |
| 18 | 18 | VMA,VDT,TDC, | F,K,T,R,C,D,E,N,G,Q,S,Y,V,A,P,L,H,I, |
| 18 | 18 | VMA,VDC,TDC, | F,K,T,R,C,D,E,N,G,Q,S,Y,V,A,P,H,L,I, |
| 18 | 18 | VDT,VMG,TDC, | F,K,T,R,C,D,E,N,G,Q,S,Y,V,A,P,L,H,I, |
| 18 | 18 | VDC,VMG,TDC, | F,K,T,R,C,D,E,N,G,Q,S,Y,V,A,P,H,L,I, |
| 18 | 18 | VHA,VRT,TDC, | F,K,T,R,D,C,E,N,G,S,Q,Y,V,A,P,L,H,I, |
| 18 | 18 | VRT,VHG,TDC, | F,K,T,R,M,D,C,E,N,G,S,Q,Y,V,A,P,L,H, |
| 18 | 18 | VHA,VRC,TDC, | F,K,T,R,D,C,E,N,G,S,Q,Y,V,A,P,L,H,I, |
| 18 | 18 | VRC,VHG,TDC, | F,K,T,R,M,D,C,E,N,G,S,Q,Y,V,A,P,L,H, |
| 18 | 18 | VVT,VWA,TDC, | F,K,R,T,D,C,E,N,G,Q,S,V,Y,A,P,H,L,I, |
| 18 | 18 | VWA,VVC,TDC, | F,K,T,R,D,C,E,N,G,Q,S,V,Y,A,P,H,L,I, |
| 18 | 18 | VWG,VVT,TDC, | F,K,R,T,M,D,C,E,N,G,Q,S,V,Y,A,P,H,L, |
| 18 | 18 | VWG,VVC,TDC, | F,K,T,R,M,D,C,E,N,G,Q,S,V,Y,A,P,H,L, |
| 18 | 18 | NDT,GMA,MMA, | F,K,T,R,C,D,E,N,G,Q,S,Y,V,A,P,L,H,I, |
| 18 | 18 | NDC,GMA,MMA, | F,K,T,R,C,D,E,N,G,Q,S,V,Y,A,P,H,L,I, |
| 18 | 18 | NDT,GMA,MMG, | F,K,T,R,C,D,E,N,G,Q,S,Y,V,A,P,L,H,I, |
| 18 | 18 | NDC,GMA,MMG, | F,K,T,R,C,D,E,N,G,Q,S,V,Y,A,P,H,L,I, |
| 18 | 18 | NDT,GMG,MMA, | F,K,T,R,C,D,E,N,G,Q,S,Y,V,A,P,L,H,I, |
| 18 | 18 | NDC,GMG,MMA, | F,K,T,R,C,D,E,N,G,Q,S,V,Y,A,P,H,L,I, |
| 18 | 18 | NDT,GMG,MMG, | F,K,T,R,C,D,E,N,G,Q,S,Y,V,A,P,L,H,I, |
| 18 | 18 | NDC,GMG,MMG, | F,K,T,R,C,D,E,N,G,Q,S,V,Y,A,P,H,L,I, |
| 18 | 18 | GDT,VMA,HDT, | F,K,T,R,C,D,E,N,G,Q,S,V,Y,A,P,L,H,I, |
| 18 | 18 | GDT,VMA,HDC, | F,K,T,R,C,D,E,N,G,Q,S,V,Y,A,P,H,L,I, |
| 18 | 18 | GDT,HDT,VMG, | F,K,T,R,C,D,E,N,G,Q,S,V,Y,A,P,L,H,I, |
| 18 | 18 | GDT,HDC,VMG, | F,K,T,R,C,D,E,N,G,Q,S,V,Y,A,P,H,L,I, |
| 18 | 18 | GDC,VMA,HDT, | F,K,T,R,C,D,E,N,G,Q,S,V,Y,A,P,L,H,I, |
| 18 | 18 | GDC,VMA,HDC, | F,K,T,R,C,D,E,N,G,Q,S,V,Y,A,P,H,L,I, |
| 18 | 18 | GDC,HDT,VMG, | F,K,T,R,C,D,E,N,G,Q,S,V,Y,A,P,L,H,I, |
| 18 | 18 | GDC,HDC,VMG, | F,K,T,R,C,D,E,N,G,Q,S,V,Y,A,P,H,L,I, |
| 18 | 18 | NDT,RMA,CMA, | F,K,T,R,C,D,E,N,G,Q,S,Y,V,A,P,L,H,I, |

(continued)

| Codons | AA | Degenerate Codons | Amino Acids |
|--------|-----|-------------------|-------------|
| 18 | 18 | NDC,RMA,CMA, | F,K,T,R,C,D,E,N,G,Q,S,V,Y,A,P,H,L,I, |
| 18 | 18 | NDT,RMG,CMA, | F,K,T,R,C,D,E,N,G,Q,S,Y,V,A,P,L,H,I, |
| 18 | 18 | NDC,RMG,CMA, | F,K,T,R,C,D,E,N,G,Q,S,V,Y,A,P,H,L,I, |
| 18 | 18 | NDT,CMG,RMA, | F,K,T,R,C,D,E,N,G,Q,S,Y,V,A,P,L,H,I, |
| 18 | 18 | NDC,CMG,RMA, | F,K,T,R,C,D,E,N,G,Q,S,V,Y,A,P,H,L,I, |
| 18 | 18 | NDT,RMG,CMG, | F,K,T,R,C,D,E,N,G,Q,S,Y,V,A,P,L,H,I, |
| 18 | 18 | NDC,RMG,CMG, | F,K,T,R,C,D,E,N,G,Q,S,V,Y,A,P,H,L,I, |
| 18 | 18 | VMA,DDT,CDT, | F,K,T,R,D,C,E,N,G,Q,S,Y,V,A,P,H,L,I, |
| 18 | 18 | DDC,VMA,CDT, | F,K,T,R,D,C,E,N,G,Q,S,V,Y,A,P,H,L,I, |
| 18 | 18 | DDT,VMG,CDT, | F,K,T,R,D,C,E,N,G,Q,S,Y,V,A,P,H,L,I, |
| 18 | 18 | DDC,VMG,CDT, | F,K,T,R,D,C,E,N,G,Q,S,V,Y,A,P,H,L,I, |
| 18 | 18 | VMA,DDT,CDC, | F,K,T,R,D,C,E,N,G,Q,S,Y,V,A,P,L,H,I, |
| 18 | 18 | DDC,VMA,CDC, | F,K,T,R,D,C,E,N,G,Q,S,V,Y,A,P,H,L,I, |
| 18 | 18 | DDT,VMG,CDC, | F,K,T,R,D,C,E,N,G,Q,S,Y,V,A,P,L,H,I, |
| 18 | 18 | DDC,VMG,CDC, | F,K,T,R,D,C,E,N,G,Q,S,V,Y,A,P,L,H,I, |
| 18 | 18 | VMA,MDT,KDT, | F,K,T,R,C,D,E,N,G,S,Q,Y,V,A,P,L,H,I, |
| 18 | 18 | MDT,VMG,KDT, | F,K,T,R,C,D,E,N,G,S,Q,Y,V,A,P,L,H,I, |
| 18 | 18 | VMA,MDC,KDT, | F,K,T,R,C,D,E,N,G,S,Q,Y,V,A,P,H,L,I, |
| 18 | 18 | MDC,VMG,KDT, | F,K,T,R,C,D,E,N,G,S,Q,Y,V,A,P,H,L,I, |
| 18 | 18 | VMA,MDT,KDC, | F,K,T,R,C,D,E,N,G,S,Q,V,Y,A,P,L,H,I, |
| 18 | 18 | MDT,VMG,KDC, | F,K,T,R,C,D,E,N,G,S,Q,V,Y,A,P,L,H,I, |
| 18 | 18 | VMA,MDC,KDC, | F,K,T,R,C,D,E,N,G,S,Q,V,Y,A,P,H,L,I, |
| 18 | 18 | MDC,VMG,KDC, | F,K,T,R,C,D,E,N,G,S,Q,V,Y,A,P,H,L,I, |
| 18 | 18 | VMA,YDT,RDT, | F,K,T,R,C,D,E,N,G,S,Q,Y,V,A,P,L,H,I, |
| 18 | 18 | VMG,YDT,RDT, | F,K,T,R,C,D,E,N,G,S,Q,Y,V,A,P,L,H,I, |
| 18 | 18 | VMA,YDC,RDT, | F,K,T,R,C,D,E,N,G,S,Q,Y,V,A,P,H,L,I, |
| 18 | 18 | YDC,VMG,RDT, | F,K,T,R,C,D,E,N,G,S,Q,Y,V,A,P,H,L,I, |
| 18 | 18 | VMA,YDT,RDC, | F,K,T,R,C,D,E,N,G,S,Q,Y,V,A,P,L,H,I, |
| 18 | 18 | VMG,YDT,RDC, | F,K,T,R,C,D,E,N,G,S,Q,Y,V,A,P,L,H,I, |
| 18 | 18 | VMA,YDC,RDC, | F,K,T,R,C,D,E,N,G,S,Q,Y,V,A,P,H,L,I, |
| 18 | 18 | YDC,VMG,RDC, | F,K,T,R,C,D,E,N,G,S,Q,Y,V,A,P,H,L,I, |
| 18 | 18 | VWG,WHT,SVT, | F,K,T,R,M,D,E,N,G,S,Q,V,Y,A,P,L,H,I, |
| 18 | 18 | WHC,VWG,SVT, | F,K,T,R,M,D,E,N,G,S,Q,V,Y,A,P,L,H,I, |
| 18 | 18 | VWG,SVT,WDT, | F,K,R,M,C,D,E,N,G,S,Q,V,Y,A,P,L,H,I, |
| 18 | 18 | VWG,SVT,WDC, | F,K,R,M,C,D,E,N,G,S,Q,V,Y,A,P,L,H,I, |
| 18 | 18 | VWG,WHT,SVC, | F,K,T,R,M,D,E,N,G,S,Q,V,Y,A,P,L,H,I, |
| 18 | 18 | WHC,VWG,SVC, | F,K,T,R,M,D,E,N,G,S,Q,V,Y,A,P,L,H,I, |
| 18 | 18 | VWG,SVC,WDT, | F,K,R,M,C,D,E,N,G,S,Q,V,Y,A,P,L,H,I, |

(continued)

| Codons | AA | Degenerate Codons | Amino Acids |
|---|---|---|---|
| 18 | 18 | VWG,SVC,WDC, | F,K,R,M,C,D,E,N,G,S,Q,V,Y,A,P,L,H,I, |
| 18 | 18 | VMA,SDT,WDT, | F,K,T,R,C,D,E,N,G,S,Q,Y,V,A,P,H,L,I, |
| 18 | 18 | SDT,VMG,WDT, | F,K,T,R,C,D,E,N,G,S,Q,Y,V,A,P,H,L,I, |
| 18 | 18 | VMA,SDT,WDC, | F,K,T,R,C,D,E,N,G,S,Q,Y,V,A,P,H,L,I, |
| 18 | 18 | SDT,VMG,WDC, | F,K,T,R,C,D,E,N,G,S,Q,Y,V,A,P,H,L,I, |
| 18 | 18 | VMA,SDC,WDT, | F,K,T,R,C,D,E,N,G,S,Q,Y,V,A,P,L,H,I, |
| 18 | 18 | SDC,VMG,WDT, | F,K,T,R,C,D,E,N,G,S,Q,Y,V,A,P,L,H,I, |
| 18 | 18 | VMA,SDC,WDC, | F,K,T,R,C,D,E,N,G,S,Q,Y,V,A,P,L,H,I, |
| 18 | 18 | SDC,VMG,WDC, | F,K,T,R,C,D,E,N,G,S,Q,Y,V,A,P,L,H,I, |
| 18 | 18 | VMA,WTK,NRT, | F,K,T,R,M,D,C,E,N,G,Q,S,Y,A,P,H,L,I, |
| 18 | 18 | VMA,WTK,NRC, | F,K,T,R,M,D,C,E,N,G,Q,S,Y,A,P,H,L,I, |
| 18 | 18 | WTK,VMG,NRT, | F,K,T,R,M,D,C,E,N,G,Q,S,Y,A,P,H,L,I, |
| 18 | 18 | WTK,VMG,NRC, | F,K,T,R,M,D,C,E,N,G,Q,S,Y,A,P,H,L,I, |
| 18 | 18 | WTS,VMA,NRT, | F,K,T,R,M,D,C,E,N,G,Q,S,Y,A,P,H,L,I, |
| 18 | 18 | WTS,VMA,NRC, | F,K,T,R,M,D,C,E,N,G,Q,S,Y,A,P,H,L,I, |
| 18 | 18 | WTS,VMG,NRT, | F,K,T,R,M,D,C,E,N,G,Q,S,Y,A,P,H,L,I, |
| 18 | 18 | WTS,VMG,NRC, | F,K,T,R,M,D,C,E,N,G,Q,S,Y,A,P,H,L,I, |
| 18 | 18 | VMA,WGK,NWT, | F,K,T,R,D,C,E,N,Q,S,Y,V,A,W,P,L,H,I, |
| 18 | 18 | VMA,WGK,NWC, | F,K,T,R,D,C,E,N,Q,S,V,Y,A,W,P,H,L,I, |
| 18 | 18 | WGK,VMG,NWT, | F,K,T,R,D,C,E,N,Q,S,Y,V,A,W,P,L,H,I, |
| 18 | 18 | WGK,VMG,NWC, | F,K,T,R,D,C,E,N,Q,S,V,Y,A,W,P,H,L,I, |
| 18 | 18 | WGS,VMA,NWT, | F,K,T,R,D,C,E,N,Q,S,Y,V,A,W,P,L,H,I, |
| 18 | 18 | WGS,VMA,NWC, | F,K,T,R,D,C,E,N,Q,S,V,Y,A,W,P,H,L,I, |
| 18 | 18 | WGS,VMG,NWT, | F,K,T,R,D,C,E,N,Q,S,Y,V,A,W,P,L,H,I, |
| 18 | 18 | WGS,VMG,NWC, | F,K,T,R,D,C,E,N,Q,S,V,Y,A,W,P,H,L,I, |
| 18 | 18 | VMA,WKK,NAT, | F,K,R,T,M,D,C,E,N,S,Q,Y,A,W,P,H,L,I, |
| 18 | 18 | VMA,WKK,NAC, | F,K,R,T,M,D,C,E,N,S,Q,Y,A,W,P,H,L,I, |
| 18 | 18 | WKK,NAT,VMG, | F,K,R,T,M,D,C,E,N,S,Q,Y,A,W,P,H,L,I, |
| 18 | 18 | WKK,NAC,VMG, | F,K,R,T,M,D,C,E,N,S,Q,Y,A,W,P,H,L,I, |
| 18 | 18 | VMA,NAT,WKS, | F,K,R,T,M,D,C,E,N,S,Q,Y,A,W,P,H,L,I, |
| 18 | 18 | VMA,NAC,WKS, | F,K,R,T,M,D,C,E,N,S,Q,Y,A,W,P,H,L,I, |
| 18 | 18 | NAT,WKS,VMG, | F,K,R,T,M,D,C,E,N,S,Q,Y,A,W,P,H,L,I, |
| 18 | 18 | NAC,WKS,VMG, | F,K,R,T,M,D,C,E,N,S,Q,Y,A,W,P,H,L,I, |
| 18 | 18 | BGG,VHG,WDT, | F,K,T,R,M,C,E,N,G,S,Q,Y,V,A,W,P,L,I, |
| 18 | 18 | VMA,DKG,WDT, | F,K,R,T,M,C,E,N,G,Q,S,Y,V,A,W,P,L,I, |
| 18 | 18 | DKG,VMG,WDT, | F,K,R,T,M,C,E,N,G,Q,S,Y,V,A,W,P,L,I, |
| 18 | 18 | DGG,VHG,WDT, | F,K,R,T,M,C,E,N,G,S,Q,Y,V,A,W,P,L,I, |
| 18 | 18 | BGG,VHG,WDC, | F,K,T,R,M,C,E,N,G,S,Q,Y,V,A,W,P,L,I, |

(continued)

| Codons | AA | Degenerate Codons | Amino Acids |
|---|---|---|---|
| 18 | 18 | VMA,DKG,WDC, | F,K,R,T,M,C,E,N,G,Q,S,Y,V,A,W,P,L,I, |
| 18 | 18 | DKG,VMG,WDC, | F,K,R,T,M,C,E,N,G,Q,S,Y,V,A,W,P,L,I, |
| 18 | 18 | DGG,VHG,WDC, | F,K,R,T,M,C,E,N,G,S,Q,Y,V,A,W,P,L,I, |
| 18 | 18 | BGT,VAA,NHT, | F,K,R,T,C,D,E,N,G,Q,S,Y,V,A,P,L,H,I, |
| 18 | 18 | BGT,VAA,NHC, | F,K,R,T,C,D,E,N,G,Q,S,V,Y,A,P,H,L,I, |
| 18 | 18 | VAG,BGT,NHT, | F,K,R,T,C,D,E,N,G,Q,S,Y,V,A,P,L,H,I, |
| 18 | 18 | VAG,BGT,NHC, | F,K,R,T,C,D,E,N,G,Q,S,V,Y,A,P,H,L,I, |
| 18 | 18 | BGG,VAA,NHT, | F,K,R,T,D,E,N,G,Q,S,Y,V,A,W,P,L,H,I, |
| 18 | 18 | BGG,VAA,NHC, | F,K,R,T,D,E,N,G,Q,S,V,Y,A,W,P,H,L,I, |
| 18 | 18 | VAG,BGG,NHT, | F,K,R,T,D,E,N,G,Q,S,Y,V,A,W,P,L,H,I, |
| 18 | 18 | VAG,BGG,NHC, | F,K,R,T,D,E,N,G,Q,S,V,Y,A,W,P,H,L,I, |
| 18 | 18 | BGC,VAA,NHT, | F,K,R,T,C,D,E,N,G,Q,S,Y,V,A,P,L,H,I, |
| 18 | 18 | BGC,VAA,NHC, | F,K,R,T,C,D,E,N,G,Q,S,V,Y,A,P,H,L,I, |
| 18 | 18 | VAG,BGC,NHT, | F,K,R,T,C,D,E,N,G,Q,S,Y,V,A,P,L,H,I, |
| 18 | 18 | VAG,BGC,NHC, | F,K,R,T,C,D,E,N,G,Q,S,V,Y,A,P,H,L,I, |
| 18 | 18 | NDT,VCA,VAA, | F,K,T,R,C,D,E,N,G,Q,S,Y,V,A,P,L,H,I, |
| 18 | 18 | NDC,VCA,VAA, | F,K,T,R,C,D,E,N,G,Q,S,V,Y,A,P,L,H,I, |
| 18 | 18 | VCT,NDT,VAA, | F,K,T,R,C,D,E,N,G,Q,S,Y,V,A,P,L,H,I, |
| 18 | 18 | NDC,VCT,VAA, | F,K,T,R,C,D,E,N,G,Q,S,V,Y,A,P,L,H,I, |
| 18 | 18 | NDT,V AA,VCG, | F,K,T,R,C,D,E,N,G,Q,S,Y,V,A,P,L,H,I, |
| 18 | 18 | NDC,VAA,VCG, | F,K,T,R,C,D,E,N,G,Q,S,V,Y,A,P,L,H,I, |
| 18 | 18 | NDT,VCC,VAA, | F,K,T,R,C,D,E,N,G,Q,S,Y,V,A,P,L,H,I, |
| 18 | 18 | NDC,VCC,VAA, | F,K,T,R,C,D,E,N,G,Q,S,V,Y,A,P,L,H,I, |
| 18 | 18 | DGG,VAA,NHT, | F,K,R,T,D,E,N,G,Q,S,Y,V,A,W,P,L,H,I, |
| 18 | 18 | DGG,VAA,NHC, | F,K,R,T,D,E,N,G,Q,S,V,Y,A,W,P,H,L,I, |
| 18 | 18 | NDT,V AG,VCA, | F,K,T,R,C,D,E,N,G,Q,S,Y,V,A,P,L,H,I, |
| 18 | 18 | NDC,VAG,VCA, | F,K,T,R,C,D,E,N,G,Q,S,V,Y,A,P,L,H,I, |
| 18 | 18 | VCT,NDT,VAG, | F,K,T,R,C,D,E,N,G,Q,S,Y,V,A,P,L,H,I, |
| 18 | 18 | NDC,VCT,VAG, | F,K,T,R,C,D,E,N,G,Q,S,V,Y,A,P,L,H,I, |
| 18 | 18 | NDT,V AG,VCG, | F,K,T,R,C,D,E,N,G,Q,S,Y,V,A,P,L,H,I, |
| 18 | 18 | NDC,VAG,VCG, | F,K,T,R,C,D,E,N,G,Q,S,V,Y,A,P,L,H,I, |
| 18 | 18 | NDT,V AG,VCC, | F,K,T,R,C,D,E,N,G,Q,S,Y,V,A,P,L,H,I, |
| 18 | 18 | NDC,VAG,VCC, | F,K,T,R,C,D,E,N,G,Q,S,V,Y,A,P,L,H,I, |
| 18 | 18 | VAG,DGG,NHT, | F,K,R,T,D,E,N,G,Q,S,Y,V,A,W,P,L,H,I, |
| 18 | 18 | VAG,DGG,NHC, | F,K,R,T,D,E,N,G,Q,S,V,Y,A,W,P,H,L,I, |
| 18 | 18 | VMA,NAT,NKT, | F,K,R,T,C,D,E,N,G,Q,S,Y,V,A,P,L,H,I, |
| 18 | 18 | VMA,NAT,NKC, | F,K,R,T,C,D,E,N,G,Q,S,Y,V,A,P,L,H,I, |
| 18 | 18 | VMA,NAC,NKT, | F,K,R,T,C,D,E,N,G,Q,S,Y,V,A,P,L,H,I, |

(continued)

| Codons | AA | Degenerate Codons | Amino Acids |
|--------|-----|-------------------|-------------|
| 18 | 18 | VMA,NAC,NKC, | F,K,R,T,C,D,E,N,G,Q,S,Y,V,A,P,L,H,I, |
| 18 | 18 | VMA,NTT,NRT, | F,K,T,R,D,C,E,N,G,Q,S,V,Y,A,P,H,L,I, |
| 18 | 18 | VMA,NTT,NRC, | F,K,T,R,D,C,E,N,G,Q,S,V,Y,A,P,H,L,I, |
| 18 | 18 | VMA,NTC,NRT, | F,K,T,R,D,C,E,N,G,Q,S,V,Y,A,P,H,L,I, |
| 18 | 18 | VMA,NTC,NRC, | F,K,T,R,D,C,E,N,G,Q,S,V,Y,A,P,H,L,I, |
| 18 | 18 | VMA,NGT,NWT, | F,K,R,T,D,C,E,N,G,S,Q,Y,V,A,P,L,H,I, |
| 18 | 18 | VMA,NGT,NWC, | F,K,R,T,D,C,E,N,G,S,Q,V,Y,A,P,H,L,I, |
| 18 | 18 | VMA,NGC,NWT, | F,K,R,T,D,C,E,N,G,S,Q,Y,V,A,P,L,H,I, |
| 18 | 18 | VMA,NGC,NWC, | F,K,R,T,D,C,E,N,G,S,Q,V,Y,A,P,H,L,I, |
| 18 | 18 | NAT,VMG,NKT, | F,K,R,T,C,D,E,N,G,Q,S,Y,V,A,P,L,H,I, |
| 18 | 18 | NAT,VMG,NKC, | F,K,R,T,C,D,E,N,G,Q,S,Y,V,A,P,L,H,I, |
| 18 | 18 | NAC,VMG,NKT, | F,K,R,T,C,D,E,N,G,Q,S,Y,V,A,P,L,H,I, |
| 18 | 18 | NAC,VMG,NKC, | F,K,R,T,C,D,E,N,G,Q,S,Y,V,A,P,L,H,I, |
| 18 | 18 | NTT,VMG,NRT, | F,K,T,R,D,C,E,N,G,Q,S,V,Y,A,P,H,L,I, |
| 18 | 18 | NTT,VMG,NRC, | F,K,T,R,D,C,E,N,G,Q,S,V,Y,A,P,H,L,I, |
| 18 | 18 | NTC,VMG,NRT, | F,K,T,R,D,C,E,N,G,Q,S,V,Y,A,P,H,L,I, |
| 18 | 18 | NTC,VMG,NRC, | F,K,T,R,D,C,E,N,G,Q,S,V,Y,A,P,H,L,I, |
| 18 | 18 | NGT,VMG,NWT, | F,K,R,T,D,C,E,N,G,S,Q,Y,V,A,P,L,H,I, |
| 18 | 18 | NGT,VMG,NWC, | F,K,R,T,D,C,E,N,G,S,Q,V,Y,A,P,H,L,I, |
| 18 | 18 | NGC,VMG,NWT, | F,K,R,T,D,C,E,N,G,S,Q,Y,V,A,P,L,H,I, |
| 18 | 18 | NGC,VMG,NWC, | F,K,R,T,D,C,E,N,G,S,Q,V,Y,A,P,H,L,I, |

Many additional useful degenerate codon sets are available. These are provided, e.g., by selecting appropriate parameters and running the codon selection algorithm described herein. Solutions using 2, 3, or even 4 or more degenerate codons to encode 12, 13, 14, 15, 16, 17, 18, 19, or 20 of the canonical amino acids are possible. In general, the use of fewer degenerate codons (e.g., 2 or 3 degenerate codons) is most desirable, as this reduces overall gene synthesis complexity. Similarly, to achieve maximum amino acid diversity at any selected position, it is generally desirable for the degenerate codon set to produce as many of the canonical amino acids as possible, e.g., 17, 18, 19 or all 20 canonical amino acids. These parameters can be selected by the user in the methods and systems (e.g., including the computer program product of the disclosure) as noted herein.

The following summary provides an approximation of the total number of codon set solutions that can be identified using 2 or 3 degenerate codons, according to the present disclosure, e.g., using the computer program product herein.

| Number of Codons | Amino Acids | | | |
|------------------|-------------|-------|------|--------|
| | 18 | 19 | 20 | Totals |
| 18 | 304 | | | 304 |
| 19 | 1536 | 24 | | 1560 |
| 20 | 6030 | 162 | | 6192 |
| 21 | 9226 | 588 | | 9814 |
| 22 | 26170 | 1402 | 14 | 27586 |
| 23 | 24396 | 2090 | 40 | 26526 |
| 24 | 72226 | 6328 | 82 | 78636 |
| 25 | 45637 | 4988 | 160 | 50785 |

(continued)

| Number of Codons | Amino Acids | | | |
|---|---|---|---|---|
| | 18 | 19 | 20 | Totals |
| Grand Total | 185525 | 15582 | 296 | 201403 |

Thus, for example, for the case where 18 amino acids are encoded, there are approximately 185,525 codon sets comprising between 18 and 25 codons, using 2 or 3 degenerate codons. Of these, for example, there are 304 solutions that encode 18 amino acids using 18 codons. Similarly, there are 296 codon sets using 2 or 3 degenerate codons that encode 20 amino acids, using 25 or fewer codons. There are 24 codon sets that use 19 codons to encode 19 amino acids. Because of the large number of total solutions, and in the interest of clarity and brevity, all solutions are not listed. However, all can be specifically determined using the computer program product herein, set to any desired set of parameters (e.g., by specifying the number of degenerate codons, the number of total codons and the number of amino acids to be encoded).

Methods and Program Products to Identify Degenerate Codon Sets

Systems and methods, including computer implemented methods for identifying codon sets such as those denoted in the tables above are a feature of the disclosure. These systems and methods can include user executable instructions, e.g., embodied in a computer memory or computer readable medium, e.g., in a computer, information appliance, or other system. In one aspect, the disclosure includes a method (e.g., a computer assisted method) that allows a user to determine codon sets comprising degenerate codons that encode a desired set of amino acids. The output from the methods and systems can yield, e.g., useful degenerate codon sets, along with the number of total codons and the number and composition of amino acids encoded by the degenerate codon set.

For example, the method can include providing a user interface that permits a user to input a desired number (and/or composition) of amino acids to be encoded, a total number of codons to encode the desired number of amino acids, and a number of degenerate codons to be used in codon sets comprising the total number of codons. This interface can be embodied in an information appliance such as a personal computer, or can be a web interface, which executes a server across the internet or across an intranet. The interface can include a typical keyboard for entry of user parameters, or can include a touch screen, pointing device or other typical apparatus for entering information from a user.

Computer executable instructions are provided to yield a list of degenerate codon sets, amino acids, and/or codons to the user, to determine which codons encode the amino acids specified by the user. An example program is provided below. This list can be displayed on a standard computer display, or can be printed for review by a user.

For example, the number of degenerate codons specified by the user can be between, e.g., 2 and 5, but is more typically between 2 and 4 and usually between 2 and 3. The number of amino acids can be between 12 and 20, and, in many preferred instances, is typically between 18 and 20 (e.g., to provide maximum diversity at each position to be varied in a variant polynucleotide or polypeptide). The number of total codons is less than 32, and is typically less than 25, and, in some preferred aspects, can be 22 or less. All ranges herein are inclusive, unless specified otherwise. Thus, "between 2 and 4" includes the numbers 2 and 4, as well as the number 3.

A corresponding system comprising a computer readable medium containing computer interpretable logic or instructions is also a feature of the disclosure. The system can, e.g., include or be embodied within a computer, a network (e.g., comprising a client interface and a server), or can be internet based (e.g., comprising a web interface for user input and a remote server). The system accepts a user instruction specifying a desired number of amino acids to be encoded, a total number of codons to encode the desired number of amino acids, and a number of degenerate codons to be used in the codon sets. The logic or instructions outputs a list of degenerate codons to the user. In one typical example, disclosed is a system comprising a computer program, e.g., embodied or residing in a computer readable medium, or on a computer or on a server or information appliance.

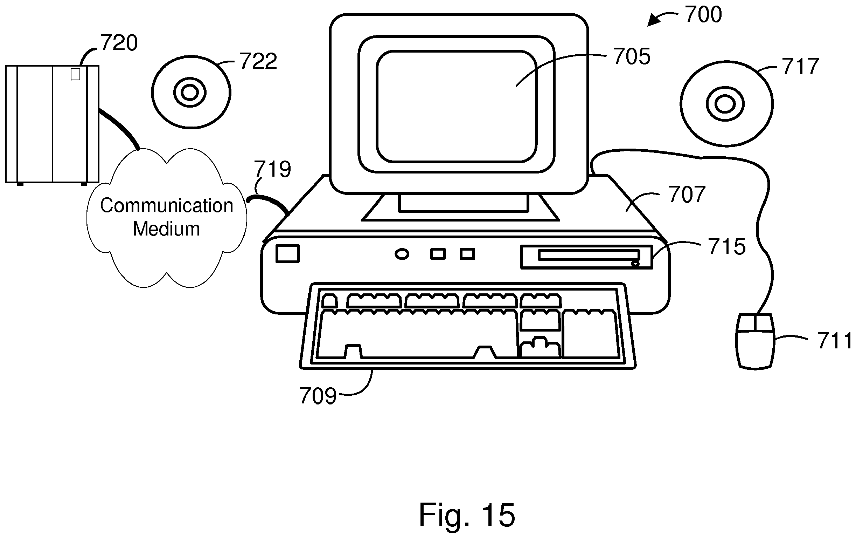

FIG. 15 shows an information appliance (or digital device) 700 that may be understood as a logical apparatus that can read instructions from media 717 and/or network port 719, e.g., relating to degenerate codon number, number of total codons, and/or number of amino acids to be encoded, along with, e.g., any computer executable instructions to deliver an output (e.g., a list of degenerate codons, encoded amino acids, or the like) to the user. Port 719 can optionally be connected to server 720 having fixed media 722, which can include the relevant instructions. This connection can be across a network, or, e.g., across the internet (or both). Apparatus 700 can thereafter use those instructions to direct server or client logic, as understood in the art, to embody aspects of the disclosure, e.g., to provide a relevant set or list of sets of degenerate codons to a user. One type of logical apparatus that may embody the disclosure is a computer system as illustrated in 700, containing CPU 707, optional input devices 709 and 711, disk drives 715 and optional monitor 705. Fixed media 717, or fixed media 722 over port 719, may be used to program such a system and may represent a disk-type optical or magnetic media, magnetic tape, solid state dynamic or static memory, etc.. In specific instances, the disclosure can be embodied in whole or in part as software recorded on this fixed media. Communication port 719 can also be used to initially receive instructions that are used to program such a system (e.g., with a number of codons or number or type of amino acids to be encoded) and may represent any type of communication connection.

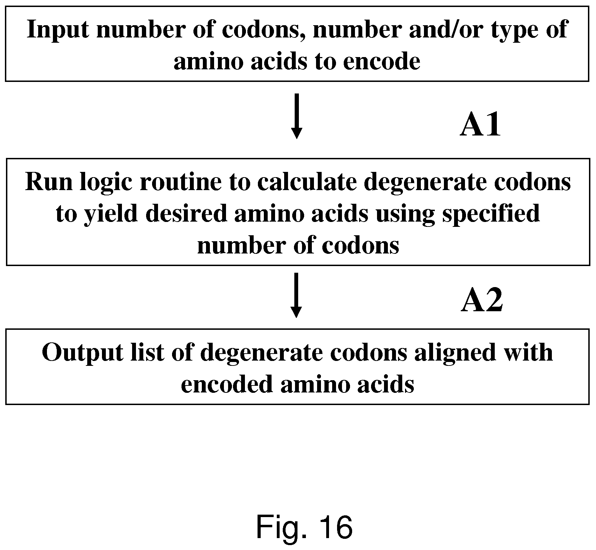

An overall process flow chart for the system and related method is provided in Figure 16, in which a number of codons and a number and/or type of amino acids to be encoded is entered by a user. A computer executable logic routing (e.g., a set of instructions or a computer program) is run to calculate or otherwise determine degenerate codon sets that provide the desired amino acids using the desired number of codons. The system then outputs a list to the user, e.g., in the form of a display of the list, or of a printed output.

In one example, the following computer program was used to identify appropriate codon sets.

```
package com.codexis.codonset;

/**************************

 * Copyright (C) 2010 Richard J. Fox, Codexis, Inc.

 ***********************/

import java.util.*;

import java.io.*;

import org.biojava.bio.symbol.Symbol;

public class Finder {

      private CodonBiasTable cbt;

      private long evaluations = 0;

  private class AmbiguousCodon {

      Symbol ambiguousCodon;

      Set<Symbol> allCodons;

      Set<String> allCodonStrings;

      Set<String> aaSet;

          public Set<String> getAminoAcids() {

       if (aaSet == null) {

          aaSet = new HashSet<String>();

          for (Iterator<Symbol> iter = allCodons.iterator();
      iter.hasNext();) {

            Symbol codon = iter.next();

            Symbol aa = getCodonBiasTable().getAminoAcidForCodon(codon);

            aaSet.add(aa.getName()); } }

        return aaSet; }

      public Set<String> getAllCodonStrings() {

        if (allCodonStrings == null) {
```

```
        allCodonStrings = new TreeSet<String>();
        for (Iterator<Symbol> iter = allCodons.iterator(); iter.hasNext();)
{
          Symbol symbol = iter.next();
          String codonString = getCodonBiasTable().getStringForCodon(symbol);
          allCodonStrings.add(codonString);
        } }
      return allCodonStrings; }
    public String getAmbiguousCodon() {
      return getCodonBiasTable().getStringForCodon(ambiguousCodon); } }
  private void searchSet(List<AmbiguousCodon> fullSet, int iStart, int
currentCodons, int maxCodons, int minAminoAcids, Set<String>
currentAminoAcids, Writer writer, Set<String> currentAmbiguousCodon, int
depth, int bottom) {


          /* Call this method to launch a recursive search of all
possible codons satisfying the following criteria:
          * maxCodons = maximum number of codons a solution set can
contain.
          * minAminoAcids = minimum number of amino acids a solution
set should code for.
          * bottom = recursive depth (equal to the number of degenerate
codons in a solution set).
                      */


  try {   for (int i = iStart; i < fullSet.size(); i++) {
    AmbiguousCodon ac = fullSet.get(i);
    int totalCodons = currentCodons + ac.getAllCodonStrings().size();
    if (depth == 1) {
    System.out.println((1.0*i)/fullSet.size()); //Print state of outer
loop }
    if (totalCodons <= maxCodons) {
    Set<String> aminoAcids = ac.getAminoAcids();
    Set<String> allAminoAcids = new HashSet<String>();
    allAminoAcids.addAll(currentAminoAcids);
    allAminoAcids.addAll(aminoAcids);
    Set<String> allAmbiguousCodons = new HashSet<String>();
    allAmbiguousCodons.addAll(currentAmbiguousCodon);
    allAmbiguousCodons.add(ac.getAmbiguousCodon());
    allAminoAcids.addAll(aminoAcids);
    allAminoAcids.addAll(ac.getAminoAcids());
    if (depth == bottom) {
     evaluations++;
```

```
            if (allAminoAcids.size() >= minAminoAcids) {

            writer.write(totalCodons + "\t" + allAminoAcids.size() + "\t");

            writer.write("Codons: ");

            for (Iterator<String> iter = allAmbiguousCodons.iterator();
iter.hasNext();) {

             writer.write(iter.next() + ","); }

            writer.write("\tAA: ");

            for (Iterator<String> iter = allAminoAcids.iterator();
iter.hasNext();) {

             writer.write(iter.next() + ","); }

            writer.write("\n");

            writer.flush(); } }

          else {

           int aaNeeded = minAminoAcids - allAminoAcids.size();

           // Do not pursue infeasible solutions.  If we still need at least
aaNeeded amino acids and we already have

           // used totalCodons codons then we cannot exceed the maxCodons
constraint.

           if (totalCodons + aaNeeded <= maxCodons) {

            searchSet(fullSet, i+1, totalCodons, maxCodons, minAminoAcids,
allAminoAcids, writer, allAmbiguousCodons, depth+1, bottom);} } } }

    } catch (Exception e) {

    throw new RuntimeException(e); } }

  public void exhaustiveSearch(int maxCodons,int minAminoAcids, int bottom)
{

   try {

   FileWriter writer = new FileWriter("c:/temp/finder.txt");

   String allBases = "ATGCKMRYSWBVHDN";

   List<AmbiguousCodon> fullSet = new ArrayList<AmbiguousCodon>();

   for (int i = 0; i < allBases.length(); i++) {

    for (int j = 0; j < allBases.length(); j++) {

     for (int k = 0; k < allBases.length(); k++) {

      String codon = "" + allBases.charAt(i) + allBases.charAt(j) +
allBases.charAt(k);

      Symbol symbol = getCodonBiasTable().getCodonForString(codon);

      AmbiguousCodon dc = new AmbiguousCodon();

      Set<Symbol> allCodons =
getCodonBiasTable().getCodonsForAmbiguousCodon(symbol);

      dc.ambiguousCodon = symbol;

      dc.allCodons = allCodons;

      int numCodons = dc.allCodons.size();
```

```
        int numAminoAcids = dc.getAminoAcids().size();

        if (!dc.getAminoAcids().contains("TER")) {

                // Rule out any ambiguous codons that cannot satisfy the maximum
        codons constraint. This assumes

                // that even if the other ambiguous codons in the final coding
        set are perfect (1 codon each for

                // every amino acid), there would still be no way to satisfy the
        maximum codons constraint.

                if (minAminoAcids-numAminoAcids <= maxCodons-numCodons) {

                    fullSet.add(dc); } } } } }

        System.out.println("Full set size: " + fullSet.size());

        int iStart = 0;

        int depth = 1;

        int totalCodons = 0;

        Set<String> allAminoAcids = new HashSet<String>();

        Set<String> allAmbiguousCodons = new HashSet<String>();

        searchSet(fullSet, iStart, totalCodons, maxCodons, minAminoAcids,
        allAminoAcids, writer, allAmbiguousCodons, depth, bottom);

        writer.close();

        System.out.println("Evalutations: " + evaluations);

        } catch (Exception e) {

        throw new RuntimeException(e); } }

          public static void main(String[] args) {

        Finder finder = new Finder();

        finder.exhaustiveSearch(25,18,2); }

       public CodonBiasTable getCodonBiasTable() {

        if (this.cbt == null) {this.cbt = new CodonBiasTable(null,0.0);}

        return this.cbt; }}
```

## Making Degenerate Oligonucleotides

Degenerate oligonucleotides can be made, incorporating degenerate codons as noted above, at sites of codon diversity. Methods of making oligonucleotides generally are well known, e.g., as taught in Oligonucleotide Synthesis: Methods and Applications (Methods in Molecular Biology) (2004) Piet Herdewijn (Editor) Humana Press ISBN-10: 1588292339, or Protocols for Oligonucleotide Conjugates: Synthesis and Analytical Techniques (Methods in Molecular Biology) (1993) Sudhir Agrawal, Humana Press ISBN-10: 0896032523. Degenerate oligonucleotides can also be commercially ordered, e.g., from Invitrogen (Carlsbad, California), or Eurofins MWG Operon (Huntsville, AL).

Degenerate oligonucleotides are used in a variety of polymerase mediated variant construction methods as noted herein, including in automated high-throughput methods. In general, degenerate oligonucleotides can be used as primers for a polymerase, as in PCR, e.g., using a reference polynucleotide as a template. The oligonucleotides are incorporated into variant amplicons, e.g., by performing PCR. Any of a variety of PCR and/or cloning methods as described herein can be used to make libraries of variant polynucleotides for screening.

## CONSTRUCTING POLYNUCLEOTIDE VARIANTS

Polynucleotide variants that comprise selected degenerate codons can be constructed in any of a variety of ways, typically by incorporating degenerate oligonucleotides, made as noted above, into amplicons of interest. For example, PCR-based reassembly methods such as described in USSN 61/061,581 filed June 13, 2008; USSN 12/483,089 filed June 11, 2009; PCT/US2009/047046 filed June 11, 2009; USSN 12/562,988 filed September 18, 2009; and PCT/US2009/057507 filed September 18, 2009, can be used. In another preferred embodiment, variant construction can be combined with cloning into an expression vector, e.g., using megaprimer PCR, abutting primer PCR, or overlapping primer PCR on circular templates, as is described herein.

In one example, polynucleotide variants having a defined set of sequence differences from a reference polynucleotide sequence are generated as part of the overall library construction process. In some embodiments, these methods are applicable for generating polynucleotides encoding polypeptide variants having defined differences in amino acid sequence as compared to a reference polypeptide, e.g., using the codon sets of the invention. Optionally, the polynucleotide variants can also have defined nucleotide differences in non-coding regions, e.g., silent mutations. The polynucleotides are efficiently generated by using sets of polynucleotide fragments, where the members of the sets encode one or more of the amino acid differences as compared to a reference polypeptide sequence, and the polynucleotide fragments are designed to have overlapping adjacent regions such that selection of an appropriate set of fragments, with and/or without mutations, allows their assembly into a polynucleotide variant, e.g., via PCR-based reassembly.

In one example, generating a polynucleotide encoding a polypeptide having an amino acid sequence with one or more defined differences in amino acid residues includes: (a) selecting a plurality of defined amino acid residue differences relative to a reference amino acid sequence; (b) defining overlapping segments of a polynucleotide sequence encoding the polypeptide with the different amino acid sequence, or optionally the reference polypeptide, with each segment being bounded by a set of forward and reverse primer binding sequences, wherein a polynucleotide sequence difference encoding each of the plurality of amino acid residue differences is encompassed in the sequences of the forward and/or reverse primers that bind to the primer binding sequences; (c) amplifying each segment with the set of forward and reverse primers, wherein selected forward and/or reverse primer contain the polynucleotide sequence differences, to generate a library of amplicons comprising members encoding the defined amino acid differences and wherein the library comprises members sufficient for assembling two or more different amino acid sequence permutations of the defined amino acid residue differences; (d) assembling from the library a set of amplicons having complementary adjacent regions that together encode the polypeptide with a defined amino acid sequence permutation having one or more defined amino acid residue differences; and (e) replicating the set of assembled amplicons to synthesize the polynucleotide encoding the polypeptide. Further details on this and related reassembly strategies can be found in PCT/US2009/057507. In this set of methods, the defined amino acid residues are typically varied according to a codon set as noted above. The amplification primers are typically constructed as degenerate primers, as noted above.

A library of amplicons containing all of the defined amino acid differences allows the synthesis of a plurality of polynucleotides that encode all possible permutations of amino acid sequences in encoded polypeptides.

As will be apparent to the skilled artisan, dividing the polynucleotide into defined segments for amplification can be accomplished using techniques well known in the art. In some embodiments, since the segments are defined by primer binding sequences, which are themselves used to introduce mutations into the amplicon, division of the polynucleotide into segments can initially take into account the location of the mutations on the polynucleotide. The divisions of the polynucleotide into segments can also take into account the total length of the polynucleotide, the efficiency of replication (e.g., amplification of segments), and the desired number of amplicons for assembly. Other considerations will be apparent to the skilled artisan.

Amplification reactions can be affected by sequence, type of polymerase used, efficiency of primers, and unwanted side reactions (e.g., primer dimers). Thus, in some embodiments, depending on the total length of the polynucleotide to be assembled, the segment lengths can be 2000 bases or less, 1500 bases or less, 1200 bases or less, 1000 bases or less, 900 bases or less, 800 bases or less, 700 bases or less, 600 bases or less, 500 bases or less, 400 bases or less, 300 bases or less, 250 bases or less, or 200 bases or less to about 100 or as few as about 50 bases in length. Generally, length of the segments is from about 50 to about 1000 bases, about 200 to 1000 bases, about 300 to 700 bases, or about 400 to 600 bases, with about 500 bases being useful average length given the efficiency of polymerases used in amplification reactions. In various embodiments, the segments are overlapping such that the amplicons produced therefrom will also have overlapping adjacent regions (e.g., overlapping complementary regions) for assembling the polynucleotide.

In some embodiments, the adjacent overlapping regions are of sufficient length and complementarity to permit the formation of stable annealed (hybridized) amplicons during assembly of the polynucleotide. Thus, in some embodiments, the length of overlap can be 4 or more nucleotides, 5 or more nucleotides, 6 or more nucleotides, 8 or more nucleotides, 10 or more nucleotides, 15 or more nucleotides, 20 or more nucleotides, 25 or more nucleotides, 30 or more nucleotides, 40 or more nucleotides, 50 or more nucleotides, and 100 or less, 90 or less, 80 or less, 70 or less, 60 or less nucleotides in length as permitted by the ability to form stable annealed amplicons.

Since the overlap regions can include the primer binding sequences used to generate the amplicons, the length of overlap can account for any differences in the sequence of the primer (e.g., forward and/or reverse) used to generate the polynucleotide differences encoding the mutation to be introduced.

In some embodiments, the segments are bounded by primer binding sequences to which the forward/reverse primers anneal. Where appropriate, the primer binding sequences that define the segments can also encompass the position of the polynucleotide that encodes an amino acid sequence difference. The primer binding sequence can be of any sufficient length to anneal to the primer (forward or reverse) during the amplification reaction. Accordingly the primer binding sequence can be 100 bases or less, 90 bases or less, 80 bases or less, 70 bases or less, 60 bases or less, 50 bases or less, 40 bases or less, 30 bases or less, 20 bases or less 15 bases or less, to about 8 bases or 10 bases. In some embodiments, the length of the primer binding sequences can comprise from about 8 to 50 bases, about 8 to 40 bases, about 10 to 30 bases, or about 15 to 25 bases. The primers typically can comprise lengths complementary to the primer binding sequences described above. Accordingly, in some embodiments, the length of the forward/reverse primers can be about 60 nucleotides or less, 50 nucleotides or less, 40 nucleotides or less, 30 nucleotides or less, 20 nucleotides or less 15 nucleotides or less, to about 10 nucleotides or even 8 nucleotides. In some embodiments, the length of the forward/reverse primers can be from about 8 to 50 nucleotides, about 8 to 40 nucleotides, about 10 to 30 nucleotides, or about 15 to 25 nucleotides.

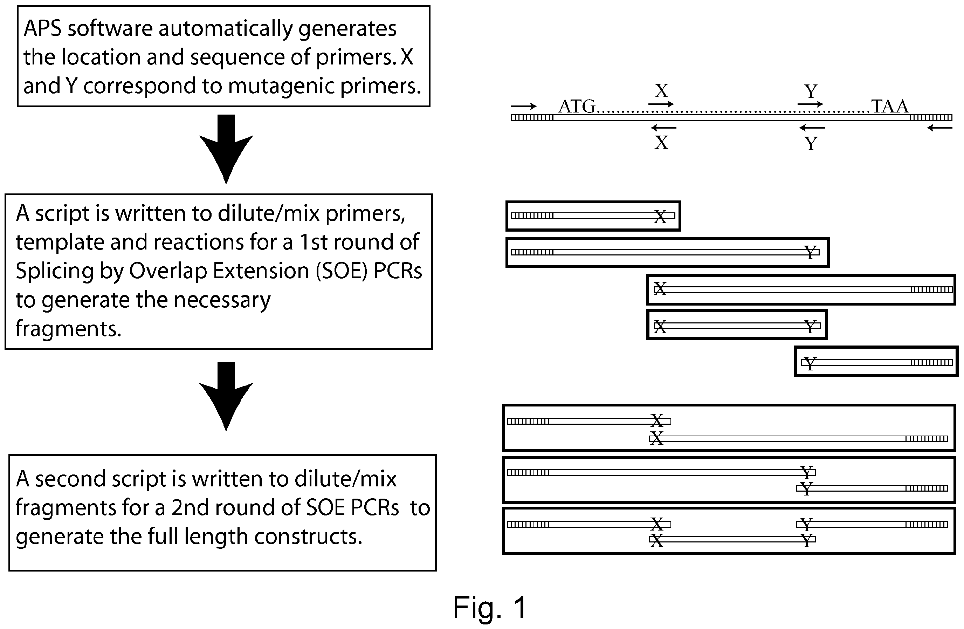

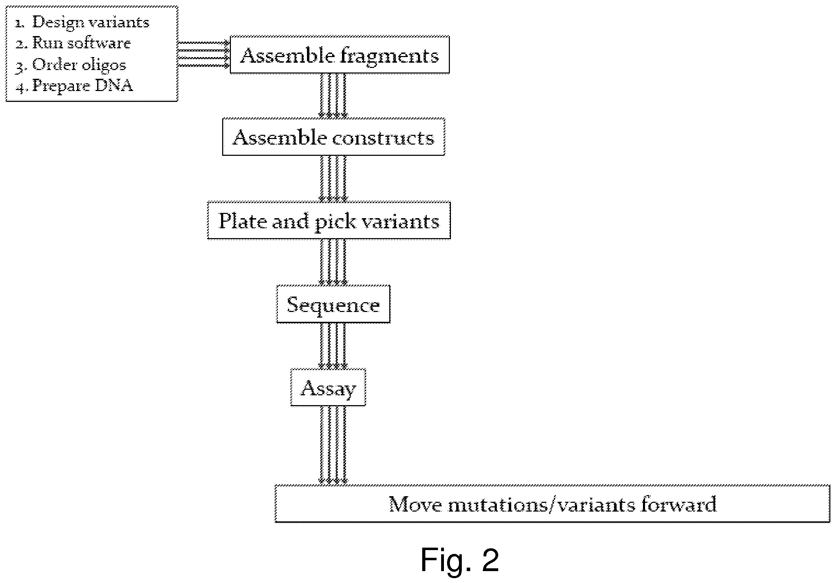

Figure 1 provides an overview of an automated example relating to assembling variants using splicing by overlap extension PCR (also known as PCR SOEing). This process is referred to as "Automated Parallel SOEing" ("APS"), or "Multiplexed Gene SOEing." As shown, APS software is used to select primers (W and Y correspond to mutagenic primers in Figure 1). APS software automatically generates the location and sequence of the primers. An automated set of instructions is used to dilute/mix primers for a first round of PCR splicing by overlap extension. The instructions, e.g., control any fluid handling/ thermocycling components of an automated system. The instructions then direct a second round of PCR splicing by overlap extension. Figure 2 provides a process flow chart for mutagenesis by APS ("MAPS"). Overall, about 85% of clones deriving from typical MAPS processes have sequences as would be predicted upon final assembly.

Further details regarding splicing by overlap extension can be found in Horton et al. (1989) "Engineering hybrid genes without the use of restriction enzymes: gene splicing by overlap extension," Gene 77(1):61-8; Horton et al. (1990) "Gene splicing by overlap extension: tailor-made genes using the polymerase chain reaction" Biotechniques 8(5):528-35; Horton et al. (1997) "Splicing by overlap extension by PCR using asymmetric amplification: an improved technique for the generation of hybrid proteins of immunological interest" Gene 186(1):29-35, and in PCR Cloning Protocols (Methods in Molecular Biology) Bing-Yuan Chen (Editor), Harry W. Janes (Editor) Humana Press; 2nd edition (2002) ISBN-10: 0896039692.

Where the primer contains a sequence encoding a defined amino acid difference, e.g., using a codon selected from a degenerate codon set as noted herein, the mutation can be located at a region of the primer that does not interfere with primer extension. In some embodiments, the mutation is located at about the middle of the mutagenic primer, e.g., where the primer has a $T_m$ that is sufficient to anneal to the template nucleic acid and serve as a primer for the polymerase mediated extension reaction. In some embodiments, the polynucleotide sequence differences can be located, depending on the length of the primer, about 5 bases, 6 bases, 8 bases, 10 bases, 12 bases, 15 bases, 20 bases, 25 bases from the 3' end of the primer. Accordingly, in some embodiments the length of the forward/reverse primers can be from about 8 to 50 nucleotides, about 8 to 40 nucleotides, about 10 to 30 nucleotides, or about 15 to 25 nucleotides, and further comprise nucleotide sequence difference at about the middle of the primer. Thus, in some embodiments the forward/reverse primers are about 50 nucleotides in length with a nucleotide difference about 25 nucleotides from the 3' end, about 40 nucleotides in length with a nucleotide difference about 20 nucleotides from the 3' end, about 30 nucleotides in length with a nucleotide difference about 15 nucleotides from the 3' end, about 25 nucleotides in length with a nucleotide difference about 12 nucleotides from the 3' end, or about 20 nucleotides in length with a nucleotide difference about 10 nucleotides from the 3' end.

The stability of the oligonucleotide primers, e.g., the thermal melting temperature, is a function of ion strength, temperature, G/C content, and the presence of chaotropic agents and can be calculated using known methods for predicting melting temperatures (see, e.g., Baldino et al., Methods Enzymology 168:761-777; Bolton et al., 1962, Proc. Natl. Acad. Sci. USA 48:1390; Bresslauer et al., 1986, Proc. Natl. Acad. Sci USA 83:8893-8897; Freier et al., 1986, Proc. Natl. Acad. Sci USA 83:9373-9377; Kierzek et al., Biochemistry 25:7840-7846; Rychlik et al., 1990, Nucleic Acids Res 18:6409-6412 (erratum, 1991, Nucleic Acids Res 19:698); Sambrook et al., 2001, Molecular Cloning: A Laboratory Manual, 3rd Ed., Cold Spring Harbor Laboratory Press, NY; Suggs et al., 1981, In Developmental Biology Using Purified Genes (Brown et al., eds.), pp. 683-693, Academic Press; and Wetmur, 1991, Crit Rev Biochem Mol Biol 26:227-259).

To generate the library of amplicons, forward and reverse primers that anneal to the primer binding sequences of each segment of the polynucleotide are used in an amplification reaction to generate amplicons. Where the amplicon has a polynucleotide difference encoding a defined amino acid change relative to the reference sequence, the sequence of the forward and/or reverse primers are designed to introduce the different sequence (*i.e.,* mutation) in the amplification reaction. Suitable combinations of forward and reverse primers are used to generate a library of amplicons comprising members that can encode each of the plurality of amino acid residue differences.

In some embodiments, the sets of forward and reverse primers can be stored in an array, for example a primer array, such that they can be easily accessed when amplicons are needed for synthesis of a polynucleotide encoding a defined amino acid sequence permutation. The oligonucleotide primers can be used to introduce any type of mutation selected in the defined plurality of amino acid residue differences, including, among others, amino acid insertions, deletions, and substitutions. The substitutions can be conservative or nonconservative mutations, as dictated by the chosen plurality of amino acid residue differences. These changes can include, or can be separate from changes introduced by incorporating degenerate codons of the invention at selected codon sites.

In many embodiments, libraries comprising more than one amino acid sequence difference at the same amino acid residue position of a polypeptide sequence are desirable. In these embodiments, different amplicons from the same overlapping segment can be generated, where each amplicon is prepared with forward and reverse primer pairs for each defined mutation at a given residue position. To prepare a polynucleotide encoding a particular sequence permutation at that specific amino acid residue position, one of the amplicons containing the desired mutation (a defined nucleotide difference) is chosen and assembled as a member of the set of amplicons to generate the polynucleotide encoding a polypeptide containing the desired mutation(s) at the specified amino acid residue position.

In some embodiments, more than one pair of primers (e.g., a set of degenerate primers) can be used to generate a set of amplicons (e.g., polynucleotide fragments) that can be used to assemble a set of polynucleotide variants encoding polypeptides having more amino acid residue changes (e.g., substitutions) at a specific defined position. The polynucleotide variants assembled from the amplicons made using degenerate primers can be sequenced before or after their encoded polypeptide is assayed in order to determine the specific sequence at the position of interest.

Optionally, an overlapping segment defined for a polynucleotide sequence may not have any associated mutations. Additionally, the same segment may in one amino acid sequence permutation encompass a specified mutation, but in some sequence permutations may not have any mutation associated with the segment. Thus in some embodiments, the library of amplicons can contain members that do not have any polynucleotide sequence differences as compared to the reference sequence for a particular segment. These bridging polynucleotides, which have no associated changes in sequence as compared to the reference sequence, can be used as a connector to assemble a complete polynucleotide.

With the appropriate choice of segments, the amplicon library comprises members that can be used to assemble at least two or more different amino acid sequence permutations of the defined amino acid differences relative to the reference sequence. For example, a plurality of mutations defined by amino acid residue differences A and B can have the following permutations: A alone, B alone, or A and B. Thus the amplicon library has sufficient members to generate an amino acid sequence permutation having independently an A mutation or B mutation. In some embodiments, the amplicon library has members sufficient to generate every amino acid sequence permutation of the defined amino acid residue differences relative to the reference sequence. Thus, for the given example, the amplicon library has sufficient members to generate amino acid sequence permutations having independently an A mutation or B mutation, or an A + B mutation.

Since the size of the amplicons will approximately correspond to the size of the segments, the amplicons can be 2000 bases or less, 1500 bases or less, 1200 bases or less, 1000 bases or less, 900 bases or less, 800 bases or less, 700 bases or less, 600 bases or less, 500 bases or less, 400 bases or less, 300 bases or less, 250 bases or less, or 200 bases or less to about 100 or as few as about 50 bases in length. Generally, length of the amplicons is from about 50 to about 1000 bases, about 200 to 1000 bases, about 300 to 700 bases, or about 400 to 600 bases, with about 500 bases or less a useful length given the efficiency of polymerases used in amplification reactions. In some embodiments, the amplicons are about 400 bases or less in length.

Generally, the amplification reaction can use any enzyme used for polymerase mediated extension reactions, such as Taq polymerase, Pfu polymerase, Pwo polymerase, Tfl polymerase, rTth polymerase, Tli polymerase, Tma polymerases, or a Klenow fragment. Conditions for amplifying a polynucleotide segment using polymerase chain reaction can follow standard conditions known in the art. See, e.g., Viljoen, et al. (2005) Molecular Diagnostic PCR Handbook Springer, ISBN 1402034032; PCR Cloning Protocols (Methods in Molecular Biology) Bing-Yuan Chen (Editor), Harry W. Janes (Editor) Humana Press; 2nd edition (2002) ISBN-10: 0896039692; Directed Enzyme Evolution: Screening and Selection Methods (Methods in Molecular Biology) Frances H. Arnold (Editor), George Georgiou (Editor) Humana Press; 1st edition (2003) ISBN-10: 58829286X; Directed Evolution Library Creation: Methods and Protocols (Methods in Molecular Biology) (Hardcover) Frances H. Arnold (Editor), George Georgiou (Editor) Humana Press; st1 edition (2003) ISBN-10: 1588292851; Short Protocols in Molecular Biology (2 volume set); Ausubel et al. (Editors) Current Protocols; 52 edition (2002) ISBN-10: 0471250929; and PCR Protocols A Guide to Methods and Applications (Innis et al. eds.) Academic Press Inc. San Diego, CA (1990) (Innis).

Desirably, amplification of each amplicon can be carried out in separate reactions, minimizing the need to isolate one amplicon product from another amplicons, and reducing PCR artifacts. However, the amplification reactions for two or more amplicons can be carried out in a single reaction and the products isolated, such as by electrophoresis or chromatography. In some embodiments, the products of the amplification reaction can be treated with various combinations of exonucleases and phosphatases to remove remaining primers and free nucleotides (e.g., combination of exonuclease I and alkaline phosphatase). DPN 1 can also be used to eliminate template nucleic acids from an amplification mixture, e.g., by cleaving template nucleic acid isolated from an in vivo source (DPN 1 cleaves methylated DNA).

To generate the polynucleotide encoding the polypeptide with the defined amino acid sequence permutation, a set of amplicons having complementary overlapping regions can be selected and assembled under conditions that permit the annealing of the complementary overlapping regions to each other. For example, the amplicons can be denatured and then allowed to anneal to form a complex of amplicons that together encode the polypeptide with a defined amino acid sequence permutation having one or more of the amino acid residue differences relative to a reference sequence. Generally, assembly of each set of amplicons can be carried out separately such that the polynucleotide encoding one amino acid sequence permutation is readily distinguished from another polynucleotide encoding a different amino acid sequence permutation. In some embodiments the assembly can be carried out in addressable locations on a substrate (e.g., an array) such that a plurality of polynucleotides encoding a plurality of defined amino acid sequence permutations can be generated simultaneously.

In some embodiments, assemblies can be prepared such that multiple (*i.e.*, 2 or more) amplicons are represented for the same fragment. The resulting product from this assembly reaction will contain a mixture of polynucleotides containing different permutations of the defined amino acid sequence differences. This mixture can be cloned directly and variants can be sequenced before or after encoded polypeptides are assayed.

The assembled amplicons are optionally replicated, e.g., using a polymerase to synthesize polynucleotides encoding the polypeptide of interest. In some embodiments, the reaction conditions can use the same conditions and polymerases used for the amplification reaction. The assembled amplicons act as primers such that a single round of replication creates a duplicate of the assembled amplicons. Generally, in the replicating step, primers that anneal to primer binding sequences that flank the polynucleotide (e.g., a terminal 5' region and a terminal 3' region) can be added to amplify the polynucleotide product by carrying out additional amplification reactions. In some embodiments, these flanking primers can incorporate recognition sequences for restriction enzymes to ease cloning of the synthesized polynucleotide product into plasmids or vectors, such as expression vectors. However, in other embodiments, this is unnecessary, e.g., where restriction enzyme free cloning is utilized.

In some embodiments, the flanking primers can have sequences that allow for direct *in vitro* expression using a coupled transcription-translation systems for synthesis of the protein product without the need for transformation into a host organism. Hence, some flanking primers can incorporate control sequences to control the expression of the polypeptide coding region. Amplification reactions using such flanking primers can operably link the control sequences to the polypeptide coding region of interest.

Once the amplicons have been synthesized, any polynucleotide encoding a specified amino acid sequence permutation based on a plurality of amino acid residue difference can be made using the amplicons. In some instances, the method of generating a polynucleotide encoding a polypeptide having an amino acid sequence with one or more defined differences in amino acid residues as compared to a reference polypeptide sequence can comprise the steps of: (a) assembling a set of amplicons having complementary overlapping adjacent regions, where the assembled set of amplicons comprise a polynucleotide sequence encoding an amino acid sequence with one or more defined amino acid residue difference as compared to a reference sequence, where the amplicons are selected from a library of amplicons having members encoding a plurality of amino acid differences, and (b) replicating the set of assembled overlapping polynucleotide fragments to synthesize the polynucleotide of interest.

Further details regarding the assembly of nucleotide variants and other topics relevant to the current disclosure can be found in USSN 61/061,581 filed June 13, 2008; USSN 12/483,089 filed June 11, 2009; PCT/US2009/047046 filed June 11, 2009; USSN 12/562,988 filed September 18, 2009; and PCT/US2009/057507 filed September 18, 2009.

In some instances, the amplicon library can be used to generate polynucleotides encoding any permutation of a defined plurality of defined amino acid differences, the method comprising: (a) generating permutations of amino acid sequences differing from a reference amino acid sequence based on a plurality of defined amino acid residue differences as compared to a reference amino acid sequence, (b) selecting a defined amino acid sequence permutation and determining a corresponding polynucleotide sequence based on a reference sequence, (c) selecting a set of overlapping polynucleotide fragments encoding the defined amino acid sequence permutations, where at least each overlapping polynucleotide fragment encoding an amino acid difference is from a plurality of polynucleotide fragments encoding different known amino acid residue differences, wherein the plurality of fragments has members sufficient to assemble polynucleotides encoding at least two different amino acid sequence permutations, (d) assembling the set of polynucleotide fragments having complementary overlapping adjacent regions, and (e) replicating the set of assembled overlapping fragments to synthesize the polynucleotide encoding the polypeptide. For each desired amino acid sequence permutation, the steps of (b) to (e) can be repeated.

An exemplary process for generating the amplicons for "n" number of variants includes : (a) importing a reference sequence and a list of mutations associated with the sequence, (b) creating a list of permutations based on the list of mutations, (c) selecting a defined permutation of the amino acid sequence (e.g., variant 1), (d) identifying overlapping polynucleotide fragments from a library of amplicons, (e) determining the number of variants and if the number of variants is less than the total number of desired variants, reiterating steps (a) to (d).

For efficient synthesis of the amplicon libraries, appropriately designed oligonucleotide primers are used in an amplification reaction. In some instances, the method of generating a library of overlapping polynucleotide fragments can comprise: (a) generating a plurality of permutations of amino acid sequences differing from a reference amino acid sequence based on a plurality of defined amino acid residue differences from a reference amino acid sequence, and for each permutation (i) determining a polynucleotide sequence encoding the amino acid sequence based on a reference polynucleotide sequence; (ii) scanning a polynucleotide sequence and identifying a change in polynucleotide sequence encoding an amino acid residue difference, and optionally determining the proximity of a next change in polynucleotide sequence encoding a next amino acid residue difference in the amino acid sequence permutation; (iii) selecting a forward oligonucleotide primer having a sequence encoding the amino acid difference, and optionally including the next change in polynucleotide sequence in the same forward primer if proximate to the change in polynucleotide sequence; (iv) scanning a polynucleotide sequence from the location of the forward primer until the next change in polynucleotide sequence is identified or until the end of the polynucleotide, and selecting a reverse oligonucleotide primer for amplifying a polynucleotide fragment with the forward oligonucleotide primer, wherein the reverse primer has a sequence that optionally encodes the next change in amino acid residue difference; (v) reiterating steps (ii) to (iv) for each change in polynucleotide sequence encoding an amino acid residue difference until all changes in polynucleotide sequence are present on oligonucleotide primers and ends of the polynucleotide sequence is reached; and (g) amplifying with each set of forward and reverse oligonucleotide primers to generate the library of overlapping amplicons having members encoding the amino acid differences. In these instances, when scanning of the polynucleotide sequence encounters the end of the polynucleotide, flanking primers can be used in combination with the internal primers to complete the generation of the amplicons.

An exemplary process for selecting the appropriate forward and reverse primers comprises: (a) selecting a variant (an amino acid sequence permutation) and generating its corresponding polynucleotide sequence based on a reference sequence, (b) creating a forward oligonucleotide primer for a fragment with a first mutation, (c) scanning the sequence from the first mutation to the next mutation or to the end of the gene and creating a reverse oligonucleotide primer for the next mutation, (d) and if the next mutation is proximate to the first mutation, placing the next mutation in the same forward oligonucleotide primer, (e) reiterating steps (b) to (d) until ends of polynucleotide variant n is reached.

As noted above, in some instances where the polynucleotide has been separated out into overlapping segments defined by a set of forward and reverse primers, the forward and reverse primers may have no associated mutations. One context in which this may occur is if the polynucleotide segments are to be restricted in size, for example about less than 1000 bases, because of a need for efficient synthesis of an amplicon, such that not all the segments have defined changes in polynucleotide sequence. In some instances, in preparing the oligonucleotides based on the method above, the search of the sequence can be limited to a particular size "1", for example by about 1200 bases in step (iv) above for selecting a reverse primer. In other words, following the identification of a forward primer based on a sequence difference, a scan is made in one or the other direction of the polynucleotide sequence to determine the nucleotide distance to the next mutation. If the distance exceeds the set limit, a segment that does not encompass any mutations can be created to bridge two segments that contain the two distant mutations. The scanning process can be reiterated at the point of the next mutation.

The oligonucleotide primers, either alone or in sets (e.g., forward and reverse oligonucleotides) as well as the corresponding amplicons can be placed on addressable substrates for automation and/or storage. Oligonucleotide primers in the addressable substrates, also described herein as primer array, can be robotically accessed to synthesize any libraries of amplicons for a defined plurality of amino acid differences. Likewise, the amplicons in the addressable substrates, also described herein as amplicon arrays, can be accessed to generate a polynucleotide sequence encoding a desired amino acid sequence permutation based on the defined plurality of amino acid residue differences. A substrate or solid support for the array can be composed of organic polymers such as polystyrene, polyethylene, polypropylene, polyfluoroethylene, polyethyleneoxy, and polyacrylamide, as well as co-polymers and grafts thereof. A solid support can also be inorganic, such as glass, silica, controlled pore glass (CPG), reverse phase silica or metal, such as gold or platinum. The configuration of a substrate can be in the form of beads, spheres, particles, granules, a gel, a membrane or a surface. Surfaces can be planar, substantially planar, or non-planar. Solid supports can be porous or non-porous, and can have swelling or non-swelling characteristics. A solid support can be configured in the form of a well, depression, or other container, vessel, feature, or location. A plurality of supports can be configured on an array at various locations, addressable for robotic delivery of reagents, or by detection methods and/or instruments. In some embodiments, the substrate is a reaction chamber. Commercially available reaction vessels contain at least one reaction chamber, but can contain 8, 24, 96 or 384 reaction chambers. An example of a reaction chamber is one of the 96 microtiter wells in a 96 well microtiter plate. In some embodiments, a robotic system and an associated computer system capable of sampling primers or primer pairs from the arrays can be used to deliver them to a reaction chamber. Reagents for polymerase mediated amplification can also be delivered to each set of primers in the reaction chamber followed by implementation of an amplification routine (such as in a automated thermocycler). This allows formation of an addressable substrate containing defined amplicons based on overlapping segments of a polynucleotide sequence. The robotic system can choose the appropriate set of amplicons based on the desired permutation of the amino acid sequence, the flanking primers for amplification of the final polynucleotide product, and deliver the reagents for the assembly and amplification reaction. An exemplary robotic system comprises instructions for (a) selecting a segment and associated amplicons for amplification, (b) identifying forward and reverse oligonucleotides for the selected fragment (e.g., amplicon), storing data information on the oligonucleotides on list of unique oligonucleotides (e.g., 96 well microtiter plate), and placing the oligonucleotides on a first addressable substrate (c) storing data information on synthesized fragment (e.g., position on array, sequence, oligonucleotides used, etc) to list of unique fragments, and placing the oligonucleotide on a second addressable substrate, (d) determining the number of fragments selected against the total number of fragments required for assembly, and reiterating steps (a) to (d) until all fragments have been selected, (e) placing the assembled gene into a third addressable substrate, and reiterating steps (a) to (d) until all desired variants have been generated.

In some embodiments, the present disclosure also provides libraries of polynucleotide fragments (e.g., amplicons) for assembling a plurality of polynucleotides encoding different amino acid sequence permutations. In some embodiments, the plurality of polynucleotides comprises: polynucleotide fragments with overlapping adjacent regions, each polynucleotide fragment being bounded by primer binding sequences for forward and reverse primers, wherein the plurality of polynucleotides have members that encode in the primer binding sequences of a specific amino acid residue difference from a defined plurality of amino acid residue differences relative to a reference amino acid sequence such that the plurality of polynucleotide fragments encode all of a selected plurality of amino acid residues differences from the defined plurality of amino acid residue differences; and wherein the plurality of polynucleotide fragment comprises members for assembling two or more different amino acid sequence permutations of the defined amino acid differences. In some embodiments, the plurality of polynucleotide fragments comprises members sufficient for assembling all of the possible amino acid sequence permutations of the selected plurality of amino acid residue differences. In some embodiments, the members of the plurality are amplicons formed using the forward and reverse primers.

Also disclosed herein are computer implemented systems in the form of computer software for carrying out the methods described above. In some instances, the computer program product comprises a machine readable storage medium having program instructions comprising codes for each of the steps of: (a) importing a reference sequence and a list of mutations associated with the sequence, (b) creating list of permutations based on the list of mutations, (c) selecting a defined permutation of the amino acid sequence, (d) identifying overlapping polynucleotide fragments from a library of amplicons, (e) determining the number of variants and if the number of variants is less than the total number of desired variants, reiterating steps (a) to (d). Further details regarding systems of the disclosure are found in PCT/US2009/057507 filed September 18, 2009.

As described herein, in some embodiments, the method can be used to synthesize polynucleotides encoding polypeptides having a defined set of mutations selected from a plurality of defined differences in amino acid residues from a reference sequence. The methods herein allow efficient synthesis of various permutations of amino acid sequences based on the amino acid residue differences. Efficient synthesis of polynucleotides encoding various amino acid sequence permutations is useful for a variety protein engineering applications. See, e.g., US application publication US20060195947; US application publication. US20050153417; and US Patent No. 7,220,566. In some embodiments, the methods can be use to synthesize polynucleotides encoding enzyme variants having improved properties based on a set of mutations known to affect different properties of the enzyme. For example, some mutations can affect, among others, enzyme activity, thermal stability, substrate specificity, stereoselectivity, stereospecificity, and refractoriness to product inhibition. While traditional techniques of random mutagenesis and protein evolution can lead to identification of mutations affecting these various enzyme properties, many of these mutations can occur independently of the others. Using the methods herein, various permutations of mutations affecting different traits, such as enzyme activity, substrate specificity, and thermal stability can be made and screened to identify engineered enzymes having desired multiple altered traits.

## CLONING POLYNUCLEOTIDE VARIANTS FOR EXPRESSION AND SCREENING

Polynucleotide variants can be cloned into expression vectors to facilitate screening of encoded polypeptide variants. Polynucleotide variants can also be cloned into standard cloning or shuttle vectors, e.g., for amplification by cloning. In either case, a variety of cloning methods are available, and applicable to cloning of polynucleotide variants. For example, PCR amplicons or other nucleic acids comprising polynucleotide variants can be cloned via standard restriction digestion, ligation into expression or other cloning vectors at a cloning site and transformed into a host cell. Available cloning methods are described in a variety of standard references, e.g., Principles and Techniques of Biochemistry and Molecular Biology Wilson and Walker (Editors), Cambridge University Press 6th edition (2005) ISBN-10: 0521535816; Sambrook et al., Molecular Cloning - A Laboratory Manual (3rd Ed.), Vol. 1-3, Cold Spring Harbor Laboratory, Cold Spring Harbor, New York, 2001 ("Sambrook I"); The Condensed Protocols from Molecular Cloning: A Laboratory Manual Joseph Sambrook Cold Spring Harbor Laboratory Press; 1st edition (2006) ISBN-10: 0879697717 ("Sambrook II"); Current Protocols in Molecular Biology, F.M. Ausubel et al., eds., Current Protocols, a joint venture between Greene Publishing Associates, Inc. and John Wiley & Sons, Inc., ("Ausubel I"); Short Protocols in Molecular Biology Ausubel et al. (Editors) Current Protocols; 52 edition (2002) ISBN-10: 0471250929 (Ausubel II); Lab Ref, Volume 1: A Handbook of Recipes, Reagents, and Other Reference Tools for Use at the Bench Jane Roskams (Author), Linda Rodgers (Author) Cold Spring Harbor Laboratory Press (2002) ISBN-10: 0879696303; and Berger and Kimmel, Guide to Molecular Cloning Techniques, Methods in Enzymology volume 152 Academic Press, Inc., San Diego, CA (Berger)). These texts describe, e.g., cloning, the use of expression vectors, promoters and many other relevant topics related to, e.g., the generation of clones that comprise polynucleotide variants of interest.

In some embodiments, traditional cloning methods are simplified by eliminating any need for restriction site-based cloning. Instead of synthesizing variants of interest and then recombining them into a restriction site of an expression vector, library construction can proceed by constructing and incorporating variants in coordinated or simultaneous processes. For example, a variant sequence of interest can serve as a primer that binds to a circular expression or cloning vector comprising a homologous sequence (e.g., a reference sequence to be varied); the variant sequence is incorporated into amplicons of the expression vector, which can be cloned into cells of interest. Three preferred examples are described below: megaprimer PCR, abutting primer PCR, and overlapping primer PCR. Other approaches are also available and can combine construction, amplification and/or cloning of a variant sequence of interest.

Megaprimer PCR

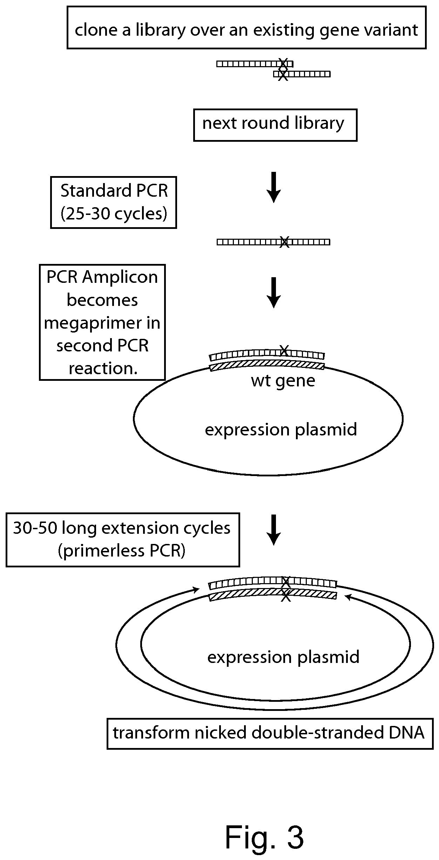

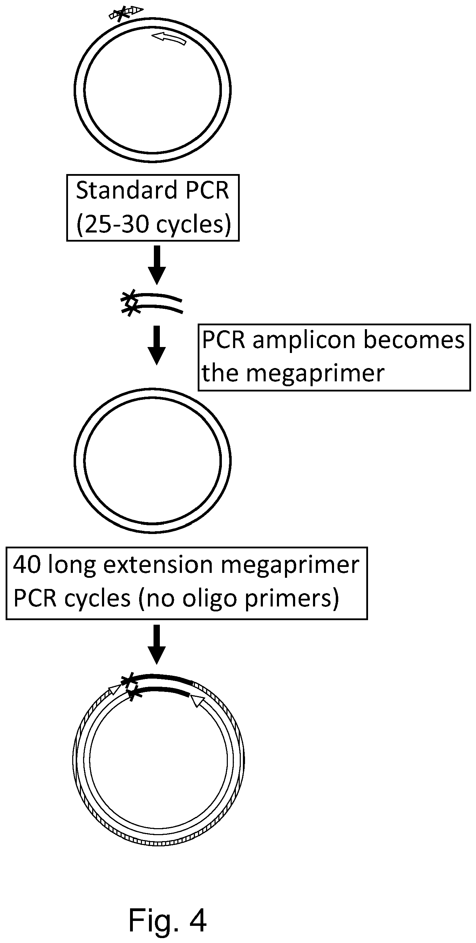

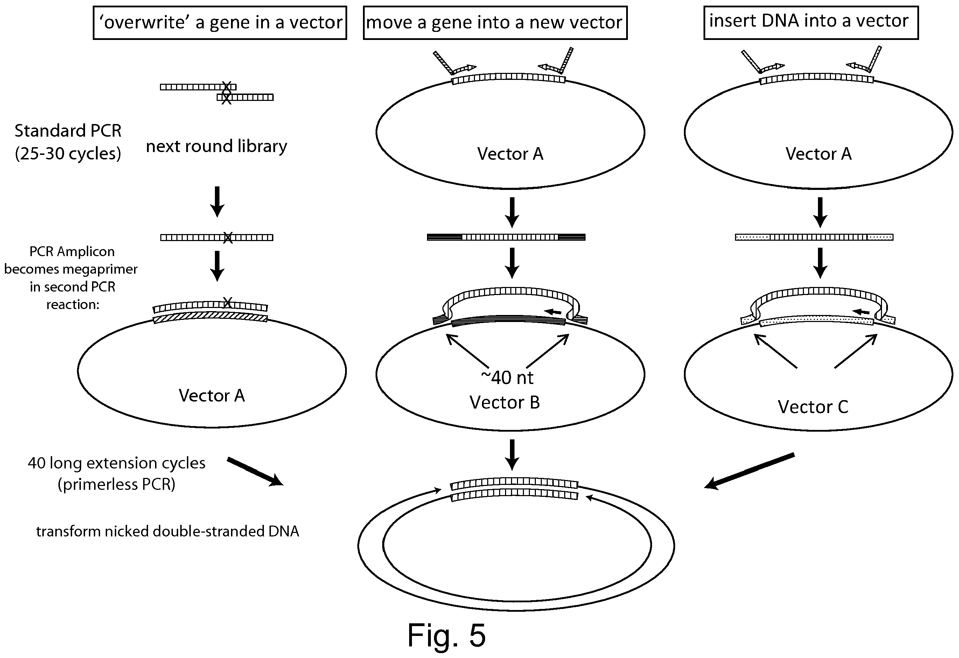

In one preferred embodiment, nucleic acid variants are cloned into expression or other vectors using MEGAWHOP (megaprimer PCR of whole plasmid, also referred to more simply as "megaprimer PCR"), or related methods. As shown in Figure 3, PCR or DNA synthesis protocols as described herein are used to produce a polynucleotide variant, or relevant fragment thereof. This variant is used as a "megaprimer" to prime extension during PCR of an expression vector that comprises a homologous nucleic acid, such as a reference polynucleotide sequence. Desirably, high ratios of megaprimer to template is used, e.g., a molar ratio on the order of about 100:1. After several long extension cycles, a "nicked" double stranded expression construct comprising the polynucleotde variant results. Desirably, as many as 40-50 or more cycles are used for the amplification. The nicks can be ligated prior to transformation using a ligase, or, more efficiently, the nicked construct can simply be transformed into a host cell, where the nicks are repaired using the host cell's endogenous DNA repair machinery. See also, Figure 4 and Figure 5. The procedure outlined in Figure 4 uses PCR to amplify a library, gene variant or gene fragment. Fragments are SOE amplified and rescued via PCR. Fragments can be viewed, e.g., on a gel, and column purified or purified using an exoSAP-it treatment of the PCR product. A second cycle of PCR of 30-50 cycles is performed, e.g., with melt and a long extension (3-6 hrs). The products can be DPN1 digested (e.g., for 60 minutes). The resulting nick-translated amplicon/ plasmid is used to transform a cell (e.g., use 1-2μl of the plasmid amplicon). For this procedure, the PCR product should be relatively free of contamination; primers should be removed before the second amplification step; a low amount of template (e.g., 10-50 ng) should be used, and amplification is desirably done with a polymerase that does not have strand displacing activity, and a Dpn1 digestion after the second PCR is helpful. Figure 5 shows three applications: 1) "overwriting a gene into a vector; 2) moving a gene into a new vector and 3) inserting DNA into a vector.

Further details related to this cloning technique are found in Miyazaki (2002) "Creating Random Mutagenesis Libraries Using Megaprimer PCR of Whole Plasmid" BioTechniques 33:1033-1038 (November 2002); and in van den Ent and Lowe (2006) "RF cloning: A restriction-free method for inserting target genes into plasmids" J. Biochem. Biophys. Methods 67:67-74. Additional useful details regarding PCR amplification for Megaprimer PCR and other applications as noted herein can be found in Sanchis et al. (2008) "Improved PCR method for the creation of saturation mutagenesis libraries in directed evolution: application to difficult to amplify templates," Appl. Microbiol. Biotechnol. 81:387-397; and Quan and Tian (2009) "Circular Polymerase Extension Cloning of Complex Gene Libraries and Pathways," PLoS ONE 4(7): e6441.

Polymerases useful for PCR methods that are adapted to long extension cycles are preferred for use with this class of embodiments, as well as with those noted below relating to abutting primer PCR and to overlapping primer PCR. This is because in several such embodiments, a polymerase may copy the entire polynucleotide variant and construct as part of the overall amplification and construction process. Polymerases with strand displacement activity are not desirably used in this embodiment. Polymerases with long read length and/ or higher fidelity are desirable. Available suitable polymerases include *Pfu* (available from Stratagene), Herculase (available from Stratagene), PfuUltra II Fusion (available from Stratagene), Phusion (available from New England Biolabs (NEB)/ Finnzymes), and KAPAHiFi (available from Kappa Bio).

Desirably, Dpn 1 digestion can be used to reduce the presence of any original plasmid DNA (e.g., an expression vector comprising a reference nucleic acid to be varied in the protocol) from the mixture of variants. Dpn 1 preferentially digests methylated nucleic acids, e.g., plasmid DNA isolated from an in vivo source. Dpn 1 digestion reduces the presence of unwanted reference/ parental plasmid in the library.

There are several advantages to Megaprimer PCR as compared to traditional restriction site-based cloning, when considered in the context of the invention. First, because no restriction sites need to be engineered into polynucleotide variants to be cloned (and/ or into degenerate oligonucleotides used in polynucleotide variant construction), the overall design process is simplified. Second, the overall cloning methodology is not limited by the presence (or absence) of specific restriction site sequences in either the vector or the polynucleotide variant sequence to be cloned. Third, oligonucleotide costs are reduced, e.g., approximately two-fold, because fewer oligonucleotides are needed in the overall cloning process, as compared to restriction-site based cloning methods.

Megprimer Example

The following provides an example protocol for megaprimer PCR for variant polynucleotide library construction.

1. Library preparation.

A library of variants is readied for cloning by megaprimer PCR following pooling of, e.g., SOE (spicing by overlap extension) PCR reactions, as described herein. First, unincorporated, e.g., SOE rescue primers are removed from the pooled SOE PCR products using, e.g., Qiagen's QIAquick PCR Purification Kit. Primer removal improves the success of the megaprimer PCR cloning reaction.

2. Megaprimer PCR cloning of library

250-500 ng of pooled PCR product is added to a PCR mix (0.2 mM each dNTP, IX Phusion HF PCR buffer - NEB, 1 unit Phusion DNA polymerase - NEB, and water to a final volume of 50 $\mu$l) containing 10 ng of the target vector carrying the wild-type target gene sequence. No additional primers need to be added to this reaction. Two-step cycling program consisting of 98°C for 30 seconds, 40 cycles of 98°C for 10 seconds and 72°C for 30 seconds/kb plasmid is then run in an MJ Research (Watertown, MA) PTC-200 thermocycler.

Following PCR, 10 units of DpnI (Promega) are added to the 50 $\mu$l reaction volume, and the sample is incubated at 37°C for 1 hour to digest the template and minimize the amount of wild-type background.

3. Transformation and analysis

*E. coli* or other relevant host cells are transformed by electroporation using, e.g., 1 $\mu$l of DpnI-treated megaprimer PCR product per 50 $\mu$l of competent cells. Megaprimer cloning can use E. coli strains capable of repairing nicked DNA, thereby omitting any need for an in vitro ligation step. Examples of appropriate strains include DH10B, XL1-Blue, TOP 10, and DH5 $\alpha$. Transformations are plated on LB agar with appropriate antibiotic (in this case, 10 $\mu$g/ml tetracycline). Surviving colonies are picked for colony PCR, and, optionally, sequence verification of the incorporation of mutations at targeted library positions.

Abutting Primer PCR

In another preferred embodiment, nucleic acid variants are simultaneously created and cloned into expression or other vectors using an abutting primer cloning strategy. In this method, phosphorylated primers encoding mutations, deletions, or small insertions are used to amplify an entire plasmid expression vector, including a target sequence (gene, coding DNA, etc.) of interest. The resulting PCR amplicon is then ligated and transformed into a cell of choice for heterologous protein expression.

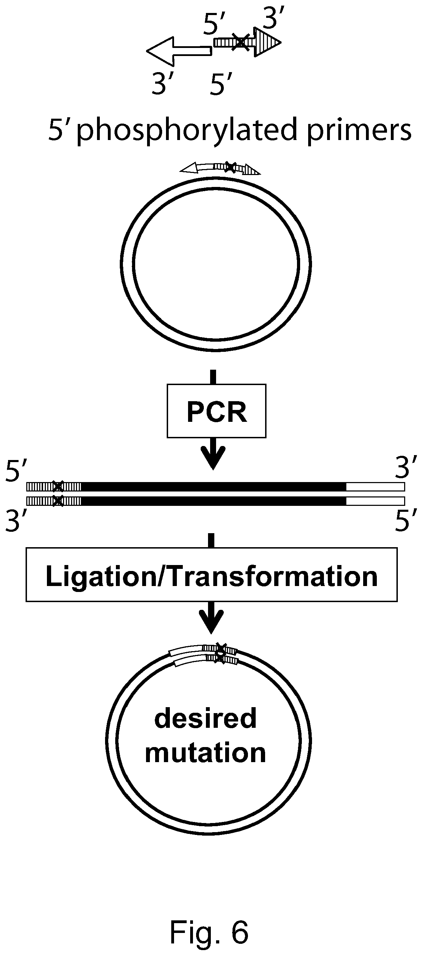

Briefly, in this class of embodiments, PCR amplification is performed using a target plasmid that includes a reference nucleic acid of interest (e.g., a gene or other target sequence for mutagensis). The PCR reaction is primed with two phosphorylated primers, e.g., desireably using a high fidelity, high read length polymerase as noted above. The primers, one or both comprising desired mutations (e.g., point or other mutations corresponding to a degenerate codon set, as compared to the reference sequence), are designed so that they anneal back to back to the plasmid. Resulting mutated PCR products can be circularized by ligation, e.g., with a T4 DNA ligase (available, e.g., from New England Biolabs). Background arising from presence of the original target plasmid in the library can be reduced by DPN 1 digestion, as noted above. Ligation, in some instances, can be omitted, e.g., when subsequent transformation is done into a host cell that will perform the ligation in vivo. The mutated PCR products, which comprise the polynucleotide variants of interest, are transformed into a host cell for expression and, optionally, screening. This method is illustrated in Figure 6. For the process shown, the abutting primer site-directed mutagenesis process includes, e.g., design of abutting primers, phosphorylation of the primers (e.g., for 30 minutes), PCR (e.g., for a total of about 3 hours), ligation of diluted PCR reactions (e.g., a 4 fold dilution) in a 30 minute ligation reaction, and transformation of a ligated amplicon (using e.g., about 1-2μl).

It is desirable to use high quality PCR primers in this method, because deletions or sequence errors in the primers (resulting, e.g., from incomplete or inefficient primer synthesis) can introduce unintended deletions and/or mutations in the final construct. Optimal primer lengths can vary, but shorter primers, on the order of 35 nucleotides or less, represent one preferred class of embodiments. This is because the percent yield for chemical synthesis of oligonucleotides synthesis declines with increasing oligonucleotide length, with a corresponding increase in sequence errors and nucleotide deletions. Longer primers can be used for the method, but should be purified by HPLC or polyacrylamide gel electrophoresis following chemical synthesis.

One commercially available abutting primer cloning kit that can be adapted to use with this method is the Phusion™ Site-Directed Mutagenesis Kit from New England Biolabs (NEB), product code: F-541.

As with the Megaprimer PCR approach, there are several advantages in using abutting primer PCR for building libraries of polynucleotide variants. These include accelerated library construction, due to a reduction in the number of steps involved in incorporating variant sequences of interest into an expression or other vector; more rapid progression to subsequent rounds of evolution due to a reduction in the number of steps for cloning, as compared to using standard restriction enzyme site cloning; greater flexibility in the types of libraries that can be designed and constructed, as the methodology is not limited by the presence (or absence) of specific restriction site sequences; reduced oligonucleotide costs in constructing libraries, as the number of required oligos is reduced as compared to standard PCR synthesis and restriction site cloning, etc.

Overlapping Abutting Primers

In a related embodiment, nucleic acid variants are cloned into expression or other vectors using an overlapping abutting primer synthesis strategy. In this approach, primers do not need to be phosphorylated; they do not abut at their 5' ends as in the previous embodiment, but, instead, overlap at their 5' ends. This creates sticky ends in the PCR amplicon, e.g., upon eventual exonuclease processing as noted below.

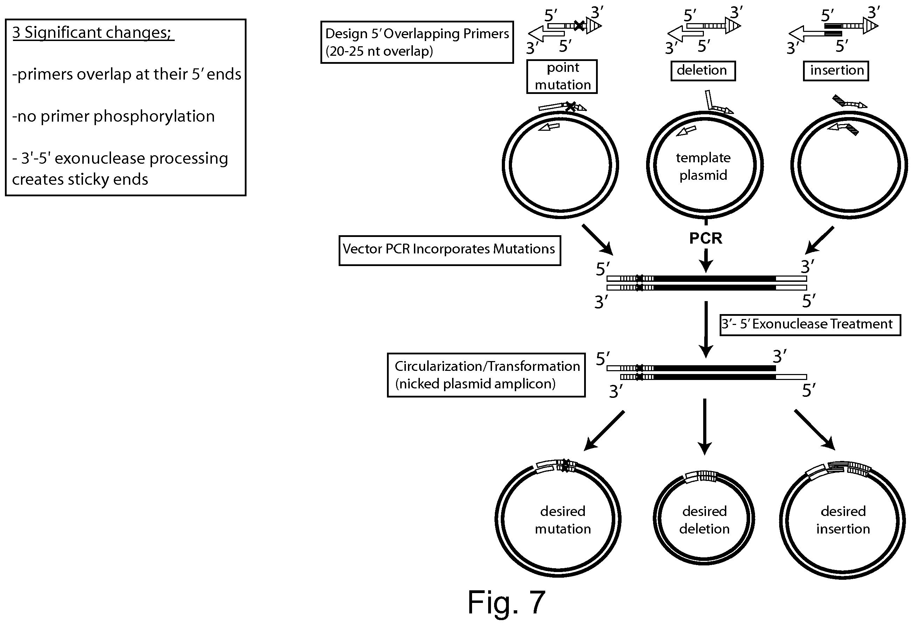

This class of embodiments is further illustrated in Figure 7. As shown, primers comprising sequence variations of interest, as compared to a reference sequence, are designed to have, e.g., about 20-25 nucleotides worth of overlap. The primers are used to prime a PCR reaction of a circular vector that includes a homologous region that the primers bind to during synthesis. The PCR reaction conditions should be optimized to account for incomplete complementarity between variant sequences and a reference sequence template (e.g., by lowering the reaction temperature).

Resulting amplicons comprise the variant of interest in a vector. Following 3'-5' exonuclease treatment, sticky ends are created at the overlapping sequences, facilitating circularization of the amplicons. Circularized constructs can be ligated in vitro or in vivo.

In addition to the advantages noted above in the context of abutting primer synthesis, the use of overlapping abutting primers has certain advantages. For example, if primer synthesis is incomplete (due, e.g., to low primer quality), a reduced frequency of unwanted variation in the final product results, owing to extension by the polymerase, e.g., using the corresponding overlapping primer as a template, correcting such lesions during amplification.

Further details applicable to this method are found in Chiu et al. (2004) "Site-directed, Ligase-Independent Mutagenesis (SLIM): a single tube methodology approaching 100% efficiency in 4 h" Nucleic Acids Research 32(21): e174, and in Li et al. (2008) "Site directed mutagenesis by combination of homologous recombination and Dpn1 digestion of the plasmid template in Escherichia coli." Analytical Biochemistry 373:389-391.

POOLING VARIANTS

After or concurrent with cloning, it can be desirable to pool polynucleotide variants for screening. However, this is not required in all cases. In some embodiments, polynucleotide variants can be assembled into an addressable library, e.g., with each address encoding a different variant polypeptide having a defined amino acid residue difference. This addressable library, e.g., of clones can be transformed into cells for translation and, optionally, automated plating and picking of colonies. Sequencing can be carried out to confirm the mutation or combination of mutations in each variant polypeptide sequence of the resulting transformed addressable library. Assay of the variant polypeptides for desired altered traits can be carried out on all of the variant polypeptides, or optionally on only those variant polypeptides confirmed by sequencing as having a desired mutation or combination of mutations.

Preferably, however, clones are pooled. A pooled library of clones can be transformed into cells for expression, plating, picking of colonies, etc. Assay of colonies from this pooled library of clones can be carried out (e.g., via high-throughput screening) before sequencing to identify polynucleotide variants encoding polypeptides having desired altered traits. Once such a "hit" for an altered trait is identified, it can be sequenced to determine the specific combination of mutations present in the polynucleotide variant sequence. Optionally, those variants encoding polypeptides not having the desired altered traits sought in assay need not be sequenced. Accordingly, the pooled library of clones method can provide more efficiency by requiring only a single transformation rather than a set of parallel transformation reactions; screening is also simplified, as a combined library can be screened without the need to keep separate library members at separate addresses.

Pooling can be performed in any of several ways. Variants can, optionally, be pooled prior to cloning, with the cloning steps being performed on pooled materials. In some protocols as noted above, this approach is not optimal, e.g., in simultaneous amplification and cloning (e.g., cloning without use of restriction sites, e.g., PCR with variant primers on circular templates), because PCR products tend to concatenate. In these and other cases, variants can be pooled after being cloned into a vector of interest, e.g., prior to transformation.

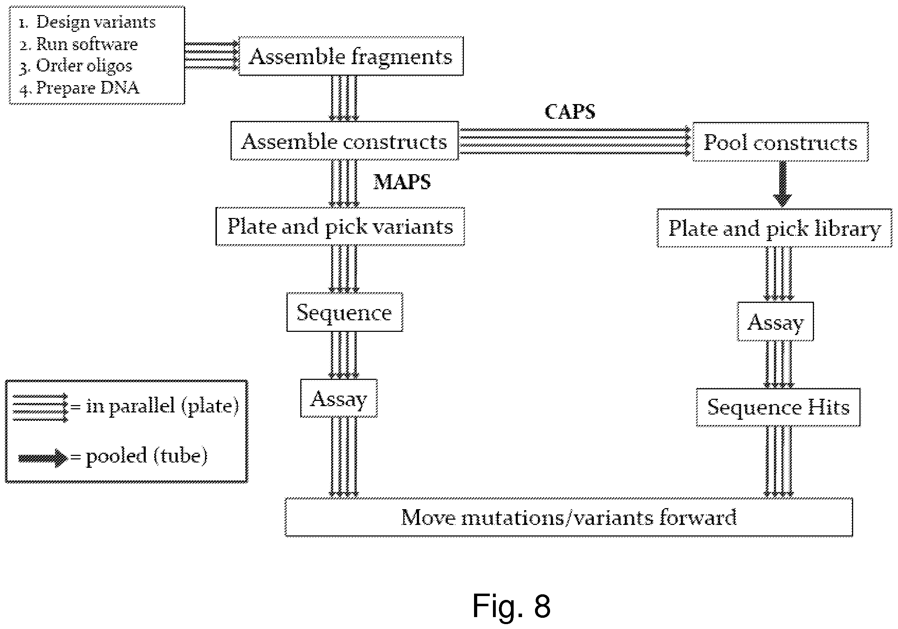

Figure 8 provides an example flow chart for a combined APS procedure (CAPS), as compared to MAPS, discussed above (See also, Figure 2). As shown, the CAPS procedure includes pooling constructs prior to plating and screening. CAPS offers several advantages, including: no need for sequence verification of variant constructs (as with MAPS, CAPS results in about 85% of variants having a sequence of interest); and pools of large numbers of constructs can be synthesized quickly. This approach is useful for quickly surveying many individual mutations.

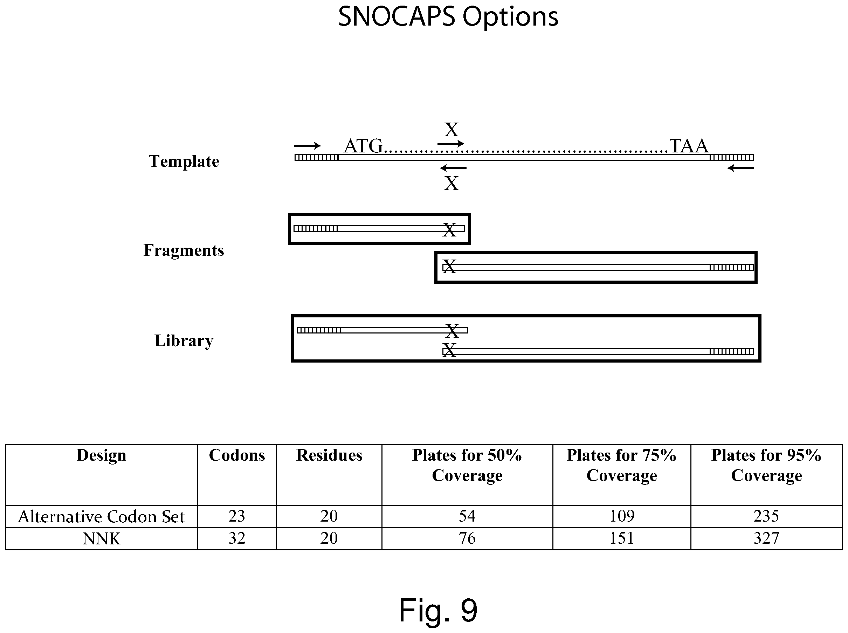

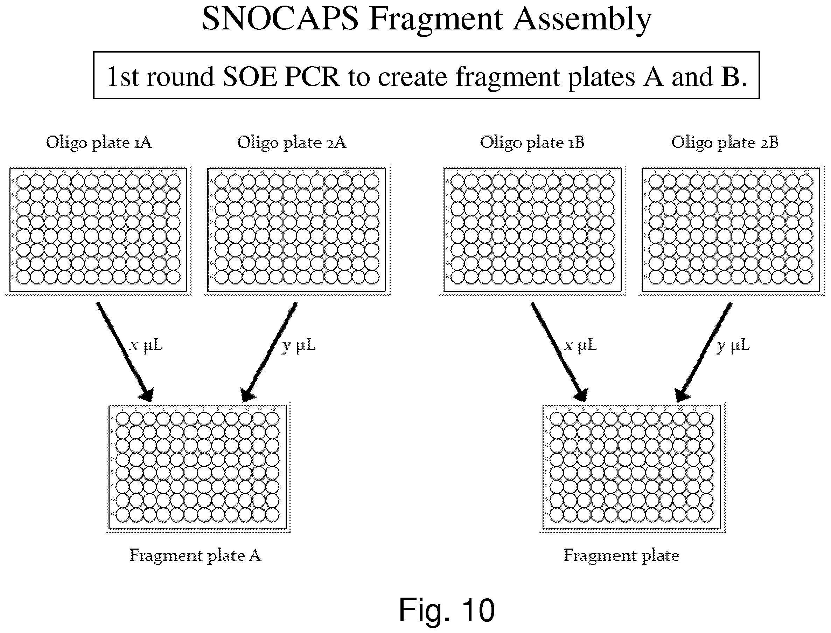

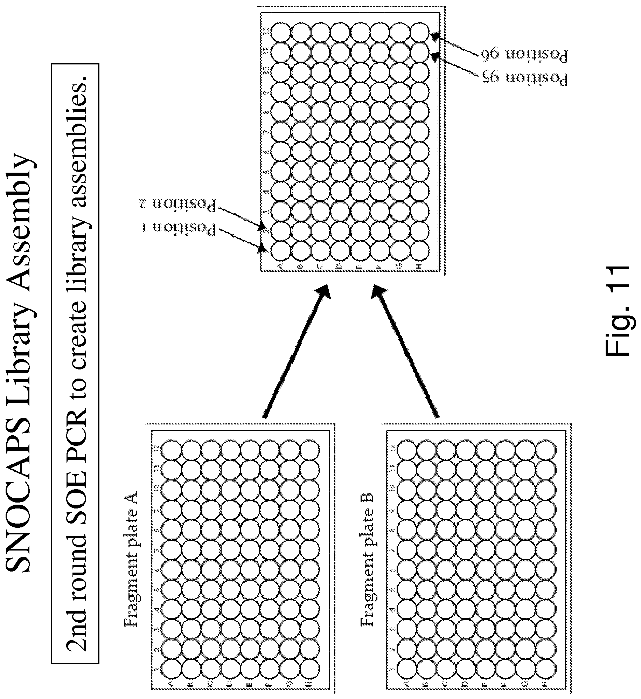

In a variation of CAPS, SaturatioN mutagenesis Of protein by CAPS ("SNOCAPS"), CAPS is performed on many positions using pools of degenerate primers, e.g., comprising a degenerate codon set as noted herein. In this preferred embodiment, the entire single amino acid mutation space of an entire protein is accessible. This is unlike random mutagenesis, which usually can not result in all amino acids occurring at all positions, due to the need for multiple changes at one codon site to achieve many amino acid changes. The SNOCAPS process is ideal for surveying, e.g., thousands of individual mutations, and provides a powerful method for creating libraries of nucleic acid variants. Figure 9 provides a schematic overview of SNOCAPS performed with a codon set of the invention. As shown, for a 300 residue protein, assuming 88 data wells per plate, just 54 standard screening plates are used to achieve 50% coverage using a 23 codon set that encodes 20 amino acids. Thus of the 300* 19=5700 possible unique amino acid mutations, one would expect to see 0.5*5700=2850 of them. Figures 10 and 11 provide a schematic of the plate manipulations involved in making the libraries, showing the resulting pooled libraries for each position on the library plates.

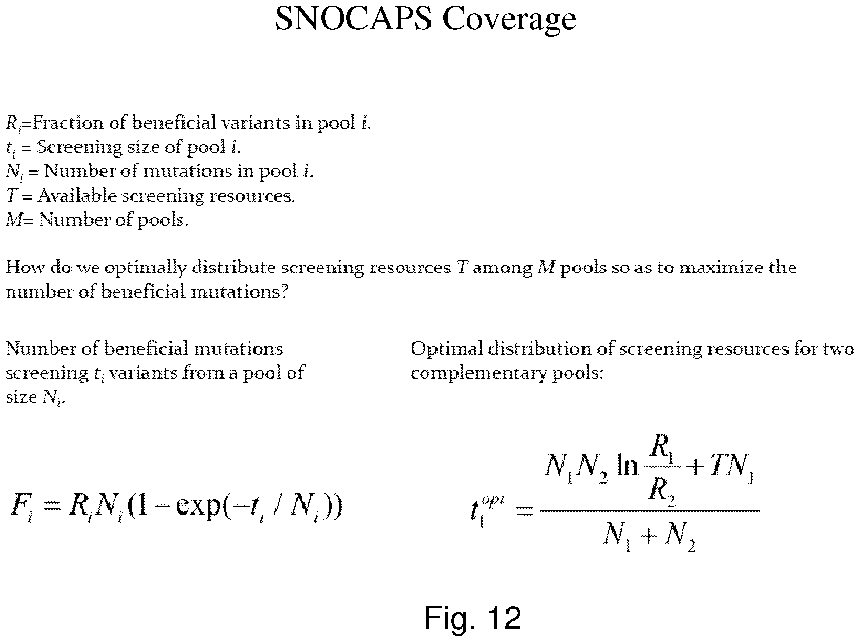

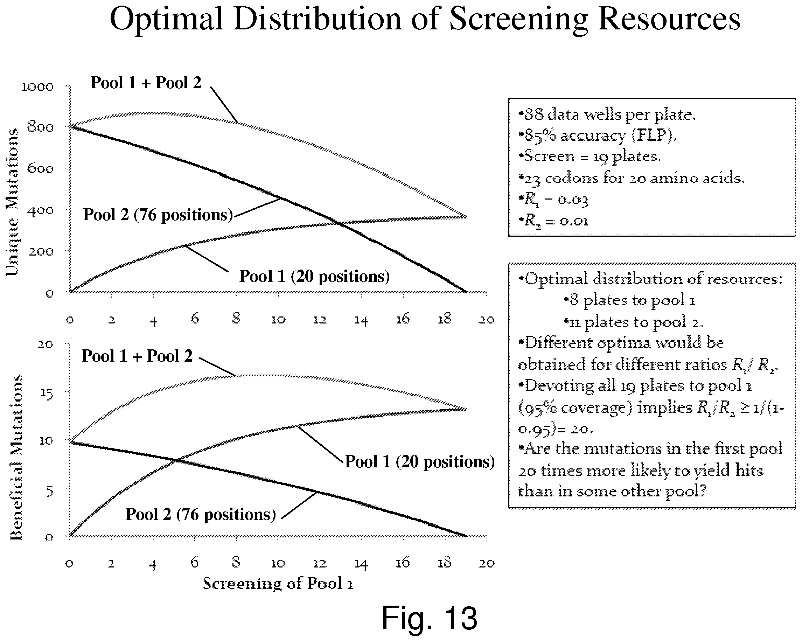

In one example cost benefit analysis, 95 % coverage can be obtained for SNOCAPS performed on 20 positions (using a 23 codon set) with 19 plates of screening and yields 361 unique amino acid mutations. The same screening effort (19 plates) devoted to SNOCAPS at 96 positions gives 47% coverage and yields 866 unique mutations. This illustrates the relative benefit of shallow screening performed on a large library, as opposed to deep oversampling on a smaller library. Figure 12 provides a screening resource model to optimize resource allocation during screening. This model addresses the issue of how screening resources (T) are to be distributed among (M) pools to maximize the number of beneficial mutations that are screened. Figure 13 graphically illustrates application of this model to a situation in which there are two separate libraries to be screened: a first comprising 20 codon sites for variation, and a second comprising 76 sites. The model puts in high relief a fact that is not widely appreciated: that committing to screen a given library at 95% coverage implies that one is ~20 times more confident that that library will yield a beneficial mutation than another library that one could construct. This is surprising to those who have not considered the costs (measured in terms of lost opportunity) associated with deep oversampling.

Several 22 codon sets are noted herein, providing a more efficient codon set than the 23 codon set noted above. Use of a 22 codon set for 20 amino acids, rather than 23 codons reduces screening burden by about 5 percent.

Additional Library Construction Methods

The methods for making libraries of polynucleotide variants can optionally employ any of a variety of methods that are known in the art, e.g., in combination with oligonucleotides comprising degenerate codons as noted herein to create variants at a codon site of interest. In general, libraries of variants can be constructed using any available mutation method that can be adapted to incorporate a degenerate codon set as noted herein. For example libraries of variants comprising the degenerate codon sets of the invention can be constructed by following any of a variety of recombination or recursive recombination methods. Available library construction methods are modified according to the invention, e.g., by incorporating degenerate oligonucleotides comprising the degenerate codons at a codon site of interest into a recombination mixture. Example applicable approaches include the use of partially or fully synthetic shuffling using degenerate oligonucleotides that comprise the degenerate codon sets as noted herein, or the use of, e.g., semi-synthetic shuffling e.g., where oligonucleotides that are used to spike a recombination reaction comprises degenerate oligonucleotides comprising the relevant codon set at a codon site of interest. A variety of recursive recombination protocols that make use of oligonucleotides for construction of libraries of polynucleotide variants are described in, e.g., WO/2000/042561 by Crameri et al. OLIGONUCLEOTIDE MEDIATED NUCLEIC ACID RECOMBINATION; WO/2000/042560 by Selifonov et al. METHODS FOR MAKING CHARACTER STRINGS, POLYNUCLEOTIDES AND POLYPEPTIDES; WO/2001/075767 by GUSTAFSSON et al. IN SILICO CROSS-OVER SITE SELECTION; and WO/2000/004190 by del Cardayre EVOLUTION OF WHOLE CELLS AND ORGANISMS BY RECURSIVE SEQUENCE RECOMBINATION. These methods can be adapted to the present invention by incorporating oligonucleotide sets, e.g.,

degenerate oligonucleotide sets, that comprise the degenerate codon sets noted herein.

Site saturation and other mutagenesis methods can also incorporate degenerate codons into libraries of variants, e.g., by incorporating degenerate oligonucleotides during variant construction during the relevant method. Approaches that can be adapted to include the codon sets of the invention include those in Fox and Huisman (2008), Trends Biotechnol. 26: 132-138; Arndt and Miller (2007) Methods in Molecular Biology, Vol. 352: Protein Engineering Protocols, Humana; Zhao (2006) Comb. Chem. High Throughput Screening 9:247-257; Bershtein et al. (2006) Nature 444: 929-932; Brakmann and Schwienhorst (2004) Evolutionary Methods in Biotechnology: Clever Tricks for Directed Evolution, Wiley-VCH, Weinheim; and Rubin- Pitel Arnold and Georgiou (2003) Directed Enzyme Evolution: Screening and Selection Methods, 230, Humana, Totowa; as well as those in, e.g., Rajpal eta 1. (2005) "A General Method for Greatly Improving the Affinity of Antibodies Using Combinatorial Libraries." PNAS 102(24): 8466-8471; Reetz et al. (2008) "Addressing the Numbers Problem in Directed Evolution" ChemBioChem 9:1797-1804 and Reetz et al. (2006) "Iterative Saturation Mutagenesis on the Basis of B Factors as a Strategy for Increasing Protein Thermostability" Angew. Chem. 118: 7907-7915). A variety of additional mutational methods that can be adapted according to the invention by incorporating degenerate codons at codon sites of interest are discussed herein and are similarly applicable in the context of library creation.

## LOGICAL FILTERS FOR LIBRARY CONSTRUCTION

In some aspects, logical filters can be applied to reduce library size, thereby decreasing the overall screening burden. Such filters include selecting which sites in a reference sequence to vary, limiting amino acid alphabets at certain codon sites, etc. These filters can be based upon physico-chemical properties of amino acids, consideration of homology information, available structure-function information for the reference nucleic acid or its encoded product, other mutagenesis experiments performed on the reference molecule or a homologue thereof, statistical or heuristic filters based upon any available data, or the like.

## Structure-Assisted design of Variants

In one example, structural data for a protein can be used as a logical filter to identify amino acid residue sites as particular candidates for mutagenesis, e.g., to create variants having modified active sites or other variant features of interest. For example, analysis of the three-dimensional structure of a reference protein can identify residues that interact at an active site, or that can be mutated to introduce a feature complementary to a non-natural feature of a substrate, e.g., by adding or altering charge, hydrophobicity, size, or the like. Similarly, mutagenesis experiments, e.g., random mutagenesis, DNA shuffling, alanine scanning, or the like, can also be used to determine which residues are optimum candidates for saturation mutagenesis, e.g., using the methods herein.

When considering structural filters, it will be appreciated that the three-dimensional structures of a large number of proteins have been determined by x-ray crystallography, nuclear magnetic resonance (NMR) spectroscopy, and the like. Many structures are freely available for download from the Protein Data Bank, at (www(dot)rcsb(dot) org/pdb. Reference protein structures, along with domain and homology information, are also freely available for search and download from the National Center for Biotechnology Information's Molecular Modeling DataBase, at www(dot)ncbi(dot)nlm(dot)nih(dot)gov/Structure/MMDB/mmdb(dot)shtml. The structures of additional proteins can be modeled, for example, based on homology with proteins whose structures have already been determined. Alternatively, the structure of a given protein, optionally complexed with a nucleotide analogue, or the like, can be determined. Once determined, structural information can be used to guide variant construction.

For example, techniques for crystal structure determination are well known. See, for example, McPherson (1999) Crystallization of Biological Macromolecules Cold Spring Harbor Laboratory; Bergfors (1999) Protein Crystallization International University Line; Mullin (1993) Crystallization Butterwoth-Heinemann; Stout and Jensen (1989) X-ray structure determination: a practical guide, 2nd Edition Wiley Publishers, New York; Ladd and Palmer (1993) Structure determination by X-ray crystallography, 3rd Edition Plenum Press, NewYork; Blundell and Johnson (1976) Protein Crystallography Academic Press, New York; Glusker and Trueblood (1985) Crystal structure analysis: A primer, 2nd Ed. Oxford University Press, NewYork; International Tables for Crystallography, Vol. F. Crystallography of Biological Macromolecules; McPherson (2002) Introduction to Macromolecular Crystallography Wiley-Liss; McRee and David (1999) Practical Protein Crystallography, Second Edition Academic Press; Drenth (1999) Principles of Protein X-Ray Crystallography (Springer Advanced Texts in Chemistry) Springer-Verlag; Fanchon and Hendrickson (1991) Chapter 15 of Crystallographic Computing, Volume 5 IUCr/Oxford University Press; Murthy (1996) Chapter 5 of Crystallo graphic Methods and Protocols Humana Press; Dauter et al. (2000) "Novel approach to phasing proteins: derivatization by short cryo-soaking with halides" Acta Cryst.D56:232-237; Dauter (2002) "New approaches to high-throughput phasing" Curr. Opin. Structural Biol. 12:674-678; Chen et al. (1991) "Crystal structure of a bovine neurophysin-II dipeptide complex at 2.8 Å determined from the single-wavelength anomalous scattering signal of an incorporated iodine atom" Proc. Natl Acad. Sci. USA, 88:4240-4244; and Gavira et al. (2002) "Ab initio crystallographic structure determination of insulin from protein to electron density without crystal handling" Acta Cryst.D58:1147-1154.

In addition, a variety of programs to facilitate data collection, phase determination, model building and refinement, and the like are publicly available. Examples include, but are not limited to, the HKL2000 package (Otwinowski and Minor (1997) "Processing of X-ray Diffraction Data Collected in Oscillation Mode" Methods in Enzymology 276:307-326), the CCP4 package (Collaborative Computational Project (1994) "The CCP4 suite: programs for protein crystallography" Acta Crystallogr D 50:760-763), SOLVE and RESOLVE (Terwilliger and Berendzen (1999) Acta Crystallogr D 55 (Pt 4):849-861), SHELXS and SHELXD (Schneider and Sheldrick (2002) "Substructure solution with SHELXD" Acta Crystallogr D Biol Crystallogr 58:1772-1779), Refmac5 (Murshudov et al. (1997) "Refinement of Macromolecular Structures by the Maximum-Likelihood Method" Acta Crystallogr D 53:240-255), PRODRG (van Aalten et al. (1996) "PRODRG, a program for generating molecular topologies and unique molecular descriptors from coordinates of small molecules" J Comput Aided Mol Des 10:255-262), and O (Jones et al. (1991) "Improved methods for building protein models in electron density maps and the location of errors in these models" Acta Crystallogr A 47 (Pt 2):110-119).

Techniques for structure determination by NMR spectroscopy are similarly well described in the literature. See, e.g., Cavanagh et al. (1995) Protein NMR Spectroscopy: Principles and Practice, Academic Press; Levitt (2001) Spin Dynamics: Basics of Nuclear Magnetic Resonance, John Wiley & Sons; Evans (1995) Biomolecular NMR Spectroscopy, Oxford University Press; Wüthrich (1986) NMR of Proteins and Nucleic Acids (Baker Lecture Series), Kurt Wiley-Interscience; Neuhaus and Williamson (2000) The Nuclear Overhauser Effect in Structural and Conformational Analysis, 2nd Edition, Wiley-VCH; Macomber (1998) A Complete Introduction to Modern NMR Spectroscopy, Wiley-Interscience; Downing (2004) Protein NMR Techniques (Methods in Molecular Biology), 2nd edition, Humana Press; Clore and Gronenborn (1994) NMR of Proteins (Topics in Molecular and Structural Biology), CRC Press; Reid (1997) Protein NMR Techniques, Humana Press; Krishna and Berliner (2003) Protein NMR for the Millenium (Biological Magnetic Resonance), Kluwer Academic Publishers; Kiihne and De Groot (2001) Perspectives on Solid State NMR in Biology (Focus on Structural Biology, 1), Kluwer Academic Publishers; Jones et al. (1993) Spectroscopic Methods and Analyses: NMR, Mass Spectrometry, and Related Techniques (Methods in Molecular Biology, Vol. 17), Humana Press; Goto and Kay (2000) Curr. Opin. Struct. Biol. 10:585; Gardner (1998) Annu. Rev. Biophys. Biomol. Struct. 27:357; Wüthrich (2003) Angew. Chem. Int. Ed. 42:3340; Bax (1994) Curr. Opin. Struct. Biol. 4:738; Pervushin et al. (1997) Proc. Natl. Acad. Sci. U.S.A. 94:12366; Fiaux et al. (2002) Nature 418:207; Fernandez and Wider (2003) Curr. Opin. Struct. Biol. 13:570; Ellman et al. (1992) J. Am. Chem. Soc. 114:7959; Wider (2000) BioTechniques 29:1278-1294; Pellecchia et al. (2002) Nature Rev. Drug Discov. (2002) 1:211-219; Arora and Tamm (2001) Curr. Opin. Struct. Biol. 11:540-547; Flaux et al. (2002) Nature 418:207-211; Pellecchia et al. (2001) J. Am. Chem. Soc. 123:4633-4634; and Pervushin et al. (1997) Proc. Natl. Acad. Sci. USA 94:12366-12371.

Modeling of the active site can involve simple visual inspection of a model of the protein, for example, using molecular graphics software such as the PyMOL viewer (open source, freely available on the World Wide Web at (www.) pymol.org) or Insight II (commercially available from Accelrys at (www(dot)accelrys(dot)com/products/insight). This can lead to the selection of sites for variation, e.g., where structural/ functional relevance of the residue is considered likely. Alternatively, modeling can involve computer-assisted docking, molecular dynamics, free energy minimization, and/or like calculations. Such modeling techniques have been well described in the literature; see, e.g., Babine and Abdel-Meguid (eds.) (2004) Protein Crystallography in Drug Design, Wiley-VCH, Weinheim; Lyne (2002) "Structure-based virtual screening: An overview" Drug Discov. Today 7:1047-1055; Molecular Modeling for Beginners, at (www(dot)usm(dot)maine(dot)edu/~rhodes/SPVTut/index(dot)html; and Methods for Protein Simulations and Drug Design at (www(dot)dddc(dot)ac(dot)cn/embo04; and references therein. Software to facilitate such modeling is widely available, for example, the CHARMm simulation package, available academically from Harvard University or commercially from Accelrys (at www(dot)accelrys(dot)com), the Discover simulation package (included in Insight II, *supra*), and Dynama (available at (www(dot)cs(dot)gsu(dot)edu/~cscrwh/progs/progs(dot)html). See also an extensive list of modeling software at (www(dot)netsci(dot)org/Resources/Software/Modeling/MMMD/top(dot)html.

Previous and Parallel Mutagenesis

A wide variety of mutational methods have been in use for several decades. As a result, considerable information regarding residues that have an effect on molecule function is available. These residues are desirably targeted for saturation mutagenesis, e.g., by the methods herein. In addition, available mutagenesis methods can be applied to any sequence to identify residues of interest. These residues of interest can be varied using the methods herein. Suitable methods for identifying sites of interest include alanine-scanning, random mutagenesis (e.g., by error prone PCR) point mutagenesis, DNA shuffling, and many others. For one example of random mutagenesis being used to identify targets for saturation mutagenesis, see e.g., May et al. (2000) "Inverting enantioselectivity by directed evolution of hydantoinase for improved production of 1-methionin" Nature Biotechnology 18,317-320.

Thus, traditional mutagenesis methods can be used to identify residues that are particularly well-suited for saturation mutagenesis by the methods herein, and/or simply to subject polynucleotide variants to any available mutagenesis method. For example, in addition to being used as a logical filter for codon site selection, a polynucleotide variant that comprises an activity of interest can also be further mutated by any available mutagenesis method, taking advantage of the features of that method.

Additional information on mutation formats is found in Sambrook 2001 and Ausubel, as well as in In Vitro Mutagenesis Protocols (Methods in Molecular Biology) Jeff Braman (Editor) Humana Press; 2nd edition (2002) ISBN-10: 0896039102; Chromosomal Mutagenesis (Methods in Molecular Biology) Gregory D. Davis (Editor), Kevin J. Kayser (Editor) Humana Press; 1st edition (2007) ISBN-10: 158829899X; PCR Cloning Protocols (Methods in Molecular Biology) Bing-Yuan Chen (Editor), Harry W. Janes (Editor) Humana Press; 2nd edition (2002) ISBN-10: 0896039692; Directed Enzyme Evolution: Screening and Selection Methods (Methods in Molecular Biology) Frances H. Arnold (Editor), George Georgiou (Editor) Humana Press; 1st edition (2003) ISBN-10: 58829286X; Directed Evolution Library Creation: Methods and Protocols (Methods in Molecular Biology) (Hardcover) Frances H. Arnold (Editor), George Georgiou (Editor) Humana Press; st1 edition (2003) ISBN-10: 1588292851; Short Protocols in Molecular Biology (2 volume set); Ausubel et al. (Editors) Current Protocols; 52 edition (2002) ISBN-10: 0471250929; and PCR Protocols A Guide to Methods and Applications (Innis et al. eds) Academic Press Inc. San Diego, CA (1990) (Innis).

The following publications and references provide additional detail on various available mutation formats: Arnold, Protein engineering for unusual environments, Current Opinion in Biotechnology 4:450-455 (1993); Bass et al., Mutant Trp repressors with new DNA-binding specificities, Science 242:240-245 (1988); Botstein & Shortle, Strategies and applications of in vitro mutagenesis, Science 229:1193-1201(1985); Carter et al., Improved oligonucleotide site-directed mutagenesis using M13 vectors, Nucl. Acids Res. 13: 4431-4443 (1985); Carter, Site-directed mutagenesis, Biochem. J. 237:1-7 (1986); Carter, Improved oligonucleotide-directed mutagenesis using M13 vectors, Methods in Enzymol. 154: 382-403 (1987); Dale et al., Oligonucleotide-directed random mutagenesis using thephosphorothioate method, Methods Mol. Biol. 57:369-374 (1996); Eghtedarzadeh & Henikoff, Use of oligonucleotides to generate large deletions, Nucl. Acids Res. 14: 5115 (1986); Fritz et al., Oligonucleotide-directed construction of mutations: a gapped duplex DNA procedure without enzymatic reactions in vitro, Nucl. Acids Res. 16: 6987-6999 (1988); Grundstrom et al., Oligonucleotide-directed mutagenesis by microscale 'shot-gun' gene synthesis, Nucl. Acids Res. 13: 3305-3316 (1985); Kunkel, The efficiency of oligonucleotide directed mutagenesis, in Nucleic Acids & Molecular Biology (Eckstein, F. and Lilley, D.M.J. eds., Springer Verlag, Berlin)) (1987); Kunkel, Rapid and efficient site-specific mutagenesis without phenotypic selection, Proc. Natl. Acad. Sci. USA 82:488-492 (1985); Kunkel et al., Rapid and efficient site-specific mutagenesis without phenotypic selection, Methods in Enzymol. 154, 367-382 (1987); Kramer et al., The gapped duplex DNA approach to oligonucleotide-directed mutation construction, Nucl. Acids Res. 12: 9441-9456 (1984); Kramer & Fritz Oligonucleotide-directed construction of mutations via gapped duplex DNA, Methods in Enzymol. 154:350-367 (1987); Kramer et al., Point Mismatch Repair, Cell 38:879-887 (1984); Kramer et al., Improved enzymatic in vitro reactions in the gapped duplex DNA approach to oligonucleotide-directed construction of mutations, Nucl. Acids Res. 16: 7207 (1988); Ling et al., Approaches to DNA mutagenesis: an overview, Anal Biochem. 254(2): 157-178 (1997); Lorimer and Pastan Nucleic Acids Res. 23, 3067-8 (1995); Mandecki, Oligonucleotide-directed double-strand break repair in plasmids of Escherichia coli: a method for site-specific mutagenesis, Proc. Natl. Acad. Sci. USA, 83:7177-7181 (1986); Nakamaye & Eckstein, Inhibition of restriction endonuclease Nci I cleavage by phosphorothioate groups and its application to oligonucleotide-directed mutagenesis, Nucl. Acids Res. 14: 9679-9698 (1986); Nambiar et al., Total synthesis and cloning of a gene coding for the ribonuclease S protein, Science 223: 1299-1301 (1984); Sakamar and Khorana, Total synthesis and expression of a gene for the a-subunit of bovine rod outer segment guanine nucleotide-binding protein (transducin), Nucl. Acids Res. 14: 6361-6372 (1988); Sayers et al., Y-T Exonucleases in phosphorothioate-based oligonucleotide-directed mutagenesis, Nucl. Acids Res. 16:791-802 (1988); Sayers et al., Strand specific cleavage of phosphorothioate-containing DNA by reaction with restriction endonucleases in the presence of ethidium bromide, (1988) Nucl. Acids Res. 16: 803-814; Sieber, et al., Nature Biotechnology, 19:456-460 (2001); Smith, In vitro mutagenesis, Ann. Rev. Genet. 19:423-462(1985); Methods in Enzymol. 100: 468-500 (1983); Methods in Enzymol. 154: 329-350 (1987); Stemmer, Nature 370, 389-91 (1994); Taylor et al., The use of phosphorothioate-modified DNA in restriction enzyme reactions to prepare nicked DNA, Nucl. Acids Res. 13: 8749-8764 (1985); Taylor et al., The rapid generation of oligonucleotide-directed mutations at high frequency using phosphorothioate-modified DNA, Nucl. Acids Res. 13: 8765-8787 (1985); Wells et al., Importance of hydrogen-bond formation in stabilizing the transition state of subtilisin, Phil. Trans. R. Soc. Lond. A 317: 415-423 (1986); Wells et al., Cassette mutagenesis: an efficient method for generation of multiple mutations at defined sites, Gene 34:315-323 (1985); Zoller & Smith, Oligonucleotide-directed mutagenesis using M13-derived vectors: an efficient and general procedure for the production ofpoint mutations in any DNA fragment, Nucleic Acids Res. 10:6487-6500 (1982); Zoller & Smith, Oligonucleotide-directed mutagenesis of DNA fragments cloned into M13 vectors, Methods in Enzymol. 100:468-500 (1983); and Zoller & Smith, Oligonucleotide-directed mutagenesis: a simple method using two oligonucleotide primers and a single-stranded DNA template, Methods in Enzymol. 154:329-350 (1987). Additional details on many of the above methods can be found in Methods in Enzymology Volume 154, which also describes various controls for trouble-shooting problems with several mutagenesis methods.

Homology

In one aspect, a homology logic filter is used to identify promising targets for variation. A variety of references describe the alignment of homologous nucleic acids to identify nucleotide targets for variation. These include WO/2000/042561 by Crameri et al. OLIGONUCLEOTIDE MEDIATED NUCLEIC ACID RECOMBINATION; WO/2000/042560 by Selifonov et al. METHODS FOR MAKING CHARACTER STRINGS, POLYNUCLEOTIDES AND POLYPEPTIDES; WO/2001/075767 by GUSTAFSSON et al. IN SILICO CROSS-OVER SITE SELECTION; and WO/2000/004190 by del Cardayre EVOLUTION OF WHOLE CELLS AND ORGANISMS BY RECURSIVE SEQUENCE RECOMBINATION. Such structural alignments can be used to identify codons sites to vary in the present invention as well. This has the advantage of focusing on residues that have previously been determined, e.g., through natural or artificial selection, to be important in the structure or function of a reference polypeptide. See also Wankhade et al. (2000) J. Biol. Chem. 275(38):29701-29708; Reddy et al. (2001) Proteins: Structure, Function, and Genetics 42:148-163; Bidwell et al. (1999) Genes and Immunity 1:3-19; Chen et al., (2003) Mol. Biol. Evo. 18:1771-1788.

Sequence alignments of homologous enzymes have been used to produce selected amino acid alphabets at particular sites. See, e.g., WO/2000/042561 by Crameri et al. OLIGONUCLEOTIDE MEDIATED NUCLEIC ACID RECOMBINATION; WO/2000/042560 by Selifonov et al. METHODS FOR MAKING CHARACTER STRINGS, POLYNUCLEOTIDES AND POLYPEPTIDES; WO/2001/075767 by GUSTAFSSON et al. IN SILICO CROSS-OVER SITE SELECTION. Other relevant approaches include the use of 2, 6, 7, and 9 amino acid alphabets at particular sites, based upon homology and other considerations (Reetz and Wu (2008) "Greatly Reduced Amino Acid Alphabets in Directed Evolution: Making the Right Choice For Saturation Mutagenesis and Homologous Amino Acid Positions" Chem. Commun. 5499-5501).

Statistical Filtering

Codon sites for variation according to the invention can be selected via any of a variety of statistical methods, e.g., that account for the physico-chemical nature of encoded amino acids, sequence difference or identity between homologous variants, weighting based upon position within or relative to an active site of an encoded protein, or the like. In addition, sites can be selected by applying statistical filters to sequences of variants, e.g., in secondary rounds of site selection and library generation, using any available statistical or heuristic filter. Selection of which codon sets are likely to be the most useful at a given postion or set of positions can also be determined via a statistical or other data analysis method. Statistical filters such as hidden Markov models (HMMs) have also been used to identify and select reduced amino acid alphabets (Susko and Roger (2007) "On Reduced Amino Acid Alphabets for Phylogenetic Inference" Mol. Biol. Evol. 24(9):2139-2150) that can be used to reduce library complexity. Nat Biotechnol. 2007 Mar;25(3):338-44. Epub 2007 Feb 18. In one useful example, PROSAR provides statistical filters for improving enzyme activity (Fox et al. (2007) "Improving catalytic function by ProSAR-driven enzyme evolution," Nat Biotechnol. 25(3):297-8.

In addition to ProSAR, various vendors, such as Partek Incorporated (St. Peters, Mo.; www.partek.com) provide software for pattern recognition which can be applied to codon site or composition interpretation or analysis to guide additional site or codon type selection. Relationships between datasets, e.g., between the sequence and activity of nucleic acid variants, the appearance or frequency of particular sites of variation in a set of variants, or the like, can be analyzed by any of a variety of methods, and the results of this analysis can be used to determine codon site selection, codon composition, relationships between codon sites or composition, or the like.

Examples of appropriate approaches include analysis by pattern recognition software, Bayes classifiers, genetic algorithms, neural networks, Monte Carlo analysis, Principal Component Analysis (PCA), Markov modeling, etc. Additional details on these and related topics that can be used to analyze, e.g., sequence and activity information can be found in David E. Goldberg (1989) Genetic Algorithms in Search, Optimization and Machine Learning Addison-Wesley Pub Co; ISBN: 0201157675; Timothy Masters (1993) Practical Neural Network Recipes in C++(Book & Disk edition) Academic Pr; ISBN: 0124790402; Kevin Gurney (1999) An Introduction to Neural Networks, UCL Press, 1 Gunpowder Square, London EC4A 3DE, UK; Christopher M. Bishop (1995) Neural Networks for Pattern Recognition Oxford Univ Press; ISBN: 0198538642; Brian D. Ripley, N. L. Hjort (Contributor) (1995) Pattern Recognition and Neural Networks Cambridge Univ Pr (Short); ISBN: 0521460867;Rubinstein, R. Y.; Kroese, D. P. (2007) Simulation and the Monte Carlo Method (2nd ed.). New York: John Wiley & Sons. ISBN 9780470177938; Tarantola, Albert (2005) Inverse Problem Theory Philadelphia: Society for Industrial and Applied Mathematics ISBN 0898715725; Steeb (2008) The Nonlinear Workbook: Chaos, Fractals, Neural Networks, Genetic Algorithms, Gene Expression Programming, Support Vector Machine, Wavelets, Hidden Markov Models, Fuzzy Logic with C++, Java and SymbolicC++ Programs: 4th edition. World Scientific Publishing. ISBN 981-281-852-9; Sergios Theodoridis, Konstantinos Koutroumbas, (2009) Pattern Recognition (4th edition), Elsevier, ISBN 978-1-59749-272-0, and in a variety of other currently available references. Any of these methods can be embodied in system instructions to facilitate codon site selection, codon composition, or the like. Computers/digital appliances that can be incorporated into the systems of the disclosure to facilitate such methods include or can be operably coupled to user viewable display systems (monitors, CRTs, printouts, etc.), printers to print data relating to signal information, peripherals such as magnetic or optical storage drives, user input devices (keyboards, microphones, pointing devices) and the like.

Sites of variation can be also obtained by consideration of various sources of information, using any of the filtering approaches herein. In some embodiments, the amino acid residue positions and corresponding mutations for a polypeptide or set of variants can be obtained from directed evolution experiments, such as those described in the references herein, and, e.g., in Crameri et al. (1998) "DNA shuffling of a family of genes from diverse species accelerates directed evolution," Nature 391:288-291; Crameri et al., (1997) "Molecular evolution of an arsenate detoxification pathway by DNA shuffling," Nature Biotech 15:436-438; Zhang et al. (1997) "Directed evolution of an effective fructosidase from a galactosidase by DNA shuffling and screening," Proc Natl Acad Sci USA 94:45-4-4509; Crameri et al., (1996) "Improved green fluorescent protein by molecular evolution using DNA shuffling, Nature Biotech 14:315-319; Stemmer, (1994) "Rapid evolution of a protein in vitro by DNA shuffling," Nature 370:389-391; Stemmer, (1994) "DNA shuffling by random fragmentation and reassembly: In vitro recombination for molecular evolution," Proc Natl Acad Sci USA 91:10747-10751 and in WO 95/22625; WO 97/0078; WO 97/35966; WO 98/27230; WO 00/42651; WO 01/75767 and U.S. Pat. 6,537,746.

Reducing Variant Amino Acid Content at Sites of Variation

In some applications, maximum amino acid diversity at each site to be varied is desirable. However, to reduce screening burden, in some instances the amino acid diversity to be encoded at a particular site can be reduced. As noted herein, high but not complete diversity sets provide reasonable tradeoffs in this regard. For example, the set VMA, NDT (which encodes 18 amino acids, not including M and W) can be used in the methods and libraries of the disclosure. Incorporation of M and W, in particular, is relatively less likely to result in gain of function mutations, making it reasonable to use codon sets that omit these amino acids. Thus, the present disclosure includes methods of making libraries that comprise all 20 cannonical amino acids, 19 cannonical amino acid (e.g., all 20 cannonical amino acids except M or W), 18 amino acids (all 20 cannonical amino acids except M and W), as well as degenerate oligonucleotides encoding all 20 amino acids at a codon site of interest, all 20 amino acids except M or W at the site, or all 20 amino acids except M and W at the site. Polynucleotides that incorporate the codon sites that encode 18, 19, or 20 amino acids, e.g., through polymerase or ligase mediated assembly of the degenerate oligonucleotides (e.g., using the oligonucleotides on a reference polynucleotide template) are also a feature of the disclosure.

Additional reductions in amino acid content are also possible, and may be more desirable when a large number of residues is to be varied, or when a clear logical basis exists to limit the amio acid set to be screened at a given site of variation. Reduced amino acid sets that represent broad functional diversity have also been identified. For example, 9 or 10 amino acid "types" have been used to identify useful mutants with an efficiency argued to be similar to the use of the typical canonical 20 amino acids. See, e.g., Li at al. (2003) "Reduction of Protein Sequence Complexity by Residue Grouping" Protein Engineering 16(5):323-330 and Akanuma et al. (2002) "Combinatorial mutagenesis to restrict amino acid usage in an enzyme to a reduced set," PNAS 99(21):13549-13553. More simply, amino acids are often grouped according to physico-chemical properties-- nonpolar, polar, basic, acidic, etc.-- by selecting representative members from each grouping, broad structural and chemical diversity can be incorporated without the screening burden of using all possible amino acids.

Knowledge regarding the structure of a reference polypeptide can be used to filter which residues are less likely to be targets for variation, and also which residues are relatively unlikely to result in a desired property. For example, if the structure of a reference polypeptide is known, or can be modeled with some degree of accuracy, then the sites that are proximal to the active site will be known. In those instances where a change in an activity of the protein is desirable, residues at the active site provide a target-rich region for targeted variants. Similarly, residues that do not fit into the active site can be excluded during the overall diversity generation process. In general, it is sometimes useful to integrate structure-guided design with some degree of evolutionary randomization; see also, Reetz et al. (2008) "Addressing the Numbers Problem in Directed Evolution" ChemBioChem 9:1797-1804 and Reetz et al. (2006) "Iterative Saturation Mutagenesis on the Basis of B Factors as a Strategy for Increasing Protein Thermostability" Angew. Chem. 118: 7907-7915.

Recursive Filtering

Typically, a variant library of polynucleotides is expressed and the resulting polypeptides screened for a desired property trait, and the mutations associated with the changes in the desired property identified. Large number of mutations affecting a polypeptide function can be readily obtained using these techniques. Any of the filtering approaches herein can be applied to the data sets that are thus generated, thereby focusing sites for further variation, or combination in subsequent rounds of mutagenesis. This process can be repeated one or more times to further refine variant construction and selection.

SCREENING POOLED LIBRARIES

Libraries of variants can be screened for one or more property of interest. In general, screening is most productively focused on libraries that comprise high levels of sequence diversity. Fisher's fundamental theorem of natural selection states: "the rate of increase in fitness of any organism at any time is equal to its genetic variance in fitness at that time." Furthermore, in the context of the invention, "deep" screening (e.g., screening with high levels of oversampling) of combinatorial libraries can be less productive than performing additional rounds of mutation, library generation, in conjunction with "shallow" screening (screening with low levels of oversampling). This is because of the relative ease of generating pooled libraries of variants according to the methods of the invention, and because sampling (or recursive sampling) from several diverse libraries increases the diversity of sequence space that is ultimately sampled.

Screening methodologies depend on the property at issue (e.g., the goal of the screening experiment). Many variants will comprise a screenable property, such as an optical feature (color, ability to fluoresce, etc.) of the variant or a substrate thereof, a survival benefit conferred by the variant (e.g., conferring resistance to a toxin, antibiotic, etc., in a cell that expresses the variant), the ability to produce or modify a substrate in a detectable way, etc. A wide variety of library screening protocols are known and can be applied by the practitioner to screening the libraries of the invention. Examples of available library screening protocols, including many examples of high throughput screening formats, are described, e.g., in Janzen and Bernasconi (2009) High Throughput Screening: Methods and Protocols (Methods in Molecular Biology) Humana Press; 2nd ed. edition ISBN-10: 1603272577; Varnek and Tropsha (Editors) (2008) Chemoinformatics: An Approach to Virtual Screening Royal Society of Chemistry; 1st edition ISBN-10: 0854041443; Lansing Taylor (Editor) (2006) High Content Screening (Methods in Molecular Biology) Humana Press; 1st edition ISBN-10: 1588297314; Hüser et al. (2006) High-Throughput Screening in Drug Discovery (Methods and Principles in Medicinal Chemistry) Wiley-VCH; 1st edition ISBN-10: 3527312838; Larson (Editor) (2005) Bioinformatics and Drug Discovery (Methods in Molecular Biology) ISBN-10: 1588293467; Arnold and Georgiou (eds) (2003) Directed Enzyme Evolution: Screening and Selection Methods (Methods in Molecular Biology) Humana Press, 1st edition ISBN-10: 58829286X; Arnold and Georgiou (Editors) (2003) Directed Evolution Library Creation: Methods and Protocols (Methods in Molecular Biology) Humana Press; 1st edition ISBN-10:1588292851; Bird and Smith (Editors) (2002) Genetic Library Construction and Screening: Advanced Techniques and Applications (Springer Lab Manuals) ISBN-10: 3540672788; English (editor) (2002) Combinatorial Library: Methods and Protocols (Methods in Molecular Biology) Humana Press; 1st edition (September 1, 2002) ISBN-10: 0896039803; and Nicolaou et al. (eds) (2002) Handbook of Combinatorial Chemistry: Drugs, Catalysts, Materials (2-Vol. Set), Wiley-VCH; 1st edition ISBN-10: 3527305092.

Additional details regarding library screening and associated methods can be found, e.g., in Kaufman, et al. (2003) Handbook of Molecular and Cellular Methods in Biology and Medicine Second Edition Ceske (ed) CRC Press (Kaufman); The Nucleic Acid Protocols Handbook Ralph Rapley (ed) (2000) Cold Spring Harbor, Humana Press Inc (Rapley); Short Protocols in Molecular Biology (2 volume set); as well as in Sambrook and Ausubel.

A variety of methods are known and can be used to isolate, detect, manipulate, detect an activity of, or otherwise handle a protein produced in a host cell library according to the invention e.g., from recombinant cultures of cells expressing variant containing proteins of the invention. A variety of protein isolation and detection methods are well known in the art, including, e.g., those set forth in R. Scopes, Protein Purification, Springer-Verlag, N.Y. (1982); Deutscher, Methods in Enzymology Vol. 182: Guide to Protein Purification, Academic Press, Inc. N.Y. (1990); Sandana (1997) Bioseparation of Proteins, Academic Press, Inc.; Bollag, et al. (1996) Protein Methods, 2nd Edition Wiley-Liss, NY; Walker (1996) The Protein Protocols Handbook Humana Press, NJ, Harris and Angal (1990) Protein Purification Applications: A Practical Approach IRL Press at Oxford, Oxford, England; Harris and Angal Protein Purification Methods: A Practical Approach IRL Press at Oxford, Oxford, England; Scopes (1993) Protein Purification: Principles and Practice 3rd Edition Springer Verlag, NY; Janson and Ryden (1998) Protein Purification: Principles, High Resolution Methods and Applications, Second Edition Wiley-VCH, NY; and Walker (1998) Protein Protocols on CD-ROM Humana Press, NJ; and the references cited therein. Additional details regarding protein purification and detection methods can be found in Satinder Ahuja ed., Handbook of Bioseparations, Academic Press (2000).

In one illustrative example, the reference polynucleotide encodes a cellulase. These enzymes, e.g., catalyze the hydrolysis of cellulose. This is a highly useful industrial process, with cellulases being used during, e.g., coffee production, textile manufacturing, conversion of biomass into biofuels, pharmaceutical processes, etc. A variety of cellulase enzymes and their coding nucleic acids are known, including those derived from fungi, bacteria, protozoans and other sources; any of these can serve as a reference polynucleotide of the invention. The cellulase is varied at any of a variety of codon sites as described herein. The resulting cellulase variants are screened for cellulase activity under one or more bioprocess conditions of interest, e.g., under industrial process conditions (temperature, pH, etc.) applicable to the production of biofuels from plant or fiber sources.

RECURSIVITY

The methods herein can be practiced in a recursive fashion. That is, any variant nucleic acid or polypeptide identified by any process herein can serve as a reference nucleic acid/ polypeptide in one or more additional rounds of directed evolution, e.g., to further improve an activity of interest. This process can be repeated in a recursive manner, until a desired level of activity is reached. Screening stringency can be iteratively increased in each round of library screening, thereby selecting for increasing levels of activity. Adding rounds of mutation and selection is often more productive than attempts to sample any particular library exhaustively.

Sequence alterations among different variants can also be combined, e.g., in each round of directed evolution. In this embodiment, different codon sites among different variants that each have a desirable activity or feature of interest are combined into a single reference nucleic acid/ polypeptide, which is used as the basis for additional rounds of mutagenesis and screening. For example, statistical analysis such as PROSAR (Fox et al. (2007) "Improving catalytic function by ProSAR-driven enzyme evolution," Nat Biotechnol. 25(3):297-8) can be used to identify residues of interest, which can be combined into a new reference sequence. Codon sites that appear to confer, e.g., activity, can be held constant, or can be varied in a non-random manner, e.g., optionally by performing conservative substitutions at these sites. However, while conservative substations do appear more frequently in active offspring, it is not always advisable to limit substitutions to conservative mutations, because this can result in beneficial mutations being missed. In any case, any of the statistical approaches noted herein can be used to determine which residues are likely candidates for being held constant, or being varied according to one or more desired criteria. Parallel library construction and screening can be used to increase overall throughput.

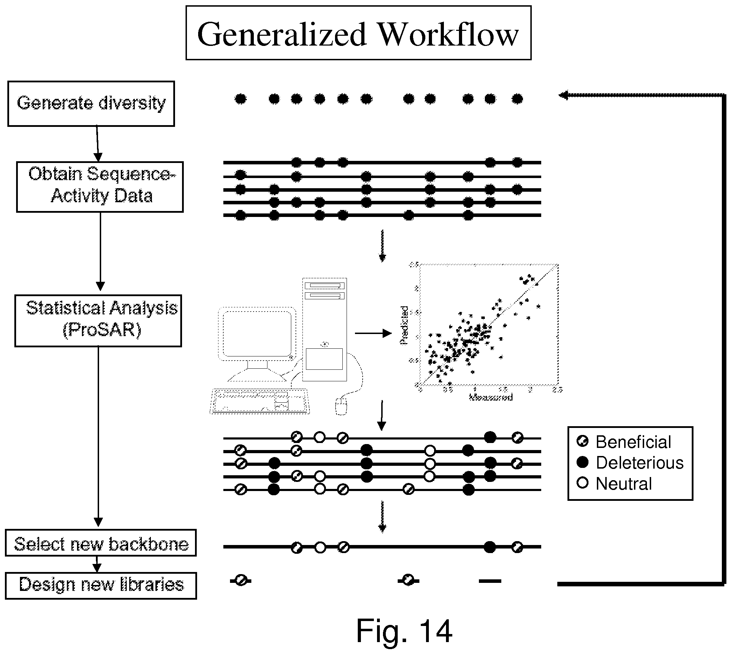

Figure 14 provides a schematic flow chart of an example recursive process flow. As shown, libraries comprising diversity are made and screened. Sequence-activity data is statistically examined, e.g., via PROSAR. Beneficial and deleterious mutations are determined, and new libraries are generated from this information, e.g., by designing one or more new reference backbone molecules.

In one example of a recursive screening approach, one or more combinatorial library is created using SNOCAPS, with an optimized degenerate codon at each site of codon variation. A screening resource allocation model can be taken into account to optimize the number of mutations in the relevant library. Beneficial mutations are identified via high throughput screening. The relationship between sequence and activity from the combinatorial libraries is used to infer mutational effects, e.g., by ProSAR. New libraries are designed, e.g., using ProSAR results and SNOCAPS mutations. Diversity generation and recombination steps can be done in parallel. These steps are repeated, until a variant comprising a desired feature is identified.

Increasing screening stringency can be used between rounds of library construction and screening. For example, one can screen for increased thermal and pH tolerance, e.g., using increasing stringency screening for recursive rounds of library generation and screening.

In one set of embodiments, recursive recombination is performed among active variants (or variants displaying one or more feature of interest) developed from one or more round(s) of mutation and screening. A wide variety of recursive recombination methods are available, e.g., as taught in Stemmer, et al. (1999) "Molecular breeding of viruses for targeting and other clinical properties" Tumor Targeting 4:1-4; Ness et al. (1999) "DNA Shuffling of subgenomic sequences of subtilisin" Nature Biotechnology 17:893-896; Chang et al. (1999) "Evolution of a cytokine using DNA family shuffling" Nature Biotechnology 17:793-797; Minshull and Stemmer (1999) "Protein evolution by molecular breeding" Current Opinion in Chemical Biology 3:284-290; Christians et al. (1999) "Directed evolution of thymidine kinase for AZT phosphorylation using DNA family shuffling" Nature Biotechnology 17:259-264; Crameri et al. (1998) "DNA shuffling of a family of genes from diverse species accelerates directed evolution" Nature 391:288-291; Crameri et al. (1997) "Molecular evolution of an arsenate detoxification pathway by DNA shuffling," Nature Biotechnology 15:436-438; Zhang et al. (1997) "Directed evolution of an effective fucosidase from a galactosidase by DNA shuffling and screening" Proc. Natl. Acad. Sci. USA 94:4504-4509; Patten et al. (1997) "Applications of DNA Shuffling to Pharmaceuticals and Vaccines" Current Opinion in Biotechnology 8:724-733; Crameri et al. (1996) "Construction and evolution of antibody-phage libraries by DNA shuffling" Nature Medicine 2:100-103; Crameri et al. (1996) "Improved green fluorescent protein by molecular evolution using DNA shuffling" Nature Biotechnology 14:315-319; Gates et al. (1996) "Affinity selective isolation of ligands from peptide libraries through display on a lac repressor 'headpiece dimer'" Journal of Molecular Biology 255:373-386; Stemmer (1996) "Sexual PCR and Assembly PCR" In: The Encyclopedia of Molecular Biology. VCH Publishers, New York. pp. 447-457; Crameri and Stemmer (1995) "Combinatorial multiple cassette mutagenesis creates all the permutations of mutant and wildtype cassettes" BioTechniques 18:194-195; Stemmer et al., (1995) "Single-step assembly of a gene and entire plasmid form large numbers of oligodeoxy-ribonucleotides" Gene, 164:49-53; Stemmer (1995) "The Evolution of Molecular Computation" Science 270: 1510; Stemmer (1995) "Searching Sequence Space" Bio/Technology 13:549-553; Stemmer (1994) "Rapid evolution of a protein in vitro by DNA shuffling" Nature 370:389-391; and Stemmer (1994) "DNA shuffling by random fragmentation and reassembly: In vitro recombination for molecular evolution." Proc. Natl. Acad. Sci. USA 91:10747-10751. Additional recursive recombination references include WO/2000/042561 by Crameri et al. OLIGONUCLEOTIDE MEDIATED NUCLEIC ACID RECOMBINATION; WO/2000/042560 by Selifonov et al. METHODS FOR MAKING CHARACTER STRINGS, POLYNUCLEOTIDES AND POLYPEPTIDES; WO/2001/075767 by GUSTAFSSON et al. IN SILICO CROSS-OVER SITE SELECTION; and WO/2000/004190 by del Cardayre EVOLUTION OF WHOLE CELLS AND ORGANISMS BY RECURSIVE SEQUENCE RECOMBINATION.

DEFINITIONS

This invention is not limited to particular devices or biological systems, which can, of course, vary. Terminology used herein is for the purpose of describing particular embodiments, and is not necessarily limiting. As used in this specification and the appended claims, the singular forms "a", "an" and "the" optionally include plural referents unless the content clearly dictates otherwise. Thus, for example, reference to "a variant" optionally includes multiple variant molecules, unless context dictates otherwise.

Unless defined otherwise, all technical and scientific terms used herein have the same meaning as commonly understood by one of ordinary skill in the art to which the invention pertains. Although many methods and materials similar or equivalent to those described herein can be used in conformity with the present invention, preferred materials and methods are described herein. In describing and claiming the present invention, the following terminology will be used in accordance with the definitions set out below.

A "codon site" is a site in a polynucleotide or polynucleotide sequence where a codon of interest appears. A "codon" is a series of nucleotides of a polynucleotide that encode an amino acid. In the context of multiple homologous molecules, a codon site is a position where a codon of interest appears, e.g., when homologous nucleotide sequences are aligned to achieve maximum similarity. A "site of codon variation" is a codon site in a set of polynucleotide variants (e.g., typically, homologs) that varies among at least some members of the set of variants.

A "degenerate codon site" is a site of codon variation where one or more nucleotides of a codon of interest are varied among variant polynucleotides according to a substitution rule. For example, a degenerate codon site amongst homologous molecules can be varied according to a stated logical substitution rule. For example, the degenerate codon "NNK" is a codon that comprises any nucleotide of a selected type (e.g., for canonical DNA, A, C, G, or T, or, for canonical RNA, A, C, G or U) at the first two positions and a G or T (or U, if the relevant polynucleotide is an RNA) at the third position (K = G or T/U). Similarly, an "NNS" degenerate codon set includes a C or G at the third position (S=C or G). An NNK or codon, for example, encodes the 20 canonical amino acids using 32 codons; these 32 codons are represented by the degenerate codon designation "NNK." A degenerate NNK codon site is a codon site in a set of related polynucleotides that is represented or described by an NNK designation. The terms "degenerate codon site" or "degenerate codon" as typically used herein are distinct from one common meaning of the phrase "codon degeneracy" used in the literature to refer to multiple codons redundantly encoding an amino acid in the genetic code (64 codons for 20 canonical amino acids).

A "polynucleotide" is a polymer of nucleotide residues, such as occurs in nature in a DNA or RNA. The polynucleotide can include either or both natural (A, C, G, T/U) and unnatural nucleotides (e.g., inosine or I). A polynucleotide can also comprise non-nucleotide elements, e.g., resulting from fusion of a polynucleotide polymer with a non-nucleotide moiety such as a molecular label. A "reference polynucleotide" is a polynucleotide of interest. The reference polynucleotide can be used as a starting point for designing homologous variants of the reference polynucleotide. Typical reference polynucleotides include wild-type polynucleotides found in nature, e.g., as specified by a sequence available in a database, or a molecule that can be determined by cloning and sequencing a molecule found in nature. Reference polynucleotides also include mutant or artificial nucleotides of interest, e.g., molecules that have been developed by recombinant technologies to comprise one or more useful property. A "polynucleotide variant" is a molecule that is related by sequence to a reference polynucleotide. In one typical example, the polynucleotide variant and reference are homologous, i.e., they descend from a common ancestor polynucleotide, either through natural or artificial evolution.

**Claims**

1. A library of polynucleotides, comprising: a mixture of polynucleotide variant molecules, the variant molecules comprising at least a first degenerate codon position, wherein degenerate codons of the first position encode any encoded amino acid in a selected molar ratio, and wherein each degenerate codon position comprises NNT, VWG, TGG wherein the ratio of codons is 16(NNT):6(VWG):1(TGG), or each degenerate codon position comprises (VMA, NDT, WKG) wherein the ratio of codons in the set is 6(VMA): 12(NDT):4(WKG).

2. The library of claim 1, wherein the variant molecules collectively comprise:

   a first set of polynucleotide variants comprising at least a first degenerate codon position; and,

a second set of polynucleotide variants comprising at least a second degenerate codon position different from the first position.

3. The library of claim 2, wherein:

(a) the first and the second position comprise the same degenerate codon set;
(b) the first set of nucleic acid variants comprises a reference codon for a plurality of members of the first set in a codon position corresponding to the second degenerate codon position in the second set;
(c) the second set of nucleic acid variants comprises a reference codon for a plurality of members of the second set in a codon position corresponding to the first degenerate codon position in the first set;
(d) both (b) and (c); or
(e) the first and second set of polynucleotide variants comprise overlapping members.

4. A method of making a library of polynucleotide variants, the method comprising:

providing a reference polynucleotide molecule or reference polynucleotide sequence;
selecting at least one site in the reference polynucleotide molecule or reference polynucleotide sequence to be varied; and,
producing a set of variant polynucleotides comprising degenerate codons at the site of interest, wherein the degenerate codons at each site of interest comprise (NNT, VWG, TGG) in a molar ratio of approximately 16:6:1, or wherein the degenerate codons at each site of interest comprise (VMA, NDT, WKG) and wherein the ratio of codons in the set is 6(VMA):12(NDT):4(WKG), thereby providing the library.

5. A library made by the method of claim 4.

6. A composition comprising a set of polynucleotide variants comprising at least one degenerate codon position, wherein the degenerate codons at the position comprise (NNT, VWG, TGG) in a molar ratio of approximately 16:6:1, or wherein the degenerate codons at the position comprise (VMA, NDT, WKG) and wherein the ratio of codons in the set is 6(VMA):12(NDT):4(WKG), wherein the polynucleotide variants collectively comprise all possible variants represented by the degenerate codon position.

7. The composition of claim 6, wherein the polynucleotide variants are synthetic oligonucleotides and/or the polynucleotide variants are nucleic acid amplicons.

8. A method of producing a set of variant polynucleotide molecules, the method comprising:

providing a reference polynucleotide sequence;
selecting a plurality of codon sites in the reference polynucleotide sequence to be varied;
selecting a degenerate codon set for the plurality of codon sites to be varied, the degenerate codon set comprising (NNT, VWG, TGG) in a molar ratio of approximately 16:6:1 or comprising (VMA, NDT, WKG) wherein the ratio of codons in the set is 6(VMA): 12(NDT):4(WKG);
producing the set of variant polynucleotide molecules, each variant polynucleotide comprising a member of the degenerate codon set at each of the plurality of sites of codon variation, wherein the set of variant polynucleotide molecules collectively comprises all of the members of the degenerate codon set at each of the sites of codon variation.

9. A method of producing a plurality of sets of variant polynucleotide molecules, the method comprising:

providing a reference polynucleotide sequence;
selecting a plurality of codon sites in the reference polynucleotide sequence to be varied;
selecting a degenerate codon set for the plurality of codon sites to be varied, the degenerate codon set comprising (NNT, VWG, TGG) in a molar ratio of approximately 16:6:1 or comprising (VMA, NDT, WKG) wherein the ratio of codons in the set is 6(VMA): 12(NDT):4(WKG);
separately producing the plurality of sets of variant polynucleotide molecules, each variant polynucleotide comprising a member of the degenerate codon set at each of the plurality of sites of codon variation, wherein the plurality of sets of variant polynucleotide molecules collectively comprises all of the members of the degenerate codon set at each of the sites of codon variation.

**10.** The method of claim 8 or claim 9, wherein:

(i) the codon sites are selected by considering one or more of: a three-dimensional structure of a reference protein encoded by the polynucleotide molecule or sequence; results of a mutagenesis experiment performed on the reference polynucleotide or sequence, or application of a statistical filter that identifies sites of interest; (ii) the codon site is selected by performing random mutagenesis, recursive recombination, or alanine scanning on the reference nucleic acid or a homolog thereof, and screening any resulting mutants for activity to identify codon sites of interest (iii) the variant polynucleotides comprise at least 10 sites of codon variation; (iv) the variant polynucleotides comprise at least 25 sites of codon variation; (v) the variant polynucleotides comprise at least 50 sites of codon variation; and/or (vi) at least about 50% of the codons in the variant polynucleotides comprise sites of codon variation.

**11.** The method of any of claims 8-10, wherein:

(i) selecting the plurality of codons comprises considering a structural model of a reference polypeptide encoded by the reference polynucleotide molecule or sequence, and selecting sites based on their relationship to one or more features of the reference polypeptide; (ii) selecting the plurality of codons comprises considering an alignment of the reference polynucleotide with one or more homologous polynucleotides, or of a reference polypeptide encoded by the reference polynucleotide with one or more homologous polypeptides, and selecting the codons for variation based on a feature of the alignment; (iii) separately producing the sets of polynucleotide variants comprises synthesizing sets of degenerate oligonucleotides comprising the degenerate codons, and separately amplifying the reference polynucleotide in separate polymerase or ligase amplification reactions using the degenerate oligonucleotides as amplification primers; and/or (iv) the method further comprises assembling the sets of polynucleotide variants into one or more expression vectors by performing a subsequent PCR with a megaprimer comprising a polynucleotide variant sequence bound to a circular template nucleic acid, optionally wherein (a) the megaprimer PCR reaction is primed from two abutting primers, at least one of which comprises a variant sequence, or (b) the megaprimer PCR reaction is primed from two overlapping primers, at least one of which comprises a variant sequence.

**12.** The method of any of claims 8-11, wherein the method comprises selecting a single degenerate codon set for all of the codons to be varied, and wherein optionally:

(i) 3 or more codons are selected to be varied using the single degenerate codon set; (ii) 5 or more codons are selected to be varied using the single degenerate codon set; or (iii) 10 or more codons are selected to be varied using the single degenerate codon set,

and/or further comprising screening the library for one or more properties of interest, optionally wherein:

(a) about 80% or less of possible variants for each site of variation within the library is screened for the one or more properties of interest; (b) about 60% or less of possible variants for each site of variation within the library is screened for the one or more properties of interest; or (c) between about 50% and about 60% of possible variants for each site of variation in the library is screened for the one or more properties of interest.

**13.** A library made by the method of any one of claims 8-12.

**Patentansprüche**

**1.** Bibliothek von Polynukleotiden, Folgendes umfassend: ein Gemisch aus Polynukleotidvariantenmolekülen, wobei die Variantenmoleküle mindestens eine erste degenerierte Codonposition umfassen, wobei degenerierte Codons der ersten Position für jede kodierte Aminosäure in einem ausgewählten Molverhältnis kodieren und wobei jede degenerierte Codonposition NNT, VWG, TGG umfasst, wobei das Verhältnis der Codons 16 (NNT) : 6 (VWG) : 1 (TGG) beträgt, oder jede degenerierte Codonposition VMA, NDT, WKG umfasst, wobei das Verhältnis der Codons in dem Satz 6 (VMA) : 12 (NDT) : 4 (WKG) beträgt.

2. Bibliothek nach Anspruch 1, wobei die Variantemoleküle gemeinsam folgendes umfassen:

einen ersten Satz von Polynukleotidvarianten, umfassend mindestens eine erste degenerierte Codonposition; und
einen zweiten Satz von Polynukleotidvarianten, umfassend mindestens eine zweite, von der ersten verschiedene, degenerierte Codonposition.

3. Bibliothek nach Anspruch 2, wobei:

(a) die erste und die zweite Position den gleichen degenerierten Codonsatz umfassen;
(b) der erste Satz von Nukleinsäurevarianten ein Referenzcodon für mehrere Elemente des ersten Satzes in einer Codonposition umfasst, die der zweiten degenerierten Codonposition in dem zweiten Satz entspricht;
(c) der zweite Satz von Nukleinsäurevarianten ein Referenzcodon für mehrere Elemente des zweiten Satzes in einer Codonposition umfasst, die der ersten degenerierten Codonposition in dem ersten Satz entspricht;
(d) sowohl (b) als auch (c); oder
(e) der erste und der zweite Satz von Polynukleotidvarianten überlappende Elemente umfassen.

4. Verfahren zum Erzeugen einer Bibliothek von Polynukleotidvarianten, wobei das Verfahren Folgendes umfasst:

Bereitstellen eines Referenzpolynukleotidmoleküls oder einer Referenzpolynukleotidsequenz;
Auswählen mindestens einer zu variierenden Stelle in dem Referenzpolynukleotidmolekül oder der Referenzpolynukleotidsequenz; und
Erzeugen eines Satzes von Variantenpolynukleotiden, die degenerierte Codons an der Stelle von Interesse umfassen, wobei die degenerierten Codons an jeder Stelle von Interesse (NNT, VWG, TGG) in einem Molverhältnis von etwa 16:6:1 umfassen, oder wobei die degenerierten Codons an jeder Stelle von Interesse (VMA, NDT, WKG) umfassen und wobei das Verhältnis der Codons in dem Satz 6 (VMA) : 12 (NDT) : 4 (WKG) beträgt, wodurch die Bibliothek bereitgestellt wird.

5. Bibliothek, erzeugt nach dem Verfahren nach Anspruch 4.

6. Zusammensetzung, umfassend einen Satz von Polynukleotidvarianten, umfassend mindestens eine degenerierte Codonposition, wobei die degenerierten Codons an der Position (NNT, VWG, TGG) in einem Molverhältnis von etwa 16:6:1 umfassen, oder wobei die degenerierten Codons an der Position (VMA, NDT, WKG) umfassen und wobei das Verhältnis der Codons in dem Satz 6 (VMA) : 12 (NDT) : 4 (WKG) beträgt, wobei die Polynukleotidvarianten gemeinsam alle möglichen Varianten umfassen, die durch die degenerierte Codonposition dargestellt werden.

7. Zusammensetzung nach Anspruch 6, wobei die Polynukleotidvarianten synthetische Oligonukleotide sind und/oder die Polynukleotidvarianten Nukleinsäureamplikons sind.

8. Verfahren zum Erzeugen eines Satzes von Variantenpolynukleotidmolekülen, wobei das Verfahren Folgendes umfasst:

Bereitstellen einer Referenzpolynukleotidsequenz;
Auswählen mehrerer Codonstellen in der zu variierenden Referenzpolynukleotidsequenz;
Auswählen eines degenerierten Codonsatzes für die mehreren zu variierenden Codonstellen, wobei der degenerierte Codonsatz (NNT, VWG, TGG) in einem Molverhältnis von etwa 16:6:1 umfasst oder (VMA, NDT, WKG) umfasst, wobei das Verhältnis der Codons in den Satz 6 (VMA) : 12 (NDT) : 4 (WKG) beträgt.
Erzeugen des Satzes von Variantenpolynukleotidmolekülen, wobei jedes Variantenpolynukleotid ein Element des degenerierten Codonsatzes an jeder der mehreren Stellen von Codonvariation umfasst, wobei der Satz von Variantenpolynukleotidmolekülen gemeinsam alle Elemente des degenerierten Codonsatzes an jeder der Stellen von Codonvariation umfasst.

9. Verfahren zum Erzeugen mehrerer Sätze von Variantenpolynukleotidmolekülen, wobei das Verfahren Folgendes umfasst:

Bereitstellen einer Referenzpolynukleotidsequenz;

Auswählen mehrerer Codonstellen in der zu variierenden Referenzpolynukleotidsequenz;

Auswählen eines degenerierten Codonsatzes für die mehreren zu variierenden Codonstellen, wobei der degenerierte Codonsatz (NNT, VWG, TGG) in einem Molverhältnis von etwa 16:6:1 umfasst oder (VMA, NDT, WKG) umfasst, wobei das Verhältnis der Codons in den Satz 6 (VMA) : 12 (NDT) : 4 (WKG) beträgt;

separates Erzeugen der mehreren Sätze von Variantenpolynukleotidmolekülen, wobei jedes Variantenpolynukleotid ein Element des degenerierten Codonsatzes an jeder der mehreren Stellen von Codonvariation umfasst, wobei die mehreren Sätze von Variantenpolynukleotidmolekülen gemeinsam alle Elemente des degenerierten Codonsatzes an jeder der Stellen von Codonvariation umfasst.

**10.** Verfahren nach Anspruch 8 oder 9, wobei:

(i) die Codonstellen ausgewählt werden, indem einer oder mehrere der folgenden Faktoren betrachtet werden: eine dreidimensionale Struktur eines Referenzproteins, für das von dem Polynukleotidmolekül oder der Polynukleotidsequenz kodiert wird; Ergebnisse eines Mutageneseexperiments, das an dem Referenzpolynukleotid oder der Referenzsequenz durchgeführt worden ist, oder Anwendung eines statistischen Filters, der Stellen von Interesse identifiziert;

(ii) die Codonstelle ausgewählt wird, indem zufällige Mutagenese, rekursive Rekombination oder Alanin-Scannen an der Referenznukleinsäure oder einem Homolog davon durchgeführt werden und alle resultierenden Mutanten auf Aktivität gescreent werden, um Codonstellen von Interesse zu identifizieren

(iii) die Variantenpolynukleotide mindestens 10 Stellen von Codonvariation umfassen;

(iv) die Variantenpolynukleotide mindestens 25 Stellen von Codonvariation umfassen;

(v) die Variantenpolynukleotide mindestens 50 Stellen von Codonvariation umfassen; und/oder

(vi) mindestens etwa 50 % der Codons in den Variantenpolynukleotiden Stellen von Codonvariation umfassen.

**11.** Verfahren nach einem der Ansprüche 8-10, wobei:

(i) das Auswählen der mehreren Codons das Betrachten eines Strukturmodells eines Referenzpolypeptids, für das von dem Referenzpolynukleotidmolekül oder der Referenzpolynukleotidsequenz kodiert wird, und das Auswählen von Stellen basierend auf ihrer Beziehung zu einem oder mehreren Merkmalen des Referenzpolypeptids umfasst;

(ii) das Auswählen der mehreren Codons das Betrachten einer Ausrichtung des Referenzpolynukleotids an einem oder mehreren homologen Polynukleotiden oder eines Referenzpolypeptids, für das durch das Referenzpolynukleotid mit einem oder mehreren homologen Polypeptiden kodiert wird, und das Auswählen der Codons zur Variation basierend auf einem Merkmal der Ausrichtung umfasst;

(iii) separates Erzeugen der Sätze von Polynukleotidvarianten das Synthetisieren von Sätzen von degenerierten Oligonukleotiden, die die degenerierten Codons umfassen, und das separate Amplifizieren des Referenzpolynukleotids in getrennten Polymerase- oder Ligaseamplifikationsreaktionen unter Verwendung der degenerierten Oligonukleotide als Amplifikationsprimer umfasst; und/oder

(iv) das Verfahren ferner das Zusammensetzen der Sätze von Polynukleotidvarianten zu einem oder mehreren Expressionsvektoren durch Durchführen einer nachfolgenden PCR mit einem Megaprimer, umfassend eine Polynukleotidvariantensequenz, die an eine kreisförmige Matrizennukleinsäure gebunden ist, umfasst, gegebenenfalls wobei (a) die Megaprimer-PCR-Reaktion von zwei aneinander angrenzenden Primern geprimet wird, von denen mindestens einer eine Variantensequenz umfasst, oder (b) die Megaprimer-PCR-Reaktion von zwei überlappenden Primern geprimet wird, von denen mindestens einer eine Variantensequenz umfasst.

**12.** Verfahren nach einem der Ansprüche 8-11, wobei das Verfahren das Auswählen eines einzelnen degenerierten Codonsatzes für alle zu variierenden Codons umfasst und wobei optional:

(i) 3 oder mehr Codons ausgewählt werden, um unter Verwendung des einzelnen degenerierten Codonsatzes variiert zu werden;

(ii) 5 oder mehr Codons ausgewählt werden, um unter Verwendung des einzelnen degenerierten Codonsatzes variiert zu werden;

(iii) 10 oder mehr Codons ausgewählt werden, um unter Verwendung des einzelnen degenerierten Codonsatzes variiert zu werden.

und/oder ferner umfassend das Screening der Bibliothek auf eine oder mehrere Eigenschaften von Interesse, gegebenenfalls wobei:

(a) höchstens etwa 80 % der möglichen Varianten für jede Variationsstelle innerhalb der Bibliothek auf die eine oder die mehreren Eigenschaften von Interesse untersucht werden;

(b) höchstens etwa 60 % der möglichen Varianten für jede Variationsstelle innerhalb der Bibliothek auf die eine oder die mehreren Eigenschaften von Interesse untersucht werden; oder

(c) zwischen etwa 50 % und etwa 60 % der möglichen Varianten für jede Variationsstelle in der Bibliothek auf die eine oder mehreren Eigenschaften von Interesse gescreent werden.

**13.** Bibliothek, erzeugt nach dem Verfahren nach einem der Ansprüche 8-12.

**Revendications**

**1.** Banque de polynucléotides, comprenant : un mélange de molécules variantes de polynucléotide, les molécules variantes comprenant au moins une première position de codon dégénéré, dans laquelle les codons dégénérés de la première position codent pour un acide aminé codé dans un rapport molaire sélectionné, et dans laquelle chaque position de codon dégénéré comprend NNT, VWG, TGG, dans laquelle le rapport de codons est 16(NNT) : 6(VWG) : 1(TGG), ou chaque position de codon dégénéré comprend (VMA, NDT, WKG) dans laquelle le rapport de codons dans l'ensemble est 6(VMA) : 12(NDT) : 4(WKG).

**2.** Banque selon la revendication 1, dans laquelle les molécules variantes comprennent collectivement :

un premier ensemble de variants de polynucléotide comprenant au moins une première position de codon dégénéré ; et,

un deuxième ensemble de variants de polynucléotide comprenant au moins une deuxième position de codon dégénéré différente de la première position.

**3.** Banque selon la revendication 2, dans laquelle :

(a) la première et la deuxième position comprennent le même ensemble de codons dégénérés ;

(b) le premier ensemble de variants d'acide nucléique comprend un codon de référence pour une pluralité de membres du premier ensemble à une position de codon correspondant à la deuxième position de codon dégénéré dans le deuxième ensemble ;

(c) le deuxième ensemble de variants d'acide nucléique comprend un codon de référence pour une pluralité de membres du deuxième ensemble à une position de codon correspondant à la première position de codon dégénéré dans le premier ensemble ;

(d) à la fois (b) et (c) ; ou

(e) les premier et deuxième ensembles de variants de polynucléotide comprennent des membres chevauchants.

**4.** Procédé de préparation d'une banque de variants de polynucléotide, le procédé comprenant :

la fourniture d'une molécule polynucléotidique de référence ou d'une séquence polynucléotidique de référence ;

la sélection d'au moins un site dans la molécule polynucléotidique de référence ou séquence polynucléotidique de référence à faire varier ; et,

la production d'un ensemble de polynucléotides variants comprenant des codons dégénérés au site d'intérêt, dans lequel les codons dégénérés à chaque site d'intérêt comprennent (NNT, VWG, TGG) dans un rapport molaire d'approximativement 16:6:1, ou dans lequel les codons dégénérés à chaque site d'intérêt comprennent (VMA, NDT, WKG) et dans lequel le rapport de codons dans l'ensemble est 6(VMA) : 12(NDT) : 4(WKG), de façon à produire la banque.

**5.** Banque préparée par le procédé selon la revendication 4.

**6.** Composition comprenant un ensemble de variants de polynucléotide comprenant au moins une position de codon dégénéré, dans laquelle les codons dégénérés à ladite position comprennent (NNT, VWG, TGG) dans un rapport molaire d'approximativement 16 : 6 : 1, ou dans laquelle les codons dégénérés à la position comprennent (VMA, NDT, WKG) et dans laquelle le rapport de codons dans l'ensemble est 6 (VMA) :12(NDT) : 4(WKG), dans laquelle les variants de polynucléotide comprennent collectivement tous les variants possibles représentés par la position de codon dégénéré.

**7.** Composition selon la revendication 6, dans laquelle les variants de polynucléotide sont des oligonucléotide synthétiques et/ou les variants de polynucléotide sont des amplicons d'acide nucléique.

**8.** Procédé de production d'un ensemble de molécules polynucléotidiques variantes, le procédé comprenant :

la fourniture d'une séquence polynucléotidique de référence ;
la sélection d'une pluralité de sites de codon dans la séquence polynucléotidique de référence à faire varier ;
la sélection d'un ensemble de codons dégénérés pour la pluralité de sites de codon à faire varier, l'ensemble de codons dégénérés comprenant (NNT, VWG, TGG) dans un rapport molaire d'approximativement 16:6:1 ou comprenant (VMA, NDT, WKG), dans lequel le rapport de codons dans l'ensemble est 6(VMA) : 12(NDT) : 4(WKG) ;
la production de l'ensemble de molécules polynucléotidiques variantes, chaque polynucléotide variant comprenant un membre de l'ensemble de codons dégénérés à chacun de la pluralité de sites de variation de codon, dans lequel l'ensemble de molécules polynucléotidiques variantes comprend collectivement tous les membres de l'ensemble de codons dégénérés à chacun des sites de variation de codon.

**9.** Procédé de production d'une pluralité d'ensembles de molécules polynucléotidiques variantes, le procédé comprenant :

la fourniture d'une séquence polynucléotidique de référence ;
la sélection d'une pluralité de sites de codon dans la séquence polynucléotidique de référence à faire varier ;
la sélection d'un ensemble de codons dégénérés pour la pluralité de sites de codon à faire varier, l'ensemble de codons dégénérés comprenant (NNT, VWG, TGG) dans un rapport molaire d'approximativement 16:6:1 ou comprenant (VMA, NDT, WKG), dans lequel le rapport de codons dans l'ensemble est 6(VMA) : 12(NDT) : 4(WKG) ;
la production séparément de la pluralité d'ensembles de molécules polynucléotidiques variantes, chaque polynucléotide variant comprenant un membre de l'ensemble de codons dégénérés à chacun de la pluralité de sites de variation de codon, dans lequel la pluralité d'ensembles de molécules polynucléotidiques variantes comprend collectivement tous les membres de l'ensemble de codons dégénérés à chacun des sites de variation de codon.

**10.** Procédé selon la revendication 8 ou la revendication 9, dans lequel :

(i) les sites de codon sont sélectionnés en tenant compte d'un ou plusieurs parmi : une structure tridimensionnelle d'une protéine de référence codée par la molécule ou séquence polynucléotidique ; les résultats d'un essai de mutagenèse conduit sur le polynucléotide ou la séquence de référence, ou l'application d'un filtre statistique qui identifie des sites d'intérêt ;
(ii) le site de codon est sélectionné en effectuant une mutagenèse aléatoire, une recombinaison récursive, ou un balayage d'alanine sur l'acide nucléique de référence ou un homologue de celui-ci, et le criblage de mutants résultants éventuels pour l'activité afin d'identifier des sites de codon d'intérêt
(iii) les polynucléotides variants comprennent au moins 10 sites de variation de codon ;
(iv) les polynucléotides variants comprennent au moins 25 sites de variation de codon ;
(v) les polynucléotides variants comprennent au moins 50 sites de variation de codon ; et/ou
(vi) au moins environ 50 % des codons dans les polynucléotides variants comprennent des sites de variation de codon.

**11.** Procédé selon l'une quelconque des revendications 8 à 10, dans lequel :

(i) la sélection de la pluralité de codons comprend la prise en compte d'un modèle structural d'un polypeptide de référence codé par la molécule ou séquence polynucléotidique de référence, et la sélection de sites sur la base de leur relation avec une ou plusieurs caractéristiques du polypeptide de référence ;
(ii) la sélection de la pluralité de codons comprend la prise en compte d'un alignement du polynucléotide de référence avec un ou plusieurs polynucléotides homologues, ou d'un polypeptide de référence codé par le polynucléotide de référence avec un ou plusieurs polypeptides homologues, et la sélection des codons pour la variation sur la base d'une caractéristique de l'alignement ;
(iii) séparément, la production des ensembles de variants de polynucléotide comprend la synthèse d'ensembles d'oligonucléotides dégénérés comprenant les codons dégénérés, et séparément l'amplification du polynucléotide de référence dans des réactions séparées d'amplification par polymérase ou ligase en utilisant les oligonucléotides dégénérés en tant qu'amorces d'amplification ; et/ou

(iv) le procédé comprend en outre l'assemblage des ensembles de variants de polynucléotide dans un ou plusieurs vecteurs d'expression en effectuant une PCR consécutive avec une méga-amorce comprenant une séquence de variant de polynucléotide liée à un acide nucléique de matrice circulaire, facultativement dans lequel (a) la réaction PCR avec méga-amorce est amorcée à partir de deux amorces en butée, dont au moins l'une comprend une séquence variante, ou (b) la réaction PCR avec méga-amorce est amorcée à partir de deux amorces chevauchantes, dont au moins une comprend une séquence variante.

12. Procédé selon l'une quelconque des revendications 8 à 11, le procédé comprenant la sélection d'un ensemble de codons dégénérés unique pour tous les codons à faire varier, et dans lequel, facultativement :

(i) 3 codons ou plus sont sélectionnés pour varier au moyen de l'ensemble de codons dégénérés unique ;
(ii) 5 codons ou plus sont sélectionnés pour varier au moyen de l'ensemble de codons dégénérés unique ; ou
(iii) 10 codons ou plus sont sélectionnés pour varier au moyen de l'ensemble de codons dégénérés unique,

et/ou comprenant en outre le criblage de la banque pour une ou plusieurs propriétés d'intérêt, facultativement dans lequel :

(a) environ 80 % ou moins de variants possibles pour chaque site de variation dans la banque sont criblés pour les une ou plusieurs propriétés d'intérêt ;
(b) environ 60 % ou moins de variants possibles pour chaque site de variation dans la banque sont criblés pour les une ou plusieurs propriétés d'intérêt ; ou
(c) entre environ 50 % et environ 60 % de variants possibles pour chaque site de variation dans la banque sont criblés pour les une ou plusieurs propriétés d'intérêt.

13. Banque préparée par le procédé selon l'une quelconque des revendications 8 à 12.

APS software automatically generates the location and sequence of primers. X and Y correspond to mutagenic primers.

A script is written to dilute/mix primers, template and reactions for a 1st round of Splicing by Overlap Extension (SOE) PCRs to generate the necessary fragments.

A second script is written to dilute/mix fragments for a 2nd round of SOE PCRs to generate the full length constructs.

Fig. 1

| 1. Design variants |
| 2. Run software |
| 3. Order oligos |
| 4. Prepare DNA |

Assemble fragments

Assemble constructs

Plate and pick variants

Sequence

Assay

Move mutations/variants forward

Fig. 2

clone a library over an existing gene variant

next round library

Standard PCR
(25-30 cycles)

PCR Amplicon
becomes
megaprimer in
second PCR
reaction.

wt gene

expression plasmid

30-50 long extension cycles
(primerless PCR)

expression plasmid

transform nicked double-stranded DNA

Fig. 3

Standard PCR
(25-30 cycles)

PCR amplicon becomes
the megaprimer

40 long extension megaprimer
PCR cycles (no oligo primers)

Fig. 4

Fig. 5

Fig. 6

Fig. 7

Fig. 8

## SNOCAPS Options

| Design | Codons | Residues | Plates for 50% Coverage | Plates for 75% Coverage | Plates for 95% Coverage |
|---|---|---|---|---|---|
| Alternative Codon Set | 23 | 20 | 54 | 109 | 235 |
| NNK | 32 | 20 | 76 | 151 | 327 |

Fig. 9

# SNOCAPS Fragment Assembly

1st round SOE PCR to create fragment plates A and B.

Fig. 10

# SNOCAPS Library Assembly

| 2nd round SOE PCR to create library assemblies. |
|---|

Fig. 11

EP 3 301 208 B1

# SNOCAPS Coverage

$R_i$=Fraction of beneficial variants in pool $i$.
$t_i$ = Screening size of pool $i$.
$N_i$ = Number of mutations in pool $i$.
$T$ = Available screening resources.
$M$= Number of pools.

How do we optimally distribute screening resources $T$ among $M$ pools so as to maximize the number of beneficial mutations?

Number of beneficial mutations screening $t_i$ variants from a pool of size $N_i$.

$$F_i = R_i N_i (1 - \exp(-t_i / N_i))$$

Optimal distribution of screening resources for two complementary pools:

$$t_1^{opt} = \frac{N_1 N_2 \ln \frac{R_1}{R_2} + T N_1}{N_1 + N_2}$$

## Fig. 12

EP 3 301 208 B1

# Optimal Distribution of Screening Resources

Fig. 13

Fig. 14

Fig. 15

EP 3 301 208 B1

| Input number of codons, number and/or type of amino acids to encode |
|---|

↓ **A1**

| Run logic routine to calculate degenerate codons to yield desired amino acids using specified number of codons |
|---|

↓ **A2**

| Output list of degenerate codons aligned with encoded amino acids |
|---|

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2000042561 A, Crameri
- WO 2000042560 A, Selifonov
- WO 2001075767 A, GUSTAFSSON
- WO 2000004190 A, del Cardayre
- US 61061581 B
- US 12483089 B
- US 2009047046 W
- US 12562988 B
- US 2009057507 W
- US 20060195947 A
- US 20050153417 A
- US 7220566 B
- WO 9522625 A
- WO 970078 A
- WO 9735966 A
- WO 9827230 A
- WO 0042651 A
- WO 0175767 A
- US 6537746 B

### Non-patent literature cited in the description

- **LANDWEHR et al.** *Chem Biol,* 2007, vol. 14 (3), 269-78
- **KUBO et al.** *Chemistry,* 2006, vol. 12 (4), 1216-20
- **HUISMAN.** *Trends Biotechnol.,* 2008, vol. 26, 132-138
- **ARNDT ; MILLER.** *Methods in Molecular Biology, Vol. 352: Protein Engineering Protocols,* 2007, vol. 352
- **ZHAO.** *Comb. Chem. High Throughput Screening,* 2006, vol. 9, 247-257
- **BERSHTEIN et al.** *Nature,* 2006, vol. 444, 929-932
- **BRAKMANN ; SCHWIENHORST.** Evolutionary Methods in Biotechnology: Clever Tricks for Directed Evolution. Wiley-VCH, 2004
- **RUBIN- PITEL ARNOLD ; GEORGIOU.** *Directed Enzyme Evolution: Screening and Selection Methods,* 2003, vol. 230
- **LI.** Reduction of Protein Sequence Complexity by Residue Grouping. *Protein Engineering,* 2003, vol. 16 (5), 323-330
- **AKANUMA et al.** Combinatorial mutagenesis to restrict amino acid usage in an enzyme to a reduced set. *PNAS,* 2002, vol. 99 (21), 13549-13553
- **SUSKO ; ROGER.** On Reduced Amino Acid Alphabets for Phylogenetic Inference. *Mol. Biol. Evol.,* 2007, vol. 24 (9), 2139-2150
- **REETZ ; WU.** Greatly Reduced Amino Acid Alphabets in Directed Evolution: Making the Right Choice For Saturation Mutagenesis and Homologous Amino Acid Positions. *Chem. Commun.,* 2008, 5499-5501
- **RAJPAL.** A General Method for Greatly Improving the Affinity of Antibodies Using Combinatorial Libraries. *PNAS,* 2005, vol. 102 (24), 8466-8471
- **REETZ et al.** Addressing the Numbers Problem in Directed Evolution. *ChemBioChem,* 2008, vol. 9, 1797-1804
- **REETZ et al.** Iterative Saturation Mutagenesis on the Basis of B Factors as a Strategy for Increasing Protein Thermostability. *Angew. Chem.,* 2006, vol. 118, 7907-7915
- **MENA ; DAUGHERTY.** Automated design of degenerate codon libraries. *Protein Engineering, Design & Selection,* 2005, vol. 18 (12), 559-561
- **WANG ; SCHULTZ.** Expanding the Genetic Code. *Angewandte Chemie Int Ed,* 2005, vol. 44, 34-66
- **XIE ; SCHULTZ.** An Expanding Genetic Code. *Methods,* 2005, vol. 36, 227-238
- **XIE ; SCHULTZ.** Adding Amino Acids to the Genetic Repertoire. *Curr Opinion in Chemical Biology,* 2005, vol. 9, 548-554
- **WANG et al.** Expanding the Genetic Code. *Annu Rev Biophys Biomol Struct,* 2006, vol. 35, 225-249
- **ALBERTS et al.** Molecular Biology of the Cell: Reference Edition. Garland Science, 2007
- Biochemical Nomenclature and Related Documents. Portland Press, 1992

- Oligonucleotide Synthesis: Methods and Applications. Methods in Molecular Biology. Humana Press, 2004
- Protocols for Oligonucleotide Conjugates: Synthesis and Analytical Techniques. **SUDHIR AGRAWAL.** Methods in Molecular Biology. Humana Press, 1993
- **HORTON et al.** Engineering hybrid genes without the use of restriction enzymes: gene splicing by overlap extension. *Gene,* 1989, vol. 77 (1), 61-8
- **HORTON et al.** Gene splicing by overlap extension: tailor-made genes using the polymerase chain reaction. *Biotechniques,* 1990, vol. 8 (5), 528-35
- **HORTON et al.** Splicing by overlap extension by PCR using asymmetric amplification: an improved technique for the generation of hybrid proteins of immunological interest. *Gene,* 1997, vol. 186 (1), 29-35
- PCR Cloning Protocols. Methods in Molecular Biology. Humana Press
- **BALDINO et al.** *Methods Enzymology,* vol. 168, 761-777
- **BOLTON et al.** *Proc. Natl. Acad. Sci. USA,* 1962, vol. 48, 1390
- **BRESSLAUER et al.** *Proc. Natl. Acad. Sci USA,* 1986, vol. 83, 8893-8897
- **FREIER et al.** *Proc. Natl. Acad. Sci USA,* 1986, vol. 83, 9373-9377
- **KIERZEK et al.** *Biochemistry,* vol. 25, 7840-7846
- **RYCHLIK et al.** *Nucleic Acids Res,* 1990, vol. 18, 6409-6412
- *Nucleic Acids Res,* 1991, vol. 19, 698
- **SAMBROOK et al.** Molecular Cloning: A Laboratory Manual. Cold Spring Harbor Laboratory Press, 2001
- **SUGGS et al.** Developmental Biology Using Purified Genes. Academic Press, 1981, 683-693
- **WETMUR.** *Crit Rev Biochem Mol Biol,* 1991, vol. 26, 227-259
- **VILJOEN et al.** Molecular Diagnostic PCR Handbook. Springer, 2005
- PCR Cloning Protocols. Methods in Molecular Biology. Humana Press, 2002
- Directed Enzyme Evolution: Screening and Selection Methods. Methods in Molecular Biology. Humana Press, 2003
- Directed Evolution Library Creation: Methods and Protocols. Methods in Molecular Biology. Humana Press, 2003
- Short Protocols in Molecular Biology. Current Protocols, 2002, vol. 2
- PCR Protocols A Guide to Methods and Applications. Academic Press Inc, 1990
- Principles and Techniques of Biochemistry and Molecular Biology. Cambridge University Press, 2005
- **SAMBROOK et al.** Molecular Cloning - A Laboratory Manual. Cold Spring Harbor Laboratory, 2001, vol. 1-3
- **JOSEPH SAMBROOK.** The Condensed Protocols from Molecular Cloning: A Laboratory Manual. Cold Spring Harbor Laboratory Press, 2006
- Current Protocols in Molecular Biology. Greene Publishing Associates, Inc. and John Wiley & Sons, Inc,
- Short Protocols in Molecular Biology. 2002
- **JANE ROSKAMS ; LINDA RODGERS.** Lab Ref, Volume 1: A Handbook of Recipes, Reagents, and Other Reference Tools for Use at the Bench. Cold Spring Harbor Laboratory Press, 2002
- **BERGER ; KIMMEL.** Guide to Molecular Cloning Techniques, Methods in Enzymology. Academic Press, Inc, vol. 152
- **MIYAZAKI.** Creating Random Mutagenesis Libraries Using Megaprimer PCR of Whole Plasmid. *BioTechniques,* November 2002, vol. 33, 1033-1038
- **VAN DEN ENT ; LOWE.** RF cloning: A restriction-free method for inserting target genes into plasmids. *J. Biochem. Biophys. Methods,* 2006, vol. 67, 67-74
- **SANCHIS et al.** Improved PCR method for the creation of saturation mutagenesis libraries in directed evolution: application to difficult to amplify templates. *Appl. Microbiol. Biotechnol.,* 2008, vol. 81, 387-397
- **QUAN ; TIAN.** Circular Polymerase Extension Cloning of Complex Gene Libraries and Pathways. *PLoS ONE,* 2009, vol. 4 (7), e6441
- **CHIU et al.** Site-directed, Ligase-Independent Mutagenesis (SLIM): a single tube methodology approaching 100% efficiency in 4 h. *Nucleic Acids Research,* 2004, vol. 32 (21), e174
- **LI et al.** Site directed mutagenesis by combination of homologous recombination and Dpn1 digestion of the plasmid template in Escherichia coli. *Analytical Biochemistry,* 2008, vol. 373, 389-391
- **FOX ; HUISMAN.** *Trends Biotechnol.,* 2008, vol. 26, 132-138
- **ZHAO.** *Chem. High Throughput Screening,* 2006, vol. 9, 247-257
- **RUBIN- PITEL ARNOLD ; GEORGIOU.** Directed Enzyme Evolution: Screening and Selection Methods. 2003, vol. 230
- **MCPHERSON.** Crystallization of Biological Macromolecules. Cold Spring Harbor Laboratory, 1999
- **BERGFORS.** Protein Crystallization. International University Line, 1999
- **MULLIN.** Crystallization. Butterwoth, 1993
- **STOUT ; JENSEN.** X-ray structure determination: a practical guide. Wiley Publishers, 1989
- **LADD ; PALMER.** Structure determination by X-ray crystallography. Plenum Press, 1993

- **BLUNDELL ; JOHNSON.** Protein Crystallography. Academic Press, 1976
- **GLUSKER ; TRUEBLOOD.** Crystal structure analysis: A primer. Oxford University Press, 1985
- Introduction to Macromolecular Crystallography. **MCPHERSON.** International Tables for Crystallography, Vol. F. Crystallography of Biological Macromolecules. Wiley-Liss, 2002
- **MCREE ; DAVID.** Practical Protein Crystallography. Academic Press, 1999
- Springer Advanced Texts in Chemistry. **DRENTH.** Principles of Protein X-Ray Crystallography. Springer-Verlag, 1999
- **FANCHON ; HENDRICKSON.** Crystallographic Computing. Oxford University Press, 1991, vol. 5 IUCr
- **MURTHY.** Crystallo graphic Methods and Protocols. Humana Press, 1996
- **DAUTER et al.** Novel approach to phasing proteins: derivatization by short cryo-soaking with halides. *Acta Cryst.,* 2000, vol. D56, 232-237
- **DAUTER.** New approaches to high-throughput phasing. *Curr. Opin. Structural Biol.,* 2002, vol. 12, 674-678
- **CHEN et al.** Crystal structure of a bovine neurophysin-II dipeptide complex at 2.8 Å determined from the single-wavelength anomalous scattering signal of an incorporated iodine atom. *Proc. Natl Acad. Sci. USA,* 1991, vol. 88, 4240-4244
- **GAVIRA et al.** Ab initio crystallographic structure determination of insulin from protein to electron density without crystal handling. *Acta Cryst.,* 2002, vol. D58, 1147-1154
- **OTWINOWSKI ; MINOR.** Processing of X-ray Diffraction Data Collected in Oscillation Mode. *Methods in Enzymology,* 1997, vol. 276, 307-326
- Collaborative Computational Project (1994) "The CCP4 suite: programs for protein crystallography. *Acta Crystallogr D,* 1994, vol. 50, 760-763
- **TERWILLIGER ; BERENDZEN.** *Acta Crystallogr,* 1999, vol. D 55, 849-861
- **SCHNEIDER ; SHELDRICK.** Substructure solution with SHELXD. *Acta Crystallogr D Biol Crystallogr,* 2002, vol. 58, 1772-1779
- **MURSHUDOV et al.** Refinement of Macromolecular Structures by the Maximum-Likelihood Method. *Acta Crystallogr D,* 1997, vol. 53, 240-255
- **VAN AALTEN et al.** PRODRG, a program for generating molecular topologies and unique molecular descriptors from coordinates of small molecules. *J Comput Aided Mol Des,* 1996, vol. 10, 255-262
- **JONES et al.** Improved methods for building protein models in electron density maps and the location of errors in these models. *Acta Crystallogr,* 1991, vol. A 47, 110-119
- **CAVANAGH et al.** Protein NMR Spectroscopy: Principles and Practice. Academic Press, 1995
- **LEVITT.** Spin Dynamics: Basics of Nuclear Magnetic Resonance. John Wiley & Sons, 2001
- **EVANS.** Biomolecular NMR Spectroscopy. Oxford University Press, 1995
- Baker Lecture Series. **WÜTHRICH.** NMR of Proteins and Nucleic Acids. Kurt Wiley-Interscience, 1986
- **NEUHAUS ; WILLIAMSON.** The Nuclear Overhauser Effect in Structural and Conformational Analysis. Wiley-VCH, 2000
- **MACOMBER.** A Complete Introduction to Modern NMR Spectroscopy. Wiley-Interscience, 1998
- Protein NMR Techniques. **DOWNING.** Methods in Molecular Biology. Humana Press, 2004
- NMR of Proteins. **CLORE ; GRONENBORN.** Topics in Molecular and Structural Biology. CRC Press, 1994
- **REID.** Protein NMR Techniques. Humana Press, 1997
- Protein NMR for the Millenium. **KRISHNA ; BERLINER.** Biological Magnetic Resonance. Kluwer Academic Publishers, 2003
- Perspectives on Solid State NMR in Biology. **KIIHNE ; DE GROOT.** Focus on Structural Biology. Kluwer Academic Publishers, 2001, vol. 1
- Spectroscopic Methods and Analyses: NMR, Mass Spectrometry, and Related Techniques. **JONES et al.** Methods in Molecular Biology. Humana Press, 1993, vol. 17
- **GOTO ; KAY.** *Curr. Opin. Struct. Biol.,* 2000, vol. 10, 585
- **GARDNER.** *Annu. Rev. Biophys. Biomol. Struct.,* 1998, vol. 27, 357
- **WÜTHRICH.** *Angew. Chem. Int. Ed.,* 2003, vol. 42, 3340
- **BAX.** *Curr. Opin. Struct. Biol.,* 1994, vol. 4, 738
- **PERVUSHIN et al.** *Proc. Natl. Acad. Sci. U.S.A.,* 1997, vol. 94, 12366
- **FIAUX et al.** *Nature,* 2002, vol. 418, 207
- **FERNANDEZ ; WIDER.** *Curr. Opin. Struct. Biol.,* 2003, vol. 13, 570
- **ELLMAN et al.** *J. Am. Chem. Soc.,* 1992, vol. 114, 7959
- **WIDER.** *BioTechniques,* 2000, vol. 29, 1278-1294
- **PELLECCHIA et al.** *Nature Rev. Drug Discov.,* 2002, vol. 1, 211-219
- **ARORA ; TAMM.** *Curr. Opin. Struct. Biol.,* 2001, vol. 11, 540-547
- **FLAUX et al.** *Nature,* 2002, vol. 418, 207-211
- **PELLECCHIA et al.** *J. Am. Chem. Soc.,* 2001, vol. 123, 4633-4634
- **PERVUSHIN et al.** *Proc. Natl. Acad. Sci. USA,* 1997, vol. 94, 12366-12371
- Protein Crystallography in Drug Design. Wiley-VCH, 2004

- **LYNE.** Structure-based virtual screening: An overview. *Drug Discov. Today,* 2002, vol. 7, 1047-1055
- **MAY et al.** Inverting enantioselectivity by directed evolution of hydantoinase for improved production of 1-methionin. *Nature Biotechnology,* 2000, vol. 18, 317-320
- In Vitro Mutagenesis Protocols. **SAMBROOK ; AUSUBEL.** Methods in Molecular Biology. Humana Press, 2001
- Chromosomal Mutagenesis. Methods in Molecular Biology. Humana Press, 2007
- Short Protocols in Molecular Biology. 2002, vol. 2
- **ARNOLD.** Protein engineering for unusual environments. *Current Opinion in Biotechnology,* 1993, vol. 4, 450-455
- **BASS et al.** Mutant Trp repressors with new DNA-binding specificities. *Science,* 1988, vol. 242, 240-245
- **BOTSTEIN ; SHORTLE.** Strategies and applications of in vitro mutagenesis. *Science,* 1985, vol. 229, 1193-1201
- **CARTER et al.** Improved oligonucleotide site-directed mutagenesis using M13 vectors. *Nucl. Acids Res.,* 1985, vol. 13, 4431-4443
- **CARTER.** Site-directed mutagenesis. *Biochem. J.,* 1986, vol. 237, 1-7
- **CARTER.** Improved oligonucleotide-directed mutagenesis using M13 vectors. *Methods in Enzymol,* 1987, vol. 154, 382-403
- **DALE et al.** Oligonucleotide-directed random mutagenesis using thephosphorothioate method. *Methods Mol. Biol.,* 1996, vol. 57, 369-374
- **EGHTEDARZADEH ; HENIKOFF.** Use of oligonucleotides to generate large deletions. *Nucl. Acids Res.,* 1986, vol. 14, 5115
- **FRITZ et al.** Oligonucleotide-directed construction of mutations: a gapped duplex DNA procedure without enzymatic reactions in vitro. *Nucl. Acids Res.,* 1988, vol. 16, 6987-6999
- **GRUNDSTROM et al.** Oligonucleotide-directed mutagenesis by microscale 'shot-gun' gene synthesis. *Nucl. Acids Res.,* 1985, vol. 13, 3305-3316
- The efficiency of oligonucleotide directed mutagenesis. **KUNKEL.** Nucleic Acids & Molecular Biology. Springer Verlag, 1987
- **KUNKEL.** Rapid and efficient site-specific mutagenesis without phenotypic selection. *Proc. Natl. Acad. Sci. USA,* 1985, vol. 82, 488-492
- **KUNKEL et al.** Rapid and efficient site-specific mutagenesis without phenotypic selection. *Methods in Enzymol.,* 1987, vol. 154, 367-382
- **KRAMER et al.** The gapped duplex DNA approach to oligonucleotide-directed mutation construction. *Nucl. Acids Res.,* 1984, vol. 12, 9441-9456
- **KRAMER ; FRITZ.** Oligonucleotide-directed construction of mutations via gapped duplex DNA. *Methods in Enzymol.,* 1987, vol. 154, 350-367
- **KRAMER et al.** Point Mismatch Repair. *Cell,* 1984, vol. 38, 879-887
- **KRAMER et al.** Improved enzymatic in vitro reactions in the gapped duplex DNA approach to oligonucleotide-directed construction of mutations. *Nucl. Acids Res.,* 1988, vol. 16, 7207
- **LING et al.** Approaches to DNA mutagenesis: an overview. *Anal Biochem.,* 1997, vol. 254 (2), 157-178
- **LORIMER ; PASTAN.** *Nucleic Acids Res,* 1995, vol. 23, 3067-8
- **MANDECKI.** Oligonucleotide-directed double-strand break repair in plasmids of Escherichia coli: a method for site-specific mutagenesis. *Proc. Natl. Acad. Sci. USA,* 1986, vol. 83, 7177-7181
- **NAKAMAYE ; ECKSTEIN.** Inhibition of restriction endonuclease Nci I cleavage by phosphorothioate groups and its application to oligonucleotide-directed mutagenesis. *Nucl. Acids Res.,* 1986, vol. 14, 9679-9698
- **NAMBIAR et al.** Total synthesis and cloning of a gene coding for the ribonuclease S protein. *Science,* 1984, vol. 223, 1299-1301
- **SAKAMAR ; KHORANA.** Total synthesis and expression of a gene for the a-subunit of bovine rod outer segment guanine nucleotide-binding protein (transducin). *Nucl. Acids Res.,* 1988, vol. 14, 6361-6372
- **SAYERS et al.** Y-T Exonucleases in phosphorothioate-based oligonucleotide-directed mutagenesis. *Nucl. Acids Res.,* 1988, vol. 16, 791-802
- **SAYERS et al.** Strand specific cleavage of phosphorothioate-containing DNA by reaction with restriction endonucleases in the presence of ethidium bromide. *Nucl. Acids Res.,* 1988, vol. 16, 803-814
- **SIEBER et al.** *Nature Biotechnology,* 2001, vol. 19, 456-460
- **SMITH.** In vitro mutagenesis. *Ann. Rev. Genet.,* 1985, vol. 19, 423-462
- *Methods in Enzymol.,* 1983, vol. 100, 468-500
- *Methods in Enzymol,* 1987, vol. 154, 329-350
- **STEMMER.** *Nature,* 1994, vol. 370, 389-91
- **TAYLOR et al.** The use of phosphorothioate-modified DNA in restriction enzyme reactions to prepare nicked DNA. *Nucl. Acids Res.,* 1985, vol. 13, 8749-8764
- **TAYLOR et al.** The rapid generation of oligonucleotide-directed mutations at high frequency using phosphorothioate-modified DNA. *Nucl. Acids Res.,* 1985, vol. 13, 8765-8787
- **WELLS et al.** Importance of hydrogen-bond formation in stabilizing the transition state of subtilisin. *Phil. Trans. R. Soc. Lond. A,* 1986, vol. 317, 415-423

- **WELLS et al.** Cassette mutagenesis: an efficient method for generation of multiple mutations at defined sites. *Gene,* 1985, vol. 34, 315-323
- **ZOLLER ; SMITH.** Oligonucleotide-directed mutagenesis using M13-derived vectors: an efficient and general procedure for the production ofpoint mutations in any DNA fragment. *Nucleic Acids Res.,* 1982, vol. 10, 6487-6500
- **ZOLLER ; SMITH.** Oligonucleotide-directed mutagenesis of DNA fragments cloned into M13 vectors. *Methods in Enzymol.,* 1983, vol. 100, 468-500
- **ZOLLER ; SMITH.** Oligonucleotide-directed mutagenesis: a simple method using two oligonucleotide primers and a single-stranded DNA template. *Methods in Enzymol.,* 1987, vol. 154, 329-350
- *Methods in Enzymology,* vol. 154
- **WANKHADE et al.** *J. Biol. Chem.,* 2000, vol. 275 (38), 29701-29708
- **REDDY et al.** *Proteins: Structure, Function, and Genetics,* 2001, vol. 42, 148-163
- **BIDWELL et al.** *Genes and Immunity,* 1999, vol. 1, 3-19
- **CHEN et al.** *Mol. Biol. Evo.,* 2003, vol. 18, 1771-1788
- *Nat Biotechnol.,* March 2007, vol. 25 (3), 338-44
- **FOX et al.** Improving catalytic function by Pro-SAR-driven enzyme evolution. *Nat Biotechnol.,* 2007, vol. 25 (3), 297-8
- **DAVID E. GOLDBERG.** Genetic Algorithms in Search, Optimization and Machine Learning. Addison-Wesley Pub Co, 1989
- **TIMOTHY MASTERS.** Practical Neural Network Recipes in C++. Academic Pr, 1993
- **KEVIN GURNEY.** An Introduction to Neural Networks. UCL Press, 1999
- **CHRISTOPHER M. BISHOP.** Neural Networks for Pattern Recognition. Oxford Univ Press, 1995
- **BRIAN D. RIPLEY ; N. L. HJORT.** Pattern Recognition and Neural Networks. Cambridge Univ Pr, 1995
- **RUBINSTEIN, R. Y. ; KROESE, D. P.** Simulation and the Monte Carlo Method. John Wiley & Sons, 2007
- **TARANTOLA ; ALBERT.** Inverse Problem Theory. Society for Industrial and Applied Mathematics, 2005
- **STEEB.** The Nonlinear Workbook: Chaos, Fractals, Neural Networks, Genetic Algorithms, Gene Expression Programming, Support Vector Machine, Wavelets, Hidden Markov Models, Fuzzy Logic with C++, Java and SymbolicC++ Programs. World Scientific Publishing, 2008
- **SERGIOS THEODORIDIS ; KONSTANTINOS KOUTROUMBAS.** Pattern Recognition. Elsevier, 2009
- **CRAMERI et al.** DNA shuffling of a family of genes from diverse species accelerates directed evolution. *Nature,* 1998, vol. 391, 288-291
- **CRAMERI et al.** Molecular evolution of an arsenate detoxification pathway by DNA shuffling. *Nature Biotech,* 1997, vol. 15, 436-438
- **ZHANG et al.** Directed evolution of an effective fructosidase from a galactosidase by DNA shuffling and screening. *Proc Natl Acad Sci USA,* 1997, vol. 94, 45-4, 4509
- **CRAMERI et al.** Improved green fluorescent protein by molecular evolution using DNA shuffling. *Nature Biotech,* 1996, vol. 14, 315-319
- **STEMMER.** Rapid evolution of a protein in vitro by DNA shuffling. *Nature,* 1994, vol. 370, 389-391
- **STEMMER.** DNA shuffling by random fragmentation and reassembly: In vitro recombination for molecular evolution. *Proc Natl Acad Sci USA,* 1994, vol. 91, 10747-10751
- High Throughput Screening: Methods and Protocols. **JANZEN ; BERNASCONI.** Methods in Molecular Biology. Humana Press, 2009
- Chemoinformatics: An Approach to Virtual Screening. Royal Society of Chemistry, 2008
- High Content Screening. Methods in Molecular Biology. Humana Press, 2006
- High-Throughput Screening in Drug Discovery. **HÜSER et al.** Methods and Principles in Medicinal Chemistry. Wiley-VCH, 2006
- Bioinformatics and Drug Discovery. Methods in Molecular Biology. 2005
- Genetic Library Construction and Screening: Advanced Techniques and Applications. Springer Lab Manuals. 2002
- Combinatorial Library: Methods and Protocols. Methods in Molecular Biology. Humana Press, 01 September 2002
- Handbook of Combinatorial Chemistry: Drugs, Catalysts, Materials. Wiley-VCH, 2002, vol. 2
- **KAUFMAN et al.** Handbook of Molecular and Cellular Methods in Biology and Medicine. CRC Press, 2003
- **2000.** The Nucleic Acid Protocols Handbook. Cold Spring Harbor, Humana Press Inc
- **SAMBROOK ; AUSUBEL.** Short Protocols in Molecular Biology. vol. 2
- **R. SCOPES.** Protein Purification. Springer-Verlag, 1982
- Methods in Enzymology Vol. 182: Guide to Protein Purification. Academic Press, Inc, 1990
- **SANDANA.** Bioseparation of Proteins. Academic Press, Inc, 1997
- **BOLLAG et al.** Protein Methods. Wiley-Liss, 1996
- **WALKER.** The Protein Protocols Handbook. Humana Press, 1996

- **NJ, HARRIS ; ANGAL.** Protein Purification Applications: A Practical Approach. IRL Press, 1990
- **HARRIS ; ANGAL.** Protein Purification Methods: A Practical Approach. IRL Press
- **SCOPES.** Protein Purification: Principles and Practice. Springer Verlag, 1993
- **JANSON ; RYDEN.** Protein Purification: Principles, High Resolution Methods and Applications. Wiley-VCH, 1998
- **WALKER.** Protein Protocols on CD-ROM. Humana Press, 1998
- Handbook of Bioseparations. Academic Press, 2000
- **STEMMER et al.** Molecular breeding of viruses for targeting and other clinical properties. *Tumor Targeting,* 1999, vol. 4, 1-4
- **NESS et al.** DNA Shuffling of subgenomic sequences of subtilisin. *Nature Biotechnology,* 1999, vol. 17, 893-896
- **CHANG et al.** Evolution of a cytokine using DNA family shuffling. *Nature Biotechnology,* 1999, vol. 17, 793-797
- **MINSHULL ; STEMMER.** Protein evolution by molecular breeding. *Current Opinion in Chemical Biology,* 1999, vol. 3, 284-290
- **CHRISTIANS et al.** Directed evolution of thymidine kinase for AZT phosphorylation using DNA family shuffling. *Nature Biotechnology,* 1999, vol. 17, 259-264
- **CRAMERI et al.** Molecular evolution of an arsenate detoxification pathway by DNA shuffling. *Nature Biotechnology,* 1997, vol. 15, 436-438

- **ZHANG et al.** Directed evolution of an effective fucosidase from a galactosidase by DNA shuffling and screening. *Proc. Natl. Acad. Sci. USA,* 1997, vol. 94, 4504-4509
- **PATTEN et al.** Applications of DNA Shuffling to Pharmaceuticals and Vaccines. *Current Opinion in Biotechnology,* 1997, vol. 8, 724-733
- **CRAMERI et al.** Construction and evolution of antibody-phage libraries by DNA shuffling. *Nature Medicine,* 1996, vol. 2, 100-103
- **CRAMERI et al.** Improved green fluorescent protein by molecular evolution using DNA shuffling. *Nature Biotechnology,* 1996, vol. 14, 315-319
- **GATES et al.** Affinity selective isolation of ligands from peptide libraries through display on a lac repressor 'headpiece dimer. *Journal of Molecular Biology,* 1996, vol. 255, 373-386
- Sexual PCR and Assembly PCR. **STEMMER.** The Encyclopedia of Molecular Biology. VCH Publishers, 1996, 447-457
- **CRAMERI ; STEMMER.** Combinatorial multiple cassette mutagenesis creates all the permutations of mutant and wildtype cassettes. *BioTechniques,* 1995, vol. 18, 194-195
- **STEMMER et al.** Single-step assembly of a gene and entire plasmid form large numbers of oligodeoxy-ribonucleotides. *Gene,* 1995, vol. 164, 49-53
- **STEMMER.** The Evolution of Molecular Computation. *Science,* 1995, vol. 270, 1510
- **STEMMER.** Searching Sequence Space. *Bio/Technology,* 1995, vol. 13, 549-553
- **STEMMER.** DNA shuffling by random fragmentation and reassembly: In vitro recombination for molecular evolution. *Proc. Natl. Acad. Sci. USA,* 1994, vol. 91, 10747-10751